(19) Europäisches Patentamt
European Patent Office
Office européen des brevets

(11) EP 4 745 237 A1

(12) EUROPEAN PATENT APPLICATION
published in accordance with Art. 153(4) EPC

(43) Date of publication:
**20.05.2026 Bulletin 2026/21**

(21) Application number: **24842287.5**

(22) Date of filing: **12.07.2024**

(51) International Patent Classification (IPC):
***C12N 15/113** (2010.01)* ***A61K 48/00** (2006.01)*
***A61K 31/713** (2006.01)* ***A61P 1/16** (2006.01)*
***A61P 3/10** (2006.01)*

(52) Cooperative Patent Classification (CPC):
**A61K 31/713; A61K 48/00; A61P 1/16; A61P 3/10; C12N 15/113**

(86) International application number:
**PCT/CN2024/105105**

(87) International publication number:
**WO 2025/016294 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310869821**

(71) Applicant: **YUN HO BIO CO., LTD**
**Shanghai 201206 (CN)**

(72) Inventors:
- **ZHU, Yun**
  **Shanghai 201206 (CN)**
- **WU, Yun**
  **Shanghai 201206 (CN)**
- **MAO, Song**
  **Shanghai 201206 (CN)**
- **LIU, Yang**
  **Shanghai 201206 (CN)**
- **DONG, Xianglei**
  **Shanghai 201206 (CN)**

(74) Representative: **Simmons & Simmons LLP (Munich)**
**Lehel Carré**
**Gewürzmühlstraße 11**
**80538 Munich (DE)**

Remarks:
The complete document including Reference Table(s) and the Sequence Listing(s) can be downloaded from the EPO website

# (54) RNAI AGENT FOR INHIBITING EXPRESSION OF KETOHEXOKINASE AND USE THEREOF

(57) Disclosed are an RNAi agent for inhibiting the expression of a KHK gene in cells and a pharmaceutical composition comprising same. The present invention also relates to a therapeutic use of the RNAi agent.



Processed by Luminess, 75001 PARIS (FR)

## Description

## Technical Filed

The present invention relates to RNAi agents that inhibit the expression of the ketohexokinase (KHK) gene in cells and pharmaceutical compositions comprising the same. The invention also relates to therapeutic uses of the RNAi agents.

## Background

The KHK gene encodes ketohexokinase, which catalyzes the phosphorylation of fructose and is the first enzyme in the fructose metabolic pathway. Studies have shown that knocking out the KHK gene can inhibit fructose-induced fatty liver, inflammation, and intestinal damage, suggesting the therapeutic potential of KHK inhibitors for metabolic syndrome. Fructose is primarily metabolized in the intestine and liver. KHK promotes fructose conversion and lipogenesis, which can lead to intrahepatic fat accumulation and is associated with the development of non-alcoholic steatohepatitis (NASH). In the intestine, KHK promotes fructose metabolism, preventing excessive fructose from entering the liver and thus playing a protective role. Excessive fructose intake leads to high concentrations of fructose rapidly entering the glycolytic pathway, providing substrates for triglyceride, glucose, and uric acid production. Elevated triglycerides and uric acid are high-risk factors for hypertension, diabetes, gout, and other conditions.

Specifically and efficiently inhibiting the KHK gene in liver through siRNA drugs, can provide beneficial effects for preventing and/or treating metabolic diseases, such as hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, hyperuricemia, gout, and other metabolism-related disorders, by reducing fructose metabolism, decreasing uric acid excretion, and improving insulin sensitivity. Particularly, non-alcoholic fatty liver disease (NAFLD) and non-alcoholic steatohepatitis (NASH) are recognized as potentially fatal diseases that can lead to cirrhosis, liver failure, and hepatocellular carcinoma (HCC).

Currently, no nucleic acid-based drugs targeting the same gene are on the market. There remains a need to develop such drugs with better efficacy, duration of action, specific targeting, and/or safety profiles.

## Summary

One aspect of the present invention provides an RNAi agent for inhibiting expression of a ketohexokinase (KHK) gene in a cell, comprising a sense strand and an antisense strand that form a duplex region, wherein the antisense strand comprises at least 15 contiguous nucleotides of a nucleotide sequence selected from SEQ ID NOs: 34 to 65 and nucleotide sequences having 1 to 3 nucleotide differences therefrom.

In some embodiments, the duplex region is 17 to 23 base pairs in length, preferably 18 to 21 base pairs, more preferably 19 base pairs.

In some embodiments, each of the sense strand and the antisense strand is 17 to 23 nucleotides in length, preferably 19 to 21 nucleotides.

In some embodiments, the RNAi agent comprises one or two blunt ends, preferably one blunt end.

In some embodiments, the RNAi agent comprises one or two overhangs, preferably one overhang, each overhang comprising 1 to 4 unpaired nucleotides, preferably 2 unpaired nucleotides.

In some embodiments, the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

In some embodiments, the sense strand comprises at least 15 contiguous nucleotides of any nucleotide sequence selected from SEQ ID NOs: 1 to 32 and nucleotide sequences having 1 to 3 nucleotide differences therefrom.

In some embodiments, the antisense strand has no more than 23 nucleotides and comprises a nucleotide sequence selected from SEQ ID NOs: 34 to 65; and the sense strand has no more than 21 nucleotides and comprises a nucleotide sequence from SEQ ID NOs: 1 to 32.

In some embodiments, in the RNAi agent:

the sense strand comprises or consists of a sequence set forth in SEQ ID NO: 1 or a nucleotide sequence having 1, 2, or 3 nucleotide differences therefrom, and the antisense strand comprises or consists of a sequence set forth in SEQ ID NO:34 or a nucleotide sequence having 1, 2, or 3 nucleotide differences therefrom;

the sense strand comprises or consists of a sequence set forth in SEQ ID NO:2 or a nucleotide sequence having 1, 2, or 3 nucleotide differences therefrom, and the antisense strand comprises or consists of a sequence set forth in SEQ

ID NO:35 or a nucleotide sequence having 1, 2, or 3 nucleotide differences therefrom;
the sense strand comprises or consists of a sequence set forth in SEQ ID NO:3 or a nucleotide sequence having 1, 2, or 3 nucleotide differences therefrom, and the antisense strand comprises or consists of a sequence set forth in SEQ ID NO:36 or a nucleotide sequence having 1, 2, or 3 nucleotide differences therefrom;
the sense strand comprises or consists of a sequence set forth in SEQ ID NO:4 or a nucleotide sequence having 1, 2, or 3 nucleotide differences therefrom, and the antisense strand comprises or consists of a sequence set forth in SEQ ID NO:37 or a nucleotide sequence having 1, 2, or 3 nucleotide differences therefrom;
the sense strand comprises or consists of a sequence set forth in SEQ ID NO:5 or a nucleotide sequence having 1, 2, or 3 nucleotide differences therefrom, and the antisense strand comprises or consists of a sequence set forth in SEQ ID NO:38 or a nucleotide sequence having 1, 2, or 3 nucleotide differences therefrom;
the sense strand comprises or consists of a sequence set forth in SEQ ID NO:6 or a nucleotide sequence having 1, 2, or 3 nucleotide differences therefrom, and the antisense strand comprises or consists of a sequence set forth in SEQ ID NO:39 or a nucleotide sequence having 1, 2, or 3 nucleotide differences therefrom;
the sense strand comprises or consists of a sequence set forth in SEQ ID NO:7 or a nucleotide sequence having 1, 2, or 3 nucleotide differences therefrom, and the antisense strand comprises or consists of a sequence set forth in SEQ ID NO:40 or a nucleotide sequence having 1, 2, or 3 nucleotide differences therefrom;
the sense strand comprises or consists of a sequence set forth in SEQ ID NO:8 or a nucleotide sequence having 1, 2, or 3 nucleotide differences therefrom, and the antisense strand comprises or consists of a sequence set forth in SEQ ID NO:41 or a nucleotide sequence having 1, 2, or 3 nucleotide differences therefrom;
the sense strand comprises or consists of a sequence set forth in SEQ ID NO:9 or a nucleotide sequence having 1, 2, or 3 nucleotide differences therefrom, and the antisense strand comprises or consists of a sequence set forth in SEQ ID NO:42 or a nucleotide sequence having 1, 2, or 3 nucleotide differences therefrom;
the sense strand comprises or consists of a sequence set forth in SEQ ID NO:10 or a nucleotide sequence having 1, 2, or 3 nucleotide differences therefrom, and the antisense strand comprises or consists of a sequence set forth in SEQ ID NO:43 or a nucleotide sequence having 1, 2, or 3 nucleotide differences therefrom;
the sense strand comprises or consists of a sequence set forth in SEQ ID NO:11 or a nucleotide sequence having 1, 2, or 3 nucleotide differences therefrom, and the antisense strand comprises or consists of a sequence set forth in SEQ ID NO:44 or a nucleotide sequence having 1, 2, or 3 nucleotide differences therefrom;
the sense strand comprises or consists of a sequence set forth in SEQ ID NO:12 or a nucleotide sequence having 1, 2, or 3 nucleotide differences therefrom, and the antisense strand comprises or consists of a sequence set forth in SEQ ID NO:45 or a nucleotide sequence having 1, 2, or 3 nucleotide differences therefrom;
the sense strand comprises or consists of a sequence set forth in SEQ ID NO:13 or a nucleotide sequence having 1, 2, or 3 nucleotide differences therefrom, and the antisense strand comprises or consists of a sequence set forth in SEQ ID NO:46 or a nucleotide sequence having 1, 2, or 3 nucleotide differences therefrom;
the sense strand comprises or consists of a sequence set forth in SEQ ID NO:14 or a nucleotide sequence having 1, 2, or 3 nucleotide differences therefrom, and the antisense strand comprises or consists of a sequence set forth in SEQ ID NO:47 or a nucleotide sequence having 1, 2, or 3 nucleotide differences therefrom;
the sense strand comprises or consists of a sequence set forth in SEQ ID NO:15 or a nucleotide sequence having 1, 2, or 3 nucleotide differences therefrom, and the antisense strand comprises or consists of a sequence set forth in SEQ ID NO:48 or a nucleotide sequence having 1, 2, or 3 nucleotide differences therefrom;
the sense strand comprises or consists of a sequence set forth in SEQ ID NO:16 or a nucleotide sequence having 1, 2, or 3 nucleotide differences therefrom, and the antisense strand comprises or consists of a sequence set forth in SEQ ID NO:49 or a nucleotide sequence having 1, 2, or 3 nucleotide differences therefrom;
the sense strand comprises or consists of a sequence set forth in SEQ ID NO:17 or a nucleotide sequence having 1, 2, or 3 nucleotide differences therefrom, and the antisense strand comprises or consists of a sequence set forth in SEQ ID NO:50 or a nucleotide sequence having 1, 2, or 3 nucleotide differences therefrom;
the sense strand comprises or consists of a sequence set forth in SEQ ID NO:18 or a nucleotide sequence having 1, 2, or 3 nucleotide differences therefrom, and the antisense strand comprises or consists of a sequence set forth in SEQ ID NO:51 or a nucleotide sequence having 1, 2, or 3 nucleotide differences therefrom;
the sense strand comprises or consists of a sequence set forth in SEQ ID NO:19 or a nucleotide sequence having 1, 2, or 3 nucleotide differences therefrom, and the antisense strand comprises or consists of a sequence set forth in SEQ ID NO:52 or a nucleotide sequence having 1, 2, or 3 nucleotide differences therefrom;
the sense strand comprises or consists of a sequence set forth in SEQ ID NO:20 or a nucleotide sequence having 1, 2, or 3 nucleotide differences therefrom, and the antisense strand comprises or consists of a sequence set forth in SEQ ID NO:53 or a nucleotide sequence having 1, 2, or 3 nucleotide differences therefrom;
the sense strand comprises or consists of a sequence set forth in SEQ ID NO:21 or a nucleotide sequence having 1, 2, or 3 nucleotide differences therefrom, and the antisense strand comprises or consists of a sequence set forth in SEQ ID NO:54 or a nucleotide sequence having 1, 2, or 3 nucleotide differences therefrom;

the sense strand comprises or consists of a sequence set forth in SEQ ID NO:22 or a nucleotide sequence having 1, 2, or 3 nucleotide differences therefrom, and the antisense strand comprises or consists of a sequence set forth in SEQ ID NO:55 or a nucleotide sequence having 1, 2, or 3 nucleotide differences therefrom;
the sense strand comprises or consists of a sequence set forth in SEQ ID NO:23 or a nucleotide sequence having 1, 2, or 3 nucleotide differences therefrom, and the antisense strand comprises or consists of a sequence set forth in SEQ ID NO:56 or a nucleotide sequence having 1, 2, or 3 nucleotide differences therefrom;
the sense strand comprises or consists of a sequence set forth in SEQ ID NO:24 or a nucleotide sequence having 1, 2, or 3 nucleotide differences therefrom, and the antisense strand comprises or consists of a sequence set forth in SEQ ID NO:57 or a nucleotide sequence having 1, 2, or 3 nucleotide differences therefrom;
the sense strand comprises or consists of a sequence set forth in SEQ ID NO:25 or a nucleotide sequence having 1, 2, or 3 nucleotide differences therefrom, and the antisense strand comprises or consists of a sequence set forth in SEQ ID NO:58 or a nucleotide sequence having 1, 2, or 3 nucleotide differences therefrom;
the sense strand comprises or consists of a sequence set forth in SEQ ID NO:26 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:27 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:28 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:29 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:30 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:31 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom; or
the sense strand comprises or consists of a sequence set forth in SEQ ID NO:32 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom.

In some embodiments, the RNAi agent comprises duplex 4023, 4089, 4098, 4101, 4102, 4103, 4118, 4119, 4121, 4122, 4123, 4124, 4125, 4126, 4127, 4128, 4129, 4130, 4132, 4135, 4138, 4141, 4142, 4145, 4147, 4148, 4149, 4151, 4159, 4161, 4162 or 4163.

In some embodiments, the sense strand and/or the antisense strand of the RNAi agent comprises at least one modified nucleotide, the modified nucleotide independently selected from 2'-deoxythymidine (dT) nucleotide, 2'-O-methyl-modified nucleotide, 2'-fluoro-modified nucleotide, 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide, 2'-amino-modified nucleotide, 2'-O-allyl-modified nucleotide, 2'-C-alkyl-modified nucleotide, 2'-methoxyethyl-modified nucleotide, abasic nucleotide, inverted abasic nucleotide, inverted nucleotide, morpholino nucleotide, phosphoramidate, tetrahydropyran-modified nucleotide, 1,5-anhydrohexitol-modified nucleotide, cyclohexenyl-modified nucleotide, nucleotide comprising a phosphorothioate group, nucleotide comprising a methylphosphonate group, nucleotide comprising a 5'-phosphate, nucleotide comprising a 5'-phosphate mimic, and combinations thereof; preferably selected from 2'-O-methyl-modified nucleotide, 2'-fluoro-modified nucleotide, 2'-deoxy-modified nucleotide, nucleotide comprising a phosphorothioate internucleotide linkage, and combinations thereof; preferably, each nucleotide of the sense strand and/or the antisense strand of the RNAi agent is modified.

In some embodiments, in the RNAi agent, in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent, the antisense strand comprises at least one phosphorothioate internucleotide linkage; preferably, the phosphorothioate internucleotide linkage is present at one or more of the following locations: between nucleotide positions 1 and 2 at 5'-end of the antisense strand; between nucleotide positions 2 and 3 at 5'-end of the antisense strand; between nucleotide positions 1 and 2 at 3'-end of the antisense strand; and between nucleotide positions 2 and 3 at 3'-end of the antisense strand.

In some embodiments, in the RNAi agent, in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

In some embodiments, in the RNAi agent, the sense strand comprises at least one phosphorothioate internucleotide linkage; preferably, the phosphorothioate internucleotide linkage is present (i) between nucleotide positions 1 and 2 at 5'-end of the sense strand; and/or (ii) between nucleotide positions 2 and 3 at 5'-end of the sense strand.

In some embodiments, the modification is selected from one of STC, ESC, Advanced ESC, ESC+, AD1-3, AD5 and GalXC.

In some embodiments, the antisense strand of the RNAi agent comprises or consists of a sequence set forth in any one of SEQ ID NOs: 104 to 130 or a nucleotide sequence having 1, 2 or 3 nucleotide differences from each thereof.

In some embodiments, the sense strand of the RNAi agent comprises or consists of a sequence set forth in any one of SEQ ID NOs: 132 to 153 or a nucleotide sequence having 1, 2 or 3 nucleotide differences from each thereof.

In some embodiments, in the RNAi agent:

(b1) the sense strand comprises or consists of a nucleotide sequence set forth in SEQ ID NO: 132 or a nucleotide sequence having 1, 2 or 3 nucleotide differences therefrom, and the antisense strand comprises or consists of a nucleotide sequence set forth in SEQ ID NO: 104 or a nucleotide sequence having 1, 2 or 3 nucleotide differences therefrom;

(b2) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(b3) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(b4) the sense strand comprises or consists of a nucleotide sequence set forth in SEQ ID NO: 135 or a nucleotide sequence having 1, 2 or 3 nucleotide differences therefrom, and the antisense strand comprises or consists of a nucleotide sequence set forth in SEQ ID NO: 107 or a nucleotide sequence having 1, 2 or 3 nucleotide differences therefrom;

(b5) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 108 or a nucleotide sequence having 1, 2 or 3 nucleotide differences therefrom;

(b6) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 109 or a nucleotide sequence having 1, 2 or 3 nucleotide differences therefrom;

(b7) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 110 or a nucleotide sequence having 1, 2 or 3 nucleotide differences therefrom;

(b8) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 111 or a nucleotide sequence having 1, 2 or 3 nucleotide differences therefrom;

(b9) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b10) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b11) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 114 or a nucleotide sequence having 1, 2 or 3 nucleotide differences

therefrom;

(b12) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 115 or a nucleotide sequence having 1, 2 or 3 nucleotide differences therefrom;

(b13) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 116 or a nucleotide sequence having 1, 2 or 3 nucleotide differences therefrom;

(b14) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b15) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b16) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b17) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b 18) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b19) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b20) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b21) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b22) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b23) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b24) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b25) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b26) the sense strand comprises or consists of a nucleotide sequence set forth in SEQ ID NO: 140 or a nucleotide

sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b27) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b28) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b29) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b30) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b31) the sense strand comprises or consists of a nucleotide sequence set forth in SEQ ID NO: 142 or a nucleotide sequence having 1, 2 or 3 nucleotide differences therefrom, and the antisense strand comprises or consists of a nucleotide sequence set forth in SEQ ID NO: 118 or a nucleotide sequence having 1, 2 or 3 nucleotide differences therefrom;

(b32) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b33) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b34) the sense strand comprises or consists of a nucleotide sequence set forth in SEQ ID NO: 143 or a nucleotide sequence having 1, 2 or 3 nucleotide differences therefrom, and the antisense strand comprises or consists of a nucleotide sequence set forth in SEQ ID NO: 121 or a nucleotide sequence having 1, 2 or 3 nucleotide differences therefrom;

(b35) the sense strand comprises or consists of a nucleotide sequence set forth in SEQ ID NO: 144 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b36) the sense strand comprises or consists of a nucleotide sequence set forth in SEQ ID NO: 144 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b37) the sense strand comprises or consists of a nucleotide sequence set forth in SEQ ID NO: 145 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b38) the sense strand comprises or consists of a nucleotide sequence set forth in SEQ ID NO: 145 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b39) the sense strand comprises or consists of a nucleotide sequence set forth in SEQ ID NO: 132 or a nucleotide sequence having 1, 2 or 3 nucleotide differences therefrom, and the antisense strand comprises or consists of a nucleotide sequence set forth in SEQ ID NO: 122 or a nucleotide sequence having 1, 2 or 3 nucleotide differences therefrom;

(b40) the sense strand comprises or consists of a nucleotide sequence set forth in SEQ ID NO: 146 or a nucleotide sequence having 1, 2 or 3 nucleotide differences therefrom, and the antisense strand comprises or consists of a nucleotide sequence set forth in SEQ ID NO: 123 or a nucleotide sequence having 1, 2 or 3 nucleotide differences

therefrom;

(b41) the sense strand comprises or consists of a nucleotide sequence set forth in SEQ ID NO: 147 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;

(b42) the sense strand comprises or consists of a nucleotide sequence set forth in SEQ ID NO: 148 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;

(b43) the sense strand comprises or consists of a nucleotide sequence set forth in SEQ ID NO: 149 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;

(b44) the sense strand comprises or consists of a nucleotide sequence set forth in SEQ ID NO: 150 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;

(b45) the sense strand comprises or consists of a nucleotide sequence set forth in SEQ ID NO: 151 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;

(b46) the sense strand comprises or consists of a nucleotide sequence set forth in SEQ ID NO: 152 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom; or

(b47) the sense strand comprises or consists of a nucleotide sequence set forth in SEQ ID NO: 153 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom.

In some embodiments, the RNAi agent comprises duplex 4023-60, 4122-60, 4132-60, 4149-60, 4122-50, 4122-4, 4122-64, 4122-67, 4122-10, 4122-11, 4122-70, 4122.1-60, 4122-44, 4122-18, 4122-19, 4122-74, 4122-75, 4122-101, 4122-34, 4122-35, 4122-98, 4122-26, 4122-27, 4122-97, 4122-85, 4122-86, 4122-99, 4122-80, 4122-81, 4122-100, 4122.3-60, 4132-10, 4132-11, 4132-44, 4132-26, 4132-27, 4132-34, 4132-35, 4023-65, 4023-44, 4089-60, 4098-60, 4118-60, 4123-60, 4129-60, 4135-60 or 4145-60.

In some embodiments, the RNAi agent further comprises a ligand that targets a hepatocyte; preferably, the ligand comprises a galactose moiety, a galactosamine moiety, or an N-acetylgalactosamine moiety; more preferably, the ligand is a trivalent or tetravalent N-acetylgalactosamine moiety; alternatively, the RNAi agent further comprises a ligand that targets a non-hepatocyte, for example, the ligand comprises a lipophilic monomer. In some embodiments, the ligand is L96, NAG25 or NAG37. In some embodiments, the lipophilic monomer is preferably Y132 to Y135, Y158, Y165 to Y168, L10, L57, L321, L322, Q361 to Q367, Q361s to Q367s, Q370, Q377 to Q379, or Q383.

In some embodiments, the ligand comprises a structure of formula (I):

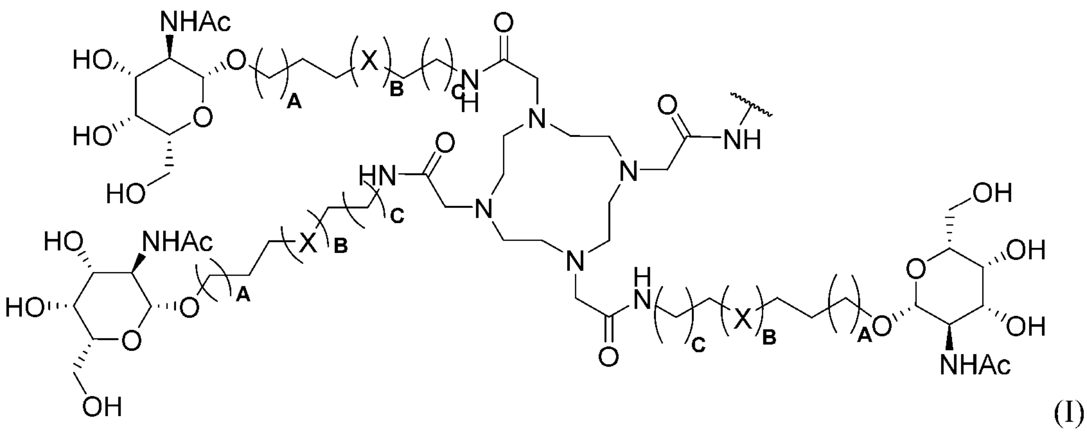

(I)

wherein,

X is -C(O)-NH-, -NH-C(O)-, $-OCH_2-CH_2O-$, -S-,

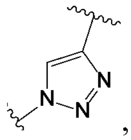, or 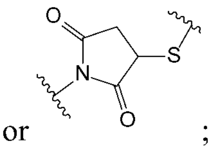;

A and C are independently 0 or an integer between 1 and 14, e.g., 0, 1, 2, 3, 4, 5, 6, 7, 8, 9, 10, 11, 12, 13 or 14;
B is 0 or an integer between 1 and 12, e.g., 0, 1, 2, 3, 4, 5, 6, 7, 8, 9, 10, 11 or 12;
the wave line represents a site to which other part of the RNAi agent is attached, wherein the ligand is connected to 5' and/or 3'-end of the sense strand and/or antisense strand; preferably, the ligand is connected to 5' and/or 3'-end of the sense strand; more preferably, the ligand is connected to 3'-end of the sense strand.

In some embodiments, the ligand has a structure of formula (II) or formula (III):

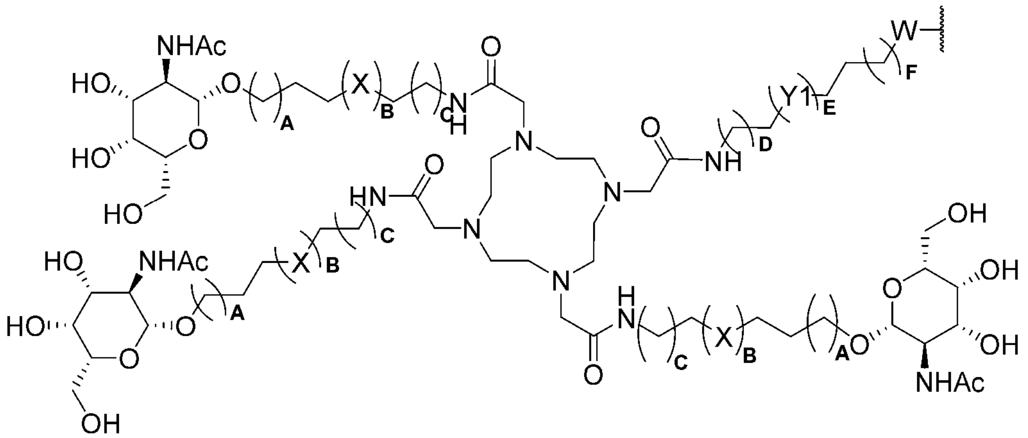
(II)

wherein,

X is -C(O)-NH-, -NH-C(O)-, $-OCH_2-CH_2O-$, -S-,

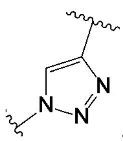, or 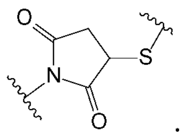;

Y1 is $-C(O)-NR_1-$, -NH-C(O)-, $-OCH_2-CH_2O-$, -S-, -S-S-,

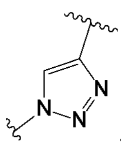, or 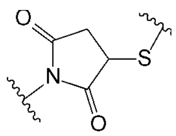,

wherein $R_1$ is an alkyl group having 1-6 (e.g., 1, 2, 3, 4, 5 or 6) carbons or hydrogen;
W is -NH-, -O- or

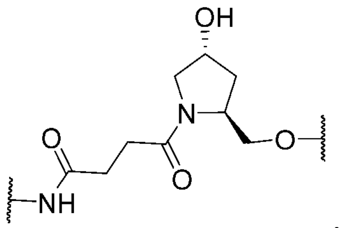

,

wherein the wave line on the right represents a site to which the sense strand or antisense strand is attached;
the wave line connected to W in formula (II) represents a site to which the sense strand or antisense strand is attached;
A, C and F are independently 0 or an integer between 1 and 14, e.g., 0, 1, 2, 3, 4, 5, 6, 7, 8, 9, 10, 11, 12, 13 or 14;
B and E are independently 0 or an integer between 1 and 12, e.g., 0, 1, 2, 3, 4, 5, 6, 7, 8, 9, 10, 11 or 12;
D is an integer between 1 and 20, e.g., 1, 2, 3, 4, 5, 6, 7, 8, 9, 10, 11, 12, 13, 14, 15, 16, 17, 18, 19 or 20;

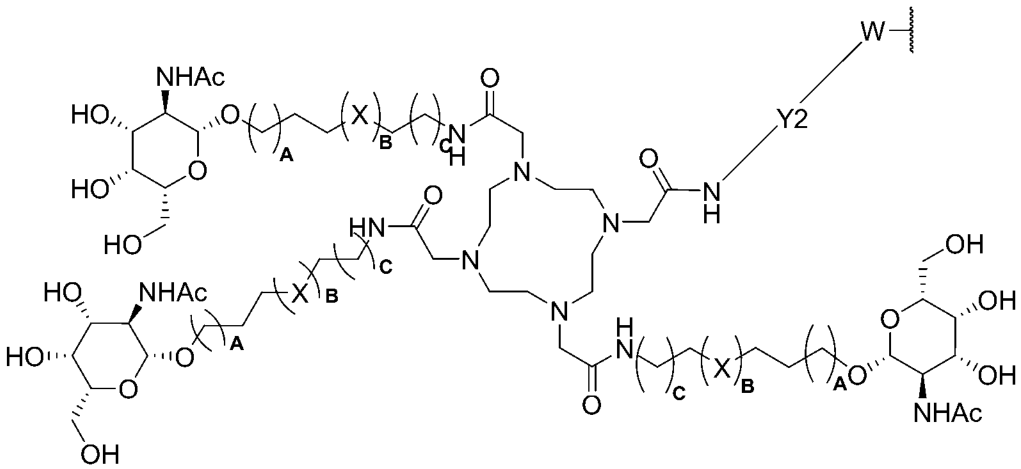

(III)

wherein,

X is -C(O)-NH-, -NH-C(O)-, $-OCH_2-CH_2O-$, -S-,

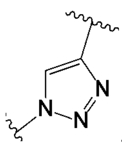 , or 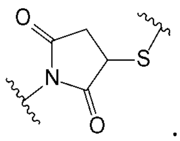 ;

Y2 is a linear short chain or polypeptide structure, selected from the group consisting of:

(1) $-(CH_2)p-(O-CH_2-CH_2)q-(CH_2)j-Z_3-$, wherein $Z_3$ is O, NH or C(O), p is an integer from 1 to 3 (e.g., 1, 2 or 3), q is an integer from 3 to 10 (e.g., 3, 4, 5, 6, 7, 8, 9 or 10); j is 0 or 1;
(2) $-CH_2-C(O)-(Sar)n_1-NH-(CH_2)r-NH-$, wherein $n_1$ is an integer between 2 and 8 (e.g., 2, 3, 4, 5, 6, 7 or 8); r is an integer from 2 to 10 (e.g., 2, 3, 4, 5, 6, 7, 8, 9 or 10);
(3) $-CH_2-C(O)-(Gly)n_2-(Z_1)m_1(Gly)o_1-NH-(CH_2)r-NH-$, wherein $Z_1$ is Ser, Thr, His, Arg, Arg(Me), Trp, Lys, Lys(Me), $Lys(Me)_2$, Orn, Orn(Me), $Orn(Me)_2$, Dap, Dap(Me), $Dap(Me)_2$, Dab, Dab(Me), $Dab(Me)_2$ or Val-Cit, $n_2$ is an integer between 1 and 5 (e.g., 1, 2, 3, 4 or 5); $m_1$ is an integer between 1 and 6 (e.g., 1, 2, 3, 4, 5 or 6); $o_1$ is an integer between 1 and 5 (e.g., 1, 2, 3, 4 or 5); r is an integer from 2 to 10 (e.g., 2, 3, 4, 5, 6, 7, 8, 9 or 10);
(4) $-CH_2-C(O)-(Sar)n_3-NH-(CH_2)r-O-$, wherein $n_3$ is an integer between 1 and 5 (e.g., 1, 2, 3, 4 or 5); r is an integer from 2 to 10 (e.g., 2, 3, 4, 5, 6, 7, 8, 9 or 10);
(5) $-CH_2-CO-(Gly)n_4-(Z_2)m_2-(Gly)o_2-NH-(CH_2)r-O-$, wherein $Z_2$ is Ser, Thr, His, Arg, Arg(Me), Trp, Lys, Lys(Me), $Lys(Me)_2$, Orn, Orn(Me), $Orn(Me)_2$, Dap, Dap(Me), $Dap(Me)_2$, Dab, Dab(Me), $Dab(Me)_2$ or Val-Cit, $n_4$ is an integer between 1 and 5 (e.g., 1, 2, 3, 4 or 5); $m_2$ is an integer between 1 and 6 (e.g., 1, 2, 3, 4, 5 or 6); $o_2$ is an integer between 1 and 5 (e.g., 1, 2, 3, 4 or 5); r is an integer from 2 to 10 (e.g., 2, 3, 4, 5, 6, 7, 8, 9 or 10);
(6)

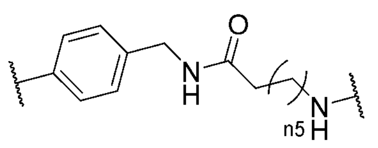 , 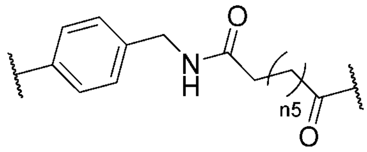 ,

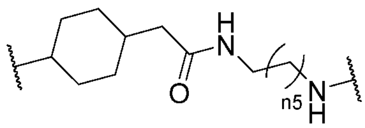 , or 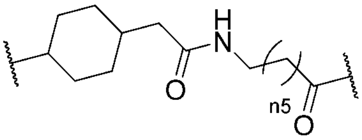 ,

wherein $n_5$ is an integer between 1 and 19 (e.g., 1, 2, 3, 4, 5, 6, 7, 8, 9, 10, 11, 12, 13, 14, 15, 16, 17, 18 or 19);
W is absent, or is

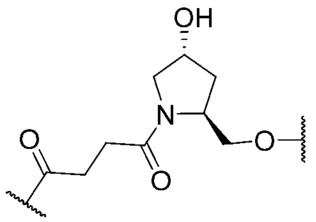 or 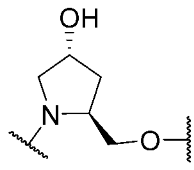,

wherein the wave line on the right represents a site to which the sense strand or antisense strand is attached; the wave line connected to W in formula (III) represents a site to which the sense strand or antisense strand is attached;
A and C are independently 0 or an integer between 1 and 14 (e.g., 0, 1, 2, 3, 4, 5, 6, 7, 8, 9, 10, 11, 12, 13 or 14);
B is 0 or an integer between 1 and 12 (e.g., 0, 1, 2, 3, 4, 5, 6, 7, 8, 9, 10, 11 or 12).

In some embodiments, the ligand has a structure of formula (II') or formula (III'):

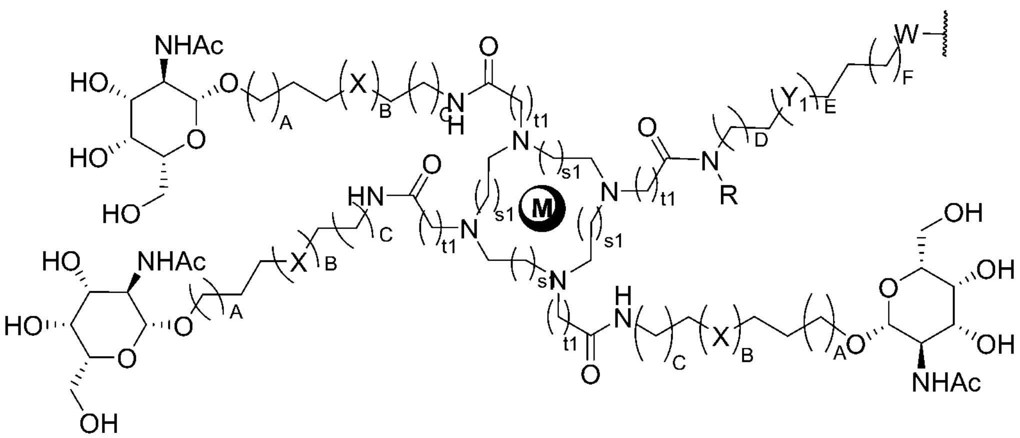

(II')

wherein,

X is -C(O)-NH-, -NH-C(O)-, $-OCH_2-CH_2O-$, -S-,

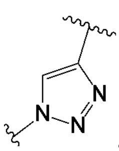 , or 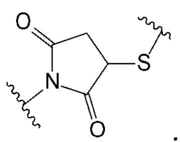 ;

each $s_1$ and each $t_1$ are independently an integer between 1 and 5, preferably independently 1 or 2;

$Y_1$ is -C(O)-$NR_1$-, -$NR_1$-C(O)-, -$OCH_2$-$CH_2$O-, -S-, -S-S-,

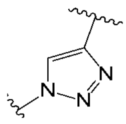

,

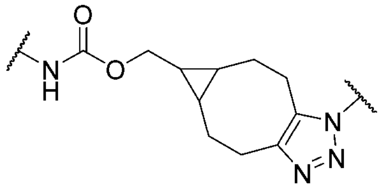

,

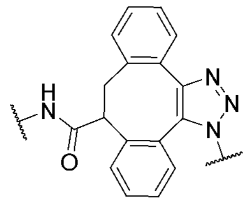

,

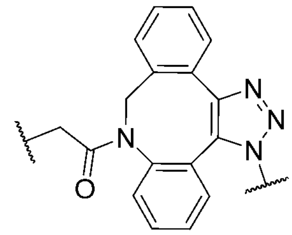

,

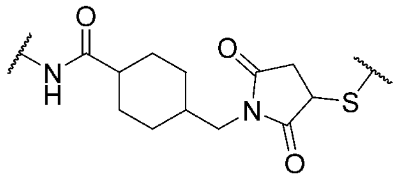

, or

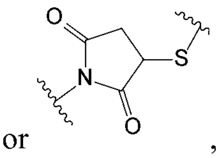

,

$R_1$ is an alkyl group having 1-6 carbons or hydrogen;
R is an alkyl group having 1-16 carbons or hydrogen;
W is -NH-, -O-,

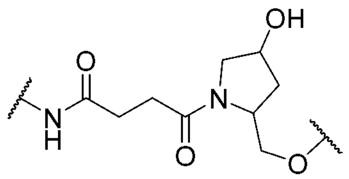

,

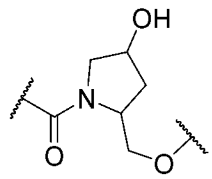

,

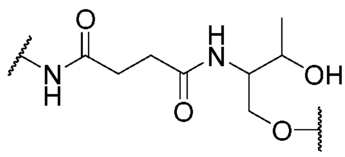

, or

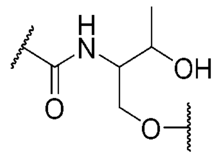

,

or a tautomer, a mesomer, a racemate, an enantiomer, or a diastereoisomer thereof, wherein the wave line on the right represents a site to which other part of the conjugate is attached;
A and C are independently 0 or an integer between 1 and 14, preferably independently 0, 1, 2 or 3;
B is 0 or an integer between 1 and 12, preferably independently 0, 1 or 2;
D is an integer between 1 and 20, preferably an integer from 1 to 5, e.g., 1, 2, 3 or 4;
E is 0 or an integer between 1 and 12, preferably 0 or an integer from 1 to 5, e.g., 0, 1, 2 or 3;
F is 0 or an integer between 1 and 14, preferably 0 or an integer from 1 to 5, e.g., 0, 1, 2 or 3; and
M is present or absent, and when present M is a cation, preferably $H^+$ or a metal cation, wherein the metal cation is preferably a monovalent metal cation, preferably $Na^+$, $K^+$, or $Ag^+$; a divalent metal cation, preferably $Ca^{2+}$, $Mg^{2+}$, $Cu^{2+}$, $Fe^{2+}$, $Zn^{2+}$, or $Mn^{2+}$; or a trivalent metal cation, preferably $Fe^{3+}$, $Al^{3+}$ or a lanthanide metal ion; alternatively, when present, M is a radionuclide, preferably Lu-177, Zr-89, Sc-44, Cu-64, Ga-67, Ga-68, Tc-99m, In-111, Sc-47, Y-90, Y-86, Sm-153, Ho-166, Re-188, Pb-212, Bi-213 or Th-232;

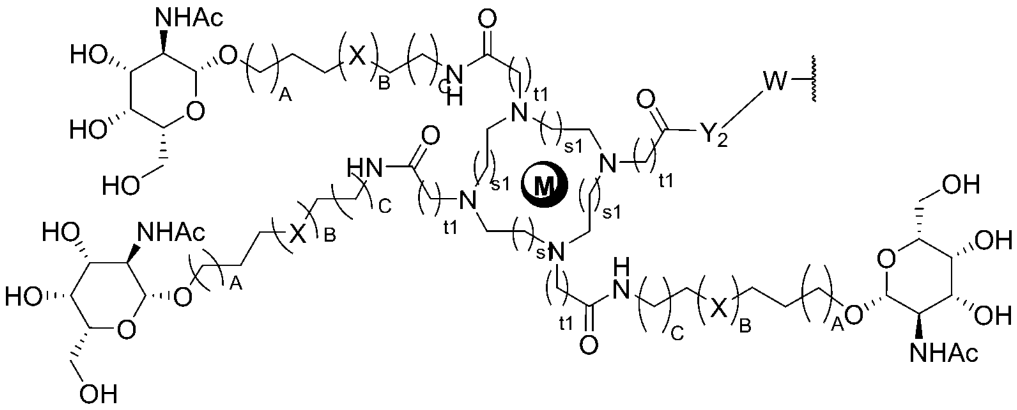

(III')

wherein,

X is -C(O)-NH-, -NH-C(O)-, $-OCH_2-CH_2O-$, -S-,

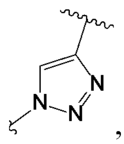

, or

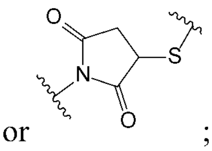

;

each $s_1$ and each $t_1$ are independently an integer between 1 and 5, preferably independently 1 or 2;
$Y_2$ is selected from the group consisting of:

(1) $NH-(CH_2)p-(O-CH_2-CH_2)q-(CH_2)j-Z_1-$, wherein $Z_1$ is O, NH, C(O) or absent, p is an integer from 1 to 3, q is an integer from 3 to 10, j is 0 or 1;
(2) $(Sar)n_1-NH-(CH_2)r-Z_2-$, wherein $n_1$ is an integer between 2 and 8, r is an integer from 2 to 10, $Z_2$ is O, NH, C(O) or absent;
(3) $(Gly)n_2-(Z_3)m_1-(Gly)o_1-NH-(CH_2)r-Z_4-$, wherein $Z_3$ is Ser, HomoSer, (NMe)Ser, Thr, (NMe)Thr, His, (NMe)His, Arg, (NMe)Arg, Arg(Me), Trp, (NMe)Trp, Trp(Me), Lys, (NMe)Lys, Lys(Me), $Lys(Me)_2$, Orn, (NMe) Orn, Orn(Me), $Orn(Me)_2$, Dap, (NMe)Dap, Dap(Me), $Dap(Me)_2$, Dab, (NMe)Dab, Dab(Me), $Dab(Me)_2$, Cit or Val-Cit, $n_2$ is an integer between 0 and 5, $m_1$ is an integer between 1 and 10, $o_1$ is an integer between 0 and 5, r is an integer from 2 to 16, $Z_4$ is O, NH, C(O) or absent;
(4)

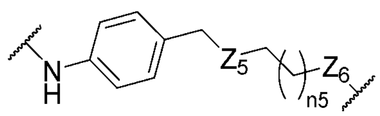

,

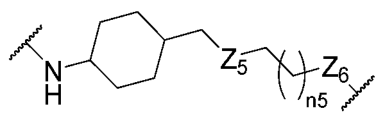

,

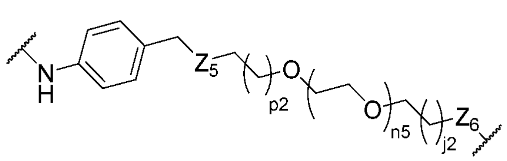

,

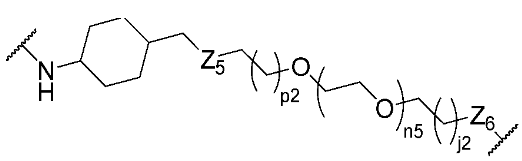

,

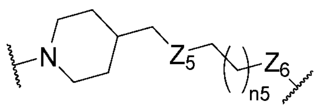

,

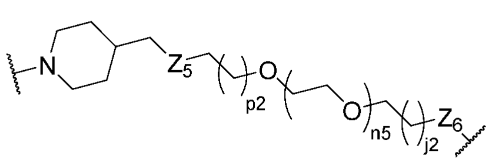

,

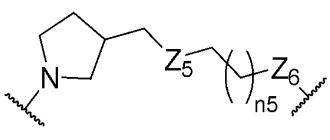,
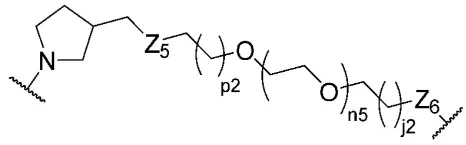,

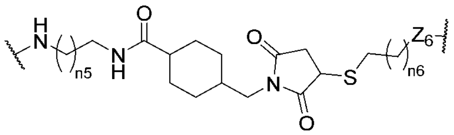,
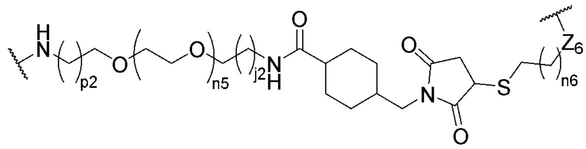,

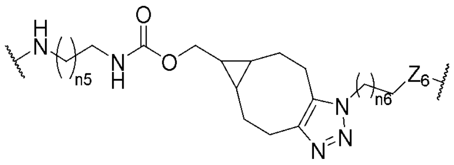,
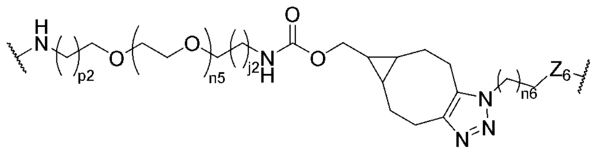,

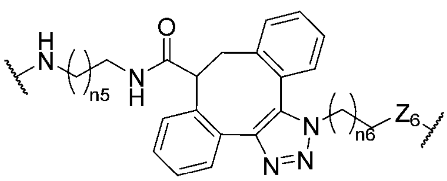,
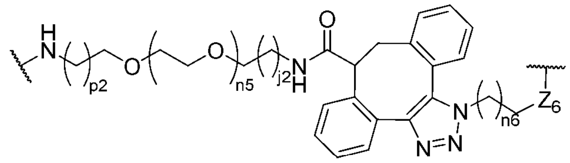,

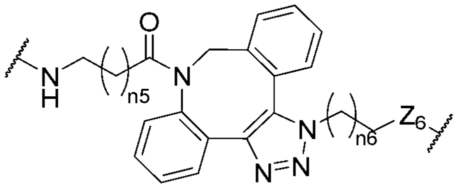,
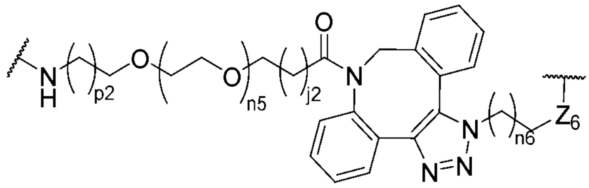,

or a tautomer, a mesomer, a racemate, an enantiomer or a diastereoisomer thereof, wherein $Z_5$ is - C(O)-NH-, -NH-C(O)- or absent, $Z_6$ is O, NH, C(O) or absent, $p_2$ is an integer from 1 to 2, $j_2$ is an integer of 1 or 2, $n_5$ is an integer between 1 and 19, $n_6$ is an integer between 1 and 19;

W is absent or is

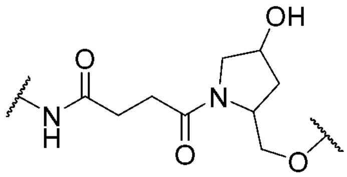,
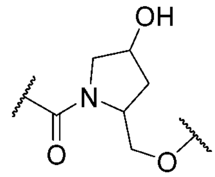,

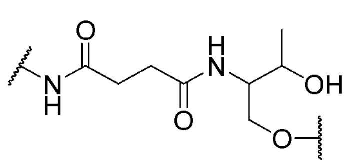, or
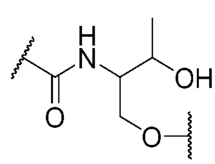,

or a tautomer, a mesomer, a racemate, an enantiomer or a diastereoisomer thereof, wherein the wave line on the right represents a site to which other part of the conjugate is attached;

A and C are independently 0 or an integer between 1 and 14, preferably independently 0, 1, 2 or 3;

B is 0 or an integer between 1 and 12, preferably independently 0, 1 or 2; and

M is present or absent, and when present M is a cation, preferably $H^+$ or a metal cation, wherein the metal cation is

preferably a monovalent metal cation, preferably $Na^+$, $K^+$, or $Ag^+$; a divalent metal cation, preferably $Ca^{2+}$, $Mg^{2+}$, $Cu^{2+}$, $Fe^{2+}$, $Zn^{2+}$, or $Mn^{2+}$; or a trivalent metal cation, preferably $Fe^{3+}$, $Al^{3+}$ or a lanthanide metal ion; alternatively, when present, M is a radionuclide, preferably Lu-177, Zr-89, Sc-44, Cu-64, Ga-67, Ga-68, Tc-99m, In-111, Sc-47, Y-90, Y-86, Sm-153, Ho-166, Re-188, Pb-212, Bi-213 or Th-232.

In some embodiments, the ligand has a structure of YHZY12001 represented by formula (IV-1), YHZY12002 represented by formula (IV-2), YHZY12003 represented by formula (IV-3), YHZY12004 represented by formula (IV-4), YHZY12005 represented by formula (IV-5), YHZY12006 represented by formula (IV-6), or YHZY12007 represented by formula (IV-7):

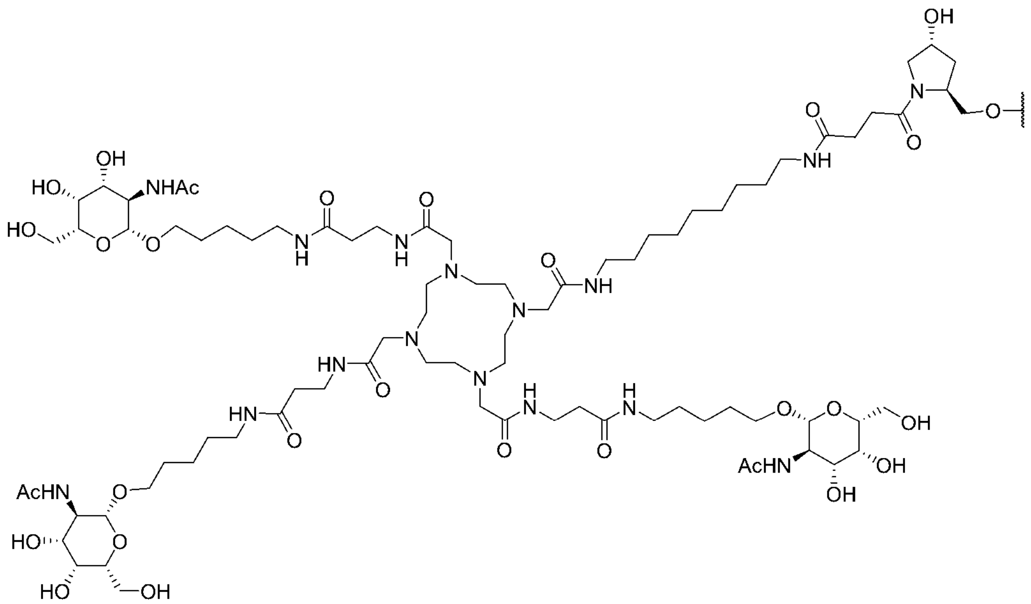

(IV-1) YHZY12001

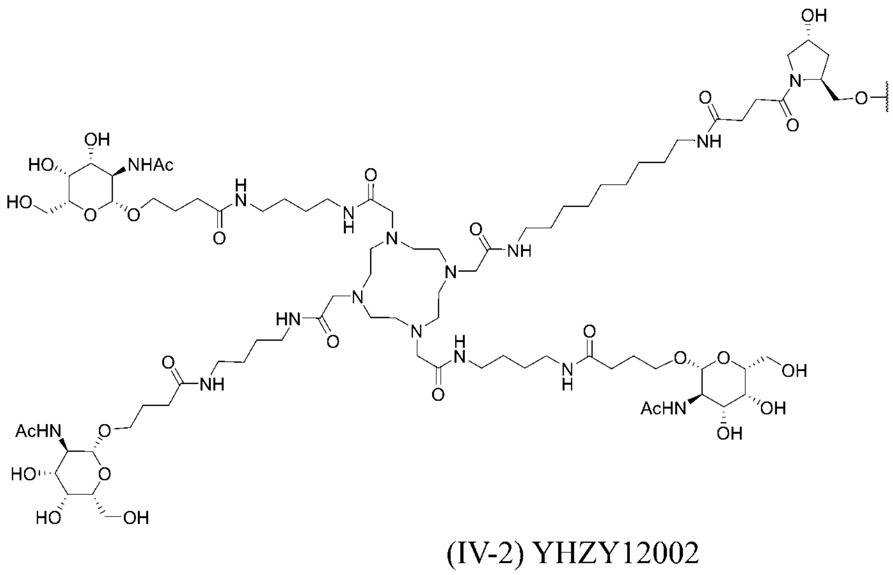

(IV-2) YHZY12002

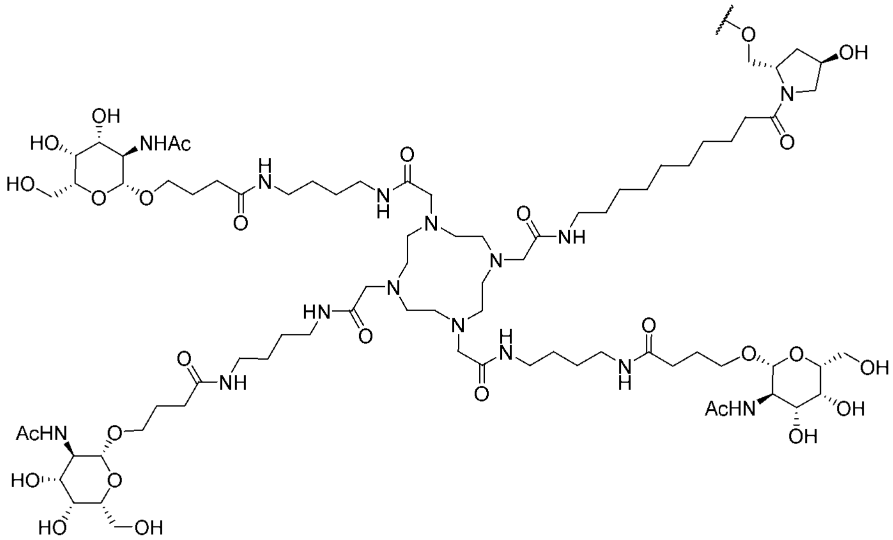

(IV-3) YHZY12003

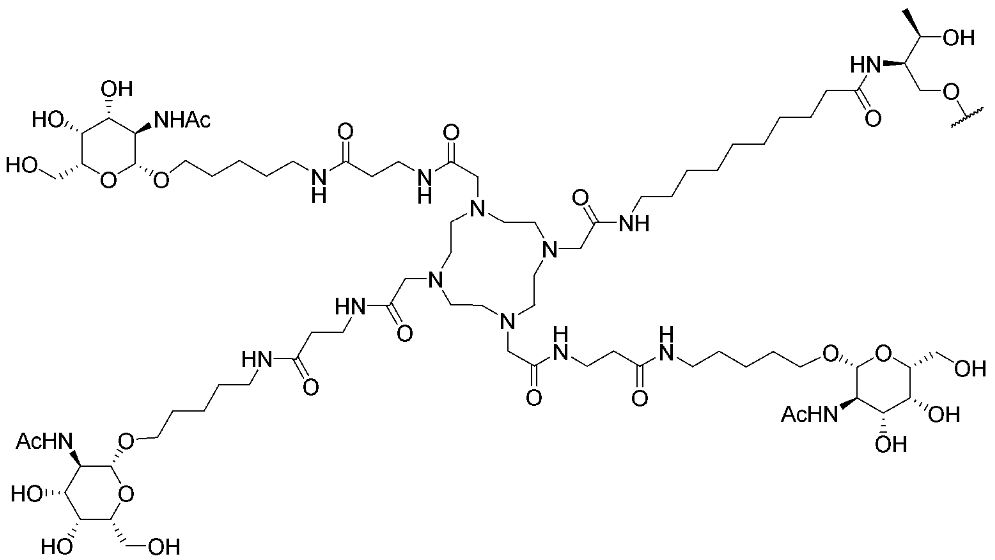

(IV-4) YHZY12004

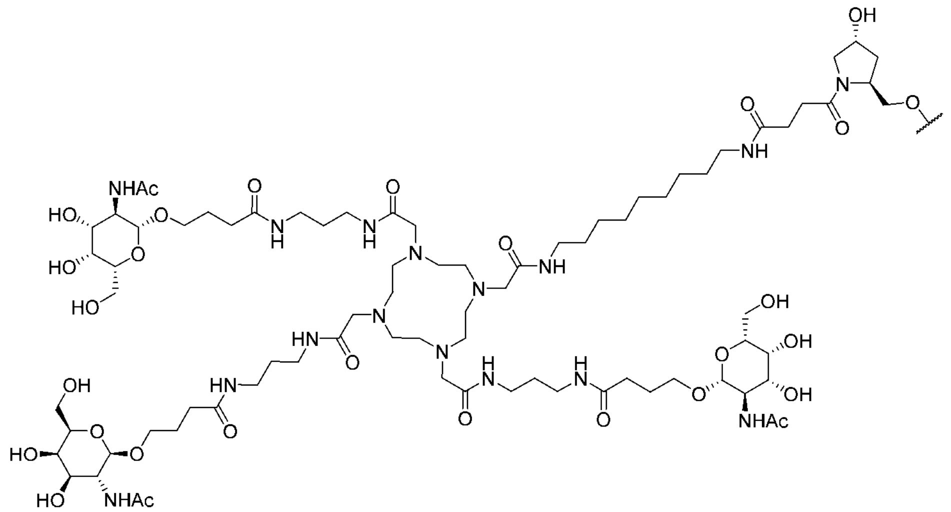

(IV-5) YHZY12005

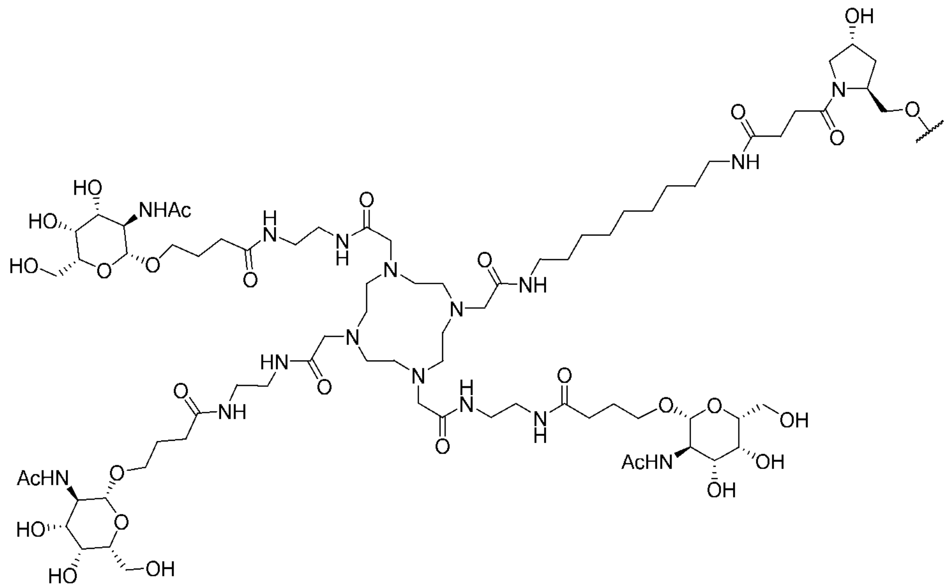

(IV-6) YHZY12006

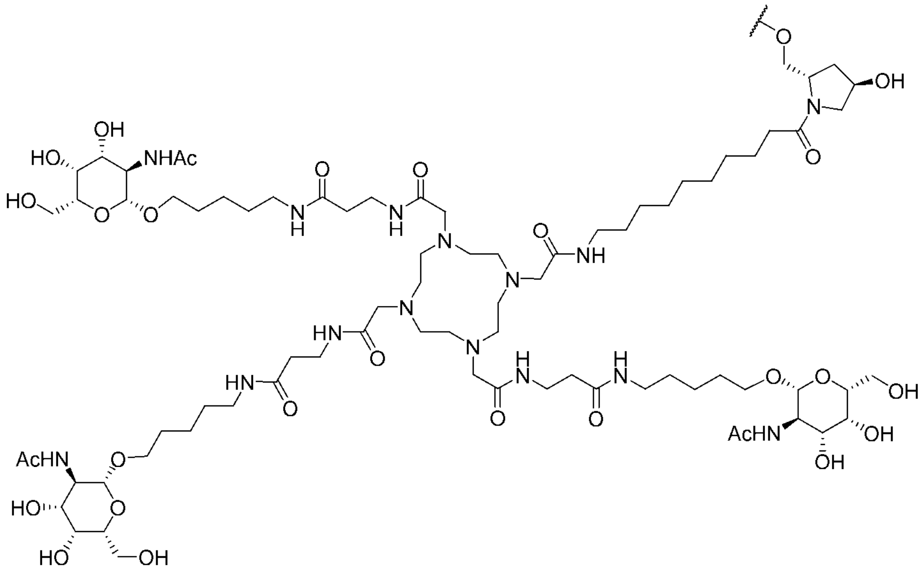

(IV-7) YHZY12007

the wave line represents a site to which the sense strand or antisense strand is attached, wherein preferably the ligand is connected to 5'- and/or 3'-end of the sense strand; preferably, the ligand is connected to 3'-end of the sense strand; preferably, wherein the ligand is connected to the sense strand or antisense strand via a phosphate ester group, a thiophosphate ester group or a phosphonate ester group.

In some embodiments, the RNAi agent has a structure of any one of the following formulas VII-1 to VII-8:

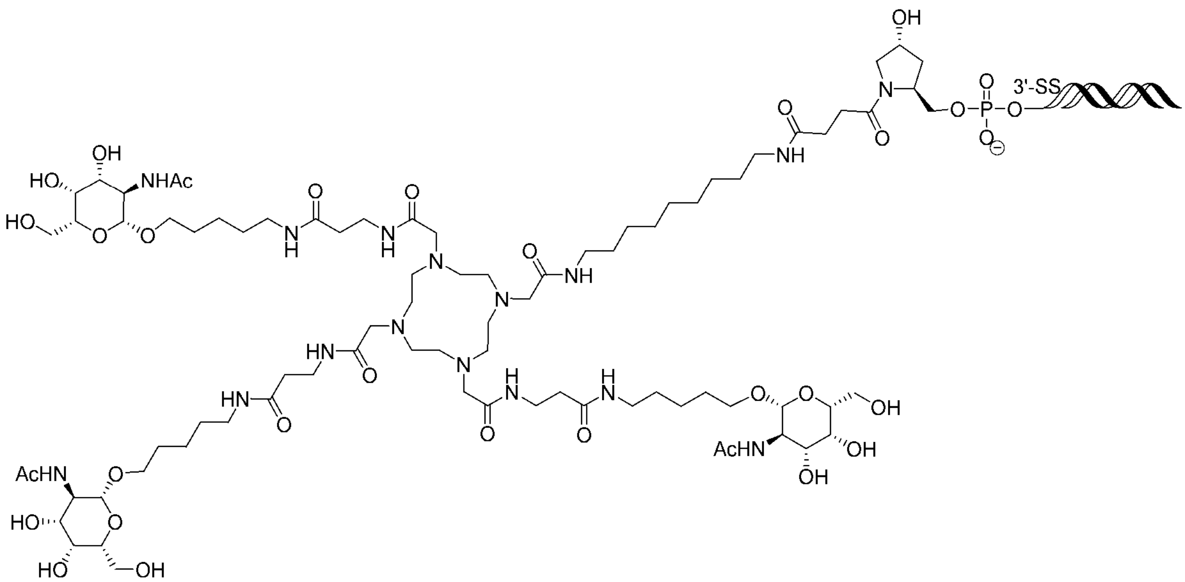

(VII-1)

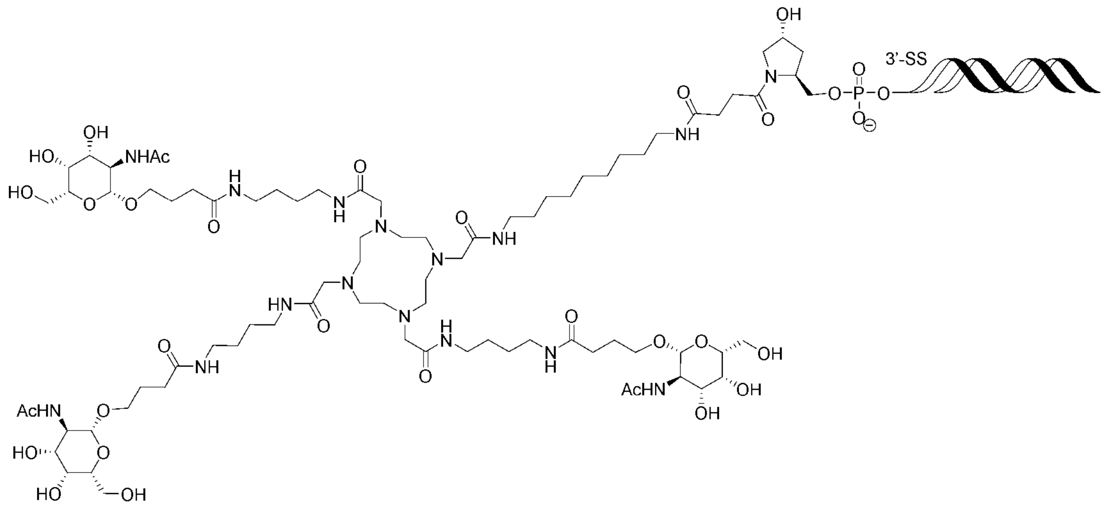

(VII-2)

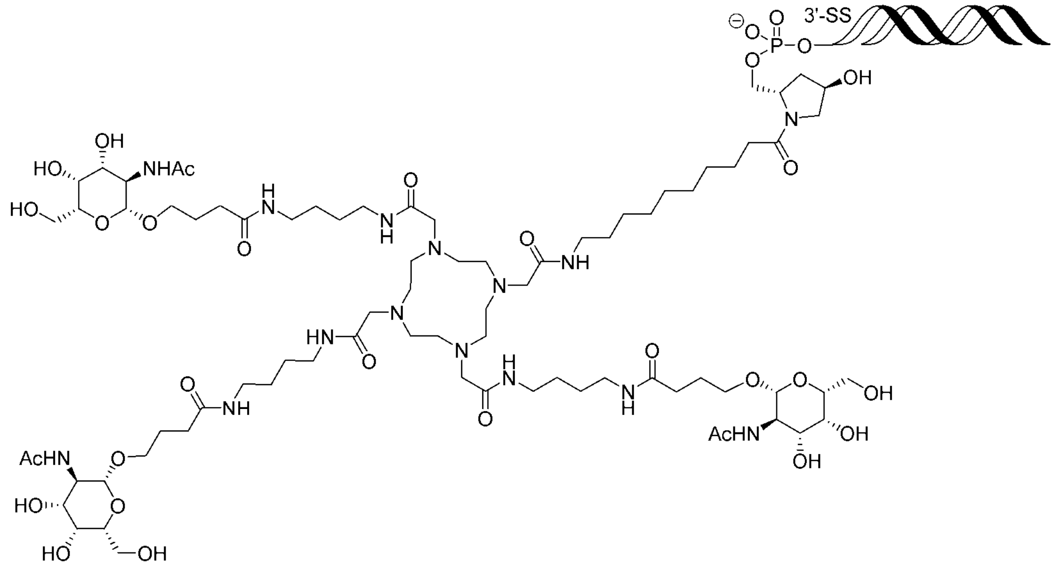

(VII-3)

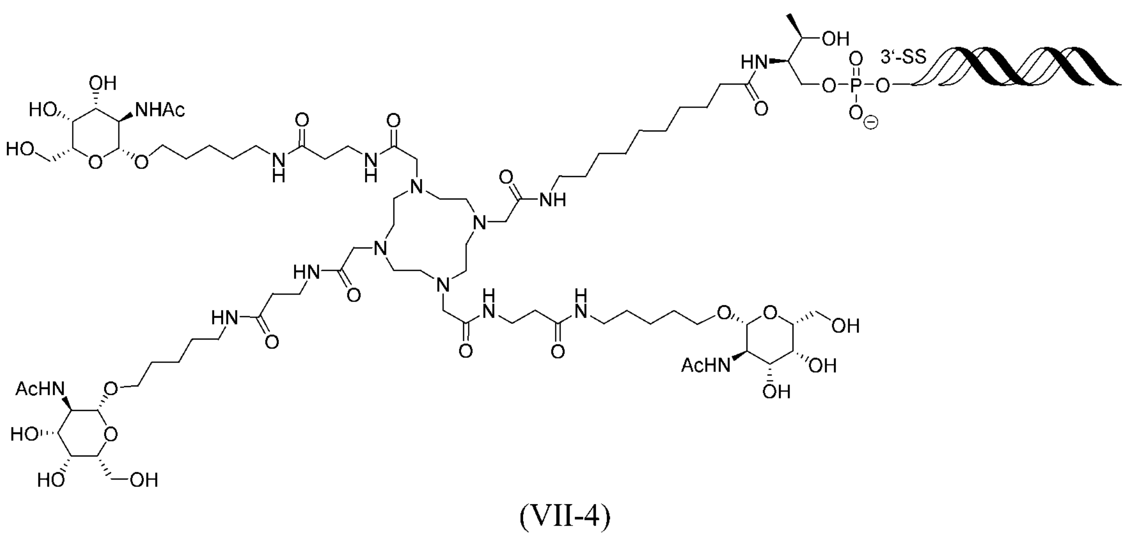

(VII-4)

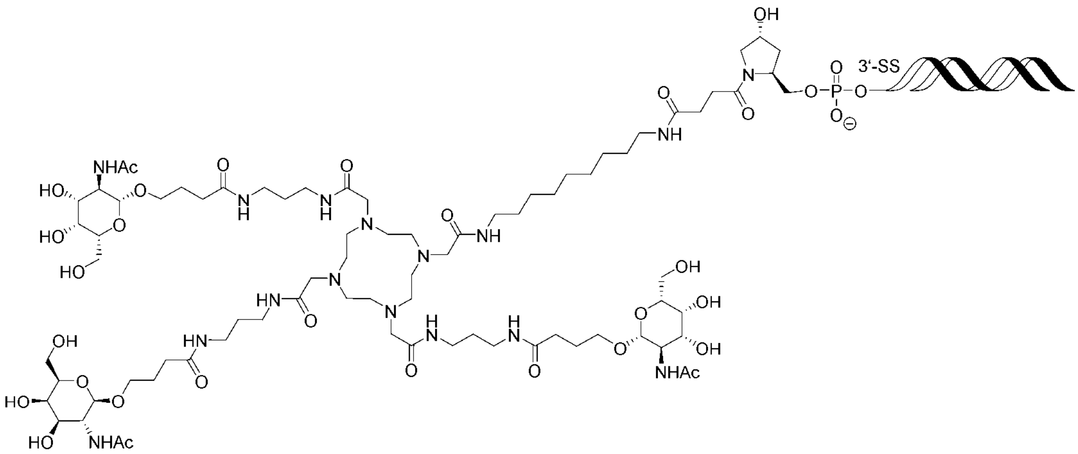

(VII-5)

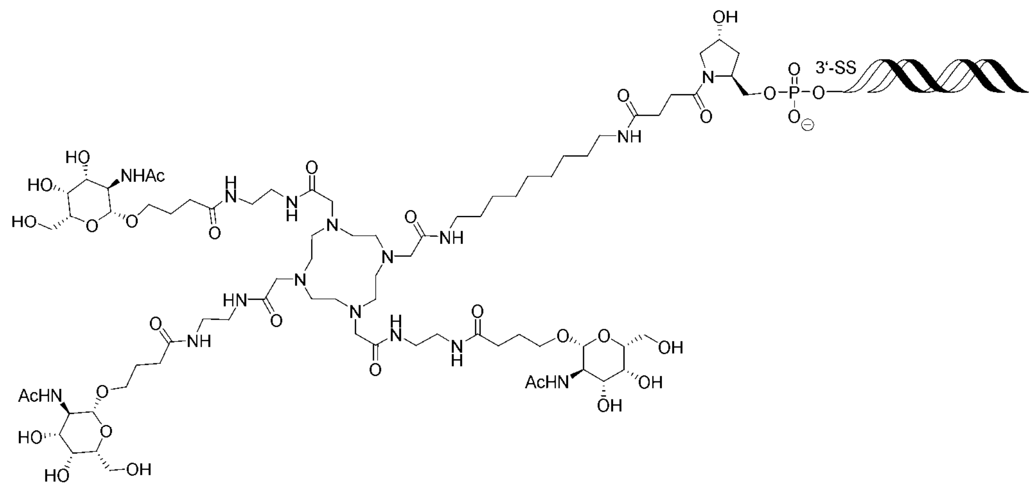

(VII-6)

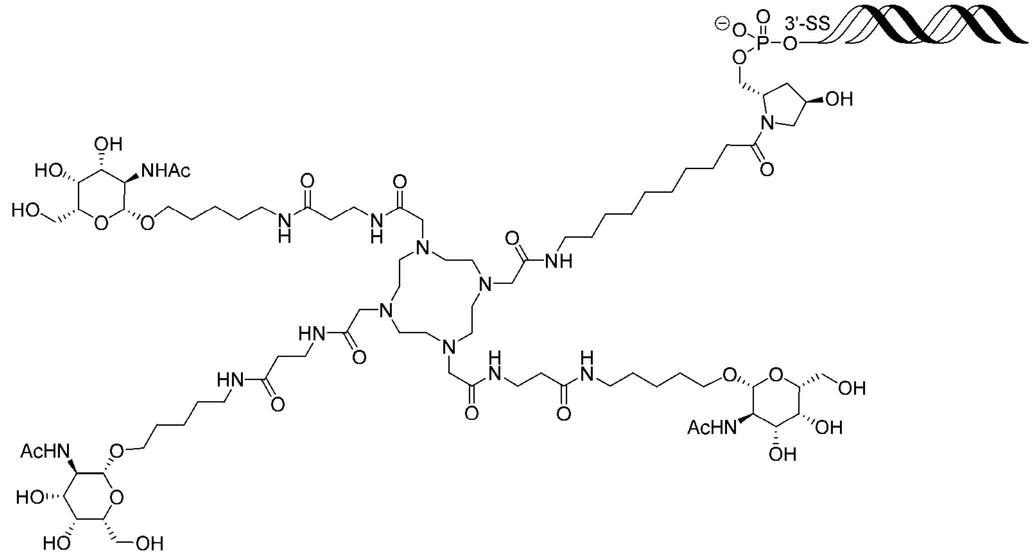

(VII-7)

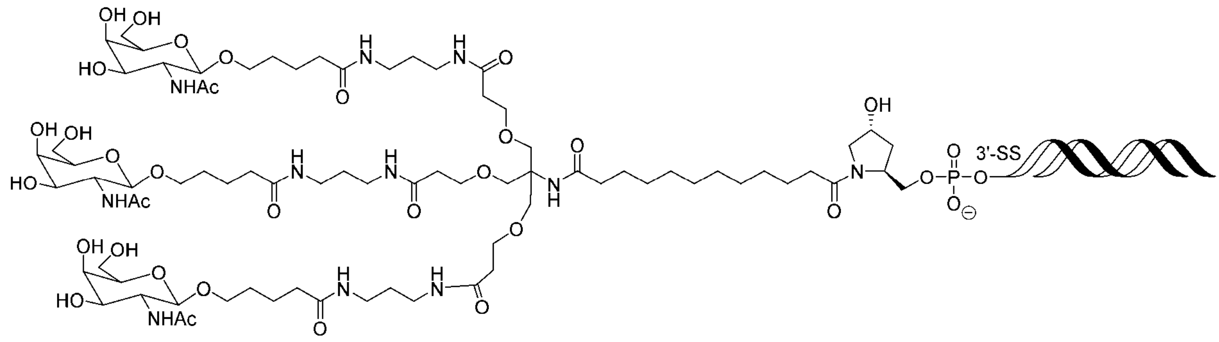

(VII-8).

In some embodiments, the sense strand comprises or consists of a nucleotide sequence set forth in any one of SEQ ID NOs: 68 to 102 or a nucleotide sequence having 1, 2 or 3 nucleotide differences from each thereof.

In some embodiments, in the RNAi agent:

(c1) the sense strand comprises or consists of a nucleotide sequence set forth in SEQ ID NO: 68 or a nucleotide sequence having 1, 2 or 3 nucleotide differences therefrom, and the antisense strand comprises or consists of a nucleotide sequence set forth in SEQ ID NO: 104 or a nucleotide sequence having 1, 2 or 3 nucleotide differences therefrom;

(c2) the sense strand comprises or consists of a nucleotide sequence set forth in SEQ ID NO: 69 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c3) the sense strand comprises or consists of a nucleotide sequence set forth in SEQ ID NO: 70 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(c4) the sense strand comprises or consists of a nucleotide sequence set forth in SEQ ID NO: 71 or a nucleotide sequence having 1, 2 or 3 nucleotide differences therefrom, and the antisense strand comprises or consists of a nucleotide sequence set forth in SEQ ID NO: 107 or a nucleotide sequence having 1, 2 or 3 nucleotide differences therefrom;

(c5) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c6) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 108 or a nucleotide sequence having 1, 2 or 3 nucleotide differences therefrom;

(c7) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 109 or a nucleotide sequence having 1, 2 or 3 nucleotide differences therefrom;

(c8) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 110 or a nucleotide sequence having 1, 2 or 3 nucleotide differences therefrom;

(c9) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 111 or a nucleotide sequence having 1, 2 or 3 nucleotide differences therefrom;

(c10) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c11) the sense strand comprises or consists of a nucleotide sequence set forth in SEQ ID NO: 72 or a nucleotide sequence

sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c12) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 114 or a nucleotide sequence having 1, 2 or 3 nucleotide differences therefrom;

(c13) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 115 or a nucleotide sequence having 1, 2 or 3 nucleotide differences therefrom;

(c14) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 116 or a nucleotide sequence having 1, 2 or 3 nucleotide differences therefrom;

(c15) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c16) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c17) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c18) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c19) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c20) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c21) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c22) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c23) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c24) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c25) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences

therefrom;

(c26) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c27) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c28) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c29) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c30) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c31) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c32) the sense strand comprises or consists of a nucleotide sequence set forth in SEQ ID NO: 79 or a nucleotide sequence having 1, 2 or 3 nucleotide differences therefrom, and the antisense strand comprises or consists of a nucleotide sequence set forth in SEQ ID NO: 118 or a nucleotide sequence having 1, 2 or 3 nucleotide differences therefrom;

(c33) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c34) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c35) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c36) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c37) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c38) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c39) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(c40) the sense strand comprises or consists of a nucleotide sequence set forth in SEQ ID NO: 82 or a nucleotide

sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(c41) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(c42) the sense strand comprises or consists of a nucleotide sequence set forth in SEQ ID NO: 83 or a nucleotide sequence having 1, 2 or 3 nucleotide differences therefrom, and the antisense strand comprises or consists of a nucleotide sequence set forth in SEQ ID NO: 121 or a nucleotide sequence having 1, 2 or 3 nucleotide differences therefrom;

(c43) the sense strand comprises or consists of a nucleotide sequence set forth in SEQ ID NO: 84 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(c44) the sense strand comprises or consists of a nucleotide sequence set forth in SEQ ID NO: 84 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(c45) the sense strand comprises or consists of a nucleotide sequence set forth in SEQ ID NO: 85 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(c46) the sense strand comprises or consists of a nucleotide sequence set forth in SEQ ID NO: 85 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(c47) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(c48) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(c49) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(c50) the sense strand comprises or consists of a nucleotide sequence set forth in SEQ ID NO: 87 or a nucleotide sequence having 1, 2 or 3 nucleotide differences therefrom, and the antisense strand comprises or consists of a nucleotide sequence set forth in SEQ ID NO: 122 or a nucleotide sequence having 1, 2 or 3 nucleotide differences therefrom;

(c51) the sense strand comprises or consists of a nucleotide sequence set forth in SEQ ID NO: 88 or a nucleotide sequence having 1, 2 or 3 nucleotide differences therefrom, and the antisense strand comprises or consists of a nucleotide sequence set forth in SEQ ID NO: 123 or a nucleotide sequence having 1, 2 or 3 nucleotide differences therefrom;

(c52) the sense strand comprises or consists of a nucleotide sequence set forth in SEQ ID NO: 89 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;

(c53) the sense strand comprises or consists of a nucleotide sequence set forth in SEQ ID NO: 90 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;

(c54) the sense strand comprises or consists of a nucleotide sequence set forth in SEQ ID NO: 91 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences

therefrom;
(c55) the sense strand comprises or consists of a nucleotide sequence set forth in SEQ ID NO: 92 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;
(c56) the sense strand comprises or consists of a nucleotide sequence set forth in SEQ ID NO: 93 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;
(c57) the sense strand comprises or consists of a nucleotide sequence set forth in SEQ ID NO: 94 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom;
(c58) the sense strand comprises or consists of a nucleotide sequence set forth in SEQ ID NO: 95 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom;
(c59) the sense strand comprises or consists of a nucleotide sequence set forth in SEQ ID NO: 96 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;
(c60) the sense strand comprises or consists of a nucleotide sequence set forth in SEQ ID NO: 97 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;
(c61) the sense strand comprises or consists of a nucleotide sequence set forth in SEQ ID NO: 98 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;
(c62) the sense strand comprises or consists of a nucleotide sequence set forth in SEQ ID NO: 99 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;
(c63) the sense strand comprises or consists of a nucleotide sequence set forth in SEQ ID NO: 100 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;
(c64) the sense strand comprises or consists of a nucleotide sequence set forth in SEQ ID NO: 101 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom; or
(c65) the sense strand comprises or consists of a nucleotide sequence set forth in SEQ ID NO: 102 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom.

In some embodiments, the RNAi agent comprises or consists of duplex 4023-60-L96, 4122-60-L96, 4132-60-L96, 4149-60-L96, 4122-60-001, 4122-50-001, 4122-4-001, 4122-64-001, 4122-67-001, 4122-10-001, 4122-11-001, 4122-70-001, 4122.1-60-001, 4122-44-001, 4122-18-001, 4122-19-001, 4122-74-001, 4122-75-001, 4122-101-001, 4122-34-001, 4122-35-001, 4122-98-001, 4122-26-001, 4122-27-001, 4122-97-001, 4122-85-001, 4122-86-001, 4122-99-001, 4122-80-001, 4122-81-001, 4122-100-001, 4122.3-60-001, 4122-60-002, 4122-10-002, 4122-11-002, 4122-60-004, 4122-10-004, 4122-11-004, 4132-60-001, 4132-10-001, 4132-11-001, 4132-44-001, 4132-26-001, 4132-27-001, 4132-34-001, 4132-35-001, 4132-60-004, 4132-10-004, 4132-11-004, 4023-65-001, 4023-44-001, 4089-60-L96, 4098-60-L96, 4118-60-L96, 4123-60-L96, 4129-60-L96, 4135-60-L96, 4145-60-L96, 4089-60-001, 4098-60-001, 4118-60-001, 4123-60-001, 4129-60-001, 4135-60-001 or 4145-60-001.
A further aspect of the present invention provides a pharmaceutical composition comprising any RNAi agent of the present invention and a pharmaceutically acceptable carrier; preferably, the pharmaceutical composition is formulated for intravenous or subcutaneous injection.

Another aspect of the present invention provides the use of any RNAi agent of the present invention in the manufacture of a medicament for:

(i) reducing expression level of KHK in a cell;
(ii) preventing or treating a disease caused by an elevated KHK expression level; or
(iii) preventing or treating a metabolic disease, wherein the metabolic disease is, for example, hypertriglyceridemia, obesity, hyperlipidemia, dyslipidemia, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, hyperuricemia or gout.

Accordingly, the present invention also provides a method for reducing the expression level of KHK in a cell, the method comprising administering a therapeutically effective amount of any RNAi agent of the present invention to a subject in need thereof. The present invention also provides a method for preventing or treating a disease caused by elevated KHK expression level, the method comprising administering a therapeutically effective amount of any RNAi agent of the present invention to a subject in need thereof. The present invention also provides a method for preventing or treating a metabolic disease, the method comprising administering a therapeutically effective amount of any RNAi agent of the present invention to a subject in need thereof, wherein the metabolic disease is, for example, one or more of hypertriglyceridemia, obesity, hyperlipidemia, dyslipidemia, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, hyperuricemia and gout. The present invention also provides the use of any RNAi agent of the present invention in the manufacture of a medicament for preventing or treating the aforementioned metabolic diseases. The present invention also provides any RNAi agent of the present invention for use in preventing or treating the aforementioned metabolic diseases.

Other aspects of the invention will be apparent from the detailed description of the specification that follows.

**Brief Description of the Figures**

Fig. 1 shows the inhibitory efficiency of exemplary KHK siRNAs of the present invention on target gene.

**Detailed Description of the Invention**

Definition

Ketohexokinase (KHK), also known as fructokinase, refers herein to the ketohexokinase protein or its encoding gene. The human KHK gene is located on chromosome 2p23. Two isoforms of ketohexokinase, KHK-A and KHK-C, have been identified, produced by alternative splicing of the full-length mRNA. KHK-C mRNA is highly expressed primarily in the liver, kidney, and small intestine. KHK-C has a much lower Km for fructose binding compared to KHK-A, enabling efficient phosphorylation of dietary fructose. An exemplary NCBI Reference Sequence for human KHK mRNA is NM_006488.3.

Some amino acid abbreviations used herein are listed below.

| | | | |
|---|---|---|---|
| Sar | sarcosine | Orn | ornithine |
| Gly | glycine | Om(Me) | N'-monomethylomithine |
| Thr | threonine | $Orn(Me)_2$ | N',N'-dimethylomithine |
| His | histidine | Dap | 2,3-diaminopropionic acid |
| Arg | arginine | Dap(Me) | N'-monomethyl-2,3-diaminopropionic acid |
| Arg(Me) | N'-methyl-arginine | $Dap(Me)_2$ | N',N'-dimethyl-2,3-diaminopropionic acid |
| Trp | tryptophan | Dab | 2,4-diaminobutyric acid |
| Lys | lysine | Dab(Me) | N'-monomethyl-2,4-diaminobutyric acid |
| Lys(Me) | N'-monomethyllysine | $Dab(Me)_2$ | N',N'-dimethyl-2,4-diaminobutyric acid |
| Val | valine | Cit | citruline |

As used herein, the term "RNAi agent" refers to a reagent comprising an RNA molecule that, when introduced into a cell, is capable of downregulating the expression of a target gene (in this context, the KHK gene) through an RNA

interference mechanism. The terms "RNAi agent of the present invention," "RNAi agent described herein," or similar expressions encompass both the modified RNAi agents of the present invention, irrespective of sequence and target gene, and the RNAi agents of the present invention with specific sequences for interfering with the KHK gene. RNA interference refers to a process whereby a nucleic acid molecule induces cleavage and degradation of a target RNA molecule (e.g., an mRNA molecule) in a sequence-specific manner, such as through the RNA-induced silencing complex (RISC) pathway. RNAi agents herein include siRNA, shRNA, and DNA/RNA hybrid molecules, also sometimes collectively referred to as double-stranded RNA (dsRNA), which comprise two antiparallel continuous nucleotide strands sufficiently complementary to hybridize and form a double-stranded region. "Hybridization" refers to the pairing of complementary polynucleotides, typically through hydrogen bonding between complementary bases in the two polynucleotides (e.g., Watson-Crick hydrogen bonding, Wobble hydrogen bonding, Hoogsteen hydrogen bonding, or reverse Hoogsteen hydrogen bonding). The "double-stranded region" refers to an area within two complementary or substantially complementary polynucleotides that form base pairs through hybridization, thereby creating a duplex between the two polynucleotide strands.

The term "antisense strand" refers to the strand in a dsRNA that contains a region substantially complementary to a target sequence. The term "sense strand" or "passenger strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at the 5' and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the terms "blunt end" or "blunt terminus" with respect to dsRNA refer to the absence of unpaired nucleotides or nucleotide analogs at a given terminus of the dsRNA, i.e., no nucleotide overhang. One end or both ends of the dsRNA may be blunt. Where both ends of the dsRNA are blunt, the dsRNA is referred to as blunt-ended. It is to be expressly understood that a "blunt-ended" dsRNA is one having blunt termini at both ends, meaning no nucleotide overhang exists at either end of the molecule. In most cases, such molecules are double-stranded throughout their entire length. As used herein, the term "nucleotide overhang" refers to at least one unpaired nucleotide projecting from the duplex structure of the dsRNA. For example, a nucleotide overhang exists when the 3' terminus of one strand of the dsRNA extends beyond the 5' terminus of the other strand, or vice versa. The overhang may comprise or consist of nucleotides/nucleosides analogs, including deoxyribonucleotides/deoxyribonucleosides. Overhangs may be present on the sense strand, antisense strand, or any combination thereof. Furthermore, the overhanging nucleotides may reside at the 5' terminus, 3' terminus, or both termini of the antisense strand or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term "dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase. Modifications suitable for the present invention include all types of modifications disclosed herein or known in the art. For example, the modified nucleotide is selected from the group consisting of: 2'-deoxythymidine (dT) nucleotide, 2'-O-methyl-modified nucleotide, 2'-fluoro-modified nucleotide, 2'-deoxy-modified nucleotide,

locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide, 2'-amino-modified nucleotide, 2'-O-allyl-modified nucleotide, 2'-C-alkyl-modified nucleotide, 2'-methoxyethyl-modified nucleotide, abasic nucleotide, inverted abasic nucleotide, inverted nucleotide, morpholino nucleotide, phosphoramidate, tetrahydropyran-modified nucleotide, 1,5-anhydrohexitol-modified nucleotide, cyclohexenyl-modified nucleotide, nucleotide comprising phosphorothioate group, nucleotide comprising methylphosphonate group, nucleotide comprising 5'-phosphate, and nucleotide comprising 5'-phosphate mimic; preferably selected from 2'-O-methyl-modified nucleotide, 2'-fluoro-modified nucleotide, 2'-deoxy-modified nucleotide, and nucleotide comprising phosphorothioate internucleoside linkage.

The term "ligand" refers to a cell or tissue targeting agent that binds to a specified cell type (e.g., hepatocytes), such as a lectin, glycoprotein, lipid, or protein (e.g., an antibody). Exemplary targeting agents include thyrotropin, melanotropin, lectins, glycoproteins, surfactant protein A, mucin carbohydrates, multivalent lactose, multivalent galactose, N-acetylgalactosamine (GalNAc), multivalent (e.g., bivalent or trivalent) GalNAc, N-acetylglucosamine, multivalent mannose, multivalent fucose, glycosylated polyamino acids, multivalent galactose, transferrin, bisphosphonates, polyglutamate, polyaspartate, cholesterol, steroids, bile acids, folate, vitamin B12, biotin, RGD peptides, and RGD peptide mimetics. In preferred embodiments, the ligand is a carbohydrate, such as a monosaccharide, disaccharide, trisaccharide, tetrasaccharide, or polysaccharide. For example, the ligand may be a derivative comprising GalNAc. In preferred embodiments, the ligand is a derivative comprising one or more N-acetylgalactosamine moieties attached via a bivalent or trivalent branched linker.

The term "therapeutically effective amount" refers to an amount of an RNAi agent or composition of the present invention that is effective to produce a desired therapeutic effect in at least a subpopulation of cells in an animal at a reasonable benefit/risk ratio applicable to any medical treatment.

The term "pharmaceutically acceptable" refers to compounds, materials, compositions, and/or dosage forms that are, within the scope of sound medical judgment, suitable for contact with tissues of humans and animals without excessive toxicity, irritation, allergic reactions, or other problems or complications and commensurate with a reasonable benefit/risk ratio.

The term "pharmaceutically acceptable carrier" refers to a pharmaceutically acceptable material, composition, or vehicle that participates in carrying or transporting an RNAi agent from one organ or part of the body to another organ or part of the body, such as a liquid or solid filler, diluent, excipient, manufacturing aid, or solvent encapsulating material. Each carrier must be "acceptable" in the sense of being compatible with other ingredients of the composition and innocuous to the patient.

The term "treatment" encompasses prevention, therapy, and cure. The patient receiving such treatment is typically any animal in need, including primates (particularly humans) and other mammals such as horses, cattle, swine, sheep, poultry, and companion animals.

## RNAi Agents For Inhibiting KHK Gene Expression

One aspect of the present invention provides an RNAi agent for inhibiting KHK gene expression, comprising a sense strand and an antisense strand that form a complementary duplex region, wherein the antisense strand comprises at least 15 contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1 to 3 nucleotide differences therefrom.

In some embodiments, the present invention provides an RNAi agent for inhibiting KHK gene expression, comprising a sense strand and an antisense strand that form a complementary duplex region, wherein the antisense strand comprises at least 15 (e.g., 15, 16, 17, 18, 19, 20, or 21) contiguous nucleotides of any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65).

In some embodiments, the present invention provides an RNAi agent for inhibiting KHK gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65).

In the present invention, when referring to 1, 2, or 3 nucleotide differences, the 1, 2, or 3 nucleotide differences may be on the sense strand and/or antisense strand outside the duplex region. In some embodiments, the 1, 2, or 3 nucleotide differences are on the sense strand and/or antisense strand within the duplex region. In some embodiments, a portion of the 1, 2, or 3 nucleotide differences are on the sense strand and/or antisense strand within the duplex region, and another portion are on the sense strand and/or antisense strand outside the duplex region. For the antisense strand, in some embodiments, the 1, 2, or 3 nucleotide differences are located at the extreme 3' end or extreme 5' end of the antisense strand. In some embodiments, the 1, 2, or 3 nucleotide differences are located between the extreme 3' end and

extreme 5' end of the antisense strand. In some embodiments, 1 or 2 of the 1, 2, or 3 nucleotide differences are located at the extreme 3' end or extreme 5' end of the antisense strand, and the other 1 or 2 are located between the extreme 3' end and extreme 5' end of the antisense strand. For the sense strand, in some embodiments, the 1, 2, or 3 nucleotide differences are located at the extreme 3' end or extreme 5' end of the sense strand. In some embodiments, the 1, 2, or 3 nucleotide differences are located between the extreme 3' end and extreme 5' end of the sense strand. In some embodiments, 1 or 2 of the 1, 2, or 3 nucleotide differences are located at the extreme 3' end or extreme 5' end of the sense strand, and the other 1 or 2 are located between the extreme 3' end and extreme 5' end of the sense strand.

In some embodiments, the present invention provides an RNAi agent for inhibiting KHK gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 1 nucleotide difference from any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65), the 1 nucleotide difference being located at the extreme 3' end of the antisense strand. In some embodiments, the present invention provides an RNAi agent for inhibiting KHK gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 2 nucleotide differences from any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65), the 2 nucleotide differences being located consecutively at the extreme 3' end of the antisense strand.

In some embodiments, the present invention provides an RNAi agent for inhibiting KHK gene expression, comprising a sense strand and an antisense strand that form a complementary duplex region, wherein the antisense strand is no more than 23 nucleotides in length and comprises at least 15 (e.g., 15, 16, 17, 18, 19, 20, or 21) contiguous nucleotides of any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65).

In some embodiments, the present invention provides an RNAi agent for inhibiting KHK gene expression, comprising a sense strand and an antisense strand that form a complementary duplex region, wherein the antisense strand is no more than 23 nucleotides in length and comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65).

One aspect of the present invention provides an RNAi agent for inhibiting KHK gene expression, comprising an antisense strand, wherein the antisense strand comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom.

In some embodiments, the present invention provides an RNAi agent for inhibiting KHK gene expression, comprising an antisense strand, wherein the antisense strand comprises at least 15 (e.g., 15, 16, 17, 18, 19, 20, or 21) contiguous nucleotides of any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65).

In some embodiments, the present invention provides an RNAi agent for inhibiting KHK gene expression, comprising an antisense strand, wherein the antisense strand comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65).

In some embodiments, the length of the duplex region is about 17 to about 23 base pairs. For example, suitable lengths for the duplex region are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to 23 base pairs, about 19 to 22 base pairs, about 19 to 21 base pairs, about 20 to 23 base pairs, about 20 to 22 base pairs, or about 21 to 23 base pairs. In certain embodiments, the length of the duplex region is about 18 to about 21 base pairs. In other embodiments, the length of the duplex region is about 19 base pairs.

Accordingly, in some embodiments of the present invention, an RNAi agent for inhibiting KHK gene expression is provided, comprising a sense strand and an antisense strand that form a complementary duplex region, the length of the duplex region being about 17 to about 23 base pairs, and wherein the antisense strand is no more than 23 nucleotides in length and comprises at least 15 contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1 to 3 nucleotide differences therefrom.

In some embodiments of the present invention, an RNAi agent for inhibiting KHK gene expression is provided,

comprising a sense strand and an antisense strand that form a complementary duplex region, the length of the duplex region being about 18 to about 21 base pairs, and wherein the antisense strand is no more than 23 nucleotides in length and comprises at least 15 contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1 to 3 nucleotide differences therefrom.

In some embodiments of the present invention, an RNAi agent for inhibiting KHK gene expression is provided, comprising a sense strand and an antisense strand that form a complementary duplex region, the length of the duplex region being about 19 base pairs, and wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom.

In some embodiments, the sense and antisense strands of the RNAi agent of the invention each independently have a length of about 17 to about 23 nucleotides, e.g., about 18 to about 23 nucleotides, about 19 to about 23 nucleotides, about 20 to about 23 nucleotides, about 21 to about 23 nucleotides, about 17 to about 22 nucleotides, about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense and antisense strands each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent for inhibiting KHK gene expression comprises two nucleotide overhangs. For example, in one embodiment, the RNAi agent for inhibiting KHK gene expression comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent for inhibiting KHK gene expression comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent for inhibiting KHK gene expression is blunt-ended and comprises (i) a sense strand and an antisense strand each having a length of 21 nucleotides, and (ii) a duplex region having a length of 21 base pairs. In another such embodiment, the RNAi agent for inhibiting KHK gene expression is blunt-ended and comprises (i) a sense strand and an antisense strand each having a length of 23 nucleotides, and (ii) a duplex region having a length of 23 base pairs. In another such embodiment, the RNAi agent for inhibiting KHK gene expression is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent for inhibiting KHK gene expression comprises at least one nucleotide overhang. For example, in some embodiments, the sense strand is 1 to 4 nucleotides longer than the antisense strand, and the duplex region formed by both strands equals the length of the antisense strand, such that the sense strand forms an overhang comprising 1 to 4 unpaired nucleotides. In other embodiments, the antisense strand is 1 to 4 nucleotides longer than the sense strand, and the duplex region formed by both strands equals the length of the sense strand, such that the antisense strand forms an overhang comprising 1 to 4 unpaired nucleotides. In some embodiments, the nucleotide overhang has a length of 1, 2, 3, or 4 nucleotides. In a specific embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent for inhibiting KHK gene expression comprises nucleotide overhangs at both 5'-end and

3'-end of the antisense strand. In another embodiment, the RNAi agent for inhibiting KHK gene expression comprises nucleotide overhangs at both 5'-end and 3'-end of the sense strand. In some embodiments, the RNAi agent for inhibiting KHK gene expression comprises nucleotide overhangs at the 5'-end of the sense strand and the 5'-end of the antisense strand. In other embodiments, the RNAi agent for inhibiting KHK gene expression comprises nucleotide overhangs at the 3'-end of the sense strand and the 3'-end of the antisense strand. In some embodiments, the RNAi agent for inhibiting KHK gene expression comprises only a nucleotide overhang at the 5'-end of the sense strand. In some embodiments, the RNAi agent for inhibiting KHK gene expression comprises only a nucleotide overhang at the 3'-end of the sense strand. In some embodiments, the RNAi agent for inhibiting KHK gene expression comprises only a nucleotide overhang at the 3'-end of the antisense strand. In some embodiments, the RNAi agent for inhibiting KHK gene expression comprises only a nucleotide overhang at the 5'-end of the antisense strand. In some embodiments, the RNAi agent for inhibiting KHK gene expression comprises only a nucleotide overhang at the 5'-end of the sense strand.

The RNAi agent for inhibiting KHK gene expression may comprise a nucleotide overhang at one end and a blunt end at the opposite end of the double-stranded RNA molecule. "Blunt end" means that the sense strand and antisense strand are fully base-paired at the molecular terminus, with no unpaired nucleotides extending beyond the duplex region. In some embodiments, the RNAi agent for inhibiting KHK gene expression comprises a nucleotide overhang at the 3'-end of the sense strand, and blunt ends at the 5'-end of the sense strand and the 3'-end of the antisense strand. In other embodiments, the RNAi agent for inhibiting KHK gene expression comprises a nucleotide overhang at the 3'-end of the antisense strand, and blunt ends at the 5'-end of the antisense strand and the 3'-end of the sense strand.

Specifically, by way of example, in one embodiment, the RNAi agent for inhibiting KHK gene expression comprises: (i) a sense strand of 19 nucleotides in length, (ii) an antisense strand of 21 nucleotides in length, wherein the strands form a duplex region equal to the length of the sense strand. In another embodiment, the RNAi agent for inhibiting KHK gene expression comprises: (i) a sense strand of 21 nucleotides in length, (ii) an antisense strand of 23 nucleotides in length, wherein the strands form a duplex region equal to the length of the sense strand.

In some embodiments, an RNAi agent for inhibiting KHK gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, and wherein the RNAi agent comprises one overhang and one blunt end, the overhang preferably having 2 unpaired nucleotides.

In some embodiments, an RNAi agent for inhibiting KHK gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting KHK gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting KHK gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, wherein the sense strand is 19 to 21 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting KHK gene expression in cells is provided, comprising a sense strand and an antisense strand that form a duplex region of 19 base pairs in length, wherein the antisense strand is 21 nucleotides in length and consists of at least 15 (e.g., 15, 16, 17, 18, 19, 20, or 21) contiguous nucleotides of a nucleotide

sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, the present invention provides an RNAi agent for inhibiting KHK gene expression in cells, comprising a sense strand and an antisense strand that form a duplex region of 19 base pairs in length, wherein the antisense strand is 21 nucleotides in length and consists of any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65), wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In preferred embodiments, the present invention provides an RNAi agent for inhibiting KHK gene expression in cells, comprising a sense strand and an antisense strand that form a duplex region, wherein the antisense strand comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, and wherein the sense strand comprises at least 15 (e.g., 15, 16, 17, 18, or 19) contiguous nucleotides of any nucleotide sequence selected from SEQ ID NOs: 1 to 32 (SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, and 32) and nucleotide sequences having 1, 2, or 3 nucleotide differences therefrom.

In preferred embodiments, the present invention provides an RNAi agent for inhibiting KHK gene expression in cells, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65) and nucleotide sequences having 1, 2, or 3 nucleotide differences therefrom, and wherein the sense strand is no more than 21 nucleotides in length and comprises at least 15 (e.g., 15, 16, 17, 18, or 19) contiguous nucleotides of any nucleotide sequence selected from SEQ ID NOs: 1 to 32 (SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, and 32) and nucleotide sequences having 1, 2, or 3 nucleotide differences therefrom.

In preferred embodiments, the present invention provides an RNAi agent for inhibiting KHK gene expression in cells, comprising a sense strand and an antisense strand that form a duplex region, wherein the antisense strand is no more than 23 nucleotides in length and comprises any nucleotide sequence selected from SEQ ID NOs: 34 to 65 (SEQ ID NO: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, and 65), and wherein the sense strand is no more than 21 nucleotides in length and comprises any nucleotide sequence selected from SEQ ID NOs: 1 to 32 (SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, and 32).

In preferred embodiments, the present invention provides an RNAi agent for inhibiting KHK gene expression in a cell, comprising a sense strand and an antisense strand that form a duplex region, wherein:

the sense strand comprises or consists of a sequence set forth in SEQ ID NO:1 or a nucleotide sequence having 1, 2, or 3 nucleotide differences therefrom, and the antisense strand comprises or consists of a sequence set forth in SEQ ID NO:34 or a nucleotide sequence having 1, 2, or 3 nucleotide differences therefrom;

the sense strand comprises or consists of a sequence set forth in SEQ ID NO:2 or a nucleotide sequence having 1, 2, or 3 nucleotide differences therefrom, and the antisense strand comprises or consists of a sequence set forth in SEQ ID NO:35 or a nucleotide sequence having 1, 2, or 3 nucleotide differences therefrom;

the sense strand comprises or consists of a sequence set forth in SEQ ID NO:3 or a nucleotide sequence having 1, 2, or 3 nucleotide differences therefrom, and the antisense strand comprises or consists of a sequence set forth in SEQ ID NO:36 or a nucleotide sequence having 1, 2, or 3 nucleotide differences therefrom;

the sense strand comprises or consists of a sequence set forth in SEQ ID NO:4 or a nucleotide sequence having 1, 2, or 3 nucleotide differences therefrom, and the antisense strand comprises or consists of a sequence set forth in SEQ ID NO:37 or a nucleotide sequence having 1, 2, or 3 nucleotide differences therefrom;

the sense strand comprises or consists of a sequence set forth in SEQ ID NO:5 or a nucleotide sequence having 1, 2, or 3 nucleotide differences therefrom, and the antisense strand comprises or consists of a sequence set forth in SEQ ID NO:38 or a nucleotide sequence having 1, 2, or 3 nucleotide differences therefrom;

the sense strand comprises or consists of a sequence set forth in SEQ ID NO:6 or a nucleotide sequence having 1, 2, or 3 nucleotide differences therefrom, and the antisense strand comprises or consists of a sequence set forth in SEQ ID NO:39 or a nucleotide sequence having 1, 2, or 3 nucleotide differences therefrom;

the sense strand comprises or consists of a sequence set forth in SEQ ID NO:7 or a nucleotide sequence having 1, 2,

or 3 nucleotide differences therefrom, and the antisense strand comprises or consists of a sequence set forth in SEQ ID NO:40 or a nucleotide sequence having 1, 2, or 3 nucleotide differences therefrom;
the sense strand comprises or consists of a sequence set forth in SEQ ID NO:8 or a nucleotide sequence having 1, 2, or 3 nucleotide differences therefrom, and the antisense strand comprises or consists of a sequence set forth in SEQ ID NO:41 or a nucleotide sequence having 1, 2, or 3 nucleotide differences therefrom;
the sense strand comprises or consists of a sequence set forth in SEQ ID NO:9 or a nucleotide sequence having 1, 2, or 3 nucleotide differences therefrom, and the antisense strand comprises or consists of a sequence set forth in SEQ ID NO:42 or a nucleotide sequence having 1, 2, or 3 nucleotide differences therefrom;
the sense strand comprises or consists of a sequence set forth in SEQ ID NO:10 or a nucleotide sequence having 1, 2, or 3 nucleotide differences therefrom, and the antisense strand comprises or consists of a sequence set forth in SEQ ID NO:43 or a nucleotide sequence having 1, 2, or 3 nucleotide differences therefrom;
the sense strand comprises or consists of a sequence set forth in SEQ ID NO:11 or a nucleotide sequence having 1, 2, or 3 nucleotide differences therefrom, and the antisense strand comprises or consists of a sequence set forth in SEQ ID NO:44 or a nucleotide sequence having 1, 2, or 3 nucleotide differences therefrom;
the sense strand comprises or consists of a sequence set forth in SEQ ID NO: 12 or a nucleotide sequence having 1, 2, or 3 nucleotide differences therefrom, and the antisense strand comprises or consists of a sequence set forth in SEQ ID NO:45 or a nucleotide sequence having 1, 2, or 3 nucleotide differences therefrom;
the sense strand comprises or consists of a sequence set forth in SEQ ID NO:13 or a nucleotide sequence having 1, 2, or 3 nucleotide differences therefrom, and the antisense strand comprises or consists of a sequence set forth in SEQ ID NO:46 or a nucleotide sequence having 1, 2, or 3 nucleotide differences therefrom;
the sense strand comprises or consists of a sequence set forth in SEQ ID NO:14 or a nucleotide sequence having 1, 2, or 3 nucleotide differences therefrom, and the antisense strand comprises or consists of a sequence set forth in SEQ ID NO:47 or a nucleotide sequence having 1, 2, or 3 nucleotide differences therefrom;
the sense strand comprises or consists of a sequence set forth in SEQ ID NO:15 or a nucleotide sequence having 1, 2, or 3 nucleotide differences therefrom, and the antisense strand comprises or consists of a sequence set forth in SEQ ID NO:48 or a nucleotide sequence having 1, 2, or 3 nucleotide differences therefrom;
the sense strand comprises or consists of a sequence set forth in SEQ ID NO:16 or a nucleotide sequence having 1, 2, or 3 nucleotide differences therefrom, and the antisense strand comprises or consists of a sequence set forth in SEQ ID NO:49 or a nucleotide sequence having 1, 2, or 3 nucleotide differences therefrom;
the sense strand comprises or consists of a sequence set forth in SEQ ID NO:17 or a nucleotide sequence having 1, 2, or 3 nucleotide differences therefrom, and the antisense strand comprises or consists of a sequence set forth in SEQ ID NO:50 or a nucleotide sequence having 1, 2, or 3 nucleotide differences therefrom;
the sense strand comprises or consists of a sequence set forth in SEQ ID NO:18 or a nucleotide sequence having 1, 2, or 3 nucleotide differences therefrom, and the antisense strand comprises or consists of a sequence set forth in SEQ ID NO:51 or a nucleotide sequence having 1, 2, or 3 nucleotide differences therefrom;
the sense strand comprises or consists of a sequence set forth in SEQ ID NO:19 or a nucleotide sequence having 1, 2, or 3 nucleotide differences therefrom, and the antisense strand comprises or consists of a sequence set forth in SEQ ID NO:52 or a nucleotide sequence having 1, 2, or 3 nucleotide differences therefrom;
the sense strand comprises or consists of a sequence set forth in SEQ ID NO:20 or a nucleotide sequence having 1, 2, or 3 nucleotide differences therefrom, and the antisense strand comprises or consists of a sequence set forth in SEQ ID NO:53 or a nucleotide sequence having 1, 2, or 3 nucleotide differences therefrom;
the sense strand comprises or consists of a sequence set forth in SEQ ID NO:21 or a nucleotide sequence having 1, 2, or 3 nucleotide differences therefrom, and the antisense strand comprises or consists of a sequence set forth in SEQ ID NO:54 or a nucleotide sequence having 1, 2, or 3 nucleotide differences therefrom;
the sense strand comprises or consists of a sequence set forth in SEQ ID NO:22 or a nucleotide sequence having 1, 2, or 3 nucleotide differences therefrom, and the antisense strand comprises or consists of a sequence set forth in SEQ ID NO:55 or a nucleotide sequence having 1, 2, or 3 nucleotide differences therefrom;
the sense strand comprises or consists of a sequence set forth in SEQ ID NO:23 or a nucleotide sequence having 1, 2, or 3 nucleotide differences therefrom, and the antisense strand comprises or consists of a sequence set forth in SEQ ID NO:56 or a nucleotide sequence having 1, 2, or 3 nucleotide differences therefrom;
the sense strand comprises or consists of a sequence set forth in SEQ ID NO:24 or a nucleotide sequence having 1, 2, or 3 nucleotide differences therefrom, and the antisense strand comprises or consists of a sequence set forth in SEQ ID NO:57 or a nucleotide sequence having 1, 2, or 3 nucleotide differences therefrom;
the sense strand comprises or consists of a sequence set forth in SEQ ID NO:25 or a nucleotide sequence having 1, 2, or 3 nucleotide differences therefrom, and the antisense strand comprises or consists of a sequence set forth in SEQ ID NO:58 or a nucleotide sequence having 1, 2, or 3 nucleotide differences therefrom;
the sense strand comprises or consists of a sequence set forth in SEQ ID NO:26 or a nucleotide sequence having 1, 2, or 3 nucleotide differences therefrom, and the antisense strand comprises or consists of a sequence set forth in SEQ

ID NO:59 or a nucleotide sequence having 1, 2, or 3 nucleotide differences therefrom;

the sense strand comprises or consists of a sequence set forth in SEQ ID NO:27 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;

the sense strand comprises or consists of a sequence set forth in SEQ ID NO:28 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;

the sense strand comprises or consists of a sequence set forth in SEQ ID NO:29 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;

the sense strand comprises or consists of a sequence set forth in SEQ ID NO:30 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;

the sense strand comprises or consists of a sequence set forth in SEQ ID NO:31 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom; or

the sense strand comprises or consists of a sequence set forth in SEQ ID NO:32 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom.

In preferred embodiments, the present invention provides an RNAi agent for inhibiting KHK gene expression, comprising a sense strand and an antisense strand that form a complementary duplex region, wherein the sense strand and antisense strand pair to form any one of the duplexes 4023, 4089, 4098, 4101, 4102, 4103, 4118, 4119, 4121, 4122, 4123, 4124, 4125, 4126, 4127, 4128, 4129, 4130, 4132, 4135, 4138, 4141, 4142, 4145, 4147, 4148, 4149, 4151, 4159, 4161, 4162 and 4163 as described herein.

**Nucleotide-Modified RNAi Agents**

For any of the embodiments of the RNAi agents of the present invention described in the section "RNAi Agents For Inhibiting KHK Gene Expression", the sense strand and/or antisense strand of the RNAi agent may comprise at least one modified nucleotide.

In some embodiments, the sense strand and the antisense strand of the RNAi agent for inhibiting KHK gene expression provided by the present invention each comprise at least one modified nucleotide.

In some embodiments, each of the nucleotides in the sense strand and the antisense strand of the RNAi agent for inhibiting KHK gene expression provided by the present invention is modified.

In any one of the above embodiments, the modified nucleotide is independently selected from the group consisting of 2'-deoxythymidine (dT) nucleotide, 2'-O-methyl-modified nucleotide, 2'-fluoro-modified nucleotide, 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide, 2'-amino-modified nucleotide, 2'-O-allyl-modified nucleotide, 2'-C-alkyl-modified nucleotide, 2'-methoxyethyl-modified nucleotide, abasic nucleotide, inverted abasic nucleotide, inverted nucleotide, morpholino nucleotide, phosphoramidate, tetrahydropyran-modified nucleotide, 1,5-anhydrohexitol-modified nucleotide, cyclohexenyl-modified nucleotide, nucleotide comprising phosphorothioate group, nucleotide comprising methylphosphonate group, nucleotide comprising 5'-phosphate, nucleotide comprising 5'-phosphate mimic and combinations thereof.

In preferred embodiments, each of the nucleotides in the sense strand and the antisense strand of the RNAi agent for inhibiting KHK gene expression provided by the present invention is modified, and the modification is selected from the group consisting of STC(Alnylam), ESC(Alnylam), Advanced ESC(Alnylam), ESC+(Alnylam), AD1-3(Arrowhead), AD5(Arrowhead) and GalXC(Dicerna) (see, e.g., Hu B, Zhong L, Weng Y, et al. Therapeutic siRNA: state of the art. *Signal Transduct Target Ther.* 2020; 5(1): 101. Published 2020 Jun 19. doi:10.1038/s41392-020-0207-x).

In some embodiments, in the RNAi agent for inhibiting KHK gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent for inhibiting KHK gene expression provided by the present invention, each of the nucleotides of the antisense strand is modified, and in the 5' to 3' direction, the nucleotides at positions 2, 5, 7, 12, and 14 of the antisense strand are 2'-fluoro-modified nucleotides, while the nucleotides at remaining positions of the antisense strand are all 2'-methoxy-modified nucleotides.

In some embodiments, in the RNAi agent for inhibiting KHK gene expression provided by the present invention, each of the nucleotides of the antisense strand is modified, and in the 5' to 3' direction, the nucleotides at positions 2, 5, 7, 14, and 16 of the antisense strand are 2'-fluoro-modified nucleotides, while the nucleotides at remaining positions of the antisense strand are all 2'-methoxy-modified nucleotides.

In some embodiments, in the RNAi agent for inhibiting KHK gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage. In preferable embodiments, the phosphorothioate internucleotide linkage is present at one or more of the following locations: (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand. In preferable embodiments, the phosphorothioate internucleotide linkage is present (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand.

In some embodiments, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides.

For example, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides.

For example, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In preferable embodiments, the sense strand comprises at least one phosphorothioate internucleotide linkage. In preferable embodiments, said phosphorothioate internucleotide linkage is present between nucleotide positions 1 and 2 at the 5'-end of the sense strand; and/or between nucleotide positions 2 and 3 at the 5'-end of the sense strand. In preferable embodiments, said phosphorothioate internucleotide linkage is present between nucleotide positions 1 and 2 at the 5'-end of the sense strand; and between nucleotide positions 2 and 3 at the 5'-end of the sense strand.

**In** preferable embodiments, the antisense strand comprises at least one phosphorothioate internucleotide linkage. In preferable embodiments, the phosphorothioate internucleotide linkage is present at one or more of the following locations: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand.

Accordingly, in some embodiments, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present at one or more of the following locations: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and/or between nucleotide positions 2 and 3 at the 5'-end of the sense strand.

Accordingly, in some embodiments, in the RNAi agent for inhibiting KHK gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and between nucleotide positions 2 and 3 at the 5'-end of the sense strand.

In preferred embodiments, the antisense strand of the RNAi agent comprises or consists of a sequence set forth in any one of SEQ ID NOs. 104 to 130 (SEQ ID NO: 104, 105, 106, 107, 108, 109, 110, 111, 112, 113, 114, 115, 116, 117, 118, 119, 120, 121, 122, 123, 124, 125, 126, 127, 128, 129, and 130) or a nucleotide sequence having 1, 2 or 3 nucleotide differences from each thereof.

In preferred embodiments, the sense strand of the RNAi agent comprises or consists of a sequence set forth in any one of SEQ ID NOs. 132 to 153 (SEQ ID NO:132, 133, 134, 135, 136, 137, 138, 139, 140, 141, 142, 143, 144, 145, 146, 147, 148, 149, 150, 151, 152, and 153) or a nucleotide sequence having 1, 2 or 3 nucleotide differences from each thereof.

In preferred embodiments, the antisense strand of the RNAi agent comprises or consists of a sequence set forth in any one of SEQ ID NOs. 104 to 130 or a nucleotide sequence having 1, 2 or 3 nucleotide differences from each thereof, and the sense strand of the RNAi agent comprises or consists of a sequence set forth in any one of SEQ ID NOs. 132 to 153 or a nucleotide sequence having 1, 2 or 3 nucleotide differences from each thereof.

In some embodiments, the present invention provides an RNAi agent for inhibiting KHK gene expression, wherein:

(b1) the sense strand comprises or consists of a nucleotide sequence set forth in SEQ ID NO: 132 or a nucleotide sequence having 1, 2 or 3 nucleotide differences therefrom, and the antisense strand comprises or consists of a nucleotide sequence set forth in SEQ ID NO: 104 or a nucleotide sequence having 1, 2 or 3 nucleotide differences therefrom;

(b2) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(b3) the sense strand comprises or consists of a nucleotide sequence set forth in SEQ ID NO: 134 or a nucleotide sequence having 1, 2 or 3 nucleotide differences therefrom, and the antisense strand comprises or consists of a

nucleotide sequence set forth in SEQ ID NO: 106 or a nucleotide sequence having 1, 2 or 3 nucleotide differences therefrom;

(b4) the sense strand comprises or consists of a nucleotide sequence set forth in SEQ ID NO: 135 or a nucleotide sequence having 1, 2 or 3 nucleotide differences therefrom, and the antisense strand comprises or consists of a nucleotide sequence set forth in SEQ ID NO: 107 or a nucleotide sequence having 1, 2 or 3 nucleotide differences therefrom;

(b5) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 108 or a nucleotide sequence having 1, 2 or 3 nucleotide differences therefrom;

(b6) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 109 or a nucleotide sequence having 1, 2 or 3 nucleotide differences therefrom;

(b7) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 110 or a nucleotide sequence having 1, 2 or 3 nucleotide differences therefrom;

(b8) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 111 or a nucleotide sequence having 1, 2 or 3 nucleotide differences therefrom;

(b9) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b10) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b11) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 114 or a nucleotide sequence having 1, 2 or 3 nucleotide differences therefrom;

(b12) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 115 or a nucleotide sequence having 1, 2 or 3 nucleotide differences therefrom;

(b13) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 116 or a nucleotide sequence having 1, 2 or 3 nucleotide differences therefrom;

(b14) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b15) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b16) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b17) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b 18) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b19) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b20) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b21) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b22) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b23) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b24) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b25) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b26) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b27) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b28) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b29) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b30) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b31) the sense strand comprises or consists of a nucleotide sequence set forth in SEQ ID NO: 142 or a nucleotide sequence having 1, 2 or 3 nucleotide differences therefrom, and the antisense strand comprises or consists of a nucleotide sequence set forth in SEQ ID NO: 118 or a nucleotide sequence having 1, 2 or 3 nucleotide differences therefrom;

(b32) the sense strand comprises or consists of a nucleotide sequence set forth in SEQ ID NO: 134 or a nucleotide sequence having 1, 2 or 3 nucleotide differences therefrom, and the antisense strand comprises or consists of a

nucleotide sequence set forth in SEQ ID NO: 119 or a nucleotide sequence having 1, 2 or 3 nucleotide differences therefrom;

(b33) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b34) the sense strand comprises or consists of a nucleotide sequence set forth in SEQ ID NO: 143 or a nucleotide sequence having 1, 2 or 3 nucleotide differences therefrom, and the antisense strand comprises or consists of a nucleotide sequence set forth in SEQ ID NO: 121 or a nucleotide sequence having 1, 2 or 3 nucleotide differences therefrom;

(b35) the sense strand comprises or consists of a nucleotide sequence set forth in SEQ ID NO: 144 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b36) the sense strand comprises or consists of a nucleotide sequence set forth in SEQ ID NO: 144 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b37) the sense strand comprises or consists of a nucleotide sequence set forth in SEQ ID NO: 145 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b38) the sense strand comprises or consists of a nucleotide sequence set forth in SEQ ID NO: 145 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b39) the sense strand comprises or consists of a nucleotide sequence set forth in SEQ ID NO: 132 or a nucleotide sequence having 1, 2 or 3 nucleotide differences therefrom, and the antisense strand comprises or consists of a nucleotide sequence set forth in SEQ ID NO: 122 or a nucleotide sequence having 1, 2 or 3 nucleotide differences therefrom;

(b40) the sense strand comprises or consists of a nucleotide sequence set forth in SEQ ID NO: 146 or a nucleotide sequence having 1, 2 or 3 nucleotide differences therefrom, and the antisense strand comprises or consists of a nucleotide sequence set forth in SEQ ID NO: 123 or a nucleotide sequence having 1, 2 or 3 nucleotide differences therefrom;

(b41) the sense strand comprises or consists of a nucleotide sequence set forth in SEQ ID NO: 147 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;

(b42) the sense strand comprises or consists of a nucleotide sequence set forth in SEQ ID NO: 148 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;

(b43) the sense strand comprises or consists of a nucleotide sequence set forth in SEQ ID NO: 149 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;

(b44) the sense strand comprises or consists of a nucleotide sequence set forth in SEQ ID NO: 150 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;

(b45) the sense strand comprises or consists of a nucleotide sequence set forth in SEQ ID NO: 151 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;

(b46) the sense strand comprises or consists of a nucleotide sequence set forth in SEQ ID NO: 152 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom; or

(b47) the sense strand comprises or consists of a nucleotide sequence set forth in SEQ ID NO: 153 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom.

In preferred embodiments, the RNAi agent provided by the present invention comprises duplex 4023-60, 4122-60, 4132-60, 4149-60, 4122-50, 4122-4, 4122-64, 4122-67, 4122-10, 4122-11, 4122-70, 4122.1-60, 4122-44, 4122-18, 4122-19, 4122-74, 4122-75, 4122-101, 4122-34, 4122-35, 4122-98, 4122-26, 4122-27, 4122-97, 4122-85, 4122-86, 4122-99, 4122-80, 4122-81, 4122-100, 4122.3-60, 4132-10, 4132-11, 4132-44, 4132-26, 4132-27, 4132-34, 4132-35, 4023-65, 4023-44, 4089-60, 4098-60, 4118-60, 4123-60, 4129-60, 4135-60 or 4145-60.

**Ligand-Conjugated RNAi Agents**

For any of the embodiments of the RNAi agent of the present invention described in the preceding sections entitled "RNAi Agents For Inhibiting KHK Gene Expression" and "Nucleotide-modified RNAi agents", the RNAi agent may comprise a ligand. As used herein, a "ligand" refers to any compound or molecule capable of interacting, directly or indirectly, with another compound or molecule. The interaction of a ligand with another compound or molecule may elicit a biological response (e.g., initiation of a signal transduction cascade, induction of receptor-mediated endocytosis) or may simply be a physical attachment. A ligand may alter one or more properties of the attached double-stranded RNA molecule, such as the pharmacodynamics, pharmacokinetics, binding, absorption, cellular distribution, cellular uptake, charge, and/or clearance of the RNA molecule.

The KHK gene is primarily expressed in the liver. Therefore, in certain embodiments, it is desirable to specifically deliver the RNAi agents of the present invention to hepatocytes. Accordingly, in certain embodiments, the ligand targets the specific delivery of the RNAi agent to hepatocytes using various methods described in more detail below. In certain embodiments, the RNAi agent is targeted to hepatocytes with a ligand that binds the surface-expressed asialoglycoprotein receptor (ASGR) or a component thereof (e.g., ASGR1, ASGR2).

In some embodiments, the RNAi agent can be specifically targeted to the liver by using a ligand that binds or interacts with a protein expressed on the surface of hepatocytes. For example, in certain embodiments, the ligand can comprise an antigen-binding protein (e.g., an antibody or binding fragment thereof such as Fab, scFv) that specifically binds a receptor expressed on hepatocytes, such as the asialoglycoprotein receptor and the LDL receptor. In one particular embodiment, the ligand comprises an antibody or binding fragment thereof that specifically binds ASGR1 and/or ASGR2. In another embodiment, the ligand comprises a Fab fragment of an antibody that specifically binds ASGR1 and/or ASGR2. In another embodiment, the ligand comprises a single-chain variable antibody fragment (scFv fragment) of an antibody that specifically binds ASGR1 and/or ASGR2. Exemplary antibodies that specifically bind ASGR1 and binding fragments thereof, which can serve as ligands for targeting the RNAi agents of the present invention to the liver, are described in WO 2017/058944, which is incorporated herein by reference in its entirety. Other antibodies or binding fragments thereof that specifically bind ASGR1, the LDL receptor, or other liver surface-expressed proteins suitable for use as ligands in the RNAi agents of the present invention are available from commercial sources.

In some embodiments, when used for extrahepatic delivery, including but not limited to, the central nervous system (CNS) (e.g., brain, spinal cord, or eye), muscle, lung, or adipose tissue, the RNAi agent may comprise one or more lipophilic monomers conjugated to one or more positions of the antisense strand and/or sense strand. For example, the lipophilic monomer may be linked to the antisense strand and/or sense strand via a nucleobase, sugar moiety, or internucleoside linkage. In some embodiments, a lipophilic monomer having a phosphoramidite group is coupled to the 3' end or the 5' end of the sense strand or antisense strand in the final synthesis cycle. In some embodiments, the lipophilic monomer has an octanol-water partition coefficient exceeding 0, 1, 1.5, 2, 3, 4, 5, or 10. Suitable lipophilic monomers may be aliphatic, alicyclic, polyalicyclic, steroidal, straight-chain or branched aliphatic. Exemplary lipophilic monomers are, for example, the lipophilic monomers Y132 to Y135, Y158, Y165 to Y168, L10, L57, L321, L322, Q361 to Q367, Q361s to Q367s, Q370, Q377 to Q379, Q383, etc., described in WO 2021/092371.

In certain embodiments, the ligand comprises a carbohydrate. A "carbohydrate" refers to a compound composed of one or more monosaccharide units having at least 6 carbon atoms (which can be linear, branched, or cyclic) with oxygen, nitrogen, or sulfur atoms attached to each carbon atom. Carbohydrates include, but are not limited to, sugars (e.g., monosaccharides, disaccharides, trisaccharides, tetrasaccharides, and oligosaccharides containing about 4, 5, 6, 7, 8, or 9 monosaccharide units) and polysaccharides (such as starch, glycogen, cellulose, and polysaccharide gums). In some embodiments, the carbohydrate incorporated into the ligand is selected from pentoses, hexoses, or heptoses, and disaccharides and trisaccharides including such monosaccharide units. In other embodiments, the carbohydrate incorporated into the ligand is an amino sugar, such as galactosamine, glucosamine, N-acetylgalactosamine, and N-acetylglucosamine.

In some embodiments, the ligand comprises a hexose or a hexosamine. The hexose can be selected from

glucose, galactose, mannose, fucose, or fructose. The hexosamine can be selected from fructosamine, galactosamine, glucosamine, or mannosamine. In certain embodiments, the ligand comprises glucose, galactose, galactosamine, or glucosamine. In one embodiment, the ligand comprises glucose, glucosamine, or N-acetylglucosamine. In another embodiment, the ligand comprises galactose, galactosamine, or N-acetylgalactosamine. In particular embodiments, ligands comprising glucose, galactose, and N-acetylgalactosamine (GalNAc) are particularly effective in targeting RNA to hepatocytes, as these ligands bind the ASGR expressed on the surface of hepatocytes. Examples of GalNAc- or galactose-containing ligands that can be incorporated into the RNAi agents of the present invention are described in U.S. Pat. Nos. 7,491,805, 8,106,022, and 8,877,917; U.S. Patent Application Publication No. US 20030130186; and WIPO Publication No. WO 2013/166155, all of which are incorporated herein by reference in their entirety.

In certain embodiments, the ligand comprises a multivalent carbohydrate moiety. As used herein, a "multivalent carbohydrate moiety" refers to a moiety comprising two or more carbohydrate units capable of independently binding or interacting with other molecules. For example, a multivalent carbohydrate moiety includes two or more binding domains composed of carbohydrates that can bind to two or more different molecules or two or more different sites on the same molecule. The "valency" of a carbohydrate moiety indicates the number of individual binding domains within the carbohydrate moiety. For example, the terms "monovalent," "bivalent," "trivalent," and "tetravalent" relative to a carbohydrate moiety refer to carbohydrate moieties having one, two, three, and four binding domains, respectively. A multivalent carbohydrate moiety can comprise a multivalent lactose moiety, a multivalent galactose moiety, a multivalent glucose moiety, a multivalent N-acetylgalactosamine moiety, a multivalent N-acetylglucosamine moiety, a multivalent mannose moiety, or a multivalent fucose moiety. In some embodiments, the ligand comprises a multivalent galactose moiety. In other embodiments, the ligand comprises a multivalent N-acetylgalactosamine moiety. In these and other embodiments, the multivalent carbohydrate moiety can be bivalent, trivalent, or tetravalent. In such embodiments, the multivalent carbohydrate moiety can be bi-branched or tri-branched. In one particular embodiment, the multivalent N-acetylgalactosamine moiety is trivalent or tetravalent. In another particular embodiment, the multivalent galactose moiety is trivalent or tetravalent. Exemplary trivalent and tetravalent GalNAc-containing ligands for incorporation into the RNAi agents of the present invention are described in detail below.

The ligand can be attached or conjugated to the RNA molecule of the RNAi agent directly or indirectly. For example, in some embodiments, the ligand is directly covalently attached to the sense strand or the antisense strand of the RNAi agent. In other embodiments, the ligand is covalently attached to the sense strand or the antisense strand of the RNAi agent via a linker. The ligand can be attached to a nucleobase, sugar moiety, or internucleotide linkage of the sense strand or antisense strand of the RNAi agent of the present invention.

In some embodiments, the ligand can be attached to 3' or 5'-end of the sense strand or antisense strand. In certain embodiments, the ligand is covalently attached to 5'-end of the sense strand. In such embodiments, the ligand is attached to 5'-terminal nucleotide of the sense strand. In these and other embodiments, the ligand is attached at 5'-position of 5'-terminal nucleotide of the sense strand. In other embodiments, the ligand is covalently attached to 3'-end of the sense strand. For example, in some embodiments, the ligand is attached to 3'-terminal nucleotide of the sense strand. In certain such embodiments, the ligand is attached at 3'-position of 3'-terminal nucleotide of the sense strand. In alternative embodiments, the ligand is attached near 3'-end but prior to one or more terminal nucleotides (i.e., one, two, three, or four nucleotides from the terminus). In some embodiments, the ligand is attached at the 2'-position of the sugar of 3'-terminal nucleotide of the sense strand. In other embodiments, the ligand is attached at the 2'-position of the sugar of 5'-terminal nucleotide of the sense strand.

In certain embodiments, the ligand is attached to the sense strand or antisense strand via a linker. A "linker" refers to an atom or group of atoms that covalently attaches the ligand to the polynucleotide component of the RNAi agent. The linker can have a length of about 1 to about 30 atoms, about 2 to about 28 atoms, about 3 to about 26 atoms, about 4 to about 24 atoms, about 6 to about 20 atoms, about 7 to about 20 atoms, about 8 to about 20 atoms, about 8 to about 18 atoms, and about 12 to about 18 atoms. In some embodiments, the linker can comprise a bifunctional linking moiety, typically comprising an alkyl group with two functional groups. One functional group is chosen to bind to the compound of interest (e.g., the sense strand or antisense strand of the RNAi agent), while the other functional group is chosen to bind to essentially any selected group, such as the ligands described herein. In certain embodiments, the linker comprises an oligomer of a chain structure or repeating units, such as ethylene glycol or amino acid units. Examples of functional groups commonly used in bifunctional linking moieties include, but are not limited to, electrophiles for reaction with nucleophilic groups and nucleophiles for reaction with electrophilic groups. In some embodiments, the bifunctional linking moiety includes an amino group, a hydroxyl group, a carboxylic acid, a thiol, an unsaturated bond (e.g., a double or triple bond), and the like.

Linkers suitable for attaching a ligand to the sense strand or antisense strand of the RNAi agent of the present invention include, but are not limited to, pyrrolidine, 8-amino-3,6-dioxaoctanoic acid, succinimidyl-4-(N-maleimidomethyl) cyclohexane-1-carboxylate, 6-aminohexanoic acid, substituted $C_1$-$C_{20}$ alkyl, substituted or unsubstituted $C_2$-$C_{20}$ alkenyl, or substituted or unsubstituted $C_2$-$C_{20}$ alkynyl. Preferred substituents for such linkers include, but are not limited to, hydroxyl, amino, alkoxy, carboxyl, benzyl, phenyl, nitro, thiol, thioalkoxy, halogen, alkyl, aryl, alkenyl, and alkynyl.

In certain embodiments, the linker is cleavable. A cleavable linker is one that is sufficiently stable outside the cell but is cleaved to release the two parts linked together by the linker upon entry into a target cell. In some embodiments, the cleavable linker is cleaved at least 10, 20, 30, 40, 50, 60, 70, 80, 90 times or more, or at least 100 times faster in the target cell or under a first reference condition (which can, for example, be selected to mimic or represent intracellular conditions) than in the blood of a subject.

Cleavable linkers are susceptible to cleavage agents, such as pH, redox potential, or the presence of degradative molecules. Generally, cleavage agents are more prevalent or found at higher levels or activity inside cells than in serum or blood. Examples of such cleavage agents include: redox agents, selected for specific substrates or lacking substrate specificity, including, for example, oxidases or reductases present in cells; esterases; endosomes or agents that can create an acidic environment, such as those resulting in a pH of 5 or lower; enzymes that can hydrolyze or degrade acid-cleavable linkers acting as general acids, peptidases (which can be substrate-specific), and phosphatases.

A cleavable linker can comprise a pH-sensitive moiety. Human serum has a pH of 7.4, while the average intracellular pH is slightly lower, ranging from about 7.1-7.3. Endosomes have a more acidic pH, in the range of 5.5-6.0, and lysosomes have an even more acidic pH (around 5.0). Some linkers will have a cleavable group that is cleaved at a preferred pH, thereby releasing the RNA molecule from the ligand inside the cell, or into a desired cellular organelle. The linker can include a cleavable group that is cleaved by a specific enzyme. The type of cleavable group incorporated into the linker can depend on the cell to be targeted. For example, a liver-targeting ligand can be linked to the RNA molecule via a linker comprising of an ester group. Hepatocytes are rich in esterases, so the linker is cleaved more efficiently in hepatocytes than in cell types not rich in esterases. Other cell types rich in esterases include cells of the lung, renal cortex, and testis. When targeting cells rich in peptidases, such as hepatocytes and synoviocytes, linkers containing peptide bonds can be used.

Other types of linkers suitable for attaching ligands to the sense strand or antisense strand in the RNAi agents of the present invention are known in the art, such as those described in U.S. Pat. Nos. 7,723,509, 8,017,762, 8,828,956, 8,877,917, and 9,181,551, all of which are incorporated herein by reference in their entirety.

In certain embodiments, the ligand covalently attached to the sense strand or antisense strand of the RNAi agent of the present invention comprises a GalNAc moiety, such as a multivalent GalNAc. In some embodiments, the multivalent GalNAc moiety is a trivalent GalNAc and is attached to 3'-end of the sense strand. In other embodiments, the multivalent GalNAc moiety is a trivalent GalNAc and is attached to 5'-end of the sense strand. In other embodiments, the multivalent GalNAc moiety is a tetravalent GalNAc moiety and is attached to 3'-end of the sense strand. In other embodiments, the multivalent GalNAc moiety is a tetravalent GalNAc moiety and is attached to 5'-end of the sense strand.

In some embodiments, the ligand is L96, NAG25, and NAG37, having the formulas shown below, respectively, wherein the wave line represents a site to which the sense strand or antisense strand of the RNAi agent is attached.

L96 =

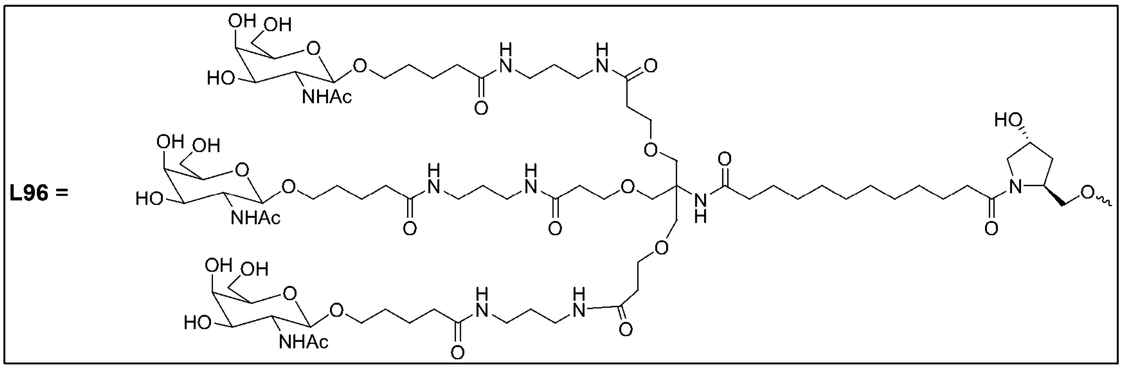

NAG25 =

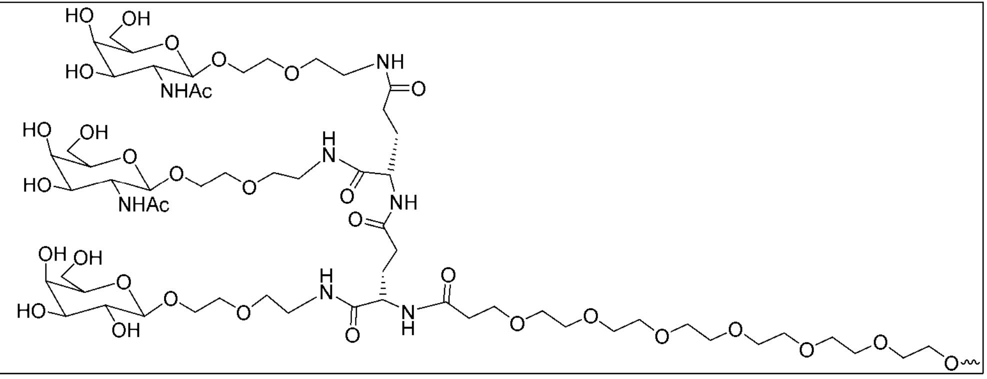

NAG37 =

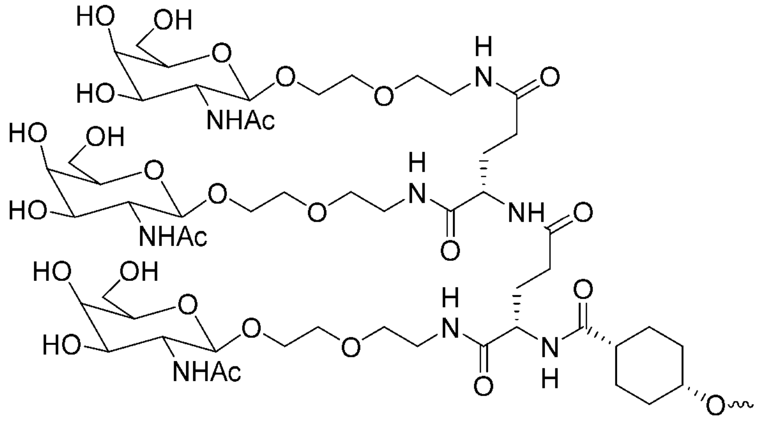

In some embodiments, the ligand comprised in the RNAi agent of the present invention comprises a structure of formula (I):

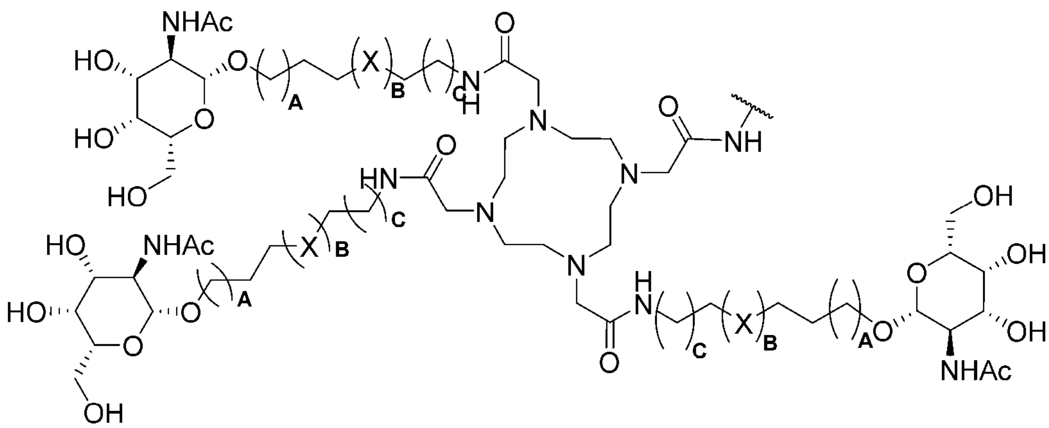

(I)

wherein,

X is -C(O)-NH-, -NH-C(O)-, $-OCH_2-CH_2O-$, -S-,

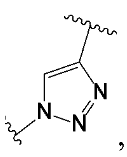 , or 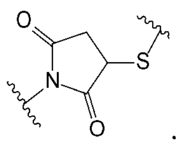 ;

A and C are independently 0 or an integer between 1 and 14, e.g., 0, 1, 2, 3, 4, 5, 6, 7, 8, 9, 10, 11, 12, 13 or 14;

B is 0 or an integer between 1 and 12, e.g., 0, 1, 2, 3, 4, 5, 6, 7, 8, 9, 10, 11 or 12;
the wave line represents a site to which other part of the RNAi agent is attached, wherein the ligand is connected to 5' and/or 3'-end of the sense strand and/or antisense strand; preferably, the ligand is connected to 5' and/or 3'-end of the sense strand; more preferably, the ligand is connected to 3'-end of the sense strand.

In a preferred embodiment, the ligand is covalently attached to the 5' and/or 3' end of the sense strand via a linker. In one embodiment, the linker is a PEG linker.

In preferred embodiments, the ligand has a structure of formula (II) or formula (III):

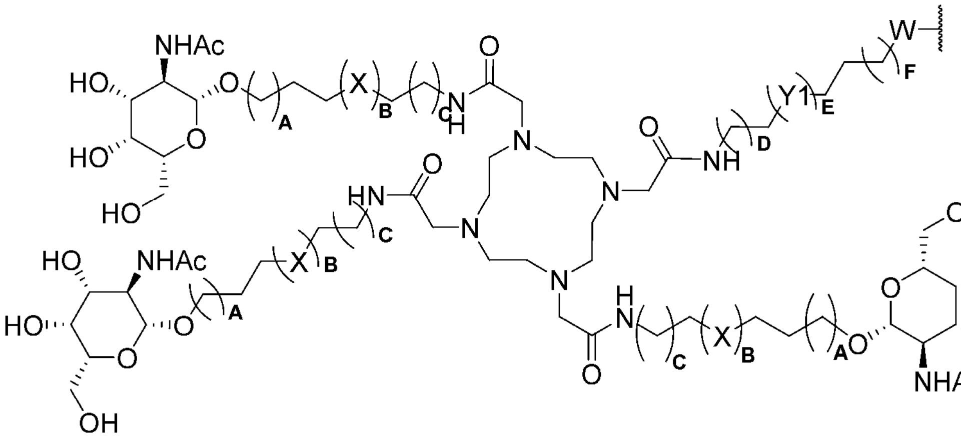

(II)

wherein,

X is -C(O)-NH-, -NH-C(O)-, $-OCH_2-CH_2O-$, -S-,

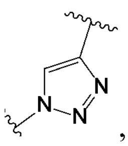
, or
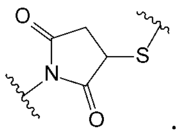
;

Y1 is $-C(O)-NR_1-$, -NH-C(O)-, $-OCH_2-CH_2O-$, -S-, -S-S-,

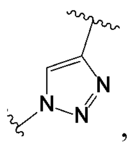
, or
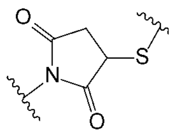
,

wherein $R_1$ is an alkyl group having 1-6 (e.g., 1, 2, 3, 4, 5 or 6) carbons or hydrogen;
W is -NH-, -O- or

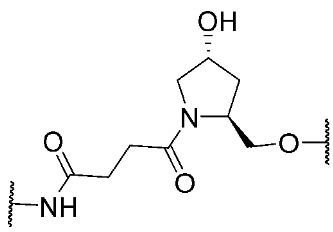
,

wherein the wave line on the right represents a site to which the sense strand or antisense strand is attached;
the wave line connected to W in formula (II) represents a site to which the sense strand or antisense strand is attached;
A, C and F are independently 0 or an integer between 1 and 14, e.g., 0, 1, 2, 3, 4, 5, 6, 7, 8, 9, 10, 11, 12, 13 or 14;
B and E are independently 0 or an integer between 1 and 12, e.g., 0, 1, 2, 3, 4, 5, 6, 7, 8, 9, 10, 11 or 12;

D is an integer between 1 and 20, e.g., 1, 2, 3, 4, 5, 6, 7, 8, 9, 10, 11, 12, 13, 14, 15, 16, 17, 18, 19 or 20;

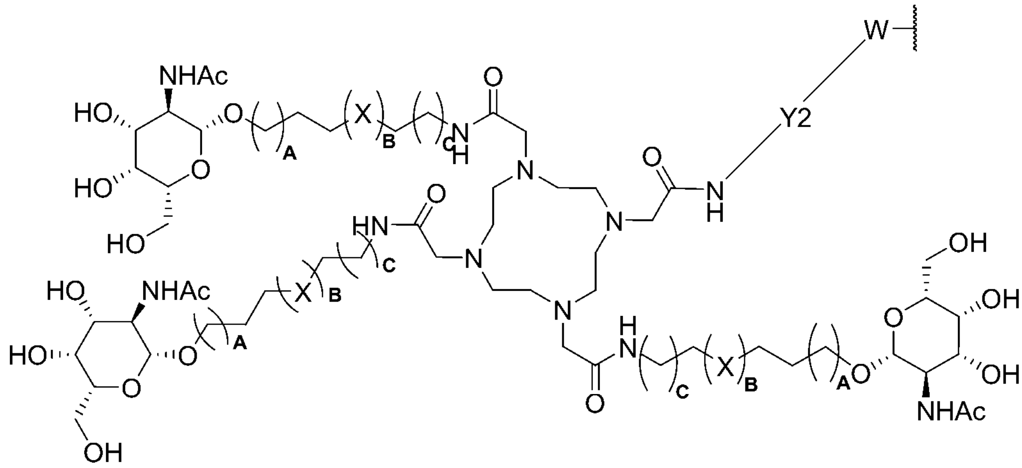

(III)

wherein,

X is -C(O)-NH-, -NH-C(O)-, $-OCH_2-CH_2O-$, -S-,

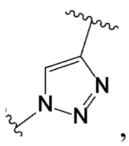, or 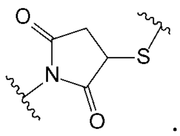;

Y2 is a linear short chain or polypeptide structure, selected from the group consisting of:

(1) $-(CH_2)p-(O-CH_2-CH_2)q-(CH_2)j-Z_3-$, wherein $Z_3$ is O, NH or C(O), p is an integer from 1 to 3 (e.g., 1, 2 or 3), q is an integer from 3 to 10 (e.g., 3, 4, 5, 6, 7, 8, 9 or 10); j is 0 or 1;
(2) $-CH_2-C(O)-(Sar)ni-NH-(CH_2)r-NH-$, wherein $n_1$ is an integer between 2 and 8 (e.g., 2, 3, 4, 5, 6, 7 or 8); r is an integer from 2 to 10 (e.g., 2, 3, 4, 5, 6, 7, 8, 9 or 10);
(3) $-CH_2-C(O)-(Gly)n_2-(Z_1)m_1-(Gly)o_1-NH-(CH_2)r-NH-$, wherein $Z_1$ is Ser, Thr, His, Arg, Arg(Me), Trp, Lys, Lys(Me), $Lys(Me)_2$, Orn, Om(Me), $Orn(Me)_2$, Dap, Dap(Me), $Dap(Me)_2$, Dab, Dab(Me), $Dab(Me)_2$ or Val-Cit, $n_2$ is an integer between 1 and 5 (e.g., 1, 2, 3, 4 or 5); $m_1$ is an integer between 1 and 6 (e.g., 1, 2, 3, 4, 5 or 6); or is an integer between 1 and 5 (e.g., 1, 2, 3, 4 or 5); r is an integer from 2 to 10 (e.g., 2, 3, 4, 5, 6, 7, 8, 9 or 10);
(4) $-CH_2-C(O)-(Sar)n_3-NH-(CH_2)r-O-$, wherein $n_3$ is an integer between 1 and 5 (e.g., 1, 2, 3, 4 or 5); r is an integer from 2 to 10 (e.g., 2, 3, 4, 5, 6, 7, 8, 9 or 10);
(5) $-CH_2-CO-(Gly)n_4-(Z_2)m_2-(Gly)o_2-NH-(CH_2)r-O-$, wherein $Z_2$ is Ser, Thr, His, Arg, Arg(Me), Trp, Lys, Lys(Me), $Lys(Me)_2$, Orn, Om(Me), $Orn(Me)_2$, Dap, Dap(Me), $Dap(Me)_2$, Dab, Dab(Me), $Dab(Me)_2$ or Val-Cit, $n_4$ is an integer between 1 and 5 (e.g., 1, 2, 3, 4 or 5); $m_2$ is an integer between 1 and 6 (e.g., 1, 2, 3, 4, 5 or 6); $o_2$ is an integer between 1 and 5 (e.g., 1, 2, 3, 4 or 5); r is an integer from 2 to 10 (e.g., 2, 3, 4, 5, 6, 7, 8, 9 or 10);
(6)

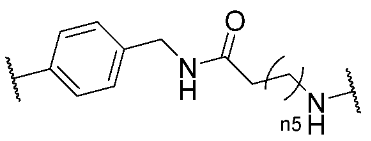, 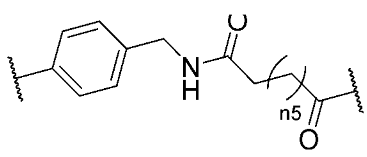,

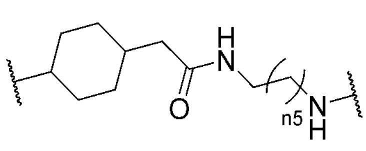, or 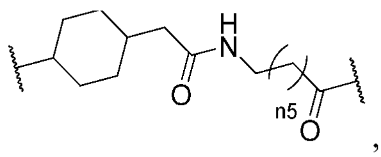,

wherein $n_5$ is an integer between 1 and 19 (e.g., 1, 2, 3, 4, 5, 6, 7, 8, 9, 10, 11, 12, 13, 14, 15, 16, 17, 18 or 19);
W is absent, or is

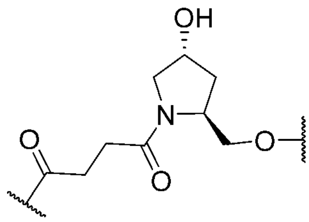 or 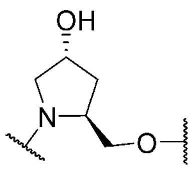,

wherein the wave line on the right represents a site to which the sense strand or antisense strand is attached; the wave line connected to W in formula (III) represents a site to which the sense strand or antisense strand is attached;
A and C are independently 0 or an integer between 1 and 14 (e.g., 0, 1, 2, 3, 4, 5, 6, 7, 8, 9, 10, 11, 12, 13 or 14);
B is 0 or an integer between 1 and 12 (e.g., 0, 1, 2, 3, 4, 5, 6, 7, 8, 9, 10, 11 or 12).

In some other embodiments, the ligand has a structure of formula (II') or formula (III'):

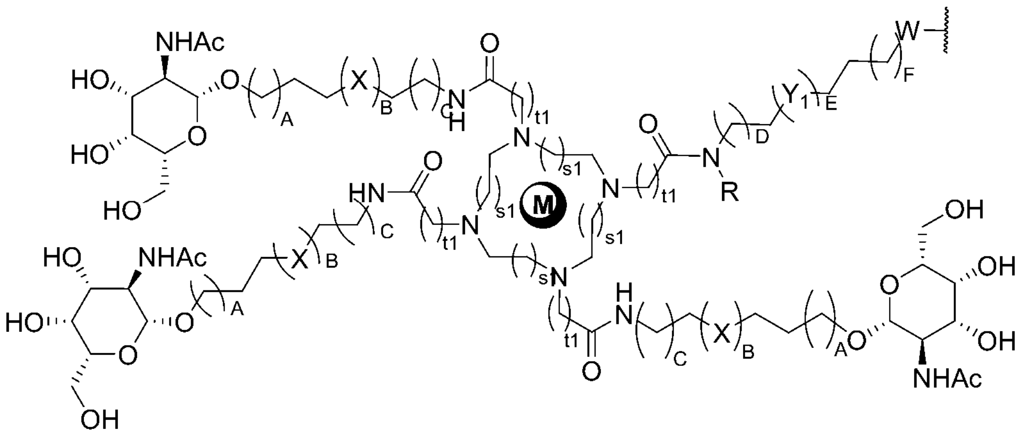

(II')

wherein,

X is -C(O)-NH-, -NH-C(O)-, $-OCH_2-CH_2O-$, -S-,

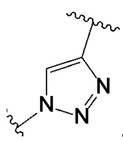, or 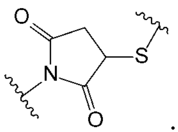;

each $s_1$ and each $t_1$ are independently an integer between 1 and 5, preferably independently 1 or 2;
$Y_1$ is $-C(O)-NR_1-$, $-NR_1-C(O)-$, $-OCH_2-CH_2O-$, -S-, -S-S-,

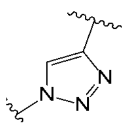,

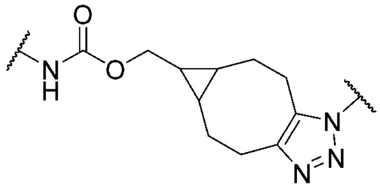, 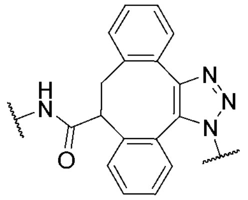, 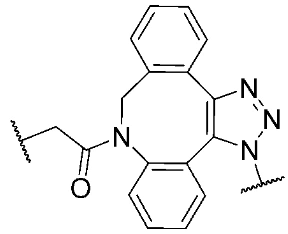,

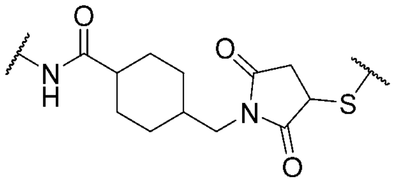, or 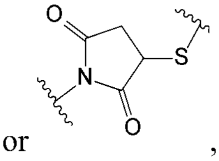,

$R_1$ is an alkyl group having 1-6 carbons or hydrogen;
R is an alkyl group having 1-16 carbons or hydrogen;
W is -NH-, -O-,

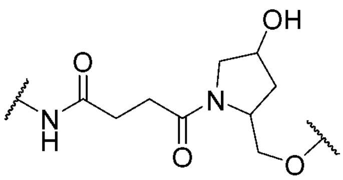, 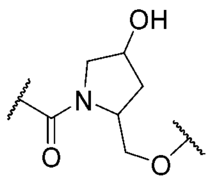,

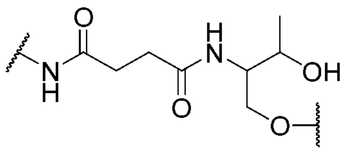, or 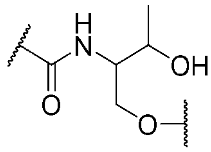,

or a tautomer, a mesomer, a racemate, an enantiomer, or a diastereoisomer thereof, wherein the wave line on the right represents a site to which other part of the conjugate is attached;
A and C are independently 0 or an integer between 1 and 14, preferably independently 0, 1, 2 or 3;
B is 0 or an integer between 1 and 12, preferably independently 0, 1 or 2;
D is an integer between 1 and 20, preferably an integer from 1 to 5, e.g., 1, 2, 3 or 4;
E is 0 or an integer between 1 and 12, preferably 0 or an integer from 1 to 5, e.g., 0, 1, 2 or 3;
F is 0 or an integer between 1 and 14, preferably 0 or an integer from 1 to 5, e.g., 0, 1, 2 or 3; and
M is present or absent, and when present M is a cation, preferably $H^+$ or a metal cation, wherein the metal cation is preferably a monovalent metal cation, preferably $Na^+$, $K^+$, or $Ag^+$; a divalent metal cation, preferably $Ca^{2+}$, $Mg^{2+}$, $Cu^{2+}$, $Fe^{2+}$, $Zn^{2+}$, or $Mn^{2+}$; or a trivalent metal cation, preferably $Fe^{3+}$, $Al^{3+}$ or a lanthanide metal ion; alternatively, when present, M is a radionuclide, preferably Lu-177, Zr-89, Sc-44, Cu-64, Ga-67, Ga-68, Tc-99m, In-111, Sc-47, Y-90, Y-86, Sm-153, Ho-166, Re-188, Pb-212, Bi-213 or Th-232;

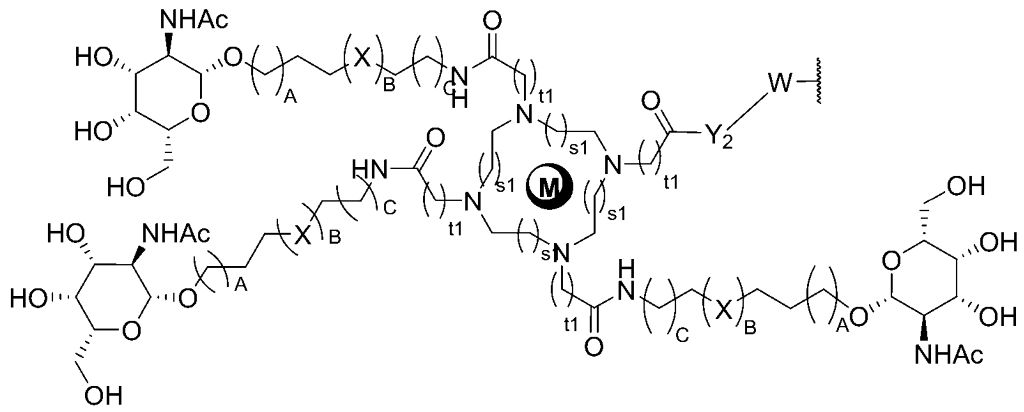

(III')

wherein,

X is -C(O)-NH-, -NH-C(O)-, $-OCH_2-CH_2O-$, -S-,

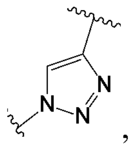, or 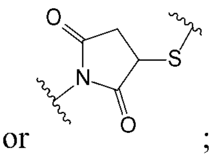;

each $s_1$ and each $t_1$ are independently an integer between 1 and 5, preferably independently 1 or 2;
$Y_2$ is selected from the group consisting of:

(1) $NH-(CH_2)p-(O-CH_2-CH_2)q-(CH_2)j-Z_1-$, wherein $Z_1$ is O, NH, C(O) or absent, p is an integer from 1 to 3, q is an integer from 3 to 10, j is 0 or 1;
(2) $(Sar)n_1-NH-(CH_2)r-Z_2-$, wherein $n_1$ is an integer between 2 and 8, r is an integer from 2 to 10, $Z_2$ is O, NH, C(O) or absent;
(3) $(Gly)n_2-(Z_3)m_1-(Gly)o_1-NH-(CH_2)r-Z_4-$, wherein $Z_3$ is Ser, HomoSer, (NMe)Ser, Thr, (NMe)Thr, His, (NMe)His, Arg, (NMe)Arg, Arg(Me), Trp, (NMe)Trp, Trp(Me), Lys, (NMe)Lys, Lys(Me), $Lys(Me)_2$, Orn, (NMe) Orn, Orn(Me), $Orn(Me)_2$, Dap, (NMe)Dap, Dap(Me), $Dap(Me)_2$, Dab, (NMe)Dab, Dab(Me), $Dab(Me)_2$, Cit or Val-Cit, $n_2$ is an integer between 0 and 5, $m_1$ is an integer between 1 and 10, $o_1$ is an integer between 0 and 5, r is an integer from 2 to 16, $Z_4$ is O, NH, C(O) or absent;
(4)

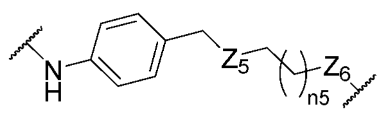, 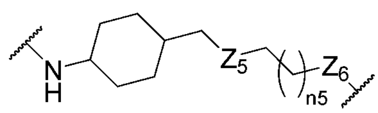,

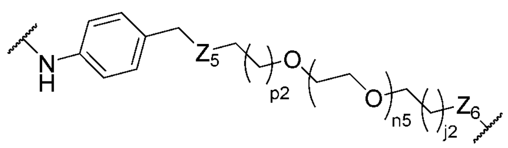, 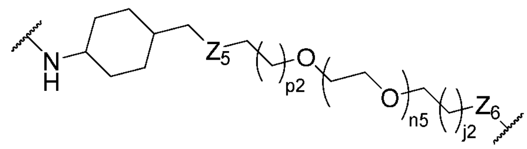,

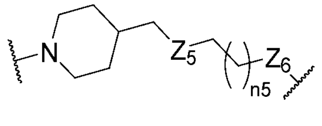, 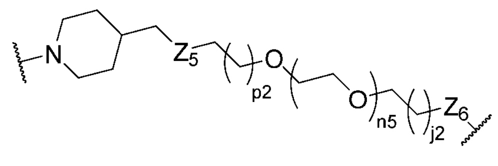,

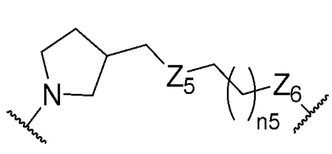, 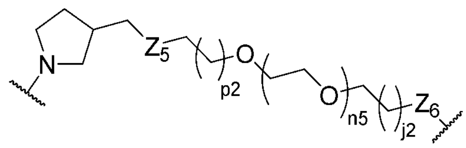,

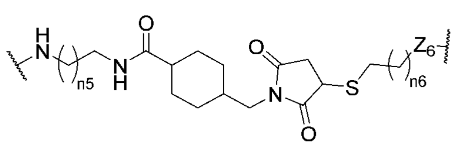, 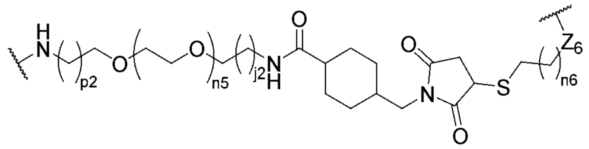,

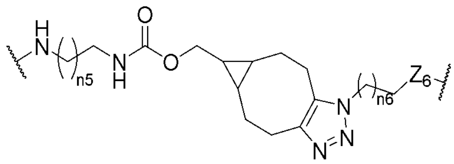, 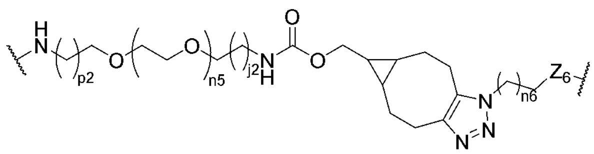,

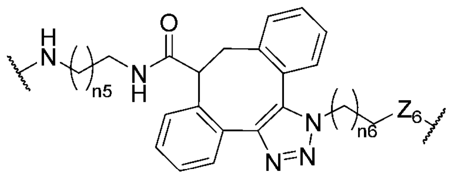, 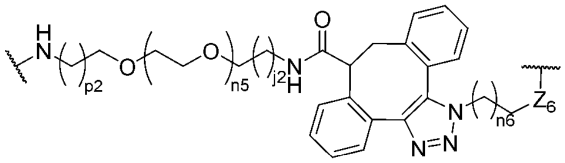,

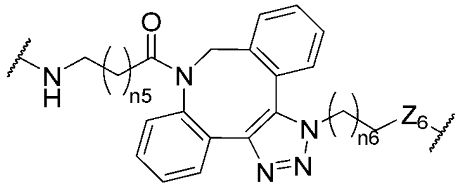, 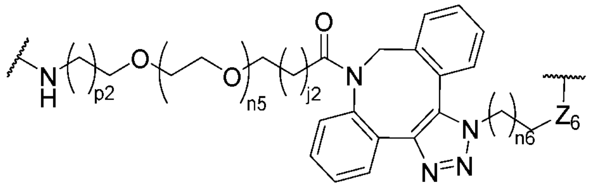,

or a tautomer, a mesomer, a racemate, an enantiomer or a diastereoisomer thereof, wherein $Z_5$ is - C(O)-NH-, -NH-C(O)- or absent, $Z_6$ is O, NH, C(O) or absent, $p_2$ is an integer from 1 to 2, $j_2$ is an integer of 1 or 2, $n_5$ is an integer between 1 and 19, $n_6$ is an integer between 1 and 19;

W is absent or is

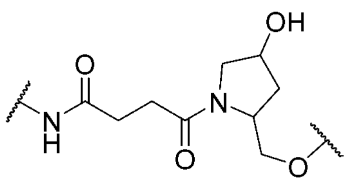, 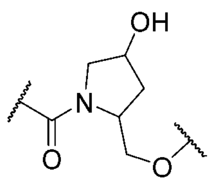,

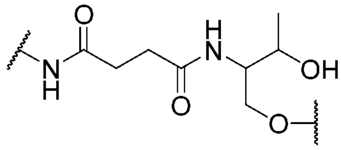, or 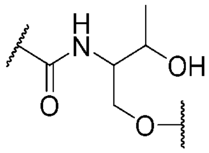,

or a tautomer, a mesomer, a racemate, an enantiomer or a diastereoisomer thereof, wherein the wave line on the right represents a site to which other part of the conjugate is attached;

A and C are independently 0 or an integer between 1 and 14, preferably independently 0, 1, 2 or 3;

B is 0 or an integer between 1 and 12, preferably independently 0, 1 or 2; and

M is present or absent, and when present M is a cation, preferably $H^+$ or a metal cation, wherein the metal cation is

preferably a monovalent metal cation, preferably $Na^+$, $K^+$, or $Ag^+$; a divalent metal cation, preferably $Ca^{2+}$, $Mg^{2+}$, $Cu^{2+}$, $Fe^{2+}$, $Zn^{2+}$, or $Mn^{2+}$; or a trivalent metal cation, preferably $Fe^{3+}$, $Al^{3+}$ or a lanthanide metal ion; alternatively, when present, M is a radionuclide, preferably Lu-177, Zr-89, Sc-44, Cu-64, Ga-67, Ga-68, Tc-99m, In-111, Sc-47, Y-90, Y-86, Sm-153, Ho-166, Re-188, Pb-212, Bi-213 or Th-232.

Preferably, exemplary trivalent and/or tetravalent GalNAc ligands that can be attached to the double-stranded RNA molecule in the RNAi agents of the present invention are provided in any one of the formulas IV-1 to IV-7 below:

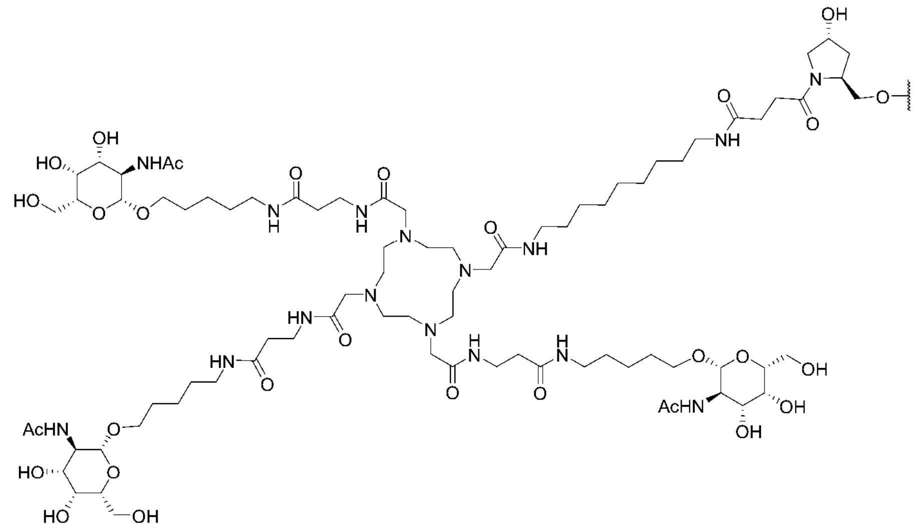

(IV-1) YHZY12001

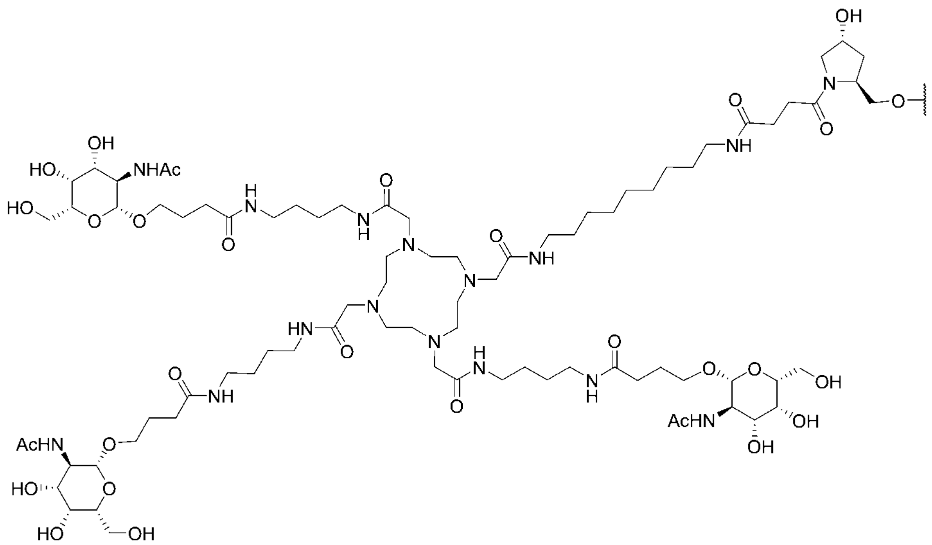

(IV-2) YHZY12002

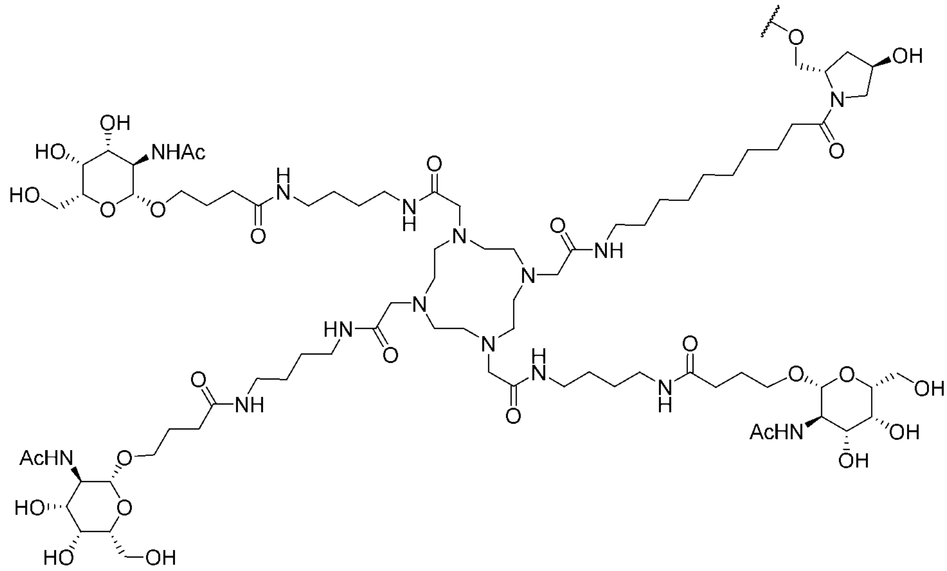

(IV-3) YHZY12003

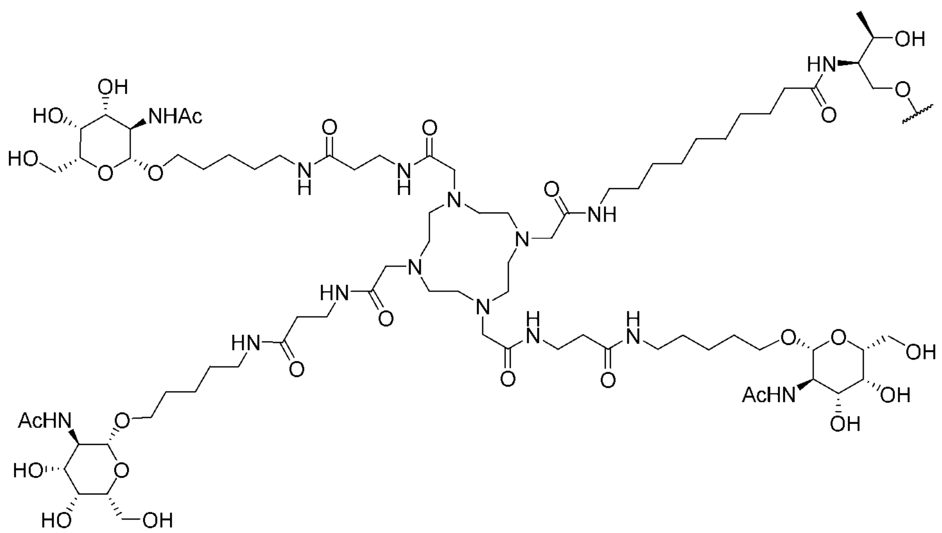

(IV-4) YHZY12004

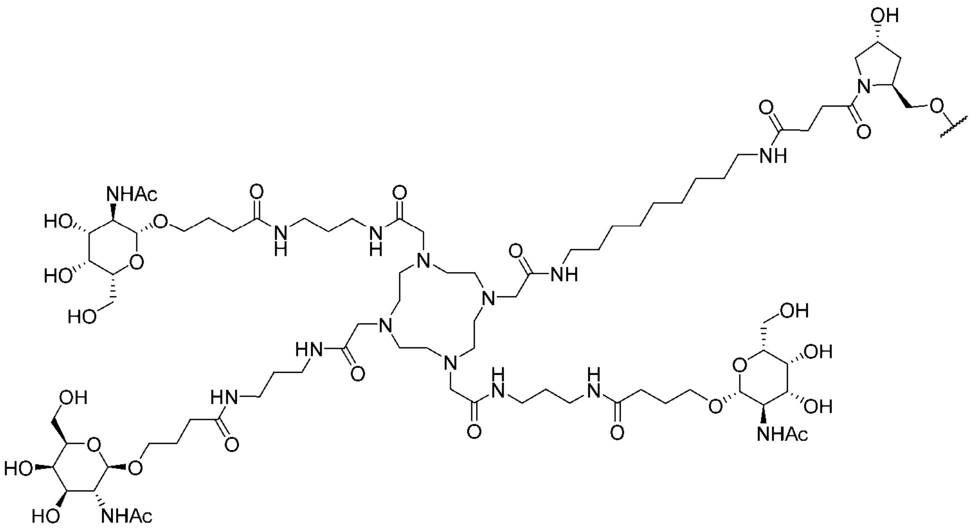

(IV-5) YHZY12005

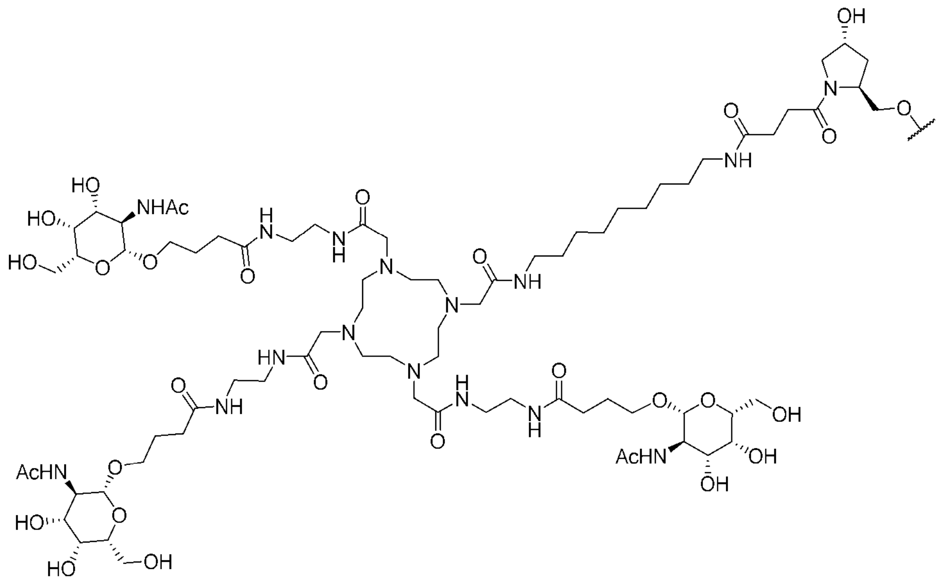

(IV-6) YHZY12006

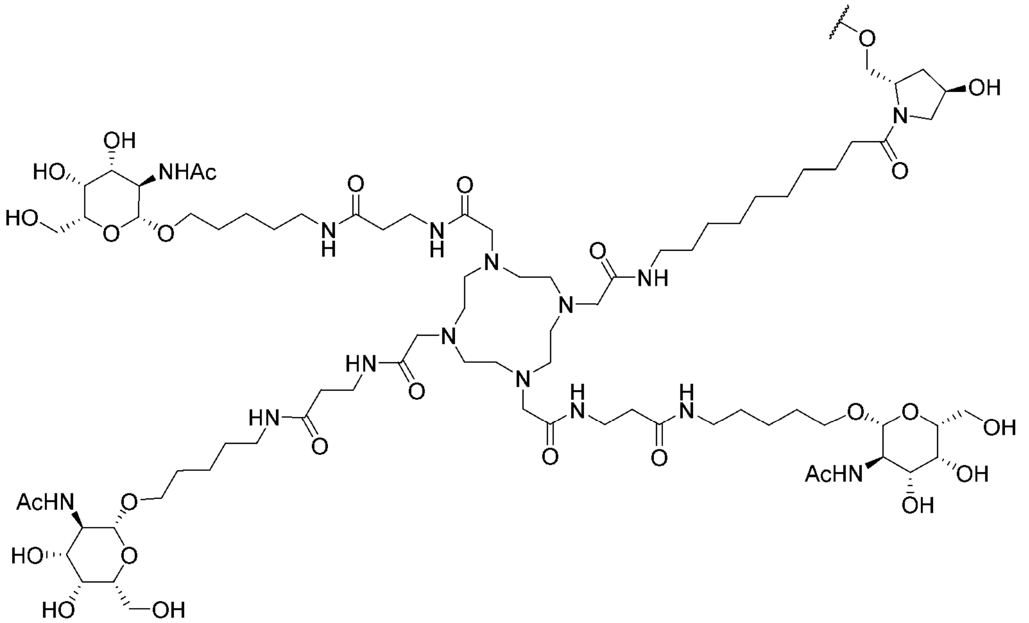

(IV-7) YHZY12007

wherein the wave line represents a site to which the sense strand or antisense strand is attached; preferably the ligand is connected to 5'- and/or 3'-end of the sense strand; more preferably, the ligand is connected to 3'-end of the sense strand; wherein the ligand is connected to the sense strand or antisense strand via a phosphate ester group, a thiophosphate ester group or a phosphonate ester group.

In preferred embodiments, the RNAi agent provided by the present invention has a structure of any one of the following formulas VII-1 to VII-8:

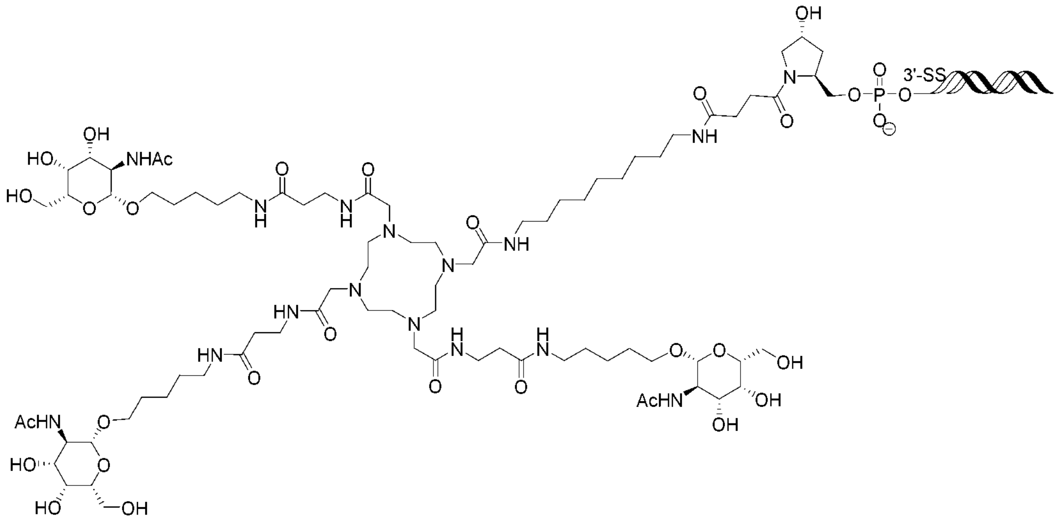

(VII-1)

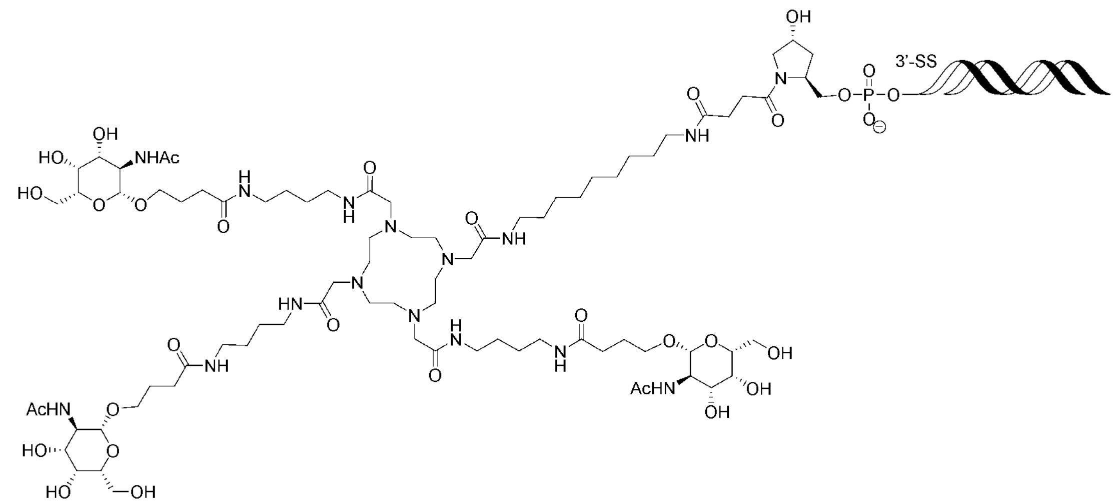

(VII-2)

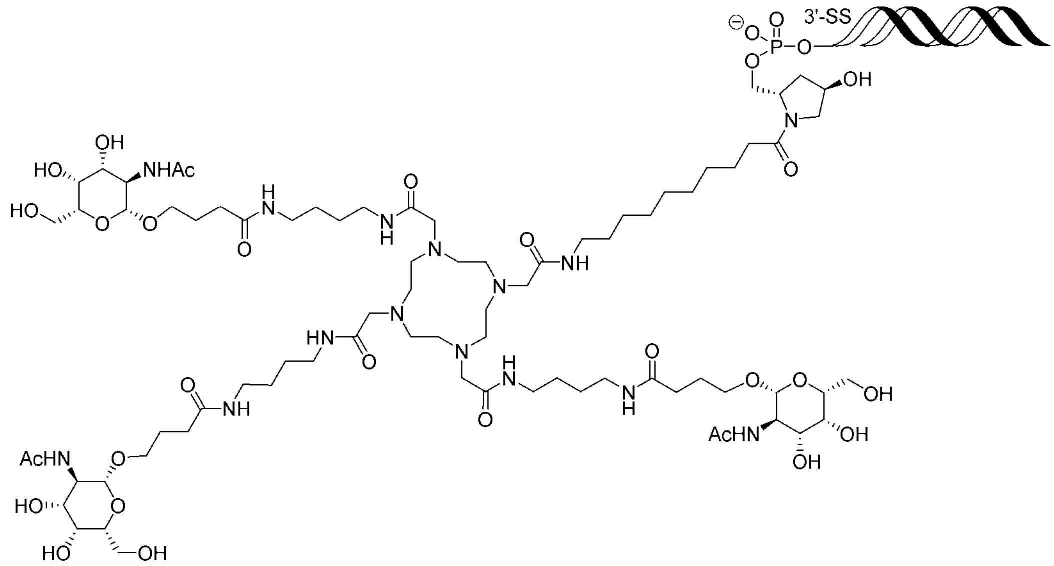

(VII-3)

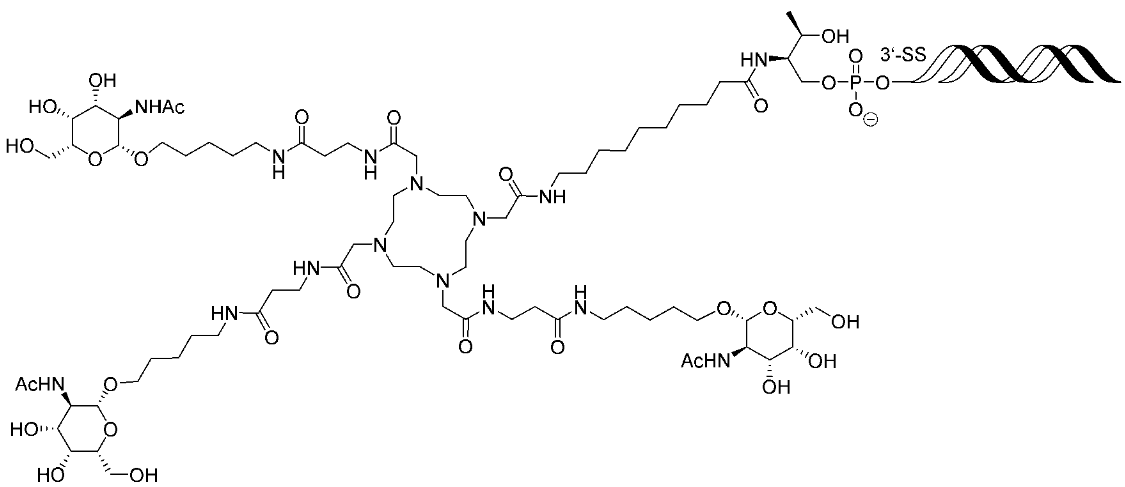

(VII-4)

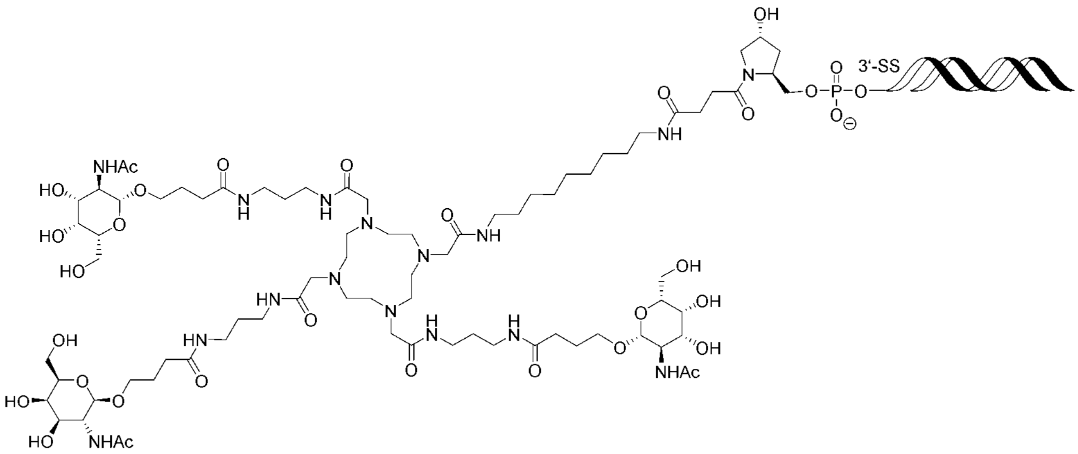

(VII-5)

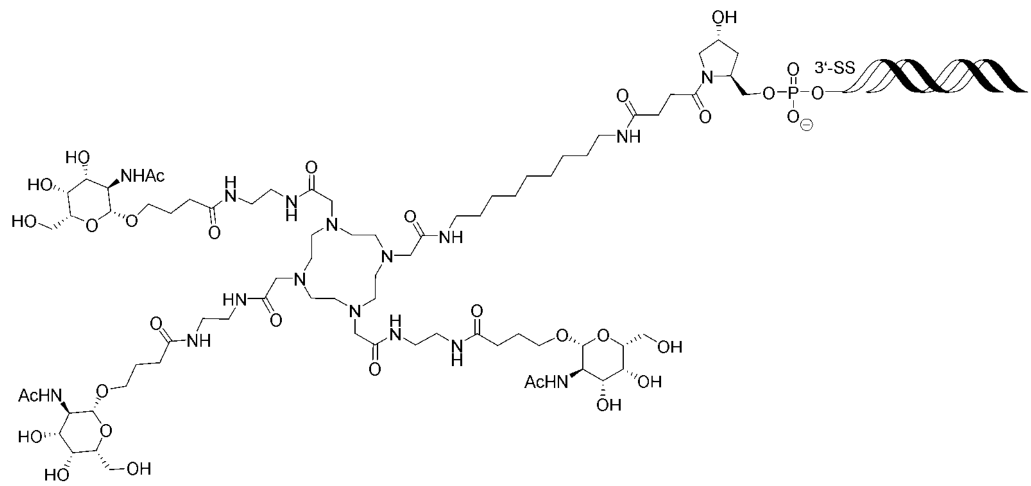

(VII-6)

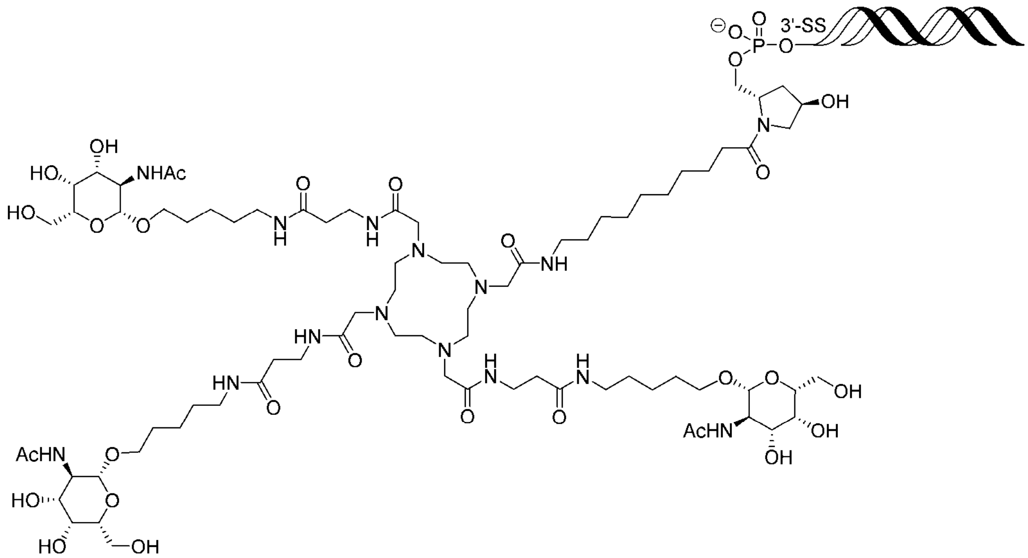

(VII-7)

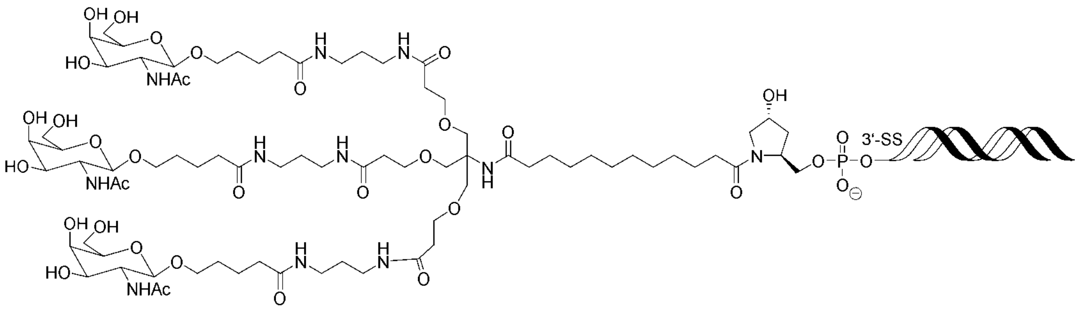

(VII-8).

In preferred embodiments, in the RNAi agent of the present invention, the sense strand comprises or consists of a nucleotide sequence set forth in any one of SEQ ID NOs: 68 to 102 (SEQ ID NOs: 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101 and 102) or a nucleotide sequence having 1, 2, or 3 nucleotide differences therefrom.

In preferred embodiments, in the RNAi agent of the present invention:

(c1) the sense strand comprises or consists of a nucleotide sequence set forth in SEQ ID NO: 68 or a nucleotide sequence having 1, 2 or 3 nucleotide differences therefrom, and the antisense strand comprises or consists of a nucleotide sequence set forth in SEQ ID NO: 104 or a nucleotide sequence having 1, 2 or 3 nucleotide differences therefrom;

(c2) the sense strand comprises or consists of a nucleotide sequence set forth in SEQ ID NO: 69 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c3) the sense strand comprises or consists of a nucleotide sequence set forth in SEQ ID NO: 70 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(c4) the sense strand comprises or consists of a nucleotide sequence set forth in SEQ ID NO: 71 or a nucleotide sequence having 1, 2 or 3 nucleotide differences therefrom, and the antisense strand comprises or consists of a nucleotide sequence set forth in SEQ ID NO: 107 or a nucleotide sequence having 1, 2 or 3 nucleotide differences therefrom;

(c5) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c6) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 108 or a nucleotide sequence having 1, 2 or 3 nucleotide differences therefrom;

(c7) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 109 or a nucleotide sequence having 1, 2 or 3 nucleotide differences therefrom;

(c8) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 110 or a nucleotide sequence having 1, 2 or 3 nucleotide differences therefrom;

(c9) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 111 or a nucleotide sequence having 1, 2 or 3 nucleotide differences therefrom;

(c10) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c11) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c12) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 114 or a nucleotide sequence having 1, 2 or 3 nucleotide differences therefrom;

(c13) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 115 or a nucleotide sequence having 1, 2 or 3 nucleotide differences therefrom;

(c14) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 116 or a nucleotide sequence having 1, 2 or 3 nucleotide differences therefrom;

(c15) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c 16) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c17) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c18) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c19) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c20) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c21) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c22) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c23) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c24) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c25) the sense strand comprises or consists of a nucleotide sequence set forth in SEQ ID NO: 76 or a nucleotide sequence having 1, 2 or 3 nucleotide differences therefrom, and the antisense strand comprises or consists of a

nucleotide sequence set forth in SEQ ID NO: 117 or a nucleotide sequence having 1, 2 or 3 nucleotide differences therefrom;

(c26) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c27) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c28) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c29) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c30) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c31) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c32) the sense strand comprises or consists of a nucleotide sequence set forth in SEQ ID NO: 79 or a nucleotide sequence having 1, 2 or 3 nucleotide differences therefrom, and the antisense strand comprises or consists of a nucleotide sequence set forth in SEQ ID NO: 118 or a nucleotide sequence having 1, 2 or 3 nucleotide differences therefrom;

(c33) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c34) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c35) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c36) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(c37) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c38) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c39) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(c40) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c41) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c42) the sense strand comprises or consists of a nucleotide sequence set forth in SEQ ID NO: 83 or a nucleotide sequence having 1, 2 or 3 nucleotide differences therefrom, and the antisense strand comprises or consists of a nucleotide sequence set forth in SEQ ID NO: 121 or a nucleotide sequence having 1, 2 or 3 nucleotide differences therefrom;
(c43) the sense strand comprises or consists of a nucleotide sequence set forth in SEQ ID NO: 84 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c44) the sense strand comprises or consists of a nucleotide sequence set forth in SEQ ID NO: 84 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c45) the sense strand comprises or consists of a nucleotide sequence set forth in SEQ ID NO: 85 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c46) the sense strand comprises or consists of a nucleotide sequence set forth in SEQ ID NO: 85 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c47) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;
(c48) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c49) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c50) the sense strand comprises or consists of a nucleotide sequence set forth in SEQ ID NO: 87 or a nucleotide sequence having 1, 2 or 3 nucleotide differences therefrom, and the antisense strand comprises or consists of a nucleotide sequence set forth in SEQ ID NO: 122 or a nucleotide sequence having 1, 2 or 3 nucleotide differences therefrom;
(c51) the sense strand comprises or consists of a nucleotide sequence set forth in SEQ ID NO: 88 or a nucleotide sequence having 1, 2 or 3 nucleotide differences therefrom, and the antisense strand comprises or consists of a nucleotide sequence set forth in SEQ ID NO: 123 or a nucleotide sequence having 1, 2 or 3 nucleotide differences therefrom;
(c52) the sense strand comprises or consists of a nucleotide sequence set forth in SEQ ID NO: 89 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;
(c53) the sense strand comprises or consists of a nucleotide sequence set forth in SEQ ID NO: 90 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;
(c54) the sense strand comprises or consists of a nucleotide sequence set forth in SEQ ID NO: 91 or a nucleotide sequence having 1, 2 or 3 nucleotide differences therefrom, and the antisense strand comprises or consists of a

nucleotide sequence set forth in SEQ ID NO: 126 or a nucleotide sequence having 1, 2 or 3 nucleotide differences therefrom;

(c55) the sense strand comprises or consists of a nucleotide sequence set forth in SEQ ID NO: 92 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;

(c56) the sense strand comprises or consists of a nucleotide sequence set forth in SEQ ID NO: 93 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;

(c57) the sense strand comprises or consists of a nucleotide sequence set forth in SEQ ID NO: 94 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom;

(c58) the sense strand comprises or consists of a nucleotide sequence set forth in SEQ ID NO: 95 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom;

(c59) the sense strand comprises or consists of a nucleotide sequence set forth in SEQ ID NO: 96 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;

(c60) the sense strand comprises or consists of a nucleotide sequence set forth in SEQ ID NO: 97 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;

(c61) the sense strand comprises or consists of a nucleotide sequence set forth in SEQ ID NO: 98 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;

(c62) the sense strand comprises or consists of a nucleotide sequence set forth in SEQ ID NO: 99 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;

(c63) the sense strand comprises or consists of a nucleotide sequence set forth in SEQ ID NO: 100 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;

(c64) the sense strand comprises or consists of a nucleotide sequence set forth in SEQ ID NO: 101 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom; or

(c65) the sense strand comprises or consists of a nucleotide sequence set forth in SEQ ID NO: 102 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom.

In some embodiments, the RNAi agent comprises or consists of duplex 4023-60-L96, 4122-60-L96, 4132-60-L96, 4149-60-L96, 4122-60-001, 4122-50-001, 4122-4-001, 4122-64-001, 4122-67-001, 4122-10-001, 4122-11-001, 4122-70-001, 4122.1-60-001, 4122-44-001, 4122-18-001, 4122-19-001, 4122-74-001, 4122-75-001, 4122-101-001, 4122-34-001, 4122-35-001, 4122-98-001, 4122-26-001, 4122-27-001, 4122-97-001, 4122-85-001, 4122-86-001, 4122-99-001, 4122-80-001, 4122-81-001, 4122-100-001, 4122.3-60-001, 4122-60-002, 4122-10-002, 4122-11-002, 4122-60-004, 4122-10-004, 4122-11-004, 4132-60-001, 4132-10-001, 4132-11-001, 4132-44-001, 4132-26-001, 4132-27-001, 4132-34-001, 4132-35-001, 4132-60-004, 4132-10-004, 4132-11-004, 4023-65-001, 4023-44-001, 4089-60-L96, 4098-60-L96, 4118-60-L96, 4123-60-L96, 4129-60-L96, 4135-60-L96, 4145-60-L96, 4089-60-001, 4098-60-001, 4118-60-001, 4123-60-001, 4129-60-001, 4135-60-001 or 4145-60-001.

**Pharmaceutical Compositions**

The present invention also encompasses pharmaceutical compositions and formulations comprising the RNAi agents described herein and a pharmaceutically acceptable carrier, excipient, or diluent. Such compositions and formulations can be used to reduce the expression of the KHK gene in a patient in need thereof. Considering clinical application, the pharmaceutical compositions and formulations will be prepared in a form suitable for the intended application. Typically, this will involve preparing compositions substantially free of pyrogens and other impurities that may be harmful to humans or animals.

The ingredients and methods for formulating pharmaceutical compositions depend on many criteria, including, but not limited to, the route of administration, the type and extent of the disease or condition to be treated, or the dosage to be administered. In some embodiments, the pharmaceutical composition is formulated based on the intended delivery route. For example, in certain embodiments, the pharmaceutical composition is formulated for parenteral delivery. Parenteral administration forms include intravenous, intra-arterial, subcutaneous, intrathecal, intraperitoneal, or intramuscular injection or infusion. In one embodiment, the pharmaceutical composition is formulated for intravenous delivery. In such embodiments, the pharmaceutical composition may comprise a lipid-based delivery vehicle. In another embodiment, the pharmaceutical composition is formulated for subcutaneous delivery. In such embodiments, the pharmaceutical composition may comprise a targeting ligand (e.g., a GalNAc-containing or antibody-containing ligand as described herein).

In some embodiments, the pharmaceutical composition comprises an effective amount of the RNAi agent described herein. An "effective amount" refers to an amount sufficient to produce a beneficial or desired clinical outcome. In some embodiments, the effective amount is an amount sufficient to reduce the expression of the KHK gene in a specific tissue or cell type of the patient (e.g., the liver or hepatocytes). The effective amount of the RNAi agent of the present invention can be from about 0.01 mg/kg body weight to about 100 mg/kg body weight, and can be administered daily, weekly, monthly, or at longer intervals. The precise determination of the specific effective dosage amount and administration frequency may be based on several factors, including the patient’s body size, age, and general condition, the type of disease to be treated (e.g., myocardial infarction, coronary artery disease, peripheral artery disease, stroke), the specific RNAi agent used, and the route of administration.

Administration of the pharmaceutical compositions of the invention can be by any common route provided the target tissue is accessible via that route. These routes include, but are not limited to, parenteral (e.g., subcutaneous, intramuscular, intraperitoneal, or intravenous), oral, nasal, buccal, intradermal, transdermal, and sublingual routes, or by direct injection into liver tissue or delivery via the hepatic portal vein. In some embodiments, the pharmaceutical composition is administered parenterally. For example, in certain embodiments, the pharmaceutical composition is administered intravenously. In other embodiments, the pharmaceutical composition is administered subcutaneously.

Colloidal dispersion systems can be used as delivery vehicles for the RNAi agents of the present invention, such as macromolecular complexes, nanocapsules, microspheres, beads, and lipid-based systems, including oil-in-water emulsions, micelles, mixed micelles, and liposomes. Commercially available fat emulsions suitable for delivering the nucleic acids of the present invention include Intralipid (Baxter International Inc.), Liposyn (Abbott Pharmaceuticals), Lipsyn II (Hospira), Liposyn III (Hospira), Nutrilipid (B. Braun Medical Inc.), and other similar fat emulsions. A preferred colloidal system for use as an in vivo delivery vehicle is the liposome (i.e., an artificial membrane vesicle). The RNAi agents of the present invention can be encapsulated within liposomes or can form complexes therewith, particularly with cationic liposomes. Alternatively, the RNAi agents of the present invention can be complexed with lipids, particularly with cationic lipids. Suitable cationic lipids are, for example, dioleoyltrimethylammonium propane (DOTAP) and dioleoylphosphatidylethanolamine (DOTMA).

Liposomal formulations are particularly suitable for topical administration, with liposomes conferring several advantages over alternative formulations. Such advantages include reduced incidence of adverse effects associated with high systemic absorption of administered therapeutics, enhanced accumulation of administered therapeutics at desired target sites, and capability for dermal delivery of RNAi agents. In some embodiments, liposomes are employed to deliver RNAi agents to epidermal cells while concurrently enhancing permeation of said RNAi agents into dermal tissues, e.g., within the skin.

The RNAi agents of the present invention may be fully encapsulated within lipid-based formulations, such as LNPs or other nucleic acid-lipid particles. As used herein, the term "LNP" refers to stable nucleic acid-lipid particles. LNPs typically comprise a cationic lipid, a non-cationic lipid, and a lipid that prevents particle aggregation (e.g., a PEG-lipid conjugate). LNPs are particularly useful for systemic applications as they exhibit prolonged circulation times following intravenous (i.v.) injection and accumulate at distal sites (e.g., sites physically separated from the administration site). LNPs include "pSPLP," which comprise encapsulated condensing agent-nucleic acid complexes as described in WO00/03683. The LNP particles of the present invention generally possess a mean diameter ranging from about 50 nm to about 150 nm, more typically from about 60 nm to about 130 nm, more typically from about 70 nm to about 110 nm, and most typically from about 70 nm to about 90 nm, and are substantially non-toxic. Additionally, when nucleic acids are

present within the nucleic acid-lipid particles of the invention, they demonstrate resistance to nuclease degradation in aqueous solutions. Nucleic acid-lipid particles and methods for their preparation are disclosed in, for example, U.S. Pat. Nos. 5,976,567; 5,981,501; 6,534,484; 6,586,410; 6,815,432; U.S. Publication No. 2010/0324120; and WO 96/40964. In one embodiment, the lipid-to-drug ratio (mass/mass basis) (e.g., lipid-to-RNAi agent ratio) will range from about 1:1 to about 50:1, about 1:1 to about 25:1, about 3:1 to about 15:1, about 4:1 to about 10:1, about 5:1 to about 9:1, or about 6:1 to about 9:1.

Cationic lipids may include, for example, N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-dioleyloxy-N,N-dimethylaminopropane (DLinDMA), 1,2-dilinoleyloxy-N,N-dimethylaminopropane (DLenDMA), 1,2-dilinoleylcarbamoyloxy-3-dimethylaminopropane (DLin-C-DAP), 1,2-dilinoleyloxy-3-(dimethylamino) acetoxypropane (DLin-DAC), 1,2-dilinoleyloxy-3-morpholinopropane (DLin-MA), 1,2-dilinolenyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleoyloxy-3-(N-methylpiperazinyl)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-(bis(2-hydroxydodecyl)amino)ethyl)(2-hydroxydodecyl)amino)ethyl)piperazin-1-yl)ethylazanediyl)didodecan-2-ol (Tech G1), or mixtures thereof. The cationic lipid may constitute about 20 mol% to about 50 mol%, or about 40 mol% of the total lipid present in the particle.

In some embodiments, the compound 2,2-dilinoleyl-4-dimethylaminomethyl-[1,3]-dioxolane may be utilized in the preparation of lipid-siRNA nanoparticles. In certain embodiments, the lipid-siRNA particles comprise 40% 2,2-dilinoleyl-4-dimethylaminomethyl-[1,3]-dioxolane : 10% DSPC : 40% cholesterol : 10% PEG-C-DOMG (mol%), with a particle size of 63.0 $\pm$ 20 nm and an siRNA-to-lipid ratio of 0.027.

Ionizable/non-cationic lipids may be anionic lipids or neutral lipids, including but not limited to distearoylphosphatidylcholine (DSPC), dioleoylphosphatidylcholine (DOPC), dipalmitoylphosphatidylcholine (DPPC), dioleoylphosphatidylglycerol (DOPG), dipalmitoyl (DP), dioleoylphosphatidylethanolamine (DOPE), palmitoyloleoylphosphatidylcholine (POPC), palmitoyloleoylphosphatidylethanolamine (POPE), N-(4-(p-maleimidophenyl)butyryl)-dioleoylphosphatidylethanolamine (DOPE-mal), dipalmitoylphosphatidylethanolamine (DPPE), dimyristoylphosphatidylethanolamine (DMPE), distearoylphosphatidylethanolamine (DSPE), 16-O-monomethyl PE, 16-O-dimethyl PE, 18-1-trans PE, 1-stearoyl-2-oleoyl-phosphatidylethanolamine (SOPE), cholesterol, or mixtures thereof. The non-cationic lipid may constitute about 5 mol% to about 90 mol%, about 10 mol%, or about 58 mol% (when cholesterol is included) of the total lipid present in the particle.

Lipids conjugated to prevent particle aggregation may include, for example, polyethylene glycol (PEG)-lipids, comprising but not limited to PEG-diacylglycerol (PEG-DAG), PEG-dialkoxypropyl (PEG-DAA), PEG-phospholipid, PEG-ceramide (PEG-Cer), or mixtures thereof. PEG-DAA conjugates may include, for instance, PEG-didodecyloxypropyl (C12), PEG-dimyristyloxypropyl (C14), PEG-dipalmityloxypropyl (C14), or PEG-distearoyloxypropyl (C18). The lipid conjugated to prevent particle aggregation may constitute 0 mol% to about 20 mol%, or about 2 mol% of the total lipid present in the particle. In some embodiments, the nucleic acid-lipid particles further comprise cholesterol, for example constituting about 10 mol% to about 60 mol%, or about 48 mol% of the total lipid present in said particle.

In one embodiment, the lipidoid ND98·4HCl (molecular weight 1487) (see U.S. Patent Application No. 12/056,230, incorporated herein by reference in its entirety), cholesterol (Sigma-Aldrich), and PEG-ceramide C16 (Avanti Polar Lipids) may be utilized to prepare lipid-dsRNA nanoparticles (i.e., LNP01 particles). Each stock solution in ethanol may be prepared as follows: ND98 at 133 mg/mL; cholesterol at 25 mg/mL; PEG-ceramide C16 at 100 mg/mL. The stock solutions of ND98, cholesterol, and PEG-ceramide C16 may then be mixed at a mol ratio of, for example, 42:48:10. The combined lipid solution may be mixed with aqueous siRNA (e.g., in sodium acetate buffer at pH 5) such that the final ethanol concentration is about 35-45% and the final sodium acetate concentration is about 100-300 mM. Lipid-siRNA nanoparticles typically form spontaneously upon mixing.

Other exemplary lipid-siRNA formulations are available from *e.g.* WO2009/127060(SNALP), PCT/US2010/022614(XTC), US 2010/0324120(MC3), PCT/US09/63933(ALNY-100) and WO2010/129709(C12-200).

Pharmaceutical compositions suitable for injectable use comprise, for example, sterile aqueous solutions or dispersions and sterile powders for the extemporaneous preparation of sterile injectable solutions or dispersions. Generally, such formulations are sterile and possess sufficient fluidity for ease of syringeability. The formulations shall remain stable under manufacturing and storage conditions and shall be preserved against the contaminating action of microorganisms such as bacteria and fungi. Suitable solvents or dispersion media may include, for instance, water,

ethanol, polyhydric alcohols (e.g., glycerol, propylene glycol, liquid polyethylene glycols, and the like), suitable mixtures thereof, and vegetable oils. Appropriate fluidity may be maintained, for example, using a coating material such as lecithin, by maintaining required particle size in dispersions, or by employing surfactants. Prevention of microbial action may be achieved through various antibacterial and antifungal agents, including but not limited to parabens, chlorobutanol, phenol, sorbic acid, thimerosal, and the like. In many cases, it is preferable to include isotonic agents, such as sugars or sodium chloride. Prolonged absorption of injectable compositions may be accomplished by incorporating agents delaying absorption in the composition, for example, aluminum monostearate and gelatin.

Sterile injectable solutions may be prepared by incorporating the requisite quantity of the active compound with any additional ingredients (e.g., those enumerated supra) into an appropriate solvent, followed by filtration sterilization. Generally, dispersions are prepared by incorporating various sterilized active ingredients into a sterile vehicle medium containing essential dispersion agents and other requisite components, for instance, as described above. In the case of sterile powders for preparing sterile injectable solutions, preferred preparation methods include vacuum desiccation and lyophilization techniques, which yield powders of the active ingredient(s) together with any additional desired constituents from their prior sterile-filtered solutions.

The compositions of the present invention may generally be formulated in neutral form or as pharmaceutically acceptable salts. Such salts include, for example, acid addition salts (formed with free amino groups) derived from inorganic acids (e.g., hydrochloric or phosphoric acids) or organic acids (e.g., acetic, oxalic, tartaric, mandelic acids, etc.). Salts formed with free carboxyl groups may likewise be derived from inorganic bases (e.g., sodium, potassium, ammonium, calcium, or ferric hydroxides) or organic bases (e.g., isopropylamine, trimethylamine, histidine, procaine, etc.). In some embodiments, the RNAi agent of the invention is formulated as a sodium salt.

For parenteral administration in aqueous solution form, for example, the solution is typically appropriately buffered, and the liquid diluent is first rendered isotonic with sufficient saline or glucose, for instance. Such aqueous solutions may be employed for intravenous, intramuscular, subcutaneous, and intraperitoneal administration, among others. Preferably, sterile aqueous media are utilized. By way of example, a unit dose may be dissolved in 1 mL of isotonic NaCl solution and either added to 1000 mL of subcutaneous infusion fluid or injected at the proposed infusion site. For administration to humans, the formulations shall comply with sterility, pyrogenicity, general safety, and purity standards as required by local Food and Drug Administration regulations. In certain embodiments, the pharmaceutical composition of the invention comprises the sterile saline solution described herein and the RNAi agent, or consists essentially of these components. In other embodiments, the pharmaceutical composition of the invention comprises the RNAi agent described herein and sterile water (e.g., Water for Injection, WFI), or consists essentially thereof. In yet other embodiments, the pharmaceutical composition of the invention comprises the RNAi agent described herein and Phosphate-Buffered Saline (PBS), or consists essentially thereof.

In certain embodiments, the pharmaceutical compositions of the present invention are packaged with or stored within an administration device. Devices for injectable formulations comprise, but are not limited to, injection ports, pre-filled syringes, auto-injectors, infusion pumps, implantable injectors, and injection pens. Devices for nebulized or powdered formulations comprise, but are not limited to, inhalers, insufflators, nebulizers, and the like. Accordingly, the present invention encompasses an administration device containing the pharmaceutical composition of the invention, for treating or preventing one or more diseases or disorders as described herein.

## Therapeutic Methods and Uses

The present invention provides a method for reducing or inhibiting the expression of KHK gene in a cell (e.g., a hepatocyte) by contacting the cell with any of the RNAi agents described herein. The cell can be *in vitro* or *in vivo.* KHK gene expression can be assessed by measuring the amount or level of KHK mRNA or KHK protein. The reduction in KHK expression in cells or animals treated with the RNAi agents of the invention can be determined relative to KHK expression in cells and animals not treated with the RNAi agent or treated with a control RNAi agent. For example, in some embodiments, the reduction in KHK expression is assessed by (a) measuring the amount or level of KHK mRNA in hepatocytes treated with an RNAi agent of the invention, (b) measuring the amount or level of KHK mRNA in hepatocytes treated with a control RNAi agent (e.g., an RNAi agent targeting an RNA molecule not expressed in hepatocytes or an RNAi agent having a nonsense or scrambled sequence) or no RNAi agent, and (c) comparing the KHK mRNA level measured in the treated cells of (a) with the KHK mRNA level in the control cells of (b). The KHK mRNA levels in the treated and control cells may be normalized to the RNA level of a control gene (e.g., 18S ribosomal RNA or a housekeeping gene) prior to comparison. KHK mRNA levels can be measured by various methods, including Northern blot analysis, nuclease protection assay, fluorescent in situ hybridization (FISH), reverse transcriptase (RT)-PCR, real-time RT-PCR, quantitative PCR, droplet digital PCR, and the like.

In some embodiments, the method for assessing KHK expression levels is performed *in vitro* in cells that naturally express the KHK gene (e.g., hepatocytes) or cells that have been engineered to express KHK. In certain embodiments, the method is performed *in vitro* in hepatocytes. Suitable hepatocytes include, but are not limited to, primary hepatocytes (e.g.,

human or non-human primate hepatocytes), HepAD38 cells, HuH-6 cells, HuH-7 cells, HuH-5-2 cells, BNLCL2 cells, Hep3B cells, or HepG2 cells. In one embodiment, the hepatocyte is a HuH-7 cell. In another embodiment, the hepatocyte is a human primary hepatocyte.

In other embodiments, the method for assessing KHK expression levels is performed *in vivo.* The RNAi agent and any control RNAi agent can be administered to an animal (e.g., a transgenic animal expressing the KHK gene or a non-human primate), and KHK mRNA or serum KHK protein levels are assessed in liver tissue harvested from the animal post-treatment. Alternatively or additionally, biomarkers or functional phenotypes associated with KHK expression can be assessed in the treated animal. For example, KHK protein is the expression product of KHK mRNA in serum or plasma. Therefore, the serum or plasma levels of KHK protein can be measured in an animal treated with an RNAi agent of the invention to assess the functional efficacy of reducing KHK expression.

In certain embodiments, the expression of KHK in hepatocytes is reduced by the RNAi agent of the invention by at least 40%, at least 45%, or at least 50%. In some embodiments, the expression of KHK in hepatocytes is reduced by the RNAi agent of the invention by at least 60%, at least 65%, at least 70%, at least 75%, at least 80%, or at least 85%. In other embodiments, the expression of KHK in hepatocytes is reduced by the RNAi agent of the invention by about 90% or more, e.g., 91%, 92%, 93%, 94%, 95%, 96%, 97%, 98%, 99%, or more. The percentage reduction in KHK expression can be measured by any of the methods described herein, as well as other methods known in the art.

The present invention provides methods for reducing or inhibiting the expression of the KHK gene, thereby reducing or inhibiting the production of KHK protein, in a patient in need thereof, as well as methods for treating or preventing a disease or disorder associated with KHK expression or activity. A "disease or disorder associated with KHK expression" refers to a disease or disorder characterized by altered levels of KHK expression, or a condition where elevated levels of KHK expression are associated with an increased risk of developing the disease or disorder. Diseases or disorders associated with KHK expression may also include those caused by abnormal changes in lipoprotein metabolism, such as changes leading to abnormal or elevated levels of KHK protein, cholesterol, lipids, triglycerides, etc., or impaired clearance of these molecules. In certain embodiments, the RNAi agents of the invention are particularly useful for treating or preventing metabolic diseases and reducing circulating levels of KHK.

Diseases and disorders associated with KHK expression that can be treated or prevented according to the methods of the invention include, but are not limited to, metabolic diseases such as hypertriglyceridemia, obesity, hyperlipidemia, dyslipidemia, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, hyperuricemia or gout.

In certain embodiments, the present invention provides a method for reducing KHK expression in a patient in need thereof, comprising administering to the patient any RNAi agent described herein. Preferably, the level of KHK expression in the patient’s hepatocytes is reduced following administration of the RNAi agent, compared to the level of KHK expression in a patient not receiving the RNAi agent, or compared to the level of KHK expression in the patient prior to administration of the RNAi agent. In some embodiments, after administration of the RNAi agent of the invention, KHK expression in the patient is reduced by at least 30%, at least 35%, at least 40%, at least 45%, at least 50%, at least 55%, at least 60%, at least 65%, at least 70%, at least 75%, at least 80%, at least 85%, or at least 90%, e.g., 91%, 92%, 93%, 94%, 95%, 96%, 97%, 98%, or 99%. The percentage reduction in KHK expression can be measured by any of the methods described herein, as well as other methods known in the art. In certain embodiments, the percentage reduction in KHK expression is determined by assessing the level of KHK protein in the patient’s serum or plasma according to the methods described herein.

In certain embodiments, the patient in need of reducing KHK expression is a patient diagnosed with or at risk of a metabolic disease. Accordingly, the present invention includes a method for treating or preventing a metabolic disease in a patient in need thereof by administering any RNAi agent of the invention. In some embodiments, the present invention includes the use of any RNAi agent described herein in the manufacture of a medicament for treating or preventing a metabolic disease in a patient in need thereof. In other embodiments, the present invention provides an KHK-targeting RNAi agent for use in a method of treating or preventing a metabolic disease in a patient in need thereof. Metabolic diseases include, but are not limited to, hypertriglyceridemia, obesity, hyperlipidemia, dyslipidemia, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, hyperuricemia or gout. In a preferred embodiment, the disease to be treated or prevented according to the method of the present invention is non-alcoholic fatty liver disease. In a preferred embodiment, the disease to be treated or prevented according to the method of the present invention is non-alcoholic steatohepatitis.

In certain other embodiments, the patient in need of reducing KHK expression is a patient with elevated circulating KHK levels. Accordingly, in some embodiments, the present invention provides a method for reducing the serum or plasma level of KHK protein in a patient in need thereof by administering to the patient any RNAi agent described herein. In some embodiments, the present invention includes the use of any RNAi agent described herein in the manufacture of a medicament for reducing the serum or plasma level of KHK protein in a patient in need thereof. In

other embodiments, the present invention provides an KHK-targeting RNAi agent for use in a method of reducing the serum or plasma level of KHK protein in a patient in need thereof.

**Sequence Listings**

**Table 1** Unmodified KHK siRNAs

| Duplex Name | SEQ ID NO. | Sense Strand (5'-3') | SEQ ID NO. | Antisense Strand (5'-3') |
|---|---|---|---|---|
| 4023 | 1 | AGUUCAAGUGGAUCCACAU | 34 | AUGUGGAUCCACUUGAACUGG |
| 4089 | 2 | UGCAGAGCCUCAGAGCAAA | 35 | UUUGCUCUGAGGCUCUGCAUC |
| 4098 | 3 | CGUGCAGGAAGCACUGAGA | 36 | UCUCAGUGCUUCCUGCACGCU |
| 4101 | 4 | UGCUCCACUCGGAUGCUUU | 37 | AAAGCAUCCGAGUGGAGCAAU |
| 4102 | 5 | UUGCUCCACUCGGAUGCUU | 38 | AAGCAUCCGAGUGGAGCAAUU |
| 4103 | 6 | AUGGCAAAUUGCUCCACUA | 39 | UAGUGGAGCAAUUUGCCAUCA |
| 4118 | 7 | CGGAGCAGGUGAAGAUGCU | 40 | AGCAUCUUCACCUGCUCCGAU |
| 4119 | 8 | CAUCGGAGCAGGUGAAGAU | 41 | AUCUUCACCUGCUCCGAUGCG |
| 4121 | 9 | UGACCCAGUUCAAGUGGAU | 42 | AUCCACUUGAACUGGGUCAGA |
| 4122 | 10 | CUGACCCAGUUCAAGUGGA | 43 | UCCACUUGAACUGGGUCAGAU |
| 4123 | 11 | AUCUGACCCAGUUCAAGUA | 44 | UACUUGAACUGGGUCAGAUCA |
| 4124 | 12 | UUGAUCUGACCCAGUUCAA | 45 | UUGAACUGGGUCAGAUCAACC |
| 4125 | 13 | GUUGAUCUGACCCAGUUCA | 46 | UGAACUGGGUCAGAUCAACCU |
| 4126 | 14 | CUGCUACAGACUUUGAGAA | 47 | UUCUCAAAGUCUGUAGCAGAC |
| 4127 | 15 | UCUGCUACAGACUUUGAGA | 48 | UCUCAAAGUCUGUAGCAGACA |
| 4128 | 16 | UGUCUGCUACAGACUUUGA | 49 | UCAAAGUCUGUAGCAGACACA |
| 4129 | 17 | GUGUCUGCUACAGACUUUA | 50 | UAAAGUCUGUAGCAGACACAU |
| 4130 | 18 | AGAUGUGUCUGCUACAGAA | 51 | UUCUGUAGCAGACACAUCUGG |
| 4132 | 19 | GAGCCUGCCAGAUGUGUCU | 52 | AGACACAUCUGGCAGGCUCGU |
| 4135 | 20 | GAGAUAAGGUGUUUGUCCA | 53 | UGGACAAACACCUUAUCUCCG |
| 4138 | 21 | CUCGGAGAUAAGGUGUUUA | 54 | UAAACACCUUAUCUCCGAGUC |
| 4141 | 22 | AGGACUCGGAGAUAAGGUA | 55 | UACCUUAUCUCCGAGUCCUUU |
| 4142 | 23 | AGGAGGACUCGGAGAUAAA | 56 | UUUAUCUCCGAGUCCUCCUUA |
| 4145 | 24 | UCAUCAGCCUGGUGGACAA | 57 | UUGUCCACCAGGCUGAUGACG |
| 4147 | 25 | UAGUGGUGCUGGACGUCAU | 58 | AUGACGUCCAGCACCACUAGC |
| 4148 | 26 | AUGGAAGAGAAGCAGAUCA | 59 | UGAUCUGCUUCUCUUCCAUGA |
| 4149 | 27 | GCCUCAUGGAAGAGAAGCA | 60 | UGCUUCUCUUCCAUGAGGCUA |
| 4151 | 28 | GGAGUAGCCUCAUGGAAGA | 61 | UCUUCCAUGAGGCUACUCCCA |
| 4159 | 29 | UGACAAGCGAGGAAACUAA | 62 | UUAGUUUCCUCGCUUGUCAGA |
| 4161 | 30 | UCAGCUGUGAGUCCAUCUA | 63 | UAGAUGGACUCACAGCUGAUG |
| 4162 | 31 | GCAUUCUCGAGAUCGCUUA | 64 | UAAGCGAUCUCGAGAAUGCAA |
| 4163 | 32 | UUUGCAUUCUCGAGAUCGA | 65 | UCGAUCUCGAGAAUGCAAAGA |

**Table 2** Modified KHK GalNAc-siRNA Sense Strands

| SEQ ID. | Sense Strand (5'-3') |
|---|---|
| 67 | mG*mG*mUmGfUmUfUfGfUmCmAmGmCmAmAmAmGmAmU-L96 |
| 68 | mA*mG*mUmUfCmAfAfGfUmGmGmAmUmCmCmAmCmAmU-L96 |
| 69 | mC*mU*mGmAfCmCfCfAfGmUmUmCmAmAmGmUmGmGmA-L96 |
| 70 | mG*mA*mGmCfCmUfGfCfCmAmGmAmUmGmUmGmUmCmU-L96 |
| 71 | mG*mC*mCmUfCmAfUfGfGmAmAmGmAmGmAmAmGmCmA-L96 |
| 72 | mC*mU*mGmAfCmCfCfAfGmUmUmCmAmAmGmUmGmGmA-YHZY12001 |
| 73 | mC*mU*mGmAmCmCfCfAfGmUmUmCmAmAmGmUmGmGmA-YHZY12001 |
| 74 | mC*mU*mGmAmCmCfCfAdGmUmUmCmAmAmGmUmGmGmA-YHZY12001 |
| 75 | mC*mU*mGmAmCmCfCmAfGmUfUmCmAmAmGmUmGmGmA-YHZY12001 |
| 76 | mC*mU*mGmAfCmCfCmAfGmUmUmCmAmAmGmUmGmGmA-YHZY12001 |
| 77 | mC*mU*mGmAfCmCfCmAdGmUmUmCmAmAmGmUmGmGmA-YHZY12001 |
| 78 | mC*mU*mGmAmCmCdCfAfGmUmUmCmAmAmGmUmGmGmA-YHZY12001 |
| 79 | mC*mU*mGmAfCmCfCfAfGmUmUmCmAmAmUmUmGmGmA-YHZY12001 |
| 80 | mC*mU*mGmAfCmCfCfAfGmUmUmCmAmAmGmUmGmGmA-YHZY12002 |
| 81 | mC*mU*mGmAfCmCfCfAfGmUmUmCmAmAmGmUmGmGmA-YHZY12004 |
| 82 | mG*mA*mGmCfCmUfGfCfCmAmGmAmUmGmUmGmUmCmU-YHZY12001 |
| 83 | mG*mA*mGmCmCmUfGfCfCmAmGmAmUmGmUmGmUmCmU-YHZY 12001 |
| 84 | mG*mA*mGmCfCmUfGmCfCmAmGmAmUmGmUmGmUmCmU-YHZY12001 |
| 85 | mG*mA*mGmCmCmUfGmCfCmAfGmAmUmGmUmGmUmCmU-YHZY12001 |
| 86 | mG*mA*mGmCfCmUfGfCfCmAmGmAmUmGmUmGmUmCmU-YHZY12004 |
| 87 | mA*mG*mUmUfCmAfAfGfUmGmGmAmUmCmCmAmCmAmU-YHZY12001 |
| 88 | mA*mG*mUmUmCmAfAfGfUmGmGmAmUmCmCmAmCmAmU-YHZY12001 |
| 89 | mU*mG*mCmAfGmAfGfCfCmUmCmAmGmAmGmCmAmAmA-L96 |
| 90 | mC*mG*mUmGfCmAfGfGfAmAmGmCmAmCmUmGmAmGmA-L96 |
| 91 | mC*mG*mGmAfGmCfAfGfGmUmGmAmAmGmAmUmGmCmU-L96 |
| 92 | mA*mU*mCmUfGmAfCfCfCmAmGmUmUmCmAmAmGmUmA-L96 |
| 93 | mG*mU*mGmUfCmUfGfCfUmAmCmAmGmAmCmUmUmUmA-L96 |
| 94 | mG*mA*mGmAfUmAfAfGfGmUmGmUmUmUmGmUmCmCmA-L96 |
| 95 | mU*mC*mAmUfCmAfGfCfCmUmGmGmUmGmGmAmCmAmA-L96 |
| 96 | mU*mG*mCmAfGmAfGfCfCmUmCmAmGmAmGmCmAmAmA-YHZY12001 |
| 97 | mC*mG*mUmGfCmAfGfGfAmAmGmCmAmCmUmGmAmGmA-YHZY12001 |
| 98 | mC*mG*mGmAfGmCfAfGfGmUmGmAmAmGmAmUmGmCmU-YHZY12001 |
| 99 | mA*mU*mCmUfGmAfCfCfCmAmGmUmUmCmAmAmGmUmA-YHZY12001 |
| 100 | mG*mU*mGmUfCmUfGfCfUmAmCmAmGmAmCmUmUmUmA-YHZY12001 |
| 101 | mG*mA*mGmAfUmAfAfGfGmUmGmUmUmUmGmUmCmCmA-YHZY12001 |
| 102 | mU*mC*mAmUfCmAfGfCfCmUmGmGmUmGmGmAmCmAmA-YHZY12001 |

**Table 3** Modified KHK siRNA Sense Strands

| SEQ ID. | Sense Strand (5'-3') |
|---|---|
| 131 | mG*mG*mUmGfUmUfUfGfUmCmAmGmCmAmAmAmGmAmU |
| 132 | mA*mG*mUmUfCmAfAfGfUmGmGmAmUmCmCmAmCmAmU |
| 133 | mC*mU*mGmAfCmCfCfAfGmUmUmCmAmAmGmUmGmGmA |
| 134 | mG*mA*mGmCfCmUfGfCfCmAmGmAmUmGmUmGmUmCmU |
| 135 | mG*mC*mCmUfCmAfUfGfGmAmAmGmAmGmAmAmGmCmA |
| 136 | mC*mU*mGmAmCmCfCfAfGmUmUmCmAmAmGmUmGmGmA |
| 137 | mC*mU*mGmAmCmCfCfAdGmUmUmCmAmAmGmUmGmGmA |
| 138 | mC*mU*mGmAmCmCfCmAfGmUfUmCmAmAmGmUmGmGmA |
| 139 | mC*mU*mGmAfCmCfCmAfGmUmUmCmAmAmGmUmGmGmA |
| 140 | mC*mU*mGmAfCmCfCmAdGmUmUmCmAmAmGmUmGmGmA |
| 141 | mC*mU*mGmAmCmCdCfAfGmUmUmCmAmAmGmUmGmGmA |
| 142 | mC*mU*mGmAfCmCfCfAfGmUmUmCmAmAmUmUmGmGmA |
| 143 | mG*mA*mGmCmCmUfGfCfCmAmGmAmUmGmUmGmUmCmU |
| 144 | mG*mA*mGmCfCmUfGmCfCmAmGmAmUmGmUmGmUmCmU |
| 145 | mG*mA*mGmCmCmUfGmCfCmAfGmAmUmGmUmGmUmCmU |
| 146 | mA*mG*mUmUmCmAfAfGfUmGmGmAmUmCmCmAmCmAmU |
| 147 | mU*mG*mCmAfGmAfGfCfCmUmCmAmGmAmGmCmAmAmA |
| 148 | mC*mG*mUmGfCmAfGfGfAmAmGmCmAmCmUmGmAmGmA |
| 149 | mC*mG*mGmAfGmCfAfGfGmUmGmAmAmGmAmUmGmCmU |
| 150 | mA*mU*mCmUfGmAfCfCfCmAmGmUmUmCmAmAmGmUmA |
| 151 | mG*mU*mGmUfCmUfGfCfUmAmCmAmGmAmCmUmUmUmA |
| 152 | mG*mA*mGmAfUmAfAfGfGmUmGmUmUmUmGmUmCmCmA |
| 153 | mU*mC*mAmUfCmAfGfCfCmUmGmGmUmGmGmAmCmAmA |

**Table 4** Modified KHK GalNAc-siRNA Antisense Strands

| SEQ ID. | Antisense Strand (5'-3') |
|---|---|
| 103 | mA*fU*mCmUmUfUmGmCmUmGmAmCmAfAmAfCmAmCmC*mA*mC |
| 104 | mA*fU*mGmUmGfGmAmUmCmCmAmCmUfUmGfAmAmCmU*mG*mG |
| 105 | mU*fC*mCmAmCfUmUmGmAmAmCmUmGfGmGfUmCmAmG*mA*mU |
| 106 | mA*fG*mAmCmAfCmAmUmCmUmGmGmCfAmGfGmCmUmC*mG*mU |
| 107 | mU*fG*mCmUmUfCmUmCmUmUmCmCmAfUmGfAmGmGmC*mU*mA |
| 108 | mU*fC*mCmAmCfUmUfGfAmAmCmUmGfGmGfUmCmAmG*mA*mU |
| 109 | mU*fC*mCmAmCmUmUmGmAmAmCmUmGfGmGfUmCmAmG*mA*mU |
| 110 | mU*fC*mCmAmC(Tgn)mUmGmAmAmCmUmGfGmGfUmCmAmG*mA*mU |
| 111 | mU*fC*mCfAmCfUmUfGmAfAmCmUmGfGmGfUmCfAmG*mA*mU |
| 112 | mU*fC*mCmAfCmUfUmGmAmAmCmUmGfGmGfUmCmAmG*mA*mU |
| 113 | mU*fC*mCmAfCmUfUmGmAmAmCfUmGfGmGmUmCmAmG*mA*mU |
| 114 | VPmU*fC*mCmAfCmUfUmGmAmAmCmUmGfGmGfUmCmAmG*mA*mU |
| 115 | mU*fC*mCmAmCfUmUmGmAmAmCmUmGfGmGfUmCmAmG*mU*mU |

(continued)

| SEQ ID. | Antisense Strand (5'-3') |
|---|---|
| 116 | mU*dC*mCmAdCmUdTmGmAmAmCdTmGfGmGmUmCmAmG*mA*mU |
| 117 | mU*fC*mCmAmCfUmUfGmAfAmCfUmGfGmGfUmCfAmG*mA*mU |
| 118 | mU*fC*mCmAmAfUmUmGmAmAmCmUmGfGmGfUmCmAmG*mA*mU |
| 119 | mA*fG*mAmCfAmCfAmUmCmUmGmGmCfAmGfGmCmUmC*mG*mU |
| 120 | mA*fG*mAmCfAmCfAmUmCmUmGfGmCfAmGmGmCmUmC*mG*mU |
| 121 | mA*dG*mAmCdAmCdAmUmCmUmGmGmCdAmGfGmCmUmC*mG*mU |
| 122 | mA*fU*mGmUmGmG(Agn)mUmCmCmAmCmUfUmGfAmAmCmU*mG*mG |
| 123 | mA*dT*mGmUdGmGdAmUmCmCmAdCmUfUmGmAmAmCmU*mG*mG |
| 124 | mU*fU*mUmGmCfUmCmUmGmAmGmGmCfUmCfUmGmCmA*mU*mC |
| 125 | mU*fC*mUmCmAfGmUmGmCmUmUmCmCfUmGfCmAmCmG*mC*mU |
| 126 | mA*fG*mCmAmUfCmUmUmCmAmCmCmUfGmCfUmCmCmG*mA*mU |
| 127 | mU*fA*mCmUmUfGmAmAmCmUmGmGmGfUmCfAmGmAmU*mC*mA |
| 128 | mU*fA*mAmAmGfUmCmUmGmUmAmGmCfAmGfAmCmAmC*mA*mU |
| 129 | mU*fG*mGmAmCfAmAmAmCmAmCmCmUfUmAfUmCmUmC*mC*mG |
| 130 | mU*fU*mGmUmCfCmAmCmCmAmGmGmCfUmGfAmUmGmA*mC*mG |

wherein m represents a 2'-O-methyl modification, f represents a 2'-fluoro modification, d represents a 2'-deoxynucleoside, * represents a phosphorothioate linkage; Tgn represents a thymidine-glycol nucleic acid (GNA) S-isomer; Agn represents an adenosine-glycol nucleic acid (GNA);

VPmU =

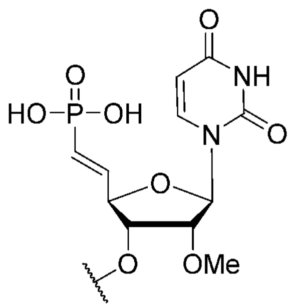

**Table 5** Modified KHK GalNAc-siRNA Compounds

| Compound ID | SS | AS | Compound ID | SS | AS |
|---|---|---|---|---|---|
| | SEQ ID No. | SEQ ID No. | | SEQ ID No. | SEQ ID No. |
| ALN212-60-L96 | 67 | 103 | 4023-60-L96 | 68 | 104 |
| 4122-60-L96 | 69 | 105 | 4132-60-L96 | 70 | 106 |
| 4149-60-L96 | 71 | 107 | 4122-60-001 | 72 | 105 |
| 4122-50-001 | 72 | 108 | 4122-4-001 | 72 | 109 |
| 4122-64-001 | 72 | 110 | 4122-67-001 | 72 | 111 |
| 4122-10-001 | 72 | 112 | 4122-11-001 | 72 | 113 |
| 4122-70-001 | 72 | 114 | 4122.1-60-001 | 72 | 115 |
| 4122-44-001 | 73 | 116 | 4122-18-001 | 73 | 112 |

(continued)

| Compound ID | SS | AS | Compound ID | SS | AS |
|---|---|---|---|---|---|
| | SEQ ID No. | SEQ ID No. | | SEQ ID No. | SEQ ID No. |
| 4122-19-001 | 73 | 113 | 4122-74-001 | 74 | 112 |
| 4122-75-001 | 74 | 113 | 4122-101-001 | 74 | 117 |
| 4122-34-001 | 75 | 112 | 4122-35-001 | 75 | 113 |
| 4122-98-001 | 75 | 117 | 4122-26-001 | 76 | 112 |
| 4122-27-001 | 76 | 113 | 4122-97-001 | 76 | 117 |
| 4122-85-001 | 77 | 112 | 4122-86-001 | 77 | 113 |
| 4122-99-001 | 77 | 117 | 4122-80-001 | 78 | 112 |
| 4122-81-001 | 78 | 113 | 4122-100-001 | 78 | 117 |
| 4122.3-60-001 | 79 | 118 | 4122-60-002 | 80 | 105 |
| 4122-10-002 | 80 | 112 | 4122-11-002 | 80 | 113 |
| 4122-60-004 | 81 | 105 | 4122-10-004 | 81 | 112 |
| 4122-11-004 | 81 | 113 | 4132-60-001 | 82 | 106 |
| 4132-10-001 | 82 | 119 | 4132-11-001 | 82 | 120 |
| 4132-44-001 | 83 | 121 | 4132-26-001 | 84 | 119 |
| 4132-27-001 | 84 | 120 | 4132-34-001 | 85 | 119 |
| 4132-35-001 | 85 | 120 | 4132-60-004 | 86 | 106 |
| 4132-10-004 | 86 | 119 | 4132-11-004 | 86 | 120 |
| 4023-65-001 | 87 | 122 | 4023-44-001 | 88 | 123 |
| 4089-60-L96 | 89 | 124 | 4098-60-L96 | 90 | 125 |
| 4118-60-L96 | 91 | 126 | 4123-60-L96 | 92 | 127 |
| 4129-60-L96 | 93 | 128 | 4135-60-L96 | 94 | 129 |
| 4145-60-L96 | 95 | 130 | 4089-60-001 | 96 | 124 |
| 4098-60-001 | 97 | 125 | 4118-60-001 | 98 | 126 |
| 4123-60-001 | 99 | 127 | 4129-60-001 | 100 | 128 |
| 4135-60-001 | 101 | 129 | 4145-60-001 | 102 | 130 |

**Table 6** Modified KHK siRNA Compounds

| Compound ID | SS | AS | Compound ID | SS | AS |
|---|---|---|---|---|---|
| | SEQ ID No. | SEQ ID No. | | SEQ ID No. | SEQ ID No. |
| ALN212-60 | 131 | 103 | 4023-60 | 132 | 104 |
| 4122-60 | 133 | 105 | 4132-60 | 134 | 106 |
| 4149-60 | 135 | 107 | 4122-50 | 133 | 108 |
| 4122-4 | 133 | 109 | 4122-64 | 133 | 110 |
| 4122-67 | 133 | 111 | 4122-10 | 133 | 112 |
| 4122-11 | 133 | 113 | 4122-70 | 133 | 114 |
| 4122.1-60 | 133 | 115 | 4122-44 | 136 | 116 |
| 4122-18 | 136 | 112 | 4122-19 | 136 | 113 |
| 4122-74 | 137 | 112 | 4122-75 | 137 | 113 |

(continued)

| Compound ID | SS | AS | Compound ID | SS | AS |
|---|---|---|---|---|---|
| | SEQ ID No. | SEQ ID No. | | SEQ ID No. | SEQ ID No. |
| 4122-101 | 137 | 117 | 4122-34 | 138 | 112 |
| 4122-35 | 138 | 113 | 4122-98 | 138 | 117 |
| 4122-26 | 139 | 112 | 4122-27 | 139 | 113 |
| 4122-97 | 139 | 117 | 4122-85 | 140 | 112 |
| 4122-86 | 140 | 113 | 4122-99 | 140 | 117 |
| 4122-80 | 141 | 112 | 4122-81 | 141 | 113 |
| 4122-100 | 141 | 117 | 4122.3-60 | 142 | 118 |
| 4132-10 | 134 | 119 | 4132-11 | 134 | 120 |
| 4132-44 | 143 | 121 | 4132-26 | 144 | 119 |
| 4132-27 | 144 | 120 | 4132-34 | 145 | 119 |
| 4132-35 | 145 | 120 | 4023-65 | 132 | 122 |
| 4023-44 | 146 | 123 | 4089-60 | 147 | 124 |
| 4098-60 | 148 | 125 | 4118-60 | 149 | 126 |
| 4123-60 | 150 | 127 | 4129-60 | 151 | 128 |
| 4135-60 | 152 | 129 | 4145-60 | 153 | 130 |
| SS represents sense strand, and AS represents antisense strand. | | | | | |

**Examples**

**Experimental Methods and Materials**

**Target Sequence Screening**

siRNAs were designed based on the full-length KHK mRNA sequence (NM_006488.3). All sequences were sourced from the NCBI Gene database.

**GalNAc Resin Synthesis**

1. GalNAc is a terminal reactive group-containing compound, including carboxylic acid-reactive compounds such as Gal-C7, Gal-C6, Gal-C5, Gal-C4, Gal-C3, etc., and amine-reactive compounds such as Gal-C7-$NH_2$, Gal-C6-$NH_2$, Gal-C5-$NH_2$, Gal-C4-$NH_2$, Gal-C3-$NH_2$. These were synthesized according to reported methods, with specific details provided in Table 7.

Table 7. List of chemical structures, nomenclature and synthesis methods for terminal reactive group-containing GalNAc compounds

| Chemical Structures (terminal reactive group-containing GalNAc compounds) | ID | References (reported synthesis methods) |
|---|---|---|
| 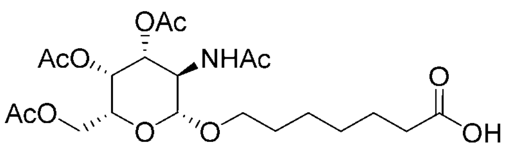 | Gal-C7 | WO2021037205, 2021 |

(continued)

| Chemical Structures (terminal reactive group-containing GalNAc compounds) | ID | References (reported synthesis methods) |
|---|---|---|
| 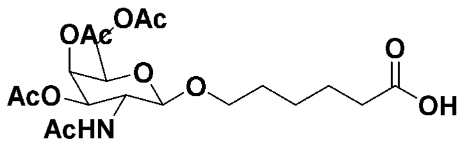 | Gal-C6 | WO2015042447A1, 2015 |
| 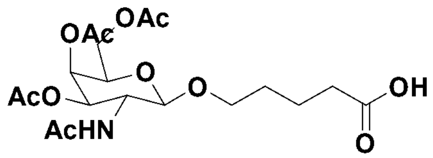 | Gal-C5 | US20120035115, 2012 |
| 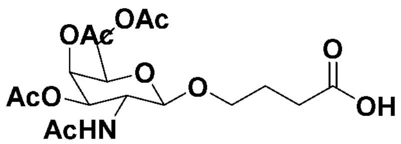 | Gal-C4 | J. Org. Chem., 2020, 85, 10, 6593-6604 |
| 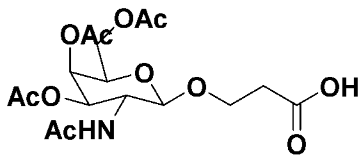 | Gal-C3 | Same method used in US20120035115 |
| 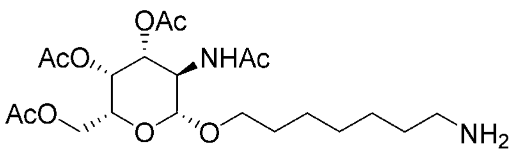 | Gal-C7-$NH_2$ | Same method used in WO2015042447A1 |
| 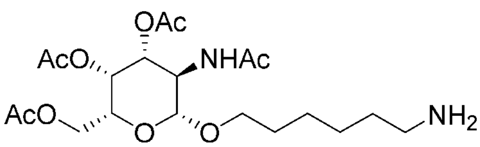 | Gal-C6-$NH_2$ | WO2015042447A1, 2015 |
| 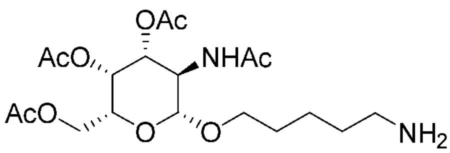 | Gal-C5-$NH_2$ | WO2021049504A1, 2021 |
| 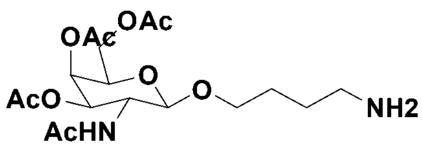 | Gal-C4-$NH_2$ | J. Med. Chem. 2016, 59, 6, 2718-2733 |
| 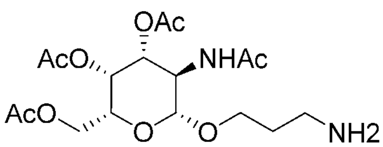 | Gal-C3-$NH_2$ | WO2021049504A1, 2021 |

(continued)

| Chemical Structures (terminal reactive group-containing GalNAc compounds) | ID | References (reported synthesis methods) |
|---|---|---|
| 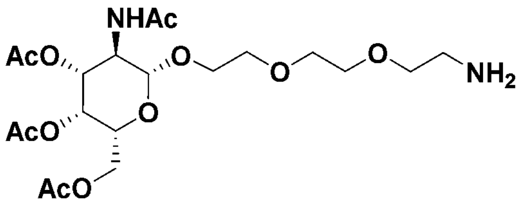 | GalNAc-AEEA | WO2020093053, 2020 |

2. Synthesis of key intermediate compound **A**

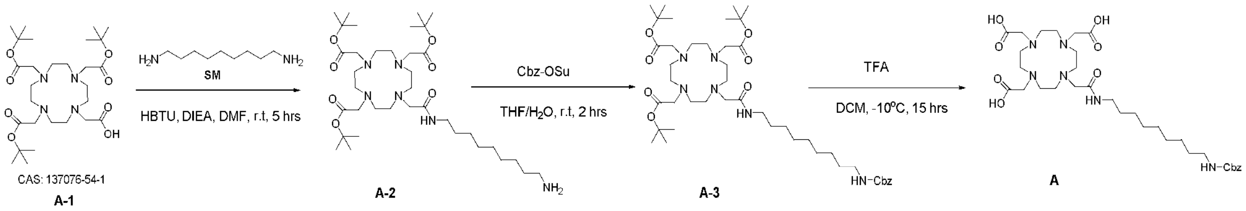


Synthesis of Compound **A-2**

Compound **A-1** (3.0 g, 5.24 mmol, 1.0 equiv.) was dissolved in DMF (20 mL), followed by sequential addition of HBTU (3.9 g, 10.5 mmol, 2.0 equiv.) and DIPEA (1.73 mL, 2.0 equiv.). The reaction mixture was stirred at room temperature for 5 minutes, whereupon 1,9-diaminononane (993 mg, 1.2 equiv.) dissolved in DMF (10 mL) was added. The reaction was stirred at room temperature for 5 hours. Upon reaction completion, the mixture was quenched with water (30 mL) and extracted with ethyl acetate (20 mL). The organic phase was sequentially washed with 5% aqueous sodium bicarbonate solution (20 mL) and saturated brine (10 mL), dried over anhydrous sodium sulfate, and concentrated to afford crude product **A-2** (3.65 g). This crude material was used directly in the subsequent reaction without further purification. LCMS: (ESI) $m/z$ = 714 $[M+H]^+$.

Synthesis of Compound **A-3**

The crude product **A-2** obtained in the previous step (3.65 g, 5.13 mmol) was dissolved in THF (10 mL), followed by sequential addition of Cbz-OSu (1.28 g, 5.13 mmol) and saturated aqueous sodium bicarbonate solution (10 mL). The mixture was stirred at room temperature for 2 hours. Upon reaction completion, the reaction mixture was diluted with water (20 mL) and extracted with ethyl acetate (3 × 20 mL). The combined organic phases were washed with saturated brine (20 mL), dried over anhydrous sodium sulfate, and concentrated. The resultant residue was purified by preparative column chromatography to afford **A-3** (1.1 g, yield: 25.6%) as a white solid. LCMS: (ESI) $m/z$ = 847.5 $[M+H]^+$.

HPLC purification conditions: column: Xbridge C18, 19 × 250 mm, 10 μm, 130 Å; mobile phase: A: water (containing 0.05% TFA), B: acetonitrile (containing 0.05% TFA); gradient program: ramp from 5% B to 95% B over 20 min.

Synthesis of Compound **A**

Intermediate **A-3** (1.1 g, 1.30 mmol) was dissolved in DCM (5 mL) and cooled to - 10°C using a low-temperature reactor. A mixture of TFA and DCM (15 mL, $v:v$ = 1:1) was slowly added. After complete addition, the reaction was stirred at -10°C for 15 hours. Upon reaction completion confirmed by LCMS, cold methyl tert-butyl ether (MTBE) (40 mL) was added, resulting in a white suspension. The mixture was centrifuged at 3200 rpm for 3 minutes, and the supernatant was decanted. The solid residue was resuspended in MTBE (40 mL), sonicated for 3 minutes, and recentrifuged (3200 rpm, 3 minutes). The supernatant was discarded, and the solid residue was dried to afford compound **A** (660 mg, yield: 74.9%) as a white solid. LCMS: (ESI) $m/z$ = 679 $[M+H]^+$.

**3.** Synthesis of **I-A** class molecules

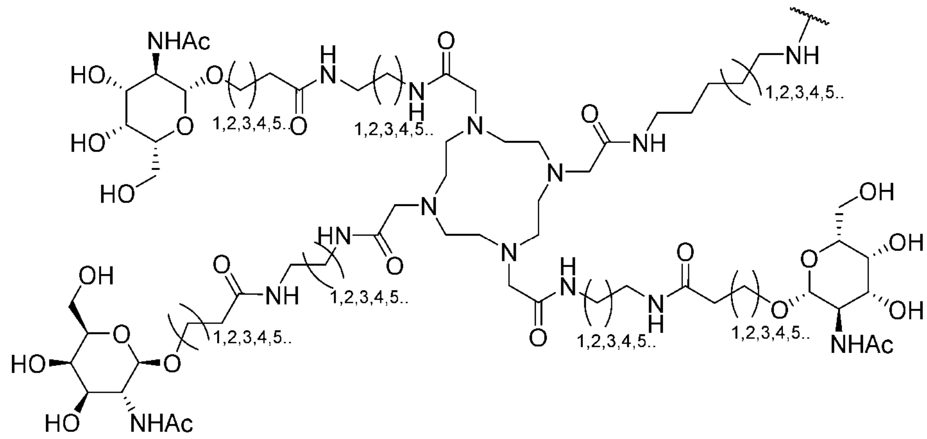

**3.1** Synthesis of **I-A10-5FAM**

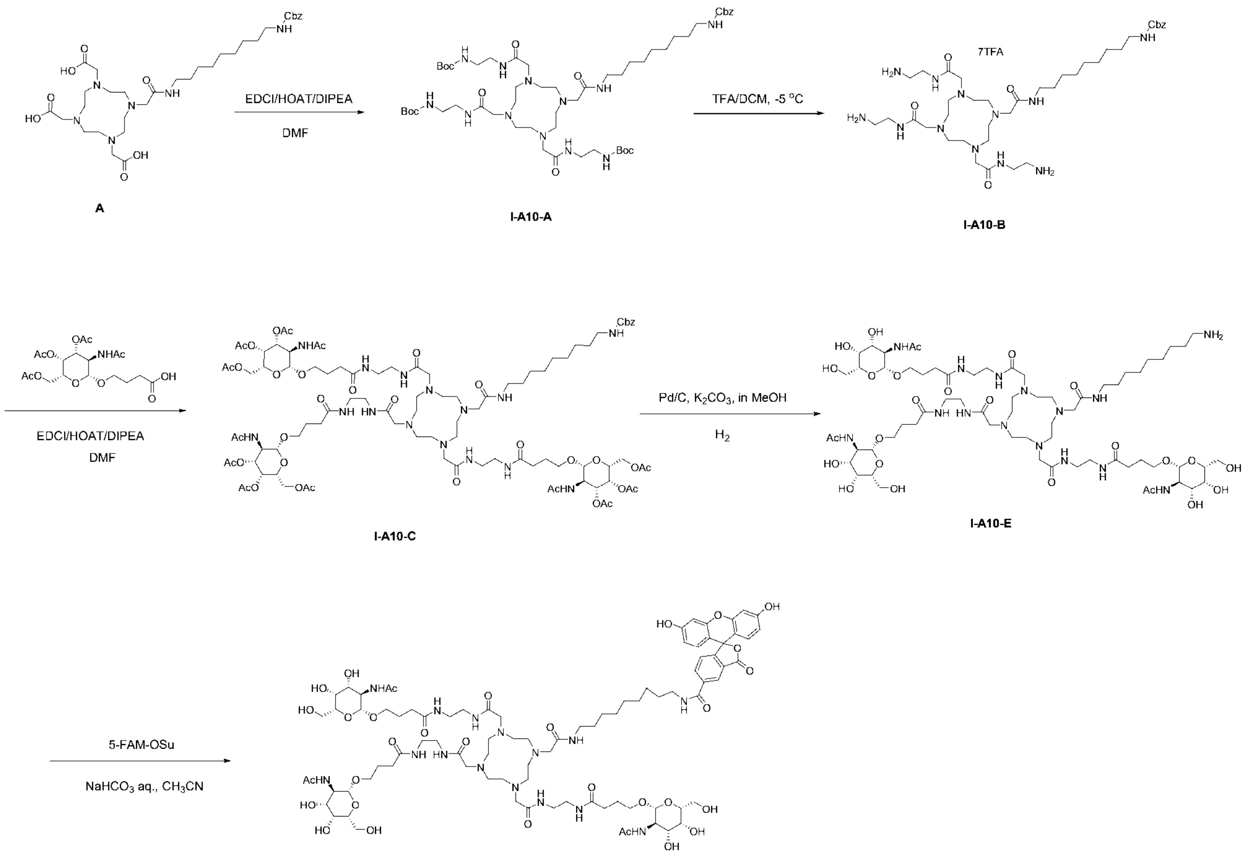


A

I-A10-A

I-A10-B

I-A10-C

I-A10-E

I-A10-5FAM

Synthesis of intermediate **I-A10-A**

1-tert-butoxycarbonylethylenediamine (71 mg, 0.442 mmol, 5.0 equiv.) and intermediate **A** (60 mg, 0.0884 mmol) were dissolved in DMF (3 mL), followed by sequential addition of EDCI·HCl (68 mg, 0.354 mmol), HOAt (48 mg, 0.354 mmol, 4.0 equiv.), and DIPEA (151 μL, 0.884 mmol). The reaction mixture was stirred at room temperature

overnight. The crude mixture was directly subjected to purification by preparative column chromatography to afford **I-A10-A** as a white solid (68 mg, yield: 68.0%). LCMS: (ESI) *m/z* = 1127.7 $[M+Na]^+$; 1105.7 $[M+H]^+$; 553.5 $[M+2H]/2^+$.

HPLC purification conditions: column: Welch Topsil C18, 21.2 × 250 mm, 5 μm, 150 Å; mobile phase: A: water (containing 0.05% TFA), B: acetonitrile (containing 0.05% TFA); gradient program: ramp from 20% B to 70% B over 20 min.

Synthesis of intermediate **I-A10-B**

**I-A10-A** (68 mg, 0.0435 mmol, 1.0 equiv.) was dissolved in DCM (3 mL) and cooled to -5 °C in an ice-salt bath. Trifluoroacetic acid (TFA, 3 mL) was added dropwise. The reaction mixture was stirred at -5°C for 2 hours. The solution was concentrated and dried to afford the trifluoroacetate salt of intermediate **I-A10-B** as a colorless transparent liquid (80 mg, yield: >100%). LCMS: (ESI) *m/z* = 805.7 $[M+H]^+$; 403.5 $[M+2H]/2^+$.

Synthesis of intermediate **I-A10-C**

Intermediate **I-A10-B** (80 mg, 0.0435 mmol) and **Gal-C4** (94 mg, 0.218 mmol) were dissolved in DMF (2 mL), followed by sequential addition of HOAt (30 mg, 0.218 mmol), EDCI-HCl (42 mg, 0.218 mmol), and DIPEA (74 μL, 0.435 mmol). The reaction mixture was stirred at room temperature overnight. Upon substantial reaction completion, the crude mixture was directly purified by preparative column chromatography to afford **I-A10-C** as a white solid (56 mg, yield: 62.7%). LCMS: (ESI) *m/z* = 1026.6 $[M+2H]/2^+$; 684.5 $[M+3H]/3^+$.

HPLC purification conditions: column: Xbridge C18 21.2 mm × 250 mm, 10 μm, 110 Å; mobile phase: A: water (containing 10 mmol/L $NH_4HCO_3$), B: acetonitrile; gradient program: ramp from 38% B to 48% B over 20 min.

Synthesis of intermediate **I-A10-E**

**I-A10-C** (25 mg, 0.0122 mmol) was dissolved in methanol (3 mL), followed by sequential addition of potassium carbonate (10 mg) and Pd/C (10% w/w on activated carbon, 5 mg). The mixture was stirred under a hydrogen atmosphere for 2 hours, with reaction progress monitored by LC-MS. The reaction mixture was filtered, and approximately 2 g of dry ice was added to the filtrate with stirring for 5 minutes. The mixture was then concentrated to afford crude **I-A10-E,** which was used directly in the subsequent reaction without further purification. LCMS: (ESI) *m/z* = 770.2 $[M+2H]/2^+$; 668.5 $[M+2H-204]/2^+$.

Synthesis of compound **I-A10-5FAM**

The crude product **I-A10-E** (0.0122 mmol) obtained in the previous step was dissolved in water (2 mL) and acetonitrile (0.5 mL), followed by sequential addition of 5-FAM-OSu (8 mg, 0.0165 mmol) and saturated aqueous sodium bicarbonate solution (0.5 mL). The reaction mixture was stirred at room temperature for 1 hour. The crude mixture was directly purified by preparative HPLC to afford **I-A10-5FAM** as an orange-yellow solid (10.0 mg, yield over two steps: 43.3%). LCMS: (ESI) *m/z* = 967.8 $[M+H+K]/2^+$; 949.3 $[M+2H]/2^+$; 645.8 $[M+2H+K]/3^+$;633.3 $[M+3H]/3^+$.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 14% B to 24% B over 20 min.

HPLC: 91.1578% (214 nm), RT = 13.049 min; 7.2549% (214 nm), RT = 13.406 min
Mobile phase: A: water (0.05%TFA), B: acetonitrile (0.05%TFA)
Gradient program: 5% B equilibration for 3 min, ramped to 65% B over 20 min, ramped to 95% B over 2 min, and 95% B for 5 min.
Flow rate: 1.0 mL/min
Column: XBridge peptide BEH column C18, 4.6 × 150 mm, 3.5 μm, 130 Å
Temperature: 60 °C

**3.2** Synthesis of **I-A11-5FAM**

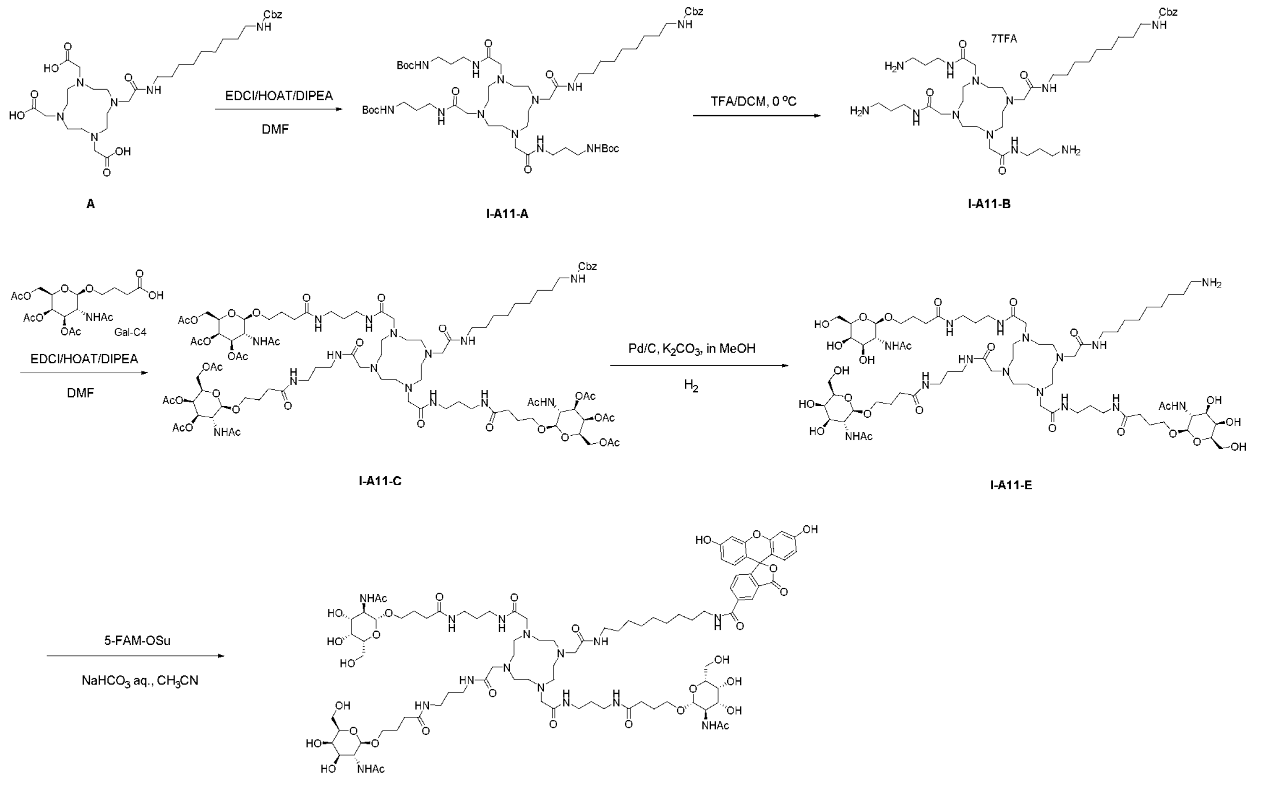


A

I-A11-A

I-A11-B

I-A11-C

I-A11-E

I-A11-5FAM

Synthesis of intermediate **I-A11-A**

Following the procedure for synthesizing intermediate **I-A10-A,** compound **A** (60 mg, 0.0884 mmol) was reacted with N-t-butyloxy carbonyl-propanediamine (73 mg, 0.442 mmol) in DMF (3 mL). After purification, **I-A11-A** was obtained as a white solid (70 mg, yield: 68.0%). LCMS: (ESI) *m/z* = 1147.8 $[M+H]^+$.

Synthesis of intermediate **I-A11-B**

Following the procedure for synthesizing intermediate **I-A10-B, I-A11-B** (80 mg, yield: quantitative) was obtained as a colorless oily liquid from **I-A11-A** (70 mg, 0.0436 mmol). LCMS: (ESI) *m/z* = 847.7 $[M+H]^+$.

Synthesis of intermediate **I-A11-C**

Following the procedure for synthesizing intermediate **I-A10-C, I-A11-C** (32 mg, yield: 35.1%) was obtained as a white solid from crude **I-A11-B.** LCMS: (ESI) *m/z* = 1047.7 $[M+2H]/2^+$.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 41% B to 51% B over 20 min.

Synthesis of intermediate **I-A11-E**

Following the procedure for synthesizing intermediate **I-A10-E, I-A11-E** (40 mg, yield: quantitative) was obtained as a white solid from **I-A11-C** (32 mg, 0.0156 mmol). LCMS: (ESI) *m/z* =791.0 $[M+2H]/2^+$.

Synthesis of compound **I-A11-5FAM**

Following the procedure for synthesizing **I-A10-5FAM, I-A11-5FAM** (6.3 mg, yield: 20.8%) was obtained as an orange solid from **I-A11-E** (40 mg). LCMS: (ESI) *m/z* = 989.2 $[M+H+K]/2^+$.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 13% B to 23% B over 20 min.

HPLC purity: 60.0611% (214 nm), RT = 12.894 min; 37.2783% (214 nm), RT = 13.256 min

Analysis conditions were the same as those for **I-A10-5FAM.**

3.3 Synthesis of **I-A12-5FAM**

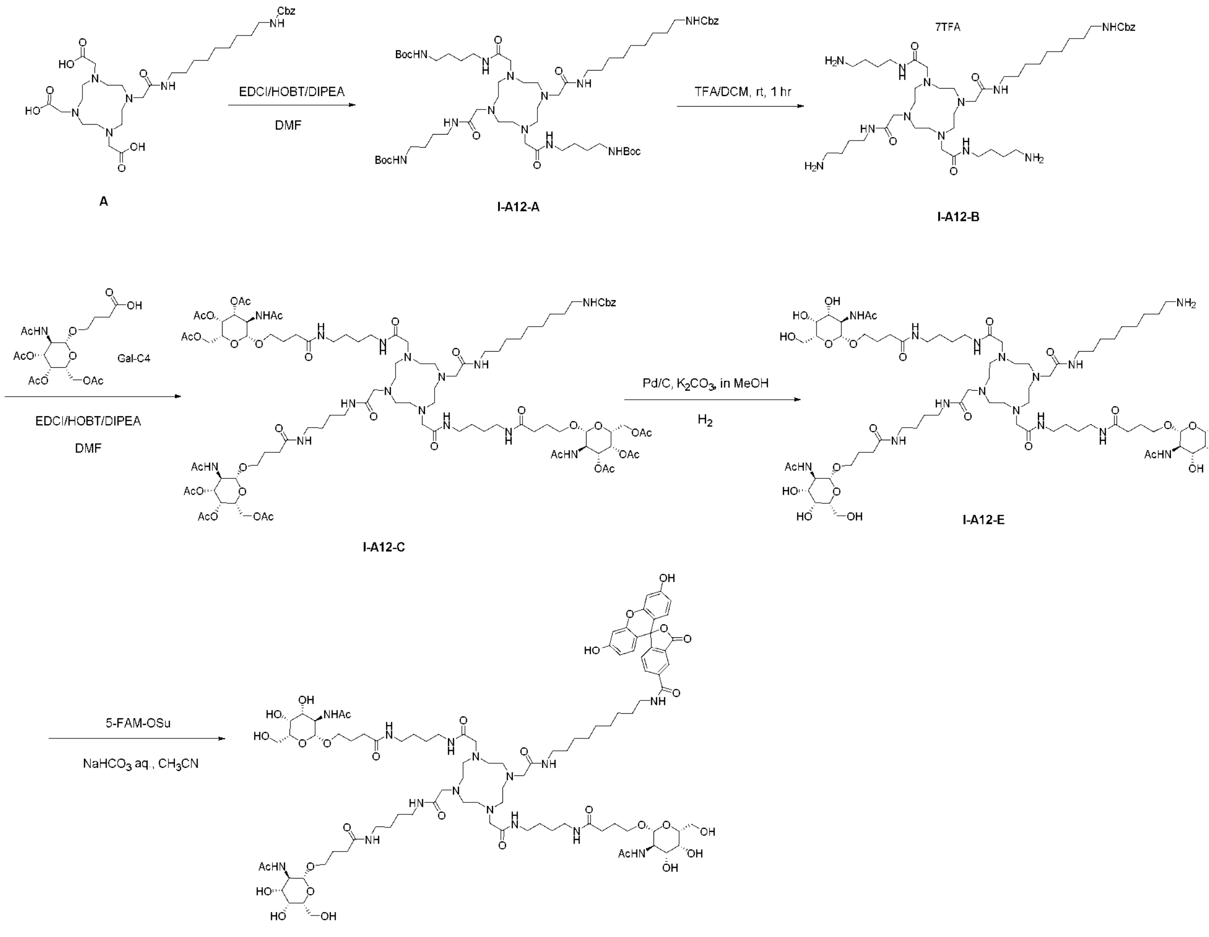


A

I-A12-A

I-A12-B

I-A12-C

I-A12-E

I-A12-5FAM

Synthesis of intermediate **I-A12-A**

Following the procedure for synthesizing intermediate **I-A10-A,** compound A (60 mg, 0.0884 mmol) was reacted with N-Boc-butanediamine (75 mg, 0.396 mmol) in DMF (2 mL). The product was purified to afford **I-A12-A** (73 mg, yield: 69.5%) as a white solid. LCMS: (ESI) *m/z* = 595.5 $[M+2H]/2^+$.

HPLC purification conditions: column: Xbridge C18, 19 × 250 mm, 10 μm, 130 Å; mobile phase: A: water (containing 0.05% TFA), B: acetonitrile (containing 0.05% TFA); gradient program: ramp from 20% B to 80% B over 20 min.

Synthesis of intermediate **I-A12-B**

**I-A12-A** (73 mg, 0.061 mmol) was dissolved in DCM (2 mL), followed by dropwise addition of TFA (2 mL). The mixture was reacted in room temperature for 1 hour and the reaction mixture was concentrated and dried to afford **I-A12-B** (53 mg yield: quantitative) as colorless oily liquid. LCMS: (ESI) *m/z* = 911.7 $[M+Na]^+$; 445.4 $[M+2H]/2^+$.

Synthesis of intermediate **I-A12-C**

**Gal-C4** (1.2 g, 2.77 mmol, 5.0 eq.) was dissolved in DMF (20 mL), followed by sequential addition of **I-A12-B** (900 mg, 0.553 mmol), EDCI (530 mg, 2.77 mmol), HOAt (376 mg, 2.77 mmol) and DIPEA (944 μL, 5.53 mmol). The reaction mixture was stirred at room temperature for 3 hours. Upon reaction completion as confirmed by LCMS, the reaction mixture was purified by preparative chromatographic column directly to afford **I-A12-C** (900 mg, yield: 76%).LCMS: (ESI) *m/z* =

1068.8 [M+2H]/2+; 903.8 [M+2H-330]/2; 712.8 [M+3H]/3+. $^1$H NMR (400 MHz, DMSO) δ 8.07-7.71 (m, 10H, 10NH), 7.39-7.19 (m, 6H, Bn-5H+1NH), 5.20 (d, J = 3.2 Hz, 3H, 3Gal-OCHO), 5.00 (s, 2H, Ph-CH2), 4.96 (dd, J = 3.2, 11.2 Hz, 3H, 3Gal-OCHC), 4.48 (d, *J* = 8.4 Hz, 3H, 3Gal-OCHC), 4.02 (brs, 9H), 3.92-3.80 (m, 3H, 3Gal-OCHC), 3.70-3.63 (m, 3H, 3Gal-OCHC), 3.47-3.39 (m, 3H, 3Gal-OCHC), 3.131-2.90 (m, 26H), 2.72-2.52 (m, 14H), 2.16-2.03 (m, 6H), 2.10 (s, 9H, 3CH3), 1.99 (s, 9H, 3CH3), 1.89 (s, 9H, 3CH3), 1.76 (s, 9H, 3CH3), 1.74-1.61 (m, 6H), 1.47-1.30 (m, 16H), 1.23 (brs, 10H).

HPLC purification conditions: column: Xbridge C18, 19 × 250 mm, 10 μm, 130 Å; mobile phase: A: water (containing 10 mmol/L $NH_4HCO_3$), B: acetonitrile; gradient program: ramp from 39% B to 49% B over 10 min.

Synthesis of intermediate **I-A12-E**

Following the procedure for synthesizing intermediate **I-A10-E, I-A12-E** (33 mg, quantitative) was obtained as a white solid from **I-A12-C** (30 mg, 0.0140 mmol). LCMS: (ESI) *m/z* = 812 [M+2H]/2+.

Synthesis of compound **I-A12-5FAM**

Following the procedure for synthesizing **I-A10-5FAM, I-A12-5FAM** (8.5 mg, yield: 21.5%) was obtained as an orange solid from **I-A12-E** (33 mg, 0.020 mmol). LCMS: (ESI) *m/z* = 991.0 [M+H+K]/2+; 674.0 [M+2H+K]/3+.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 5% B to 35% B over 10 min.

HPLC purity: 9.5195 % (214 nm), RT = 13.588 min; 87.8220 % (214 nm), RT = 13.008 min

Analysis conditions were the same as those for **I-A10-5FAM.**

**3.4** Synthesis of **I-A12-Cy5**

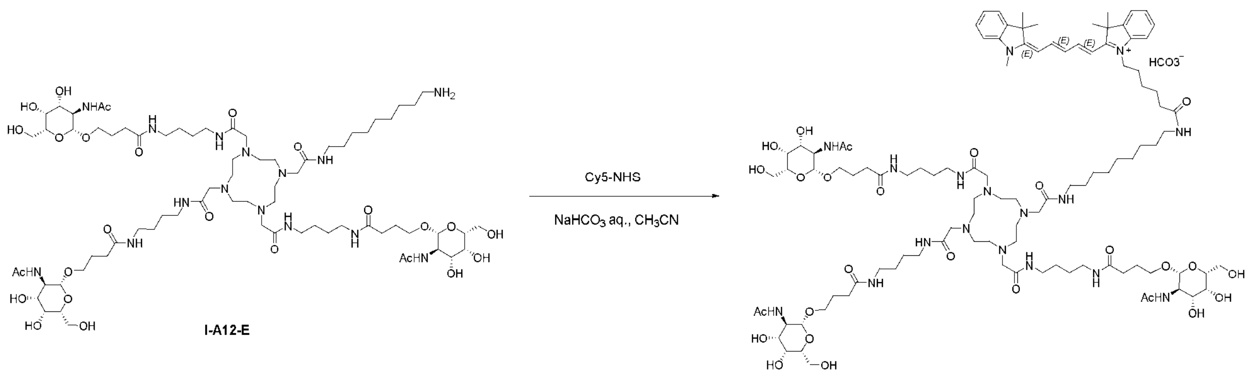


I-A12-E

I-A12-Cy5

Following the procedure for synthesizing **I-A12-5FAM, I-A12-Cy5** (8.0 mg, yield: 26.5%) was obtained as an orange solid from **I-A12-E** (30 mg, 0.020 mmol). LCMS: (ESI) *m/z* = 1044.5 [M+2H]/2+, 709.5 [M+3H+K]/3+, 696.8 [M+3H]/3+.

HPLC purification conditions: column: Gemini C18 21.2 mm × 250 mm, 10 μm, 110 Å; mobile phase: A: water (containing 10 mmol/L $NH_4HCO_3$), B: acetonitrile; gradient program: ramp from 5% B to 35% B over 10 min.

HPLC purity: 84.9 % (214 nm), RT = 17.535 min; 12.1 % (214 nm), RT = 17.945 min

**3.5** Synthesis of compound **I-A12-Ac-5FAM**

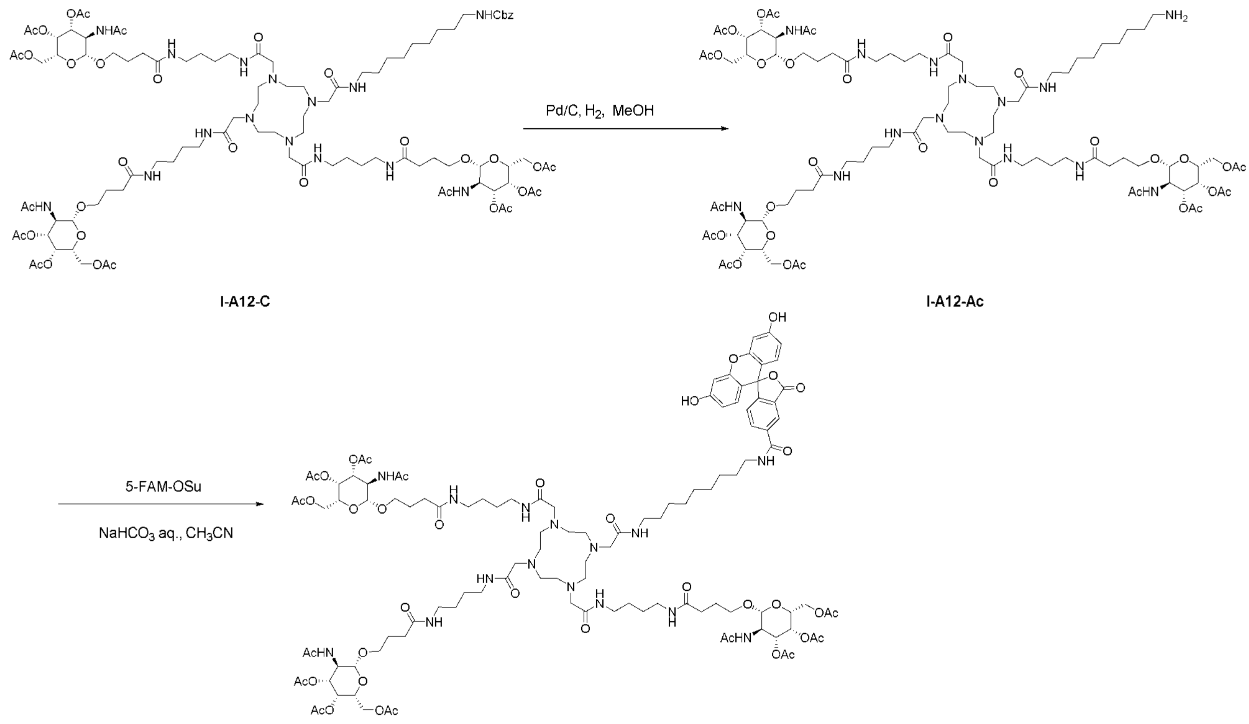


I-A12-C

I-A12-Ac

I-A12-Ac-5FAM

**I-A12-C** (40 mg, 0.0187 mmol) was dissolved in methanol (5 mL) followed by addition of Pd/C (10% w/w adsorbed on activated carbon, 8 mg). The mixture was stirred in hydrogen atmosphere for 13 hours and the reaction is monitored by LC-MS. The reaction mixture was filtered, concentrated and purified to afford **I-A12-Ac** (5 mg, yield: 13.3%) which was used directly for next step. LCMS: (ESI) *m/z* = 1001.7 $[M+2H]/2^+$; 680.7 $[M+2H+K]/3^+$.

The **I-A12-Ac** (5 mg, 0.00250 mmol) obtained in the previous step was dissolved in water (2 mL) and acetonitrile (0.5 mL), followed by subsequential addition of **5-FAM-OSu** (1.2 mg, 0.0165 mmmol) and saturated aqueous sodium bicarbonate solution (0.5 mL). The reaction mixture was stirred at room temperature for 1 hour and then purified by preparative column chromatography to afford **I-A12-Ac-5FAM** (3.5 mg, yield: 59.3%) as an orange solid. LCMS: (ESI) *m/z* = 800.4 $[M+2H+K]/3^+$; 787.5 $[M+3H]/3^+$.

HPLC purification conditions: preparative column: Gemini C18 21.2 mm × 250 mm, 10 μm, 110 Å; mobile phase: A: water (containing 10 mmol/L $NH_4HCO_3$), B: acetonitrile; gradient program: ramp from 35% B to 55% B over 20 min.

HPLC purity: 75.08% (214 nm), RT = 17.237 min; 18.82% (214 nm), RT = 17.738 min

Analysis conditions were the same as those for **I-A10-5FAM.**

**3.6** Synthesis of **I-A13-5FAM**

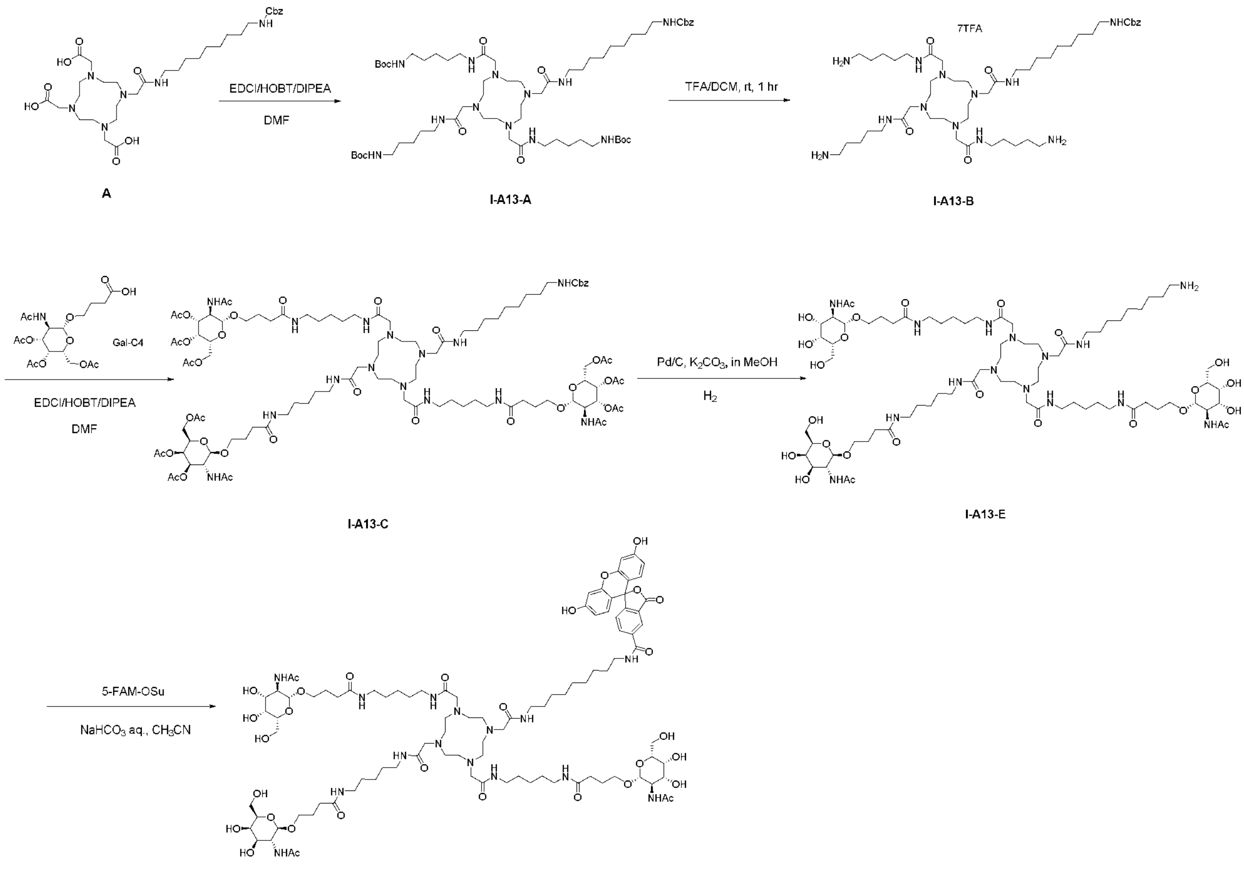


A

I-A13-A

I-A13-B

I-A13-C

I-A13-E

I-A13-5FAM

Synthesis of intermediate **I-A13-A**

Following the procedure for synthesizing intermediate **I-A10-A,** compound A (60 mg, 0.0884 mmol) was reacted with N-Boc-butanediamine (89 mg, 0.442 mmol) in DMF (2 mL) at room temperature for 2 hours. The reaction mixture was directly purified to afford **I-A13-A** (72 mg, yield: 66.7%) as a white solid. LCMS: (ESI) *m*/*z* = 1231.7 $[M+H]^+$; 616.3 $[M+2H]/2^+$.

HPLC purification conditions: preparative column: Xbridge C18, 19 × 250 mm, 10 μm, 130 Å; mobile phase: A: water (containing 10 mmol/L $NH_4HCO_3$), B: acetonitrile; gradient program: ramp from 33% B to 43% B over 20 min.

Synthesis of intermediate **I-A13-B**

Following the procedure for synthesizing intermediate **I-A12-B, I-A13-B** (65 mg, yield: quantitative) was obtained as colorless oily liquid from **I-A13-A** (72 mg, 0.058 mmol). LCMS: (ESI) *m*/*z* = 953.8 $[M+Na]^+$; 931.7 $[M+H]^+$; 466.5 $[M+2H]/2^+$.

Synthesis of intermediate **I-A13-C**

Following the procedure for synthesizing intermediate **I-A10-C, I-A13-C** (20 mg, yield: 13.4%) was obtained as a white solid from the crude **I-A13-B** as obtained in the previous step. LCMS: (ESI) *m*/*z* = 1089.4 $[M+2H]/2^+$; 924 $[M+2H-330]/2^+$.

HPLC purification conditions: preparative column: Xbridge C18, 19 × 250 mm, 10 μm, 130 Å; mobile phase: A: water (containing 10 mmol/L $NH_4HCO_3$), B: acetonitrile; gradient program: ramp from 43% B to 53% B over 10 min.

Synthesis of intermediate **I-A13-E**

Following the procedure for synthesizing intermediate **I-A10-E, I-A13-E** (20 mg, yield: quantitative) was obtained as a white solid from **I-A13-C** (20 mg, 0.0090 mmol). LCMS: (ESI) *m*/*z* = 833.0 $[M+2H]/2^+$; 731.4 $[M+2H-204]/2^+$.

Synthesis of intermediate **I-A13-5FAM**

Following the procedure for synthesizing **I-A10-5FAM, I-A13-5FAM** (10.0 mg, yield: 29.4%) was obtained as an orange solid from **I-A13-E** (20 mg, 0.0090 mmol). LCMS: (ESI) *m*/*z* = 1031 [M+H+K]/2+; 1012.7 [M+2H]/2+; 687.0 [M+2H+K]/3+; 675.3 [M+3H]/3+.

HPLC purification conditions: preparative column: Gemini C18 21.2 mm × 250 mm, 10 μm, 110 Å; mobile phase: A: water (containing 10 mmol/L $NH_4HCO_3$), B: acetonitrile; gradient program: ramp from 5% B to 35% B over 10 min.

HPLC purity: 24.2020 % (214 nm), RT = 14.195 min; 71.6996 % (214 nm), RT = 13.440 min

Analysis conditions were the same as those for **I-A10-5FAM.**

**3.7** Synthesis of **I-A12c-5FAM**

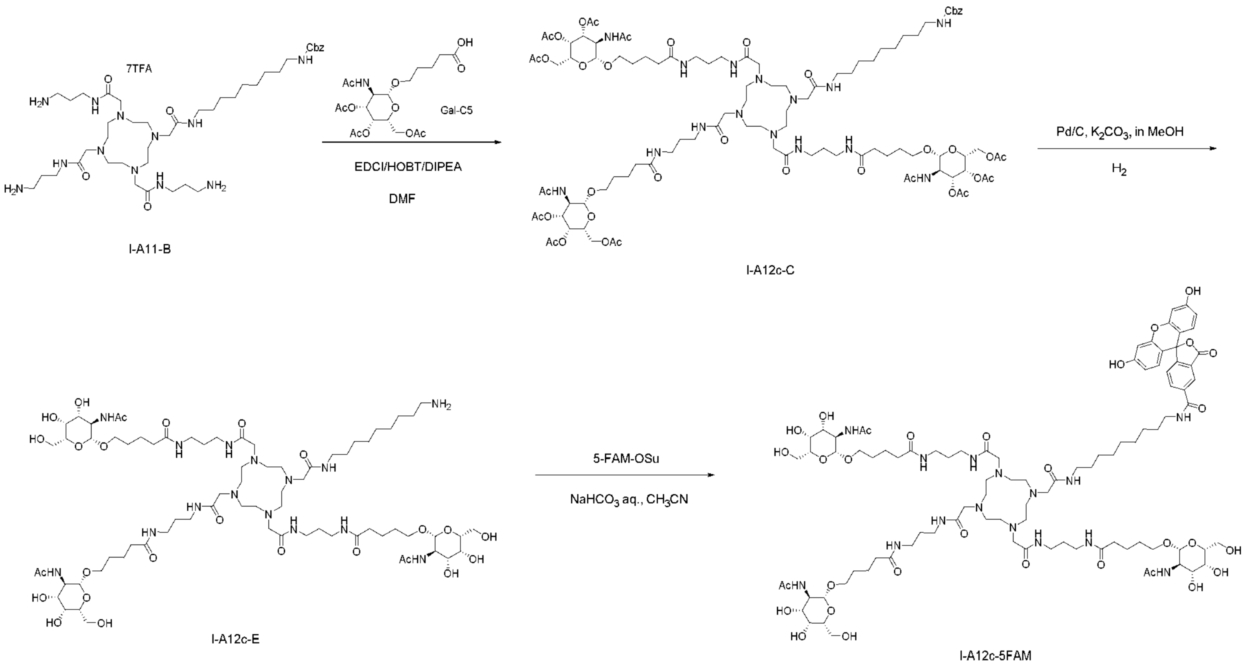


I-A11-B

I-A12c-C

I-A12c-E

I-A12c-5FAM

Following the procedure for synthesizing **I-A11-5FAM,** 4 mg of **I-A12c-5FAM** was obtained from compound **I-A11-B** by 3 steps.

LCMS: (ESI) *m*/*z* = 673.8 [M+2H+K]/3+, 661.5 [M+3H]/3+.

HPLC purity: 85.13% (214 nm), RT = 13.218 min (M); 14.87% (214 nm), RT = 13.579 min (M+K). Analysis conditions were the same as those for **I-A10-5FAM.**

**3.8** Synthesis of **I-A12d-5FAM**

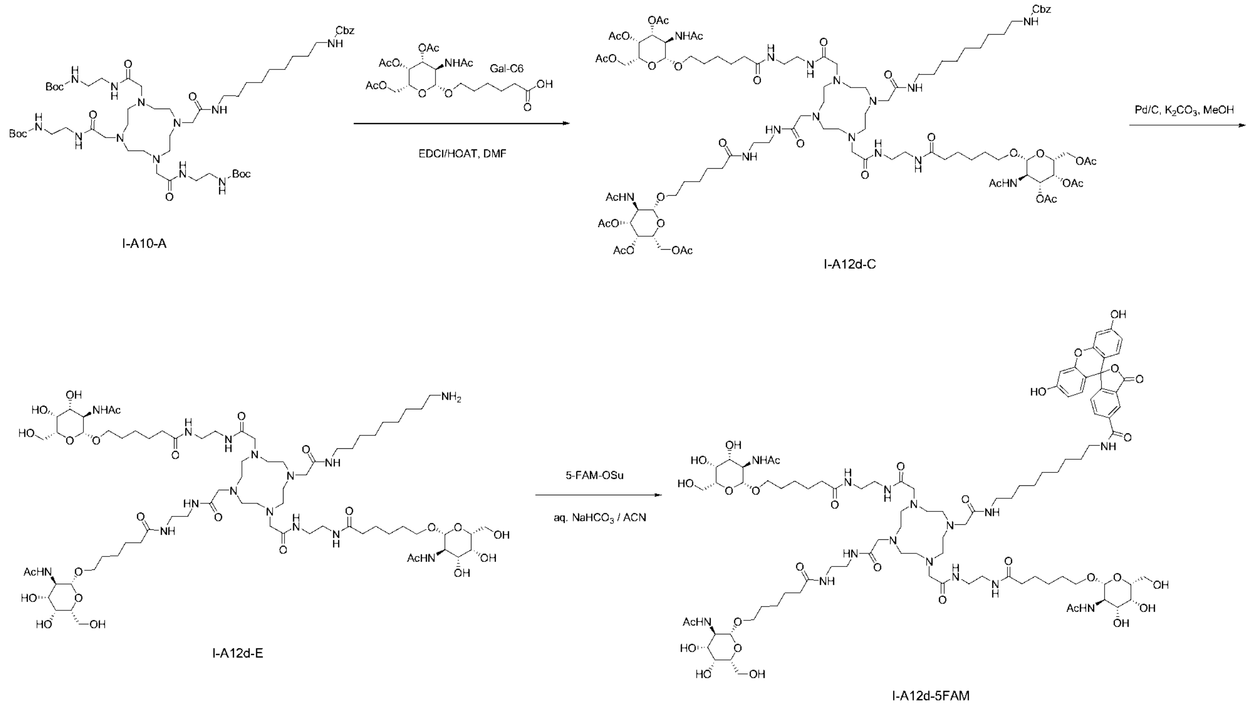


Following the procedure for synthesizing **I-A10-5FAM,** 6 mg of **I-A12d-5FAM** was obtained from compound **I-A10-A** by 3 steps.

LCMS: (ESI) *m/z* = 991.5 $[M+2H]/2^+$, 674.2 $[M+2H+K]/3^+$, 661.4 $[M+3H]/3^+$.

HPLC purity: 89.03% (214 nm), RT = 13.09 min (M); 7.83% (214 nm), RT = 13.48 min (M+K).

Analysis conditions were the same as those for **I-A10-5FAM.**

**4.** Synthesis of **I-B** class molecules

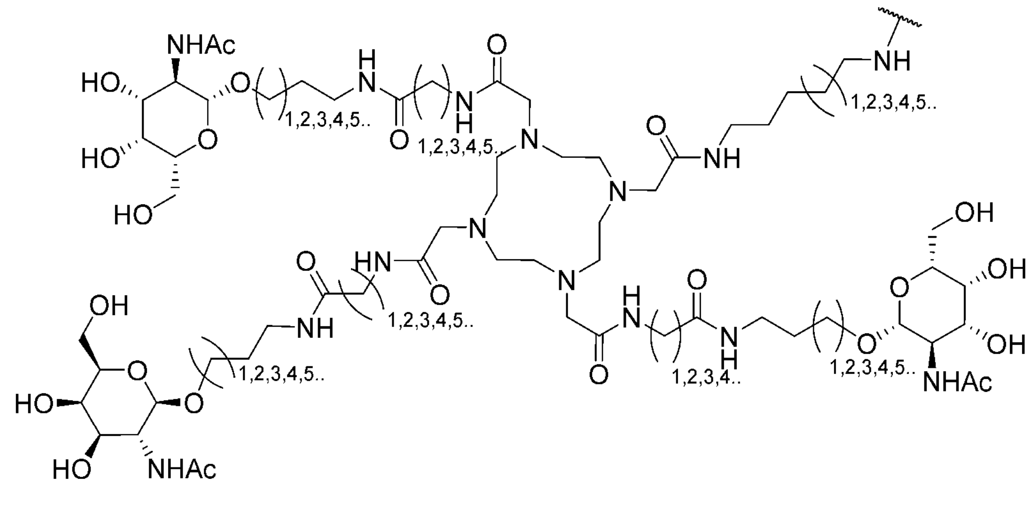

**4.1** Synthesis of **I-B11-5FAM**

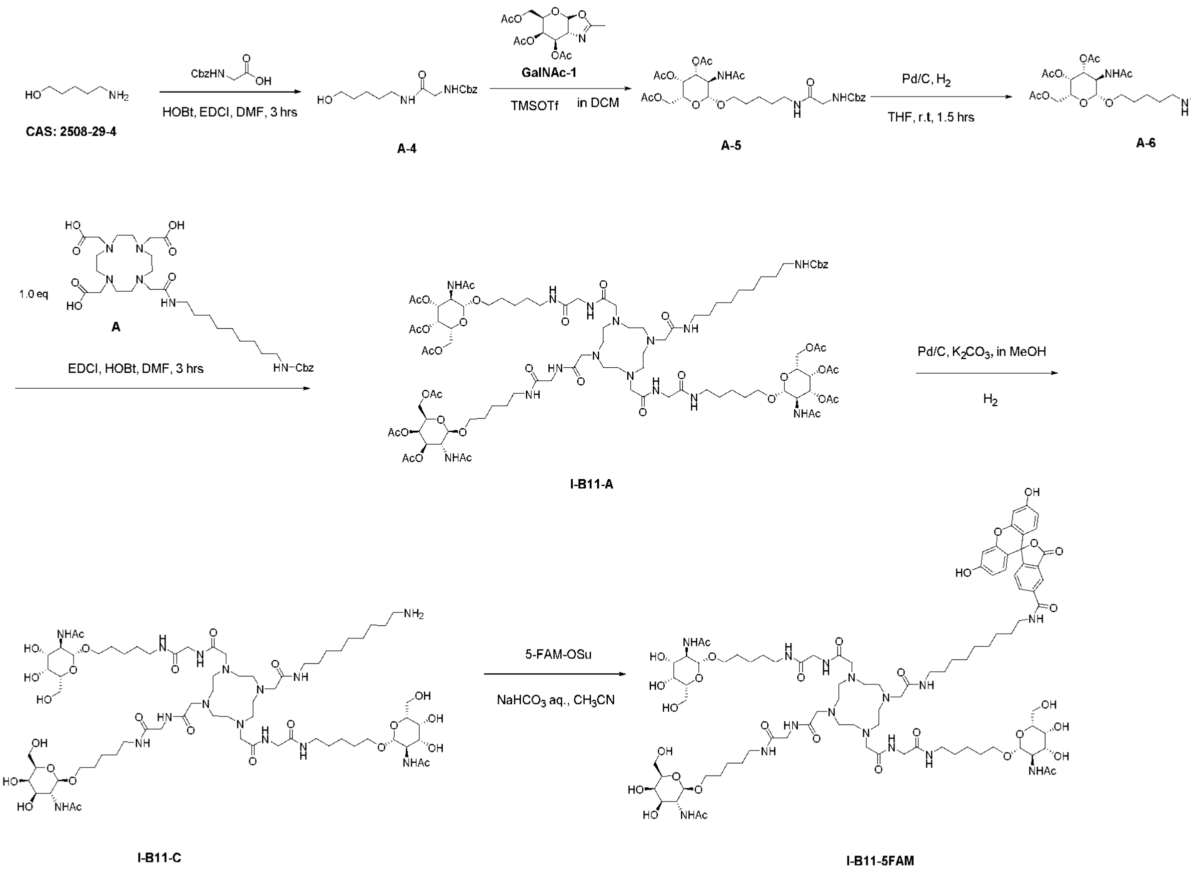

CAS: 2508-29-4 A-4 A-5 A-6 A I-B11-A I-B11-C I-B11-5FAM

Synthesis of intermediate **A-4**

5-aminopentan-1-ol (5.0 g, 23.9 mmol) was dissolved in DMF (50 mL), followed by sequential addition of N-Cbz-glycine (2.96 g, 28.7 mmol), HOBt (4.84 g, 35.9 mmol), EDCI (6.9 g, 35.9 mmol), and DIPEA (14.7 mL, 81.1 mmol). The mixture was stirred at room temperature for 3 h. The reaction was quenched with $H_2O$ (50 mL) and extracted with ethyl acetate (60 mL). The organic layer was washed with 5% aqueous $NaHCO_3$ (40 mL), dried over anhydrous $Na_2SO_4$, and concentrated. The crude product was purified by reversed-phase column chromatography to afford compound **A-4** (6.3 g, 90% yield) as a white solid. LCMS: (ESI) *m/z* = 295.1 $[M+H]^+$.

Purification conditions: column: 120 g C18; mobile phase: A: water (containing 10 mmol/L $NH_4HCO_3$), B: acetonitrile; gradient program: ramp from 5% B to 95% B over 20 min.

Synthesis of intermediate **A-5**

In a 100 mL three-necked flask, activated 4 Å molecular sieves (3.0 g), freshly prepared **GalNAc-1** (2.0 g, 6.079 mmol), and intermediate **A-4** (1.96 g, 6.687 mmol) were added. The system was purged with $N_2$ (three cycles), followed by addition of anhydrous 1,2-dichloroethane (20 mL). After stirring at room temperature for 10 min, TMSOTf (110 μL, 0.608 mmol) was added, and the mixture was stirred at room temperature for 3 h. The reaction mixture was filtered to remove molecular sieves. The filtrate was quenched with saturated aqueous $NaHCO_3$ (15 mL), and the organic layer was separated. The aqueous phase was extracted with DCM (20 mL × 2). The combined organic phases were dried over anhydrous $Na_2SO_4$ and concentrated. The crude product was purified by reversed-phase column chromatography to afford intermediate **A-5** (400 mg, yield 10.5%) as a white solid. LCMS: (ESI) *m/z* = 624.1 $[M+H]^+$.

Purification conditions: column: 120 g C18; mobile phase: A: water (containing 10 mmol/L $NH_4HCO_3$), B: acetonitrile; gradient program: ramp from 20% B to 60% B over 20 min.

Synthesis of intermediate **A-6**

In a 50 mL round-bottom flask, intermediate A-5 (400 mg, 0.642 mmol) and Pd/C (10%, 40 mg) were added. The system was purged with $H_2$ (twice), followed by addition of THF (5 mL). The mixture was stirred under $H_2$ atmosphere at room temperature for 90 min. The reaction mixture was filtered and concentrated to afford the crude product **A-6** (320 mg)

as a pale black oily liquid, which was directly used in the next step. LCMS: (ESI) *m/z* = 490.1 $[M+H]^+$.

Synthesis of intermediate **I-B11-A**

Compound **A** (50 mg, 0.074 mmol) was dissolved in DMF (2 mL), followed by sequential addition of HOBt (35 mg, 0.258 mmol), EDCI (45 mg, 0.258 mmol), DIPEA (88 μL, 0.516 mmol), and intermediate **A-6** (126 mg, 0.258 mmol). The mixture was stirred at room temperature for 3 h. The reaction mixture was directly purified by preparative reversed-phase column chromatography to afford **I-B11-A** (50 mg, yield 32.3%) as a white solid. LCMS: (ESI) *m/z* = 1047.0 $[M+2H]/2^+$.

Preparation conditions: HPLC preparative column: Xbridge C18, 19 × 250 mm, 10 μm, 130 Å; mobile phase: A: water (containing 10 mmol/L $NH_4HCO_3$), B: acetonitrile; gradient program: ramp from 40% B to 50% B over 10 min.

Synthesis of intermediate **I-B11-C**

In a 50 mL round-bottom flask, **I-B11-A** (50 mg, 0.024 mmol), $K_2CO_3$ (10 mg, 0.150 mmol), and Pd/C (10%, 12 mg) were sequentially added. Methanol (5 mL) was then introduced, and the mixture was stirred under $H_2$ atmosphere for 3 h. The reaction mixture was filtered and concentrated to afford **I-B11-C** (40 mg, crude product) as a white solid. LCMS: (ESI) *m/z* = 791.0 $[M+2H]/2^+$.

Synthesis of compound **I-B11-5FAM**

The crude product **I-B11-C** obtained from the previous step (40 mg, 0.024 mmol) was dissolved in a mixture of water (2 mL) and acetonitrile (0.5 mL), followed by sequential addition of SFAM-OSu (14 mg, 0.030 mmol) and saturated aqueous $NaHCO_3$ (0.5 mL). The mixture was stirred at room temperature for 60 min. The reaction mixture was directly purified by preparative chromatography to afford **I-B11-5FAM** (9.5 mg, yield 19.8%) as an orange-yellow solid. LCMS: (ESI) *m/z* = 989 $[M+H+K]/2^+$.

Preparation conditions: HPLC preparative column: Gemini C18 21.2 mm × 250 mm, 10 μm, 110 Å; mobile phase: A: water (containing 10 mmol/L $NH_4HCO_3$), B: acetonitrile; gradient program: ramp from 17% B to 27% B over 20 min.

HPLC purity: 9.5962% (214 nm), RT = 13.531 min; 88.8773% (214 nm), RT = 13.301 min

Analysis conditions were the same as those for **I-A10-5FAM.**

**4.2** Synthesis of **I-B12-5FAM**

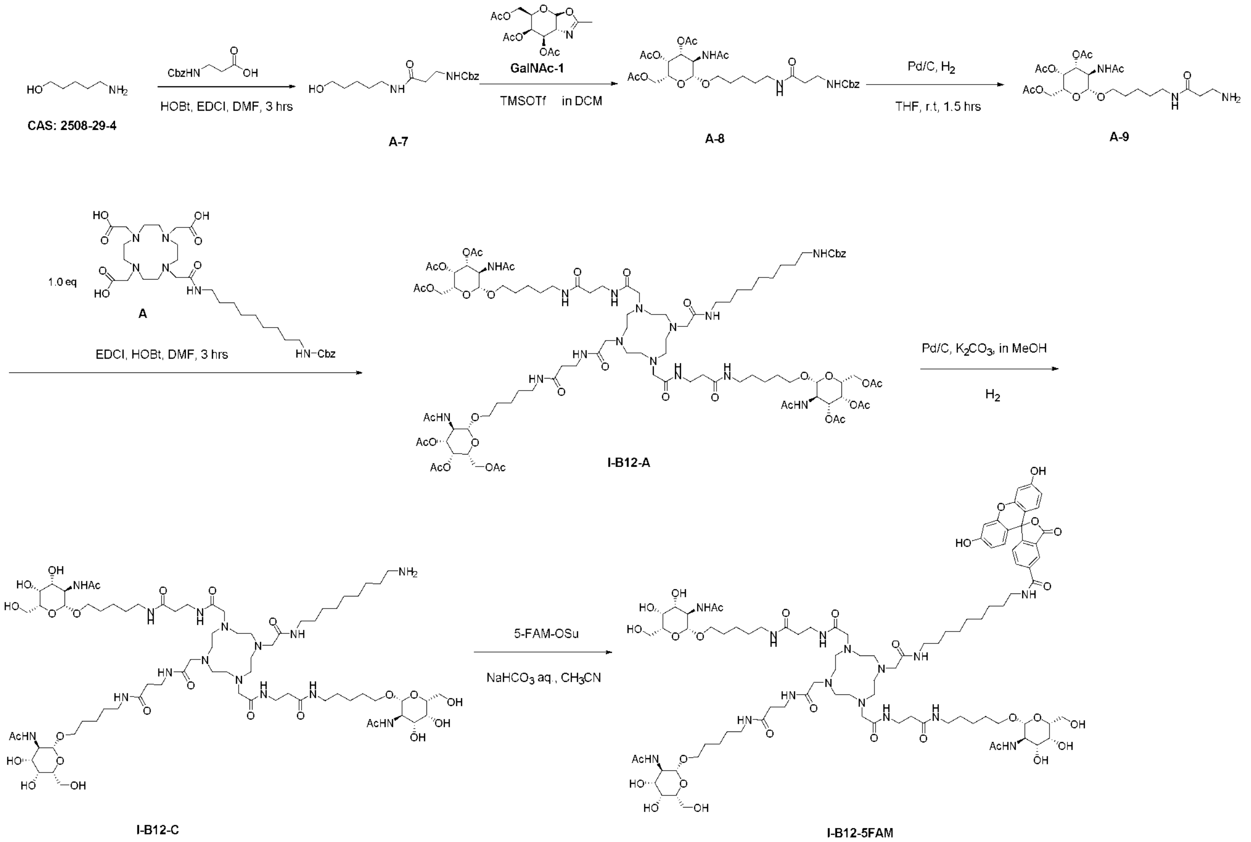


Synthesis of intermediate **A-7**

5-aminopentan-1-ol (5.0 g, 22.4 mmol) was dissolved in DMF (50 mL), followed by sequential addition of N-Cbz-β alanine (2.77 g, 26.9 mmol), HOBt (4.54 g, 33.6 mmol), EDCI (6.46 g, 33.6 mmol), and DIPEA (18.3 mL, 101. mmol). The mixture was stirred at room temperature for 3 h. The reaction was quenched with $H_2O$ (50 mL) and extracted with ethyl acetate (60 mL). The organic layer was washed with 5% aqueous $NaHCO_3$ (40 mL), dried over anhydrous $Na_2SO_4$, and concentrated. The crude product was purified by reversed-phase column chromatography to afford compound **A-7** (7.0 g, 92% yield) as a white solid. LCMS: (ESI) *m*/*z* = 309.1 $[M+H]^+$.

Purification conditions: column: 120 g C18; mobile phase: A: water (containing 10 mmol/L $NH_4HCO_3$), B: ACN; gradient program: ramp from 5% B to 95% B over 20 min.

Synthesis of intermediate **A-8**

In a 100 mL three-necked flask, activated 4 Å molecular sieves (3.0 g), DCM (20 mL), freshly prepared **GalNAc-1** (2.0 g, 6.08 mmol), and intermediate **A-7** (2.0 g, 6.69 mmol) were added. After stirring at room temperature for 10 min, TMSOTf (110 μL, 0.608 mmol) was added, and the mixture was stirred at room temperature for 3 h. The reaction mixture was filtered to remove molecular sieves. The filtrate was quenched with saturated aqueous $NaHCO_3$ (15 mL), and the organic layer was separated. The aqueous phase was extracted with DCM (20 mL × 2). The combined organic phases were dried over anhydrous $Na_2SO_4$ and concentrated. The crude product was purified by reversed-phase column chromatography to afford intermediate **A-8** (2.0 g, yield 51.7%) as a white solid. LCMS: (ESI) *m*/*z* = 638.2 $[M+H]^+$.

Purification conditions: column: 120 g C18; mobile phase: A: water (containing 10 mmol/L $NH_4HCO_3$), B: acetonitrile; gradient program: ramp from 5% B to 65% B over 20 min.

Synthesis of intermediate **A-9**

In a 50 mL round-bottom flask, intermediate **A-8** (400 mg, 0.628 mmol) and Pd/C (10%, 40 mg) were added. The system was purged with $H_2$ (twice), followed by addition of THF (5 mL). The mixture was stirred under $H_2$ atmosphere at room temperature for 90 min. The reaction mixture was filtered and concentrated to afford the crude product **A-9** (320 mg) as a pale black oily liquid, which was directly used in the next step. LCMS: (ESI) *m*/*z* = 504.1 $[M+H]^+$.

Synthesis of intermediate **I-B12-A**

Compound **A** (45 mg, 0.066 mmol) was dissolved in DMF (2 mL), followed by sequential addition of HOBt (31 mg, 0.232 mmol), EDCI (45 mg, 0.232 mmol), DIPEA (79 μL, 0.464 mmol), and intermediate **A-9** (117 mg, 0.232 mmol). The mixture was stirred at room temperature for 3 h. The reaction mixture was directly purified by preparative reversed-phase column chromatography to afford **I-B12-A** (72 mg, yield 51.1%) as a white solid. LCMS: (ESI) *m/z* = 1068.4 [M+2H]/2$^+$.
Preparation conditions: HPLC preparative column: Xbridge C18, 19 × 250 mm, 10 μm, 130 Å; mobile phase: A: water (containing 10 mmol/L $NH_4HCO_3$), B: acetonitrile; gradient program: ramp from 39% B to 49% B over 10 min.

Synthesis of intermediate **I-B12-C**

In a 50 mL round-bottom flask, **I-B12-A** (72 mg, 0.034 mmol), $K_2CO_3$ (28 mg, 0.202 mmol), and Pd/C (10%, 5 mg) were sequentially added. Methanol (2 mL) was then introduced, and the mixture was stirred under $H_2$ atmosphere for 3 h. The reaction mixture was filtered and concentrated to afford **I-B11-C** (60 mg, crude product) as a white solid. LCMS: (ESI) *m/z* = 812.1 [M+2H]/2$^+$; 711.0 [M+2H]/2$^+$; 541.8 [M+3H]/3$^+$.

Synthesis of compound **I-B12-5FAM**

The crude product **I-B12-C** obtained from the previous step (40 mg, 0.025 mmol) was dissolved in a mixture of water (1.2 mL) and acetonitrile (0.3 mL), followed by sequential addition of SFAM-OSu (14 mg, 0.029 mmol) and saturated aqueous $NaHCO_3$ (0.3 mL). The mixture was stirred at room temperature for 60 min. The reaction mixture was directly purified by preparative chromatography to afford **I-B11-5FAM** (12.5 mg, yield 25.3%) as an orange-yellow solid. LCMS: (ESI) *m/z* = 1010.3 [M+H+K]/2$^+$; 991.0 [M+2H]/2$^+$; 673.7 [M+2H+K]/3$^+$; 661.3 [M+3H]/3$^+$.
Preparation conditions: HPLC preparative column: Gemini C18 21.2 mm × 250 mm, 10 μm, 110 Å; mobile phase: A: water (containing 10 mmol/L $NH_4HCO_3$), B: acetonitrile; gradient program: ramp from 17% B to 27% B over 20 min.
HPLC purity: 93.1525 % (214 nm), RT = 13.435 min; 4.4401 % (214 nm), RT = 13.806 min
Analysis conditions were the same as those for **I-A10-5FAM.**

**4.3** Synthesis of **I-B12-Cy5**

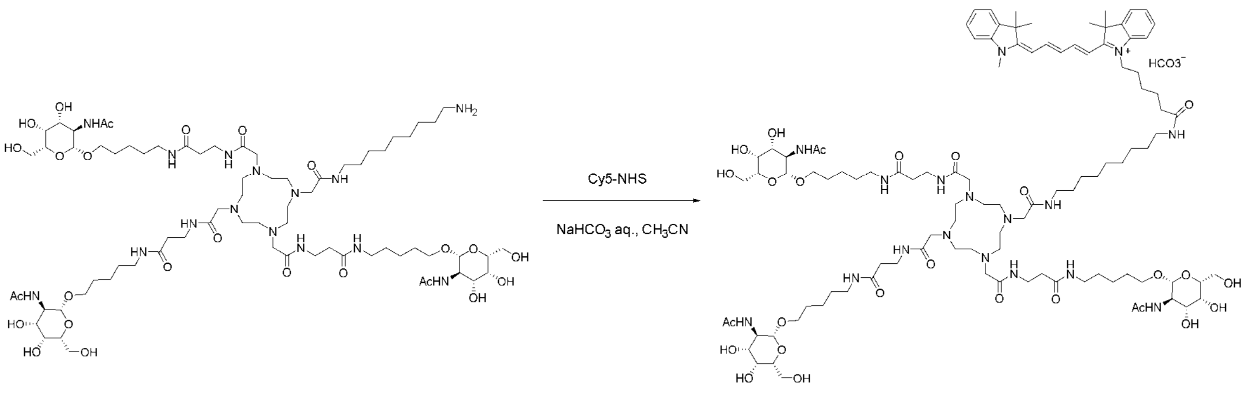


I-B12-C I-B12-Cy5

Following the procedure for synthesizing **I-A12-Cy5,** starting from **I-B12-E** (0.0124 mmol), **I-B12-Cy5** (5.0 mg, yield 19.2%) was obtained as a deep blue solid. LCMS: (ESI) *m/z* = 1045.3 [M$^+$+H]/2$^+$; 697.0 [M$^+$+2H]/3$^+$.
HPLC purification conditions: column: Xbridge C18, 19 × 250 mm, 10 μm, 130 Å; mobile phase: A: water (containing 0.05% TFA), B: acetonitrile (containing 0.05% TFA); gradient program: ramp from 31% B to 41% B over 20 min.
HPLC purity: 90.9250 % (214 nm), RT = 17.723 min (M); 8.5560 % (214 nm), RT = 17.878 min (M+K).

**4.3** Synthesis of **I-B12-Cy5**

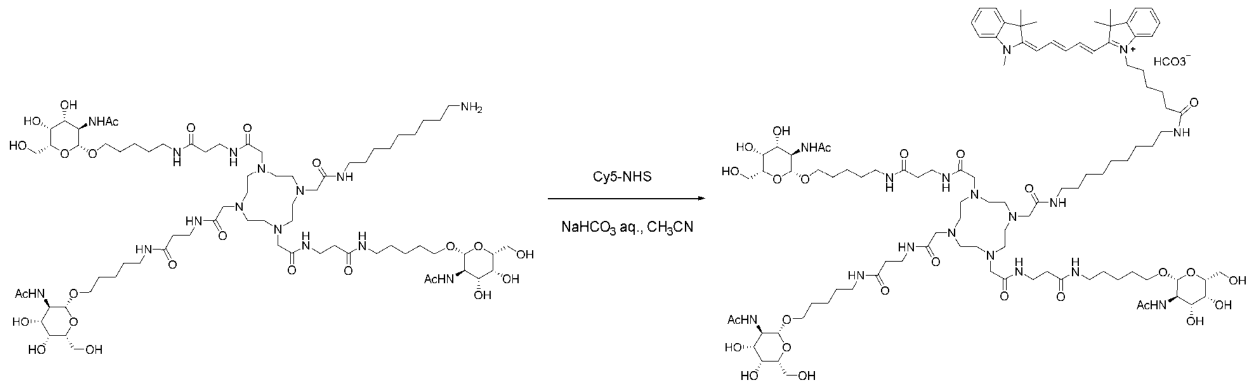


I-B12-C

I-B12-Cy5

Following the procedure for synthesizing **I-A12-Cy5,** starting from **I-B12-E** (0.0124 mmol), **I-B12-Cy5** (5.0 mg, yield 19.2%) was obtained as a deep blue solid. LCMS: (ESI) *m/z* = 1045.3 [M$^+$+H]/2$^+$; 697.0 [M$^+$+2H]/3$^+$.

HPLC purification conditions: column: Xbridge C18, 19 × 250 mm, 10 μm, 130 Å; mobile phase: A: water (containing 0.05% TFA), B: acetonitrile (containing 0.05% TFA); gradient program: ramp from 31% B to 41% B over 20 min.

HPLC purity: 90.9250 % (214 nm), RT = 17.723 min (M); 8.5560 % (214 nm), RT = 17.878 min (M+K).

**4.4** Synthesis of **I-B12b-5FAM**

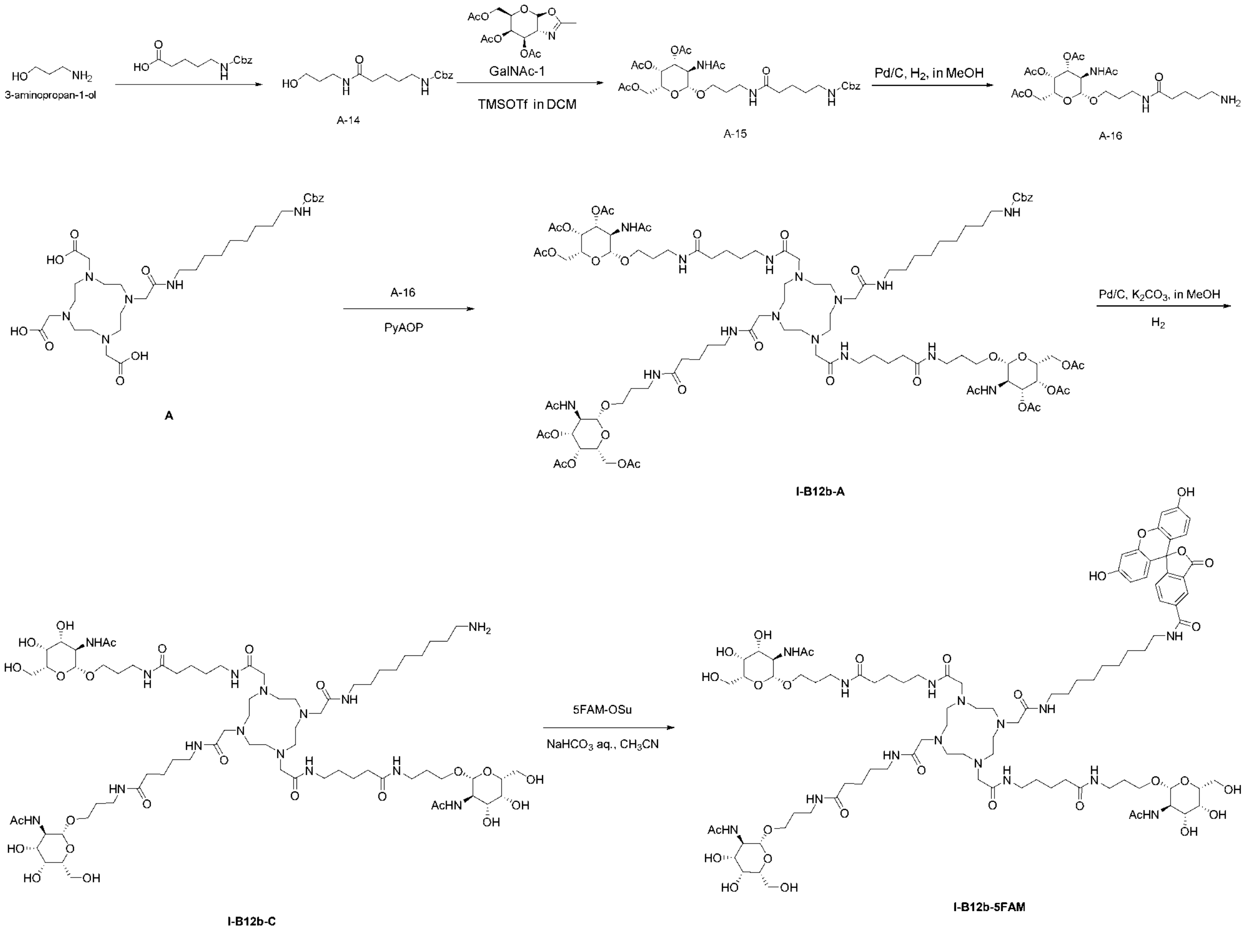


A-14

A-15

A-16

A

I-B12b-A

I-B12b-C

I-B12b-5FAM

The synthesis of intermediate **A16** was performed analogously to that for **A6** and **A9,** starting from 3-aminopropan-1-ol and proceeding through a three-step reaction sequence to afford the target compound. LCMS: (ESI) *m/z* = 504.4 [M+H]$^+$.

Following the procedure for synthesizing **I-B12-5FAM,** 4 mg **I-B12b-5FAM** was obtained starting from 5 mg **A.**

LCMS: (ESI) *m/z* = 991.5 [M+2]/2$^+$, 674.1 [M+2H+K]/3$^+$, 661.5 [M+3H]/3$^+$.

HPLC purity: 79.97% (214 nm), RT = 13.063 min (M); 18.32% (214 nm), RT = 13.685 min (M+K).

Analysis conditions were the same as those for **I-A10-5FAM.**

**4.5** Synthesis of **I-B12c-5FAM**

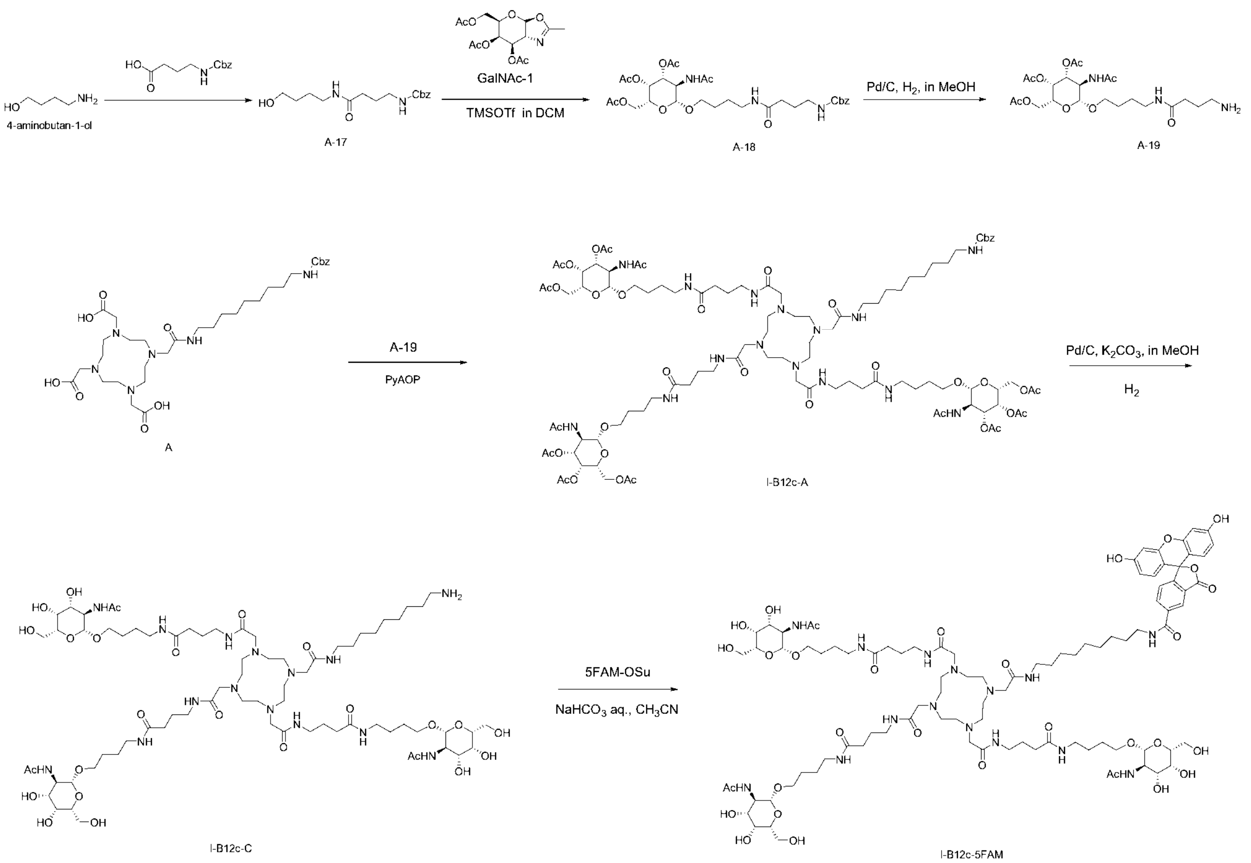


The synthesis of intermediate **A19** was performed analogously to that for **A6** and **A9,** starting from 3-aminopropan-1-ol and proceeding through a three-step reaction sequence to afford the target compound. LCMS: (ESI) *m/z* = 504.4 [M+H]$^+$.

Following the procedure for synthesizing **I-B12-5FAM,** 10 mg **I-B12c-5FAM** was obtained starting from 55 mg **A.**

LCMS: (ESI) *m/z* = 991.6 [M+2H]/2$^+$, 674.2 [M+2H+K]/3$^+$, 661.4 [M+3H]/3$^+$.

HPLC purity: 93.44% (214 nm), RT = 13.463 min (M); 5.00% (214 nm), RT = 13. 859 min (M+K).

Analysis conditions were the same as those for **I-A10-5FAM.**

**5.** Synthesis of **I-C** class molecules

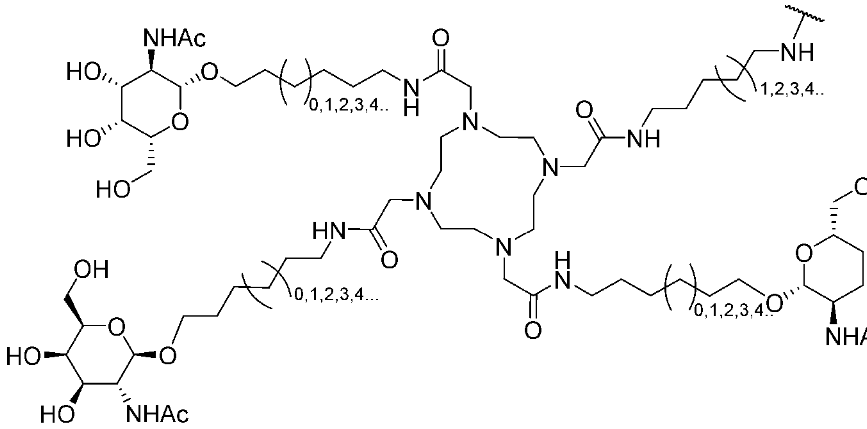

**5.1** Synthesis of compound **I-C10-5FAM**

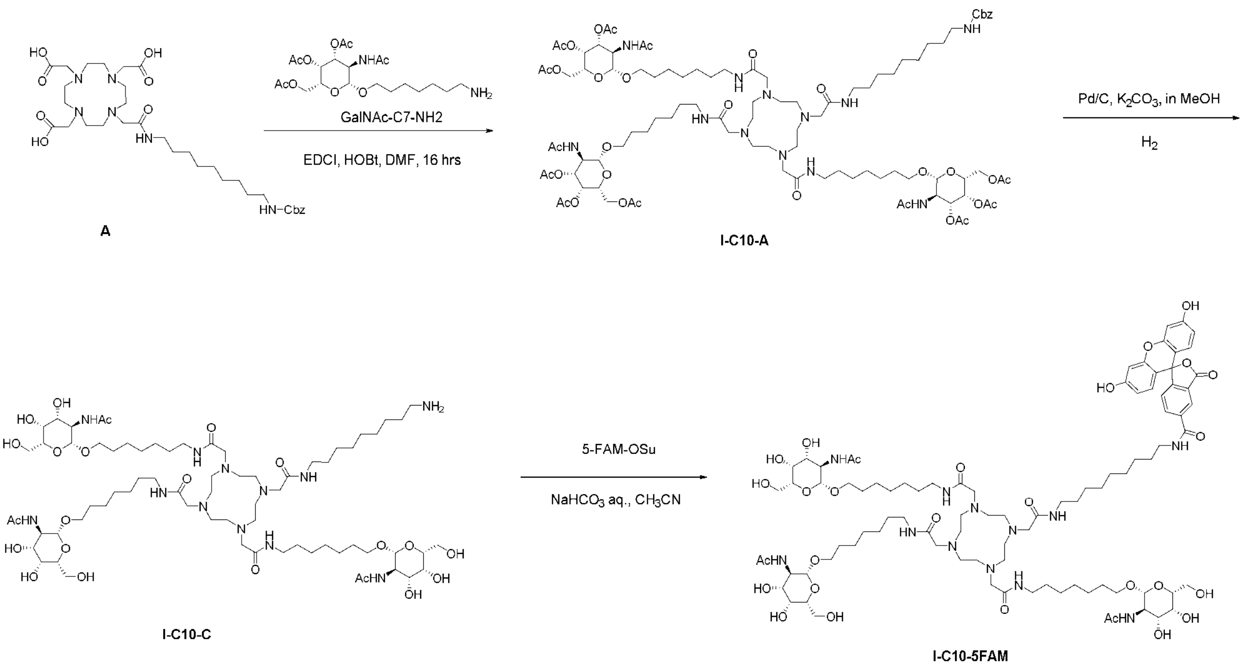


Synthesis of intermediate **I-C10-A**

Compound **A** (100 mg, 0.147 mmol) was dissolved in DMF (3 mL), followed by sequential addition of HOBt (138 mg, 1.029 mmol), EDCI (198 mg, 1.029 mmol), and **GalNAc-C7-NH$_2$** (408 mg, 0.884 mmol). The mixture was stirred at room temperature for 16 h. The reaction was quenched with $H_2O$ (5 mL) and extracted with ethyl acetate (8 mL). The organic layer was washed with 5% aqueous $NaHCO_3$ (10 mL), dried over anhydrous $Na_2SO_4$, and concentrated. The residue was purified by preparative chromatography to afford **I-C10-A** (170 mg, yield 57.6%) as a white solid. LCMS: (ESI) *m/z* = 1003.0 $[M+2H]/2^+$.

Preparation conditions: HPLC preparative column: Xbridge C18, 19 × 250 mm, 10 μm, 130 Å; mobile phase: A: water (containing 10 mmol/L $NH_4HCO_3$), B: acetonitrile; gradient program: ramp from 20% B to 80% B over 20 min.

Synthesis of intermediate **I-C10-C**

In a 50 mL round-bottom flask, **I-C10-A** (50 mg, 0.025 mmol), $K_2CO_3$ (21 mg, 0.150 mmol), and Pd/C (10%, 12 mg) were added. Methanol (5 mL) was then introduced, and the mixture was stirred under $H_2$ atmosphere for 3 h. The reaction mixture was filtered and concentrated to afford **I-C10-C** (50 mg, crude product) as a white solid. LCMS: (ESI) *m/z* = 766.5 $[M+H+K]/2^+$. 511.6 $[M+2H+K]/3^+$.

Synthesis of compound **I-C10-5FAM**

The crude product **I-C10-C** obtained from the previous step (50 mg, 0.025 mmol) was dissolved in a solution of saturated aq. $NaHCO_3$ in $CH_3CN/H_2O$ (1.8 mL, v/v = 1:4:4), followed by addition of **5-FAM-OSu** (19 mg, 0.040 mmol). The mixture was stirred at room temperature for 1 h. The reaction mixture was directly purified by preparative chromatography to afford **I-C10-5FAM** (23 mg, yield 37.7%) as an orange-yellow solid. LCMS: (ESI) *m/z* = 946 $[M+H+K]/2^+$.
Preparation conditions: HPLC preparative column: Gemini C18 21.2 mm × 250 mm, 10 μm, 110 Å; mobile phase: A: water (containing 10 mmol/L $NH_4HCO_3$), B: acetonitrile; gradient program: ramp from 22% B to 32% B over 20 min.
HPLC purity: 60.224% (214 nm), RT = 14.535 min;35.421% (214 nm), RT = 15.682 min. Analysis conditions were the same as those for **I-A10-5FAM.**

**5.2** Synthesis of compound **I-C12-5FAM**

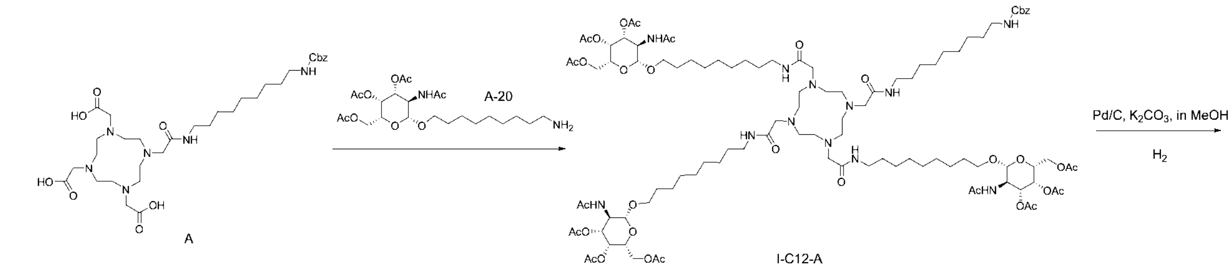


A

I-C12-A

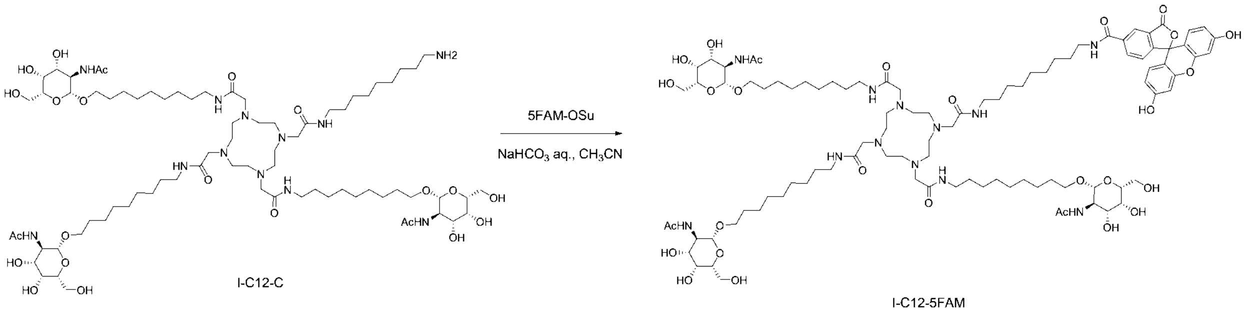


I-C12-C

I-C12-5FAM

Following the procedure for synthesizing **I-C10-5FAM,** 5.6 mg **I-C12-5FAM** was obtained.
LCMS: (ESI) *m/z* = 988.5 $[M+H+K]/2^+$, 969.2 $[M+2H]/2^+$, 659.0 $[M+2H+K]/3^+$, 646.5 $[M+3H]/3^+$.

HPLC purity: 76.78% (214 nm), RT = 8.788 min (M); 21.00% (214 nm), RT = 9.764 min (M+K); mobile phase: A: water (0.05%TFA) B: acetonitrile (0.05%TFA)
Gradient: 15%B (equilibration for 1 min), ramped to 75%B over 20 min, ramped to 95% in 1 min, and held at 95% for 5 min.
Flow rate: 1.0 mL/min
Chromatographic column: XBridge peptide BEH column C18, 4.6 × 150 mm, 3.5 μm, 130 Å
Column temperature: 60 °C

**6.** Synthesis of **I-D** class molecules

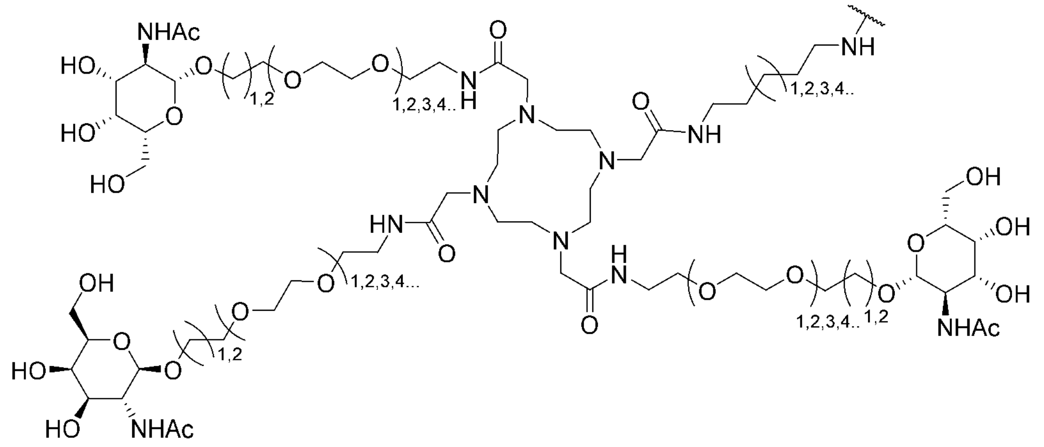

**6.1** Synthesis of compound **I-D11-5FAM**

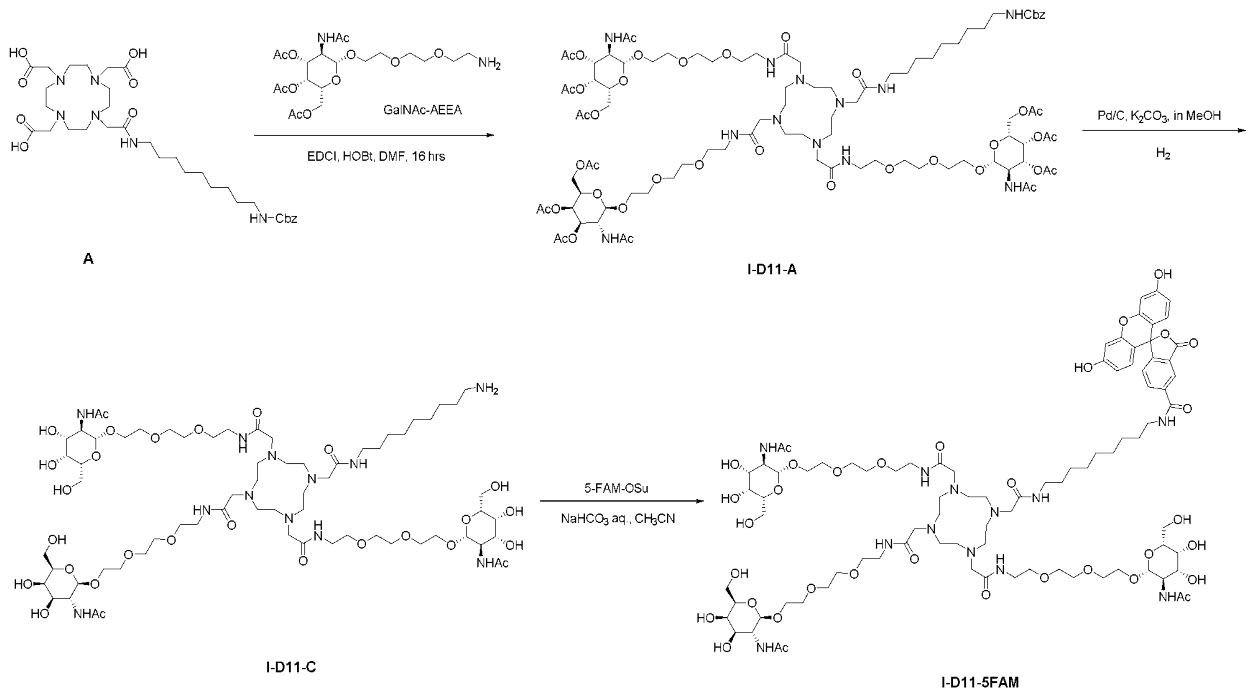


Synthesis of intermediate **I-D11-A**

**Gal-AEEA** (100 mg, 0.1688 mmol, 3.1 equiv.) was dissolved in DMF (4 mL), followed by sequential addition of **A** (37 mg, 0.0544 mmol), PyAop (113 mg, 0.2176 mmol) and DIPEA (93 μL, 0.544 mmol). The mixture was stirred at room temperature for 4 h. The reaction mixture was directly purified by preparative chromatography to afford **I-D11-A** (45 mg, yield 40%) as a white solid. LCMS: (ESI) *m*/*z* = 1031.1 $[M+2H]/2^+$.

Synthesis of intermediate **I-D11-C**

Compound **I-D11-A** (21 mg, 0.0102 mmol) was dissolved in MeOH (5 mL), followed by sequential addition of Pd/C (10%, 10 mg) and $K_2CO_3$ (10 mg). The system was purged with $H_2$ (twice), then stirred under $H_2$ atmosphere at room temperature for 2 h. The reaction mixture was concentrated and dried under vacuum to afford the crude product **I-D11-C** (35 mg) as a white solid, which was directly used in the next step. LCMS: (ESI) *m*/*z* = 793.5 $[M+H+K]/2^+$; 774.6 $[M+2H]/2^+$; 529.5 $[M+2H+K]/3^+$; 516.8 $[M+3H]/3^+$.

Synthesis of compound **I-D11-5FAM**

The crude product **I-D11-C** obtained from the previous step (ca. 0.0102 mmol, 1.0 equiv.) was dissolved in a mixture of water (2 mL) and acetonitrile (0.5 mL), followed by sequential addition of 5-FAM-OSu (6 mg, 0.0122 mmol) and saturated aq. $NaHCO_3$ (0.5 mL). The mixture was stirred at room temperature for 1 h. The reaction mixture was directly purified by preparative chromatography to afford **I-D11-5FAM** (9 mg, overall yield 46% over two steps) as an orange powder. LCMS: (ESI) *m/z* = 648.8 $[M+2H+K]/3^+$; 636.3 $[M+3H]/3^+$.

HPLC purity: 85.06 % (214 nm), RT = 14.001 min (M); 14.96 % (214 nm), RT = 14.492 min (M+K).

Analysis conditions were the same as those for **I-A10-5FAM.**

**7.** Synthesis of **I-E** class molecules

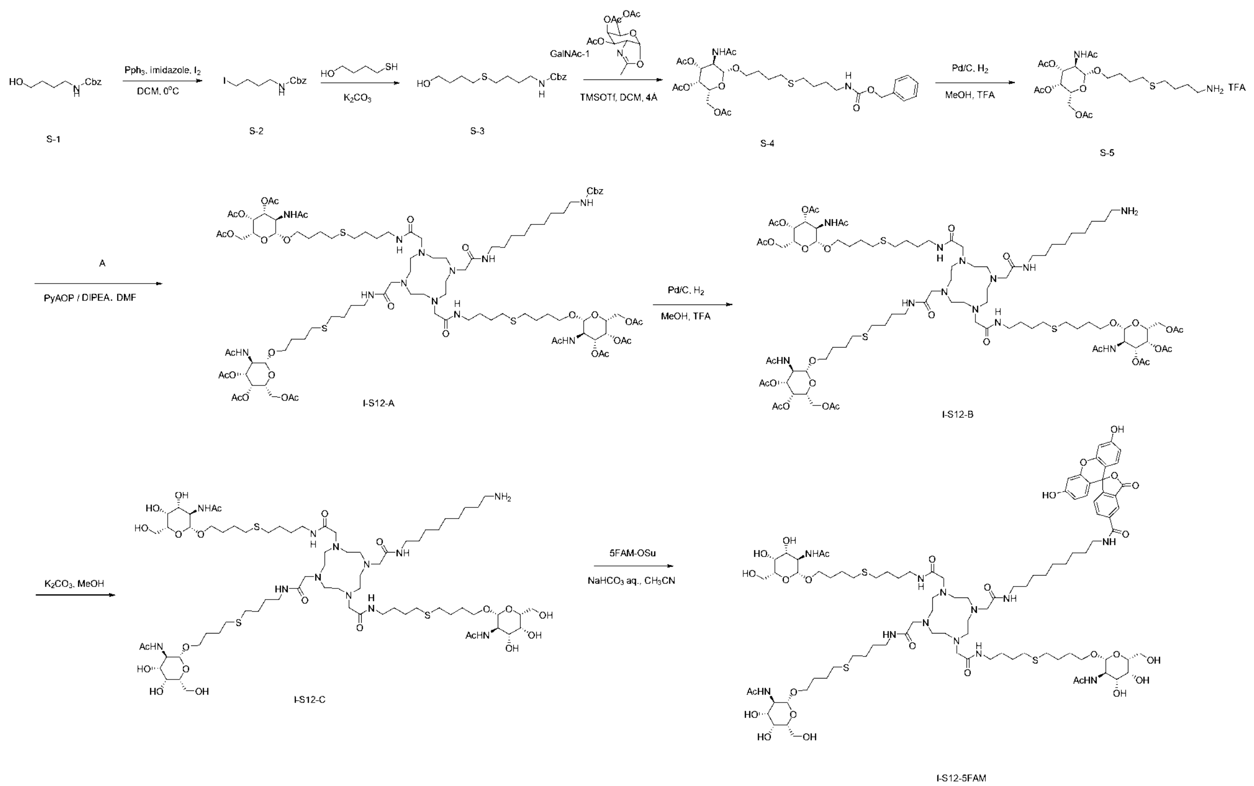


Synthesis of intermediate **S-2**

In a reaction flask were added triphenylphosphine oxide (6.82 g, 26.0 mmol) and imidazole. After drying, anhydrous DCM (200 mL) was introduced, and the mixture was cooled to 0°C. To this solution, iodine (6.6 g, 26.0 mmol) was added, and the reaction was stirred at room temperature for 10 min. The mixture was re-cooled to 0°C, followed by addition of a solution of **S-1** (4.46 g, 20 mmol) in anhydrous DCM (100 mL). The resulting mixture was stirred at 0°C for 16 h. The precipitate was filtered off, and the filtrate was washed with saturated aq. $Na_2SO_3$ (50 mL), dried over anhydrous $Na_2SO_4$, and concentrated. The crude product was purified by silica gel column chromatography to afford **S-2** (6.6 g, yield 98%) as a pale yellow solid. LCMS: (ESI) *m/z* = 356.1 $[M+Na]^+$, 334.2 $[M+H]^+$.

Synthesis of intermediate **S-3**

Compound 4-mercaptobutan-1-ol (2.12 g, 20 mmol, 1.0 equiv.) was dissolved in anhydrous DMF (20 mL), followed by addition of $K_2CO_3$ (2.76 g, 20 mmol). The mixture was stirred at room temperature for 10 min, then **S-2** (6.6 g, 20 mmol) was added. The resulting mixture was stirred at room temperature for 48 h. The reaction mixture was directly purified by reversed-phase column chromatography to afford **S-3** (4.7 g, yield 75%) as a white solid. LCMS: (ESI) *m/z* = 334.3 $[M+Na]^+$, 312.3 $[M+H]^+$.

H-NMR(400 MHz, DMSO): $\delta$: 7.40-7.22 (m, 6H),5.01 (s, 2H), 4.40 (t, J = 5.2 Hz, 1H), 3.39 (q, J = 6.0 Hz, 2H), 3.00

(q, J = 6.0 H, 2H), 2.52-2.41 (m, 4H), 1.58-1.40 (m, 8H).

Synthesis of intermediate **S-4**

**GalNAc-1** (1.8 g, 5.46 mmol, 1.0 equiv.) was dissolved in anhydrous DCM (100 mL), followed by sequential addition of **S-3** (1.7 g, 5.46 mmol) and 4 Å molecular sieves (1.0 g). After stirring for 5 min, TMSOTf (0.4 mL, 2 mmol) was added. The mixture was stirred at room temperature for 48 h. The reaction mixture was filtered, and the filtrate was neutralized to pH 7 with saturated aq. $NaHCO_3$. The organic layer was separated, and the aqueous phase was extracted with DCM (200 mL). The combined organic phases were dried over anhydrous $Na_2SO_4$ and concentrated. The crude product was purified by reversed-phase column chromatography to afford **S-4** (1.6 g, yield 45%) as a white solid. LCMS: (ESI) *m/z* = 641.4 $[M+H]^+$. $^1$H-NMR(400 MHz, DMSO): δ: 7.82 (d, *J* = 9.2 Hz, 1H), 7.39-7.24 (m, 6H), 5.21 (d, *J* = 7.6 Hz, 1H), 5.00 (s, 2H), 4.95 (dd, *J* = 3.6, 11.2 Hz, 1H), 4.47 (d, *J* = 8.8 Hz, 1H), 4.07-3.99 (m, 3H), 3.92-3.83 (m, 1H), 3.76-3.68 (m, 1H), 3.47-3.38 (m, 1H), 3.04-2.95 (m, 2H), 2.49-2.41 (m, 4H), 2.10 (s, 3H), 2.00 (s, 3H), 1.89 (s, 3H), 1.78 (s, 3H), 1.58-1.42, 8H).

Synthesis of intermediate **S-5**

**S-4** (0.2 g, 0.31 mmol, 1.0 equiv.) was dissolved in methanol (50 mL), followed by sequential addition of Pd/C (300 mg) and TFA (26 μL). The system was purged with $H_2$ (twice), then stirred under $H_2$ atmosphere at room temperature for 16 h. The reaction mixture was filtered and concentrated to afford **S-5** (180 mg, yield 93%) as a colorless viscous liquid. LCMS: (ESI) *m/z* = 507.3 $[M+H]^+$.

Synthesis of intermediate **I-S12-A**

**S-5** (176 mg, 0.283 mmol, 3.0 equiv.) was dissolved in DMF (3 mL), followed by sequential addition of **A** (55 mg, 0.081 mmol), PyAOP (169 mg, 0.33 mmol) and DIPEA (0.14 mL, 0.81 mmol). The mixture was stirred at room temperature for 2 h. The reaction mixture was directly purified by preparative chromatography to afford **I-S12-A** (100 mg, yield 57%) as a white solid. LCMS: (ESI) m/z = 715.7 $[M+3H]/3^+$, 1073.2 $[M+2H]/2^+$.

Preparation conditions: chromatography column: Xbridge C18, 19 × 250 mm, 10 μm, 130 Å; mobile phase: A: water (containing 10 mmol/L $NH_4HCO_3$), B: ACN; gradient program: ramp from 35% B to 45% B over 20 min.

Synthesis of intermediate **I-S12-B**

Compound **I-S12-A** (80 mg, 0.0467 mmol, 1.0 equiv.) was dissolved in MeOH (5 mL), followed by sequential addition of Pd/C (10%, 100 mg) and TFA (14 μL). The system was purged with $H_2$ (twice), then stirred under $H_2$ atmosphere for 16 h. LCMS analysis indicated 30% conversion of starting material with 70% remaining. The reaction mixture was filtered, concentrated, and the crude product was purified by preparative chromatography to afford **I-S12-B** (8 mg, yield 6.7%) as a white solid. LCMS: (ESI) *m/z* = 1005.8 $[M+2H]/2^+$; 671.2 $[M+3H]/3^+$.

Preparation conditions: chromatography column: Xbridge C 18, 19 × 250 mm, 10 μm, 130 Å; mobile phase: A: water (containing 0.05% TFA), B: ACN (containing 0.05% TFA); gradient program: ramp from 5% B to 95% B over 20 min.

Synthesis of intermediate **I-S12-C**

Compound **I-S12-B** (8 mg, 0.004 mmol, 1.0 equiv.) was dissolved in methanol (25 mL), followed by addition of $K_2CO_3$ (5 mg); the mixture was stirred at room temperature for 2 h. The reaction mixture was concentrated to afford the crude product **I-S12-C,** which was directly used in the next step. LCMS: (ESI) *m/z* = 835.8 $[M+H+K]/2^+$; 816.8 $[M+2H]/2^+$; 545.1 $[M+3H]/3^+$.

Synthesis of compound **I-S12-5FAM**

Compound **I-S12-C** (crude, 0.004 mmol, 1.0 equiv.) was dissolved in a mixture of water (2 mL) and acetonitrile (0.5 mL), followed by sequential addition of 5-FAM-OSu (3 mg, 0.006 mmol) and saturated aq. $NaHCO_3$ (0.5 mL); the mixture was stirred at room temperature for 1 h. The reaction mixture was directly purified by preparative chromatography to afford **IS12-5FAM** (2 mg, yield 25%) as an orange powder.

Preparation conditions: chromatography column: Gemini C18 21.2 mm × 250 mm, 10 μm, 110 Å; mobile phase: A: water (containing 10 mmol/L $NH_4HCO_3$), B: ACN; gradient program: ramp from 26% B to 36% B over 20 min.

LCMS: (ESI) *m/z* = 1015.1 $[M+H+K]/2^+$; 677.2 $[M+2H+K]/3^+$.
HPLC purity: 96.33% (214 nm), RT = 16.71 min (M).

Analysis conditions were the same as those for **I-A10-5FAM.**

**8.** Synthesis of Class **II** molecules

8.1 Synthesis of key intermediate **B**

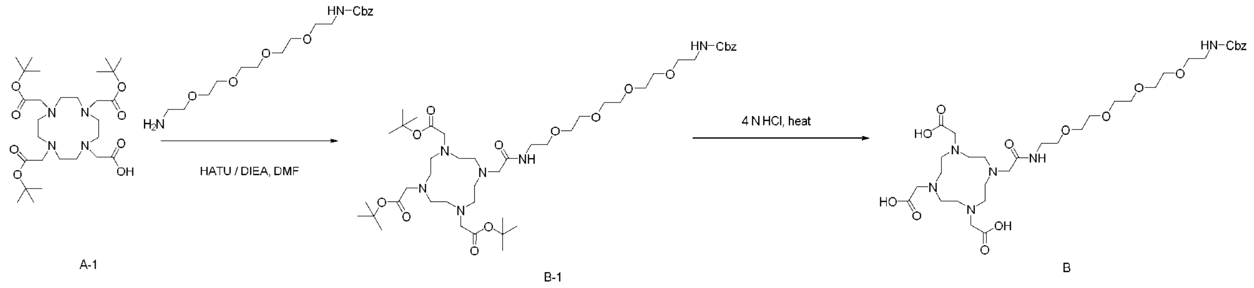


A-1 B-1 B

Compound **A-1** (0.572 g, 1 mmol, 1.0 equiv.) was dissolved in DMF (5 mL), followed by sequential addition of HATU (380 mg, 1 mmol, 1.0 equiv.) and DIPEA (0.51 mL, 3 mmol, 3.0 equiv.); the mixture was stirred at room temperature for 10 min, followed by addition of $NH_2$-$PEG_5$-NHCbz (1.88 g, 5.0 mmol). The resulting mixture was stirred at room temperature for 2 h. The reaction mixture was directly purified by reversed-phase column chromatography to afford the Boc-protected product (1.2 g) as a white solid. LCMS: (ESI) *m/z* = 925.7 $[M+H]^+$; 463.5 $[M+2H]/2^+$. The obtained white solid was dissolved in THF (20 mL), followed by addition of 6 N HCl (20 mL); the mixture was stirred at 50°C for 4 h. The reaction mixture was concentrated and freeze-dried. The resulting crude product was purified by reversed-phase column chromatography to afford compound **B** (0.6 g, yield 79%) as a white solid.

Reversed-phase purification conditions: column: 120 g C18 column; Mobile phase: A: water (containing 10 mmol/L $NH_4HCO_3$); B: ACN; Gradient: ramped from 5% B to 50% B over 20 min. LCMS: (ESI) *m/z* = 757.5 $[M+H]^+$; 779.5 $[M+Na]^+$; 379.4 $[M+2H]/2^+$.

8.2 Synthesis of **IIB-B12-5FAM**

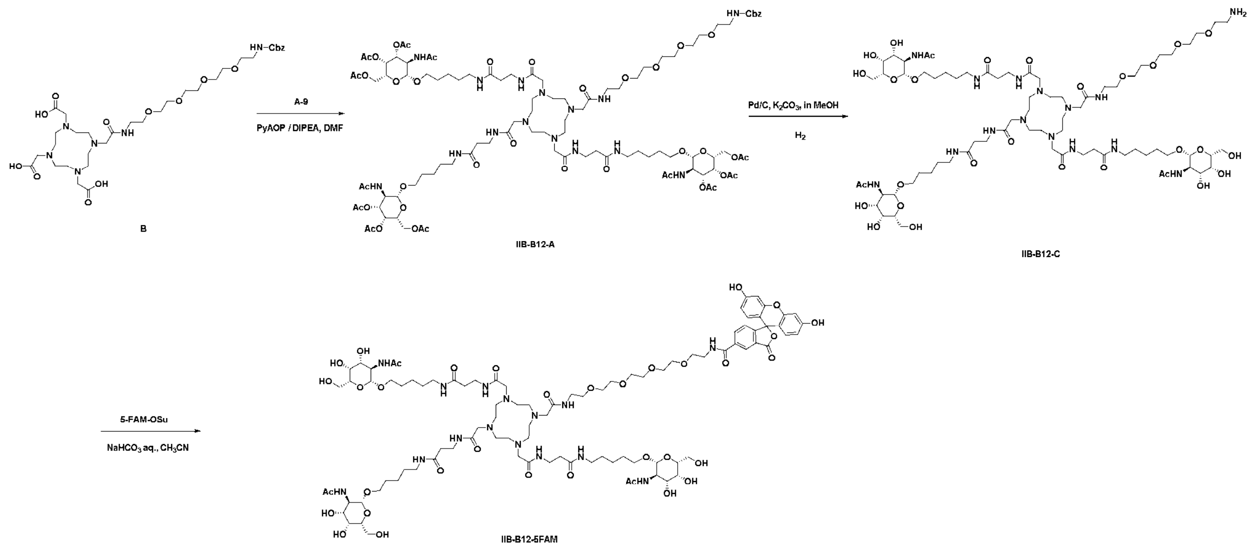


B IIB-B12-A IIB-B12-C IIB-B12-5FAM

Synthesis of intermediate **IIB-B12-A**

Compound **A-9** (0.49 g, 0.793 mmol, 3.0 equiv.) was dissolved in DMF (5 mL), followed by sequential addition of compound **B** (0.20 g, 0.264 mmol), PyAOP (0.55 g, 1.056 mmol) and DIPEA (0.45 mL, 2.64 mmol). The mixture was stirred at room temperature for 16 h. The reaction mixture was directly purified by preparative chromatography to afford **IIB-B12-A**

(225 mg, yield 38.5%) as a white solid. LCMS: (ESI) *m/z* = 1107.7 [M+2H]/$2^+$; 738.8 [M+3H]/$3^+$.

Preparation conditions: column: Xbridge C18, 19 × 250 mm, 10 μm, 130 Å; mobile phase: A: water (containing 10 mmol/L $NH_4HCO_3$), B: ACN; gradient program: ramp from 36% B to 46% B over 20 min.

Synthesis of intermediate **IIB-B12-C**

Intermediate **IIB-B12-A** (25 mg, 0.0113 mmol) was dissolved in methanol (5 mL), followed by sequential addition of Pd/C (10%, 10 mg) and $K_2CO_3$ (10 mg); the system was purged with $H_2$ (twice), then stirred under $H_2$ atmosphere at room temperature for 2 h. The reaction mixture was filtered and concentrated to afford the crude product **IIB-B12-C** (35 mg) as a white solid, which was directly used in the next step. LCMS: (ESI) *m/z* = 851.1 [M+2H]/$2^+$; 568.0 [M+3H]/$3^+$; 870.2 [M+H+K]/$2^+$; 881.0 [M+2H+K]/$3^+$.

Synthesis of compound **IIB-B12-5FAM**

Compound **IIB-B12-C** (crude, 0.0113 mmol, 1.0 equiv.) was dissolved in a mixture of water (2 mL) and acetonitrile (0.5 mL), followed by sequential addition of 5-FAM-OSu (6 mg, 0.0135 mmol) and saturated aq. $NaHCO_3$ (0.5 mL). The mixture was stirred at room temperature for 1 h. The reaction mixture was directly purified by preparative chromatography to afford **IIB-B12-5FAM** (7 mg, overall yield 30% over two steps) as an orange powder. LCMS: (ESI) *m/z* = 700.3 [M+2H+K]/$3^+$; 687.3 [M+3H]/$3^+$.

Preparation conditions: column: Gemini C18 21.2 mm × 250 mm, 10 μm, 110 Å; mobile phase: A: water (containing 10 mmol/L $NH_4HCO_3$), B: ACN; gradient program: ramp from 11% B to 21% B over 20 min.

HPLC: 75.99 % (214 nm), RT = 12.427 min (M); 23.83 % (214 nm), RT = 12.677 min (M+K). Analysis conditions were the same as those for **I-A10-5FAM.**

8.3 Synthesis of compound **IIB-B12b-5FAM**

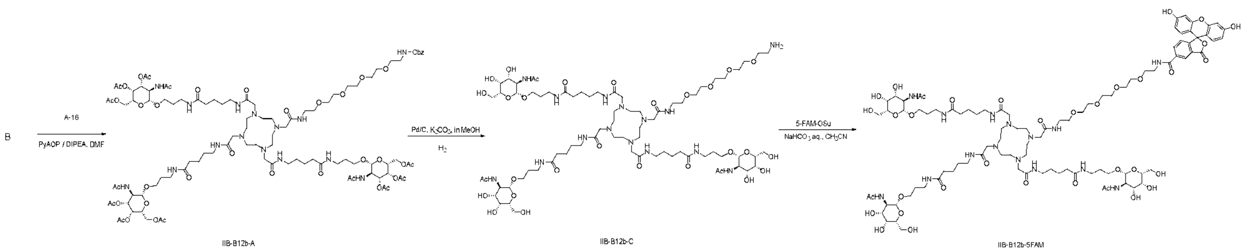


**IIB-B12b-5FAM** (7 mg) was obtained from **B** (22 mg) through similar synthetic procedures.

LCMS: (ESI) *m/z* = 700.0 [M+2H+K]/$3^+$, 687.5 [M+3H]/$3^+$.

HPLC purity: 75.99% (214 nm), RT = 12.427 min (M); 23.83% (214 nm), RT = 12.677 min (M+K). Analysis conditions were the same as those for **I-A10-5FAM.**

8.4 Synthesis of compound **IIB-B12c-5FAM**

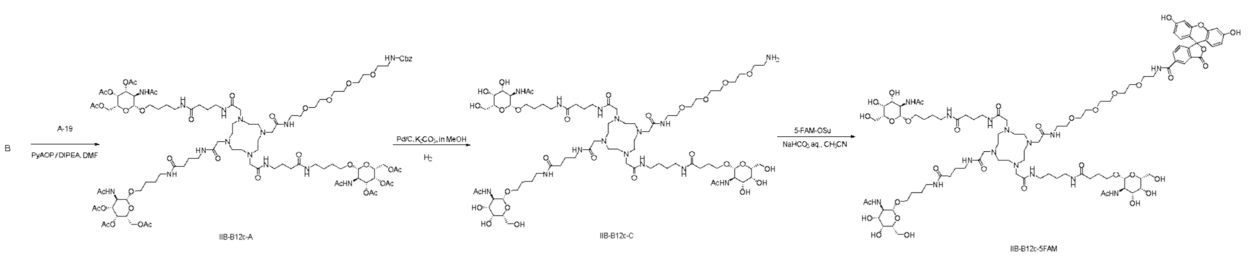


**IIB-B12b-5FAM** (5 mg) was obtained from **B** (28 mg) through similar synthetic procedures.

LCMS: (ESI) *m/z* = 1130.2 [M+2H]/$2^+$; 700.0 [M+2H+K]/$3^+$, 687.7 [M+3H]/$3^+$.

HPLC purity: 79.38 % (214 nm), RT = 12.082 min (M); 18.88 % (214 nm), RT = 12.399 min (M+K). Analysis

conditions were the same as those for **I-A10-5FAM.**

8.5 Synthesis of compound **IIB-C12-5FAM**

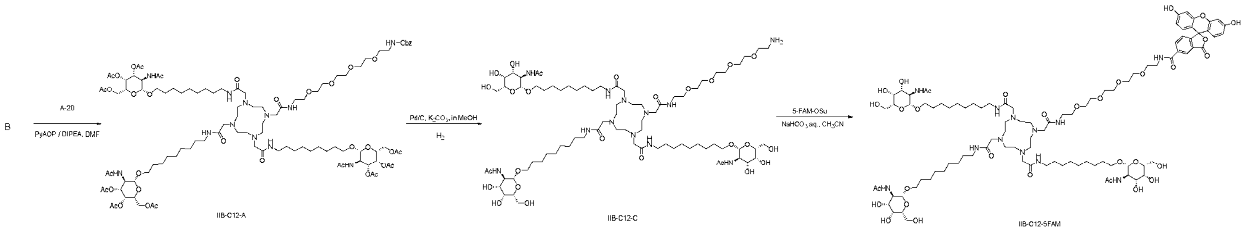


**IIB-C12-5FAM** (4.1 mg) was obtained from **B** (20 mg) through similar synthetic procedures.

LCMS: (ESI) *m/z* = 1027.1 $[M+H+K]/2^+$, 685.2 $[M+2H+K]/3^+$, 672.5 $[M+3H]/3^+$.

HPLC purity: 70.54% (214 nm), RT = 14.623 min (M); 28.75% (214 nm), RT = 15.526 min (M+K). Analysis conditions were the same as those for **I-A10-SFAM.**

8.6 Synthesis of compound **IIB-A12-5FAM**

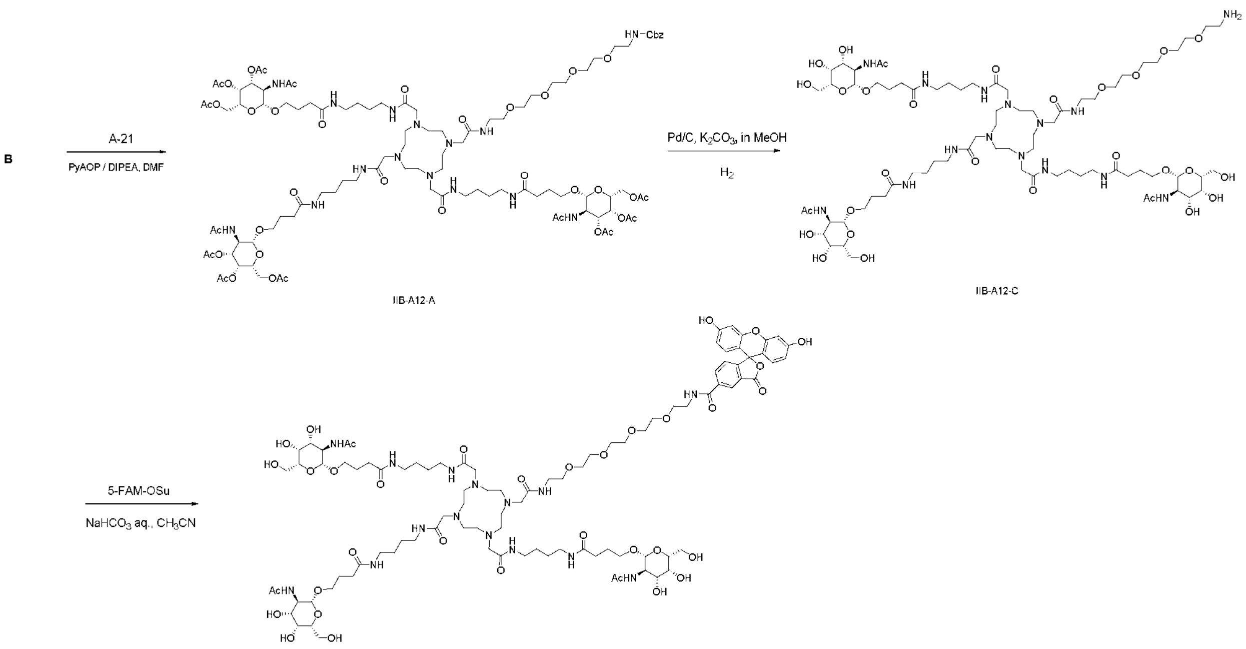


**IIB-A12-5FAM** (10 mg) was obtained from **B** (28 mg) through similar synthetic procedures.

LCMS: (ESI) *m/z* = 700.1 $[M+2H+K]/3^+$, 687.7 $[M+3H]/3^+$.

HPLC purity: 84.33% (214 nm), RT = 9.103 min (M); 15.67% (214 nm), RT = 9.431 min (M+K). Analysis conditions were the same as those for **I-A10-SFAM.**

**9.** Synthesis of Class **III** molecules

**9.1** Synthesis of compound **IIIA-B12-5FAM**

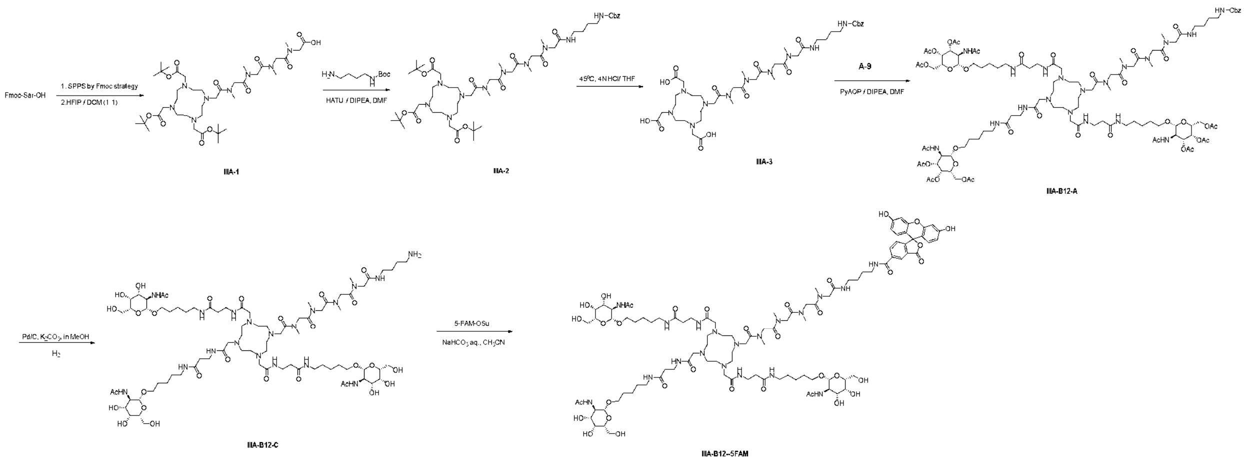


Synthesis of intermediate **IIIA-1**

The intermediate IIIA-1 was synthesized via Fmoc solid-phase peptide synthesis on 2-CTC resin through consecutive couplings of four Fmoc-Sar-OH units and compound **A-1.** Upon synthesis completion, the resin was cleaved with HFIP/DCM (1:1, v/v). The mixture was filtered under vacuum, and the filtrate was collected. The resin was washed with DMF three times. The combined filtrates were concentrated under reduced pressure to remove organic solvents and afford a crude product. The crude product was dissolved in a water/acetonitrile (1:1, v/v) mixture, frozen at -80°C, and lyophilized to afford intermediate **IIIA-1** (330 mg, yield 38.5%) as a white solid, which was directly used in the next step. LCMS: (ESI) *m*/*z* = 857.5 $[M+H]^+$; 879.5 $[M+Na]^+$; 429.5 $[M+2H]/2^+$.

Synthesis of intermediate **IIIA-2**

The intermediate **IIIA-1** (220 mg, 0.257 mmol) was dissolved in DMF (5 mL), followed by sequential addition of HATU (102 mg, 0.270 mmol) and DIPEA (131 μL, 0.771 mmol). The mixture was stirred at room temperature for 5 min, followed by addition of N-Cbz-1,4-butanediamine (95 mg, 0.283 mmol, 1.1 equiv.). The mixture was stirred at room temperature for an additional 1 h. The reaction was quenched with $H_2O$ (20 mL), and the precipitate was filtered off. The organic layer was washed with $H_2O$ and dried to afford IIIA-2 (270 mg, yield 99%) as a pale yellow solid, which was directly used in the next step. LCMS: (ESI) *m*/*z* = 1061.7 $[M+H]^+$;531.5 $[M+2H]/2^+$.

Synthesis of intermediate **IIIA-3**

The intermediate **IIIA-2** (270 mg, 0.257 mmol) was dissolved in THF (5 mL), followed by addition of 6 N HCl (5 mL). The mixture was stirred at 50°C for 2 h. The reaction mixture was concentrated and vacuum dried to afford a crude product **IIIA-3** as a pale yellow solid, which was directly used in the next step. LCMS: (ESI) *m*/*z* = 915.5 $[M+Na]^+$; 893.5 $[M+H]^+$; 447.5 $[M+2H]/2^+$.

Synthesis of intermediate **IIIA-B12-A**

Compound **A-9** (476 mg, 0.771 mmol, 3.0 equiv.) was dissolved in DMF (10 mL), followed by sequential addition of the intermediate **IIIA-3** (crude, 0.257 mmol), PyAOP (536 mg, 1.03 mmol) and DIPEA (0.44 mL, 2.57 mmol). The mixture was stirred at room temperature for 2 h. The reaction mixture was directly purified by preparative chromatography to afford **IIIA-B12-A** (90 mg, overall yield 15% over two steps) as a white solid. LCMS: (ESI) *m*/*z* = 1194.2 $[M+H+K]/2^+$; 1175.3 $[M+2H]/2^+$; 797.2 $[M+2H+K]/3^+$; 784.2 $[M+3H]/3^+$.

Synthesis of intermediate **IIIA-B12-C**

The intermediate **IIIA-B12-A** (25 mg, 0.0106 mmol, 1.0 equiv.) was dissolved in methanol (5 mL), followed by sequential addition of Pd/C (10%, 15 mg) and $K_2CO_3$ (10 mg). The system was purged with $H_2$ (twice), then stirred under $H_2$ atmosphere at room temperature for 1 h. The reaction mixture was filtered and concentrated to afford the crude product **IIIA-B12-C,** which was directly used in the next step. LCMS: (ESI) *m*/*z* = 919.2 $[M+2H]/2^+$; 613.5 $[M+3H]/3^+$.

Synthesis of compound **IIIA-B12-5FAM**

The crude product **IIIA-B12-C** obtained from the previous step (ca. 0.0106 mmol, 1.0 equiv.) was dissolved in a mixture of water (2 mL) and acetonitrile (0.5 mL), followed by sequential addition of 5-FAM-OSu (6 mg, 0.0127 mmol) and saturated aq. $NaHCO_3$ (0.5 mL). The mixture was stirred at room temperature for 1 h. The reaction mixture was directly purified by preparative chromatography to afford **IIIA-B12-5FAM** (7 mg, overall yield 30% over two steps) as an orange-yellow solid. LCMS: (ESI) *m/z* = 745.3 $[M+2H+K]/3^+$; 732.8 $[M+3H]/3^+$.

Preparation conditions: column: Xbridge C18, 19 × 250 mm, 10 μm, 130 Å; mobile phase: A: water (containing 10 mmol/L $NH_4HCO_3$), B: acetonitrile; gradient program: ramp from 12% B to 22% B over 20 min.

HPLC purity: 76.77% (214 nm), RT = 12.104 min (M); 21.43 % (214 nm), RT = 12.313 min (M+K). Analysis conditions were the same as those for **I-A10-5FAM.**

**9.2** Synthesis of compound **IIIB-B12-5FAM**

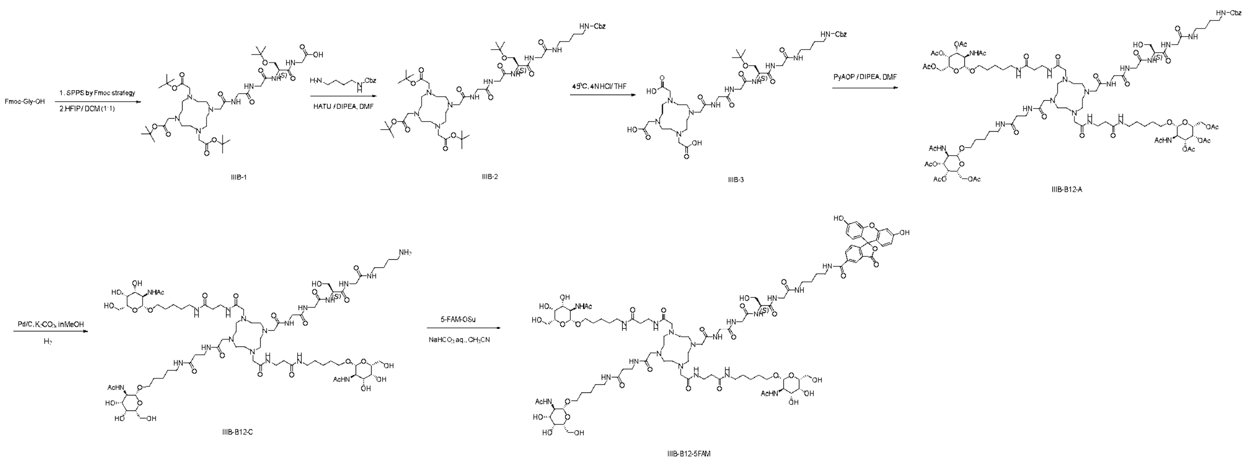


Synthesis of intermediate **IIIB-1**

The intermediate **IIIB-1** was synthesized via Fmoc solid-phase peptide synthesis on 2-CTC resin by consecutive coupling of four amino acids (Fmoc-Gly-OH, Fmoc-Ser(tBu)-OH, Fmoc-Gly-OH, Fmoc-Gly-OH) followed by compound **A-1.** After synthesis, the resin was cleaved with HFIP/DCM (1:1, v/v). The mixture was vacuum-filtered, and the filtrate was collected. The resin was washed with DMF (3 ×). All filtrates were combined and concentrated under reduced pressure to remove organic solvents to afford a crude product. The crude product was redissolved in a water/acetonitrile (1:1, v/v) mixture and lyophilized to afford intermediate **IIIB-1** (850 mg, yield 96%) as a white solid, which was directly used in the next step. LCMS: (ESI) *m/z* = 887.7 $[M+H]^+$; 444.5 $[M+2H]/2^+$; 416.4 $[M+2H-56]/2^+$.

Synthesis of intermediate **IIIB-3**

Compound **IIIB-1** (850 mg, 0.96 mmol) was dissolved in DMF (10 mL), followed by sequential addition of HATU (365 mg, 0.96 mmol) and DIPEA (391 μL, 2.88 mmol). The mixture was stirred at room temperature for 5 min, followed by addition of N-Cbz-1,3-propanediamine (200 mg, 0.96 mmol, 1.0 equiv.). The mixture was stirred at room temperature for 1 h. The reaction was quenched with $H_2O$ (20 mL), and the resulting white precipitate was filtered and vacuum dried to afford **IIIB-2** (862 mg, yield 83%) as a light brown solid, which was directly used in the next step. LCMS: (ESI) *m/z* = 539.5 $[M+2H]/2^+$. The crude **IIIB-2** (860 mg, 0.8 mmol) was dissolved in THF (10 mL), followed by addition of 4 N HCl (10 mL). The mixture was stirred at 45°C for 3 h. After completion, the reaction mixture was concentrated to afford 870 mg crude product. This crude product was purified by reversed-phase column chromatography to afford **IIIB-3** (500 mg, yield 62%) as a white solid. LCMS: (ESI) *m/z* = 853.3 $[M+H]^+$; 427.4 $[M+2H]/2^+$.

Synthesis of intermediate **IIIB-B12-A**

Compound **IIIB-3** (160 mg, 0.16 mmol, 1.0 equiv.) was dissolved in DMF (10 mL), followed by addition of DIPEA (0.27 mL, 1.6 mmol). The mixture was stirred at room temperature for 1 h. To this mixture were added **A-9** (395 mg, 0.64

mmol) and PyAOP (334 mg, 0.64 mmol) and the mixture was stirred at room temperature for 2 h. The reaction mixture was directly purified by preparative chromatography to afford **IIIB-B12-A** (170 mg, yield 46%) as a white solid. LCMS: (ESI) m/z = 1155.6 $[M+2H]/2^+$; 771.0 $[M+3H]/3^+$.

Preparation conditions: column: Xbridge C18, 19 × 250 mm, 10 μm, 130 Å; mobile phase: A: water (containing 0.05% TFA), B: ACN (containing 0.05% TFA); gradient program: ramp from 5% B to 65% B over 20 min.

Synthesis of compound **IIIB-B12-5FAM**

Compound **IIIB-B12-A** (25 mg, 0.0108 mmol, 1.0 equiv.) was dissolved in methanol (5 mL), followed by sequential addition of Pd/C (10%, 15 mg) and $K_2CO_3$ (10 mg). The system was purged with $H_2$ (twice), then stirred under $H_2$ atmosphere at room temperature for 1 h. The reaction mixture was filtered and concentrated to afford a crude product **IIIB-B12-C,** which was directly used in the next step. LCMS: (ESI) *m/z* = 899.2 $[M+2H]/2^+$; 599.5 $[M+3H]/3^+$. The crude product was dissolved in a mixture of water and acetonitrile, followed by sequential addition of 5-FAM-OSu (6 mg, 0.0130 mmol) and saturated aq. $NaHCO_3$ (0.5 mL). The mixture was stirred at room temperature for 1 h. The reaction mixture was directly purified by preparative chromatography to afford **IIIB-B12-5FAM** (6 mg, overall yield 26% over two steps) as an orange solid. LCMS: (ESI) *m/z* = 1078.2 $[M+2H]/2^+$; 719.3 $[M+3H]/3^+$; 1096.6 $[M+H+K]/2^+$; 731.8 $[M+2H+K]/3^+$.

Preparation conditions: column: Xbridge C18, 19 × 250 mm, 10 μm, 130 Å; mobile phase: A: water (containing 10 mmol/L $NH_4HCO_3$), B: ACN; gradient program: ramp from 12% B to 22% B over 20 min.

HPLC: 85.18 % (214 nm), RT = 11.511 min (M); 10.09 % (214 nm), RT = 11.670 min (M+K). Analysis conditions were the same as those for **I-A10-5FAM.**

**10.** Class **IV** molecules

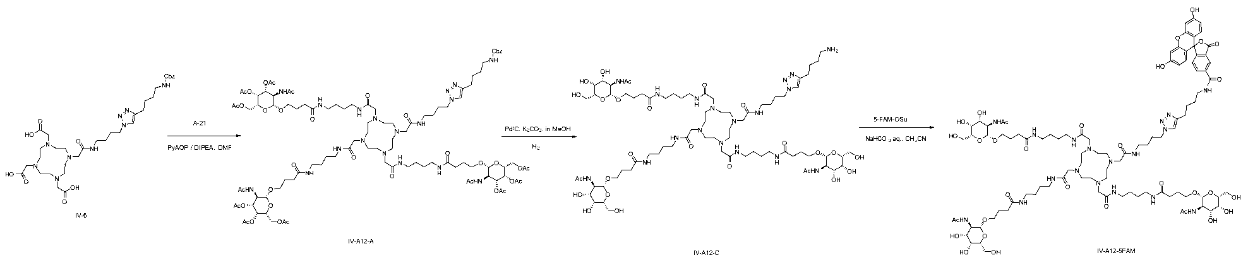


Compound **IV-A12-5FAM** (6 mg) was obtained following the synthetic procedure as indicated in the above and the analysis data is shown in the following.

LCMS: (ESI) m/z = 1037.1 $[M+2H+K]/2^+$; 679.0 $[M+3H]/3^+$.

HPLC purity: 88.19% (214 nm), RT = 11.495 min (M); 11.81% (214 nm), RT = 11.842 min (M+K). Analysis conditions were the same as those for **I-A10-SFAM.**

**11.** Synthesis of Class **V** molecules

**11.1** Synthesis of compound **VA-A12-5FAM**

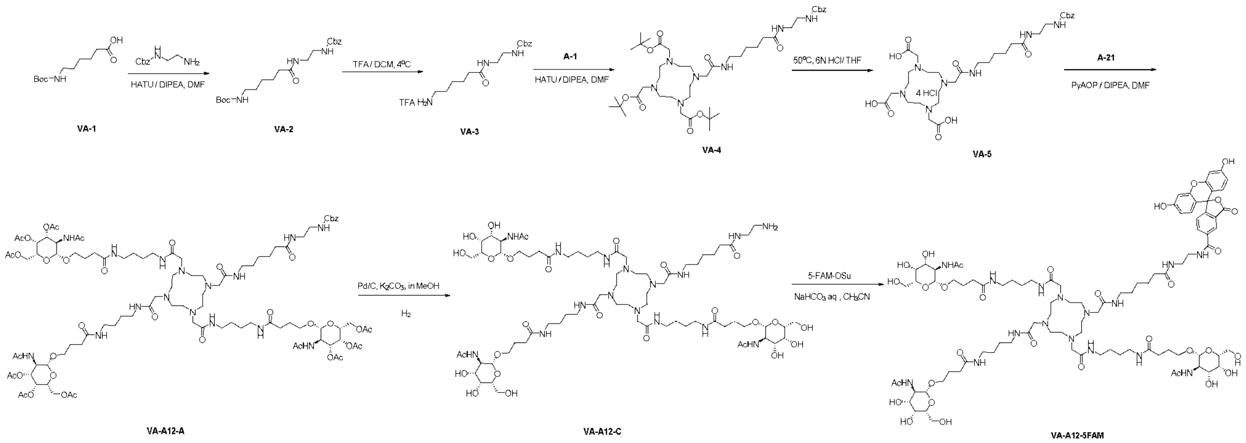


Synthesis of intermediate **VA-2**

Compound 6-(tert-butoxycarbonylamino)hexanoic acid (1.16 g, 5 mmol, 1.0 equiv.) was dissolved in DMF (10 mL), followed by sequential addition of N-Cbz-ethylenediamine (0.97 g, 5 mmol), HATU (1.9 g, 5 mmol), and DIPEA (2.6 mL, 16 mmol). The mixture was stirred at room temperature for 16 h. The reaction was quenched with $H_2O$ (50 mL), and the resulting white precipitate was filtered, washed with $H_2O$ ($2 \times 20$ mL), and vacuum dried to afford **VA-2** (1.83 g, yield 90%) as a white solid. LCMS: (ESI) *m*/*z* = 408.3 $[M+H]^+$; 308.3 $[M+H-Boc]^+$.

Synthesis of intermediate **VA-4**

Compound **VA-2** (408 mg, 1 mmol, 1.0 equiv.) was dissolved in DCM (6 mL) and cooled to 0°C, followed by addition of trifluoroacetic acid (3 mL) and the reaction was performed at 0°C for 2 h. The reaction mixture was concentrated and vacuum dried to afford crude **VA-3** (420 mg, quantitative yield) as an anhydrous oily liquid. LCMS: (ESI) *m*/*z* = 308.3 $[M+H]^+$. This crude **VA-3** was added to a solution of compound **A-1** (573 mg, 1 mmol, 1.0 equiv.) in DMF (10 mL), followed by sequential addition of DIPEA (1.0 mL, 6 mmol) and PyAOP (625 mg, 1.2 mmol). The mixture was allowed to react at room temperature for 3 h. The reaction mixture was directly purified by preparative chromatography to afford **VA-4** (540 mg, yield 41%) as a white solid. LCMS: (ESI) *m*/*z* = 862.7 $[M+H]^+$; 431.9 $[M+2H]/2^+$.

Reversed-phase purification conditions: column: 120 g C18 column; Mobile phase: A: water (containing 0.05% TFA); B: ACN; Gradient: ramped from 5% B to 50% B over 20 min.

Synthesis of intermediate **VA-5**

Compound **VA-4** (540 mg, 0.410 mmol, 1.0 equiv.) was dissolved in THF (10 mL), followed by addition of 6 N HCl (10 mL). The mixture was reacted at 50°C for 3 h. The reaction mixture was concentrated and dried to afford **VA-5** (340 mg, quantitative yield) as a light yellow solid. LCMS: (ESI) *m*/*z* = 694.5 $[M+H]^+$; 716.5 $[M+Na]^+$.

Synthesis of intermediate **VA-A12-A**

Compound **A-21** (235 mg, 0.38 mmol, 3.2 equiv.) was dissolved in DMF (5 mL), followed by sequential addition of **VA-5** (100 mg, 0.12 mmol), PyAOP (250 mg, 0.48 mmol), and DIPEA (0.21 mL, 1.2 mmol). The mixture was stirred at room temperature for 2 h. The reaction mixture was directly purified by preparative chromatography to afford **VA-A12-A** (60 mg, yield 23%) as a white solid. LCMS: (ESI) *m*/*z* = 1075.8 $[M+2H]/2^+$; 717.8 $[M+3H]/3^+$.

Preparation conditions: column: Xbridge C18, $19 \times 250$ mm, 10 $\mu$m, 130 Å; mobile phase: A: water (containing 0.05% TFA), B: ACN (containing 0.05% TFA); gradient program: ramp from 5% B to 65% B over 20 min.

Synthesis of compound **VA-A12-SFAM**

Compound **VA-A12-A** (25 mg, 0.0113 mmol, 1.0 equiv.) was dissolved in methanol (5 mL), followed by sequential addition of Pd/C (10%, 10 mg) and $K_2CO_3$ (10 mg). The system was purged with $H_2$ (twice), then stirred under $H_2$ atmosphere at room temperature for 1 h. The reaction mixture was filtered, concentrated, and vacuum dried to afford a crude product **VA-A12-C,** which was directly used in the next step. LCMS: (ESI) *m*/*z* = 819.8 $[M+2H]/2^+$; 559.9 $[M+3H]/3^+$. The crude **VA-A12-C** was dissolved in a mixture of water (2 mL) and acetonitrile (0.5 mL), followed by sequential addition of 5-FAM-OSu (6 mg, 0.0128 mmol) and saturated aq. $NaHCO_3$ (0.5 mL). The mixture was stirred at room temperature for 1

h. The reaction mixture was directly purified by preparative chromatography to afford **VA-A12-5FAM** (8 mg, overall yield 34% over two steps) as an orange powder. LCMS: (ESI) *m/z* = 679.2 [M+2H+K]/3$^+$; 666.3 [M+3H]/3$^+$.

Column: Gemini C18 21.2 mm × 250 mm, 10 μm, 110 Å; mobile phase: A: water (containing 10 mmol/L $NH_4HCO_3$), B: ACN (containing 0.05% TFA); gradient program: ramp from 10% B to 20% B over 20 min.

HPLC: 91.33% (214 nm), RT = 10.490 min (M); 7.59% (214 nm), RT = 10.803 min (M+K). Analysis conditions were the same as those for **I-A10-5FAM.**

**11.2** Synthesis of compound **VB-A12-5FAM**

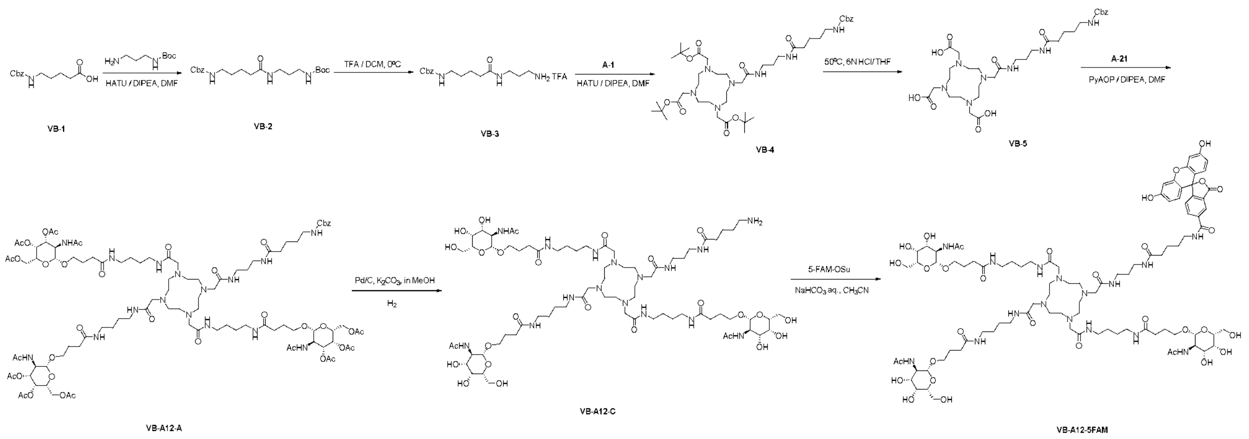


VB-1 VB-2 VB-3 VB-4 VB-5

VB-A12-A VB-A12-C VB-A12-5FAM

**VB-A12-5FAM** was synthesized by similar procedures. LCMS: (ESI) *m/z* = 679.3 [M+2H+K]/3$^+$; 666.5 [M+3H]/3$^+$.

HPLC: 73.66% (214 nm), RT = 10.736 min (M); 22.06% (214 nm), RT = 10.960 min (M+K). Analysis conditions were the same as those for **I-A10-5FAM.**

12. Synthesis of GalNAc-Ligand-Loaded Resin for Oligonucleotide Solid-Phase Synthesis

12.1 Synthesis of intermediate **A-13**

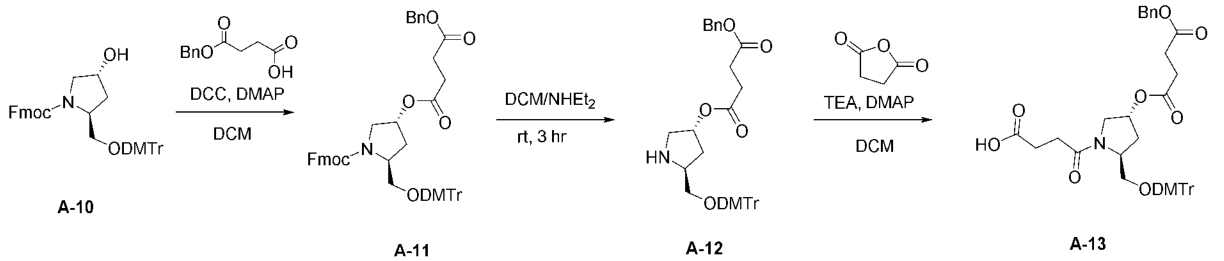


A-10 A-11 A-12 A-13

Compound **A-10** (3.2 g, 5.0 mmol, 1.0 equiv.) was dissolved in DCM (50 mL), followed by sequential addition of benzyl succinate (1.25 g, 6.0 mmol, 1.2 equiv.), DCC (1.24 g, 6.0 mmol, 1.2 equiv.), and DMAP (122 mg, 1 mmol, 0.2 equiv.). The reaction mixture was stirred at room temperature overnight, then concentrated. The residue was dissolved in DCM (20 mL) with stirring, and the insoluble white solid was filtered off. The filtrate was concentrated and dried to afford crude product **A-11** as a pale-yellow oily liquid, which was directly used in the next step.

The crude intermediate **A-11** from the previous step was dissolved in DCM (25 mL), followed by addition of diethylamine (25 mL). The mixture was stirred at room temperature for 3 h. After reaction completion confirmed by monitoring, the mixture was concentrated and dried to afford a crude product **A-12** as a pale-yellow oily liquid, which was directly used in the next step.

The crude intermediate **A-12** from the previous step was dissolved in DCM (100 mL), followed by sequential addition of succinic anhydride (1.5 g, 15 mmol), triethylamine (2.1 mL, 15 mmol), and DMAP (122 mg). The reaction mixture was stirred at room temperature overnight and concentrated. The residue was purified by reversed-phase column

chromatography to afford intermediate **A-13** (2.95 g, overall yield 83% over three steps) as a white solid. LCMS: (ESI) *m/z* = 708.2 $[M-H]^-$.

Reversed-phase purification conditions: column: 120 g C18 column; Mobile phase: A: water (containing 10 mmol/L $NH_4HCO_3$); B: ACN; Gradient: ramped from 20% B to 60% B over 20 min.

12.2 Synthesis of resin **I-A12-H-PS**

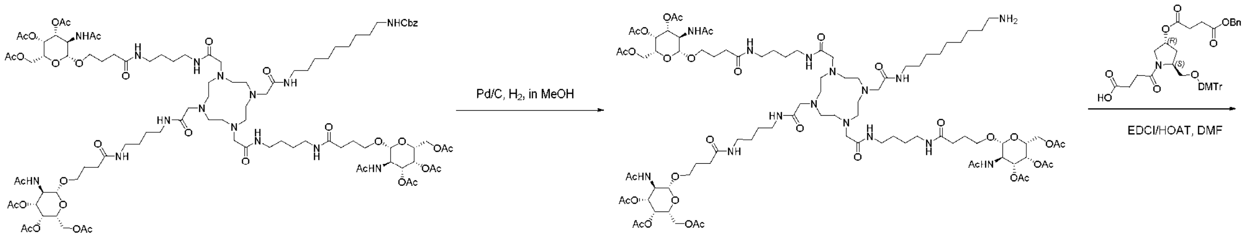


I-A12-C I-A12-D

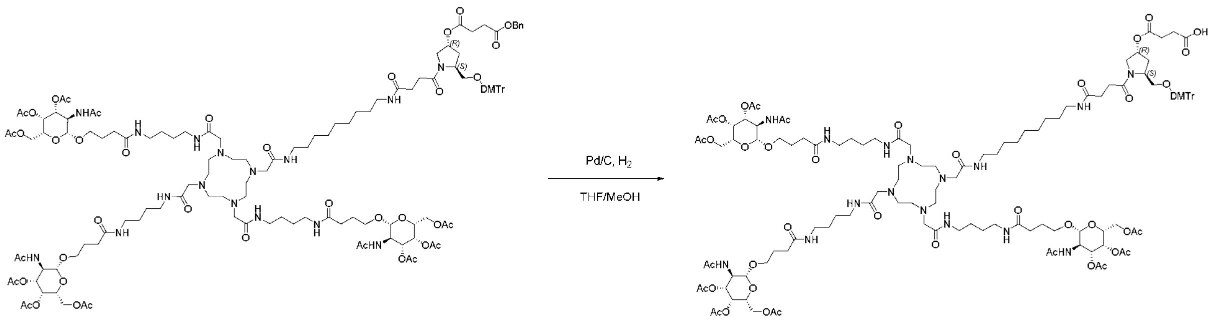


I-A12-G I-A12-H

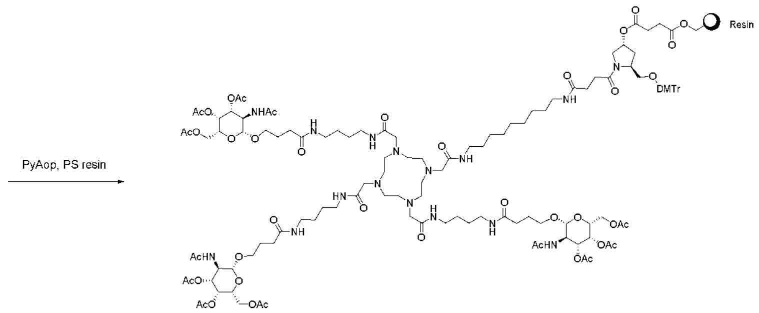


I-A12-H-PS

Synthesis of intermediate **I-A12-D**

The intermediate **I-A12-C** (900 mg, 0.422 mmol, 1.0 equiv.) was dissolved in a mixture of THF (40 mL) and methanol (5 mL), followed by addition of Pd/C (10%, 200 mg). The reaction was stirred under $H_2$ atmosphere for 3 h. The mixture was filtered and concentrated to afford a crude product **I-A12-D,** which was directly used in the next step. LCMS: (ESI) *m/z* = 1001.7 $[M+2H]/2^+$; 681.1 $[M+3H]/3^+$.

Synthesis of intermediate **I-A12-G**

The intermediate **A-13** (359 mg, 0.506 mmol, 1.2 equiv.) was dissolved in DMF (10 mL), followed by sequential addition of **I-A12-D** (863 mg, 0.422 mmol), EDCI (97 mg, 0.506 mmol), HOAt (69 mg, 0.506 mmol), and DIPEA (430 μL, 2.52 mmol). The mixture was stirred at room temperature for 3 h. Reaction completion was confirmed by LCMS, and the mixture was directly purified by preparative chromatography to afford **I-A12-G** (420 mg, yield 37%) as a white solid. LCMS: (ESI) *m/z*= 1196.1 $[M+2H]/2^+$; 797.7 $[M+3H]/3^+$. $^1H$ NMR (400 MHz, DMSO) δ 8.08-7.73 (m, 11 H, 11NH), 7.37-7.15 (m, 14 H, DMTr+Bn), 6.88 (d, *J* = 7.2 Hz, 4 H, DMTr), 5.38-5.31 (m, 1H, proline-CH-OH), 5.21 (d, *J* = 3.2 Hz, 3H, GalNAc-OCH̲O-),

5.09 (s, 2H, Ph-CH2-), 4.96 (dd, *J* = 11.6, 3.2 Hz, 3H, GalNAc), 4.48 (d, *J* = 7.6 Hz, 3H, GalNAc), 4.25-4.07 (m, 2H), 4.02 (brs, 9H), 3.93-3.70 (m, 5H), 3.73 (brs, 6H), 3.70-3.53 (m, 4H), 3.49-3.37 (m, 5H), 3.23-2.87 (m, 28H), 2.70-2.53 (m, 17H), 2.46-2.02 (m, 5H), 2.10 (s, 9H, GalNAc-OCOCH3), 1.99 (s, 9H, GalNAc-OCOCH3), 1.89 (s, 9H, GalNAc-OCOCH3), 1.78 (s, 9H, GalNAc-NHCOCH3), 1.74-1.61 (m, 7H), 1.36 (brs, 17H), 1.21 (brs, 11H).

Preparation conditions: HPLC column: Xbridge C18, 19 × 250 mm, 10 μm, 130 Å; mobile phase: A: water (containing 10 mmol/L $NH_4HCO_3$), B: acetonitrile; gradient program: ramp from 51% B to 61% B over 20 min.

Synthesis of intermediate **I-A12-H**

The intermediate **I-A12-G** (400 mg, 0.149 mmol, 1.0 equiv.) was dissolved in a mixture of THF (12 mL) and methanol (4.0 mL), followed by addition of Pd/C (10%, 100 mg). The reaction was stirred under $H_2$ atmosphere for 3 h. The mixture was filtered, concentrated, and the crude product was purified by preparative chromatography to afford **I-A12-H** (350 mg, yield 90%) as a white solid. LCMS: (ESI) *m/z* = 1321.2 $[M+H+K]/2^+$, 1150.7 $[M+2H-DMTr]/2^+$. $^1H$ NMR (400 MHz, DMSO) δ 8.08-7.73 (m, 11 H, 11NH), 7.34-7.15 (m, 9H, DMTr), 6.88 (d, *J* = 8.8 Hz, 4 H, DMTr), 5.38-5.31 (m, 1H, proline-CH-OH), 5.21 (d, *J* = 3.2 Hz, 3H, GalNAc-OCHO-), 4.95 (dd, *J* = 11.2, 3.2 Hz, 3H, GalNAc), 4.48 (d, *J* = 8.0 Hz, 3H, GalNAc), 4.25-4.07 (m, 2H), 4.02 (brs, 9H), 3.93-3.70 (m, 5H), 3.73 (brs, 6H), 3.70-3.53 (m, 5H), 3.49-3.37 (m, 5H), 3.23-2.87 (m, 28H), 2.70-2.39 (m, 19H), 2.46-2.02 (m, 5H), 2.10 (s, 9H, GalNAc-OCOCH3), 1.99 (s, 9H, GalNAc-OCOCH3), 1.89 (s, 9H, GalNAc-OCOCH3), 1.78 (s, 9H, GalNAc-NHCOCH3), 1.74-1.61 (m, 7H), 1.37 (brs, 17H), 1.22 (brs, 11H).

HPLC preparation conditions: column: Xbridge C18, 19 × 250 mm, 10 μm, 130 Å; mobile phase: A: water (containing 10 mmol/L $NH_4HCO_3$), B: acetonitrile; gradient program: ramp from 39% B to 49% B over 20 min.

HPLC purity: >99% % (214 nm), RT = 14.992 min
Mobile phase: A: water (10 mM $NH_4HCO_3$), B: acetonitrile
Gradient: 5% B (equilibration for 1 min), ramped to 95% B over 20 min, ramp to 95% B over 2min and held for 5 min.
Flow rate: 1.0 mL/min
HPLC analysis column: XBridge peptide BEH column C18, 4.6 × 150 mm, 3.5 μm, 300 Å
Column temperature: 45°

Synthesis of resin **I-A12-H-PS**

Macroporous aminomethyl resin (250 mg, 0.4 mmol/g, 2.17 equiv.) was placed in a 5 mL fritted plastic syringe, soaked in DCM (3 mL) for 30 min, and washed via aspiration-shaking-expulsion cycles with DCM (3 mL × 3) followed by DMF (3 mL × 3). A solution **of I-A12-H** (125 mg, 0.0461 mmol, 1.0 equiv.), DIPEA (39 μL, 0.231 mmol, 5.0 equiv.), and PyAOP (52 mg, 0.101 mmol, 2.2 equiv.) in DMF (3 mL) was aspirated into the pre-washed resin. The mixture was agitated at 25°C for 24 h. Kaiser test showed faint blue coloration. The resin was washed with DMF (3 mL × 6), then capped by agitation with pyridine/acetic anhydride mixture (3 mL, 2:1) for 3 h. After washing with DMF (3 mL × 6), Kaiser test indicated no coloration. The resin was further washed with DCM (3 mL × 3) and MTBE (3 mL × 3), then vacuum-dried overnight to afford off-white resin **I-A12-H-PS** (415 mg).

**Quantitation of Resin Loading**

Exactly 18.2 mg of the resin was treated with 8.0 mL of 0.48 N HCl/MeCN solution under gentle agitation for 10 min; 2.0 mL of the supernatant was collected, and LCMS analysis showed an absorption peak area of 1340.29. The loading capacity was calculated as 164.8 μmol/g using the formula:

$$\text{Loading} = \frac{\frac{1340.29 - 51.991}{3435.6} \times 8}{18.2 \times 10^{-3}}\ \mu\text{mol/g} = 164.8\ \mu\text{mol/g}$$

**Resin Cleavage**

The residual resin after DMTr removal was washed with acetonitrile (2 mL × 3), treated with concentrated aqueous ammonia (2 mL), and agitated at 60°C for 3 h. The mixture was filtered, and the filtrate was analyzed by HPLC, confirming the resin-bound product as high-purity GalNAc ligand **(I-A12-I).** LCMS: (ESI) *m/z* = 931.0 $[M+H+K]/2^+$; 911.7 $[M+2H]/2^+$; 608.3 $[M+3H]/3^+$. HPLC purity: 87.80% % (214 nm), RT = 9.588 min (M); 12.20% % (214 nm), RT = 10.398 min (M+K).

Mobile phase: A: water (10 mM $NH_4HCO_3$), B: acetonitrile

Gradient: 5%B (equilibration for 3 min), ramped to 65% B over 20 min and held for 5 min.
Flow rate: 1.0 mL/min
HPLC analysis column: XBridge peptide BEH column C18, 4.6 × 150 mm, 3.5 μm, 300 Å
Column temperature: 60°C

12.3 Synthesis of resin **I-B12-F-PS**

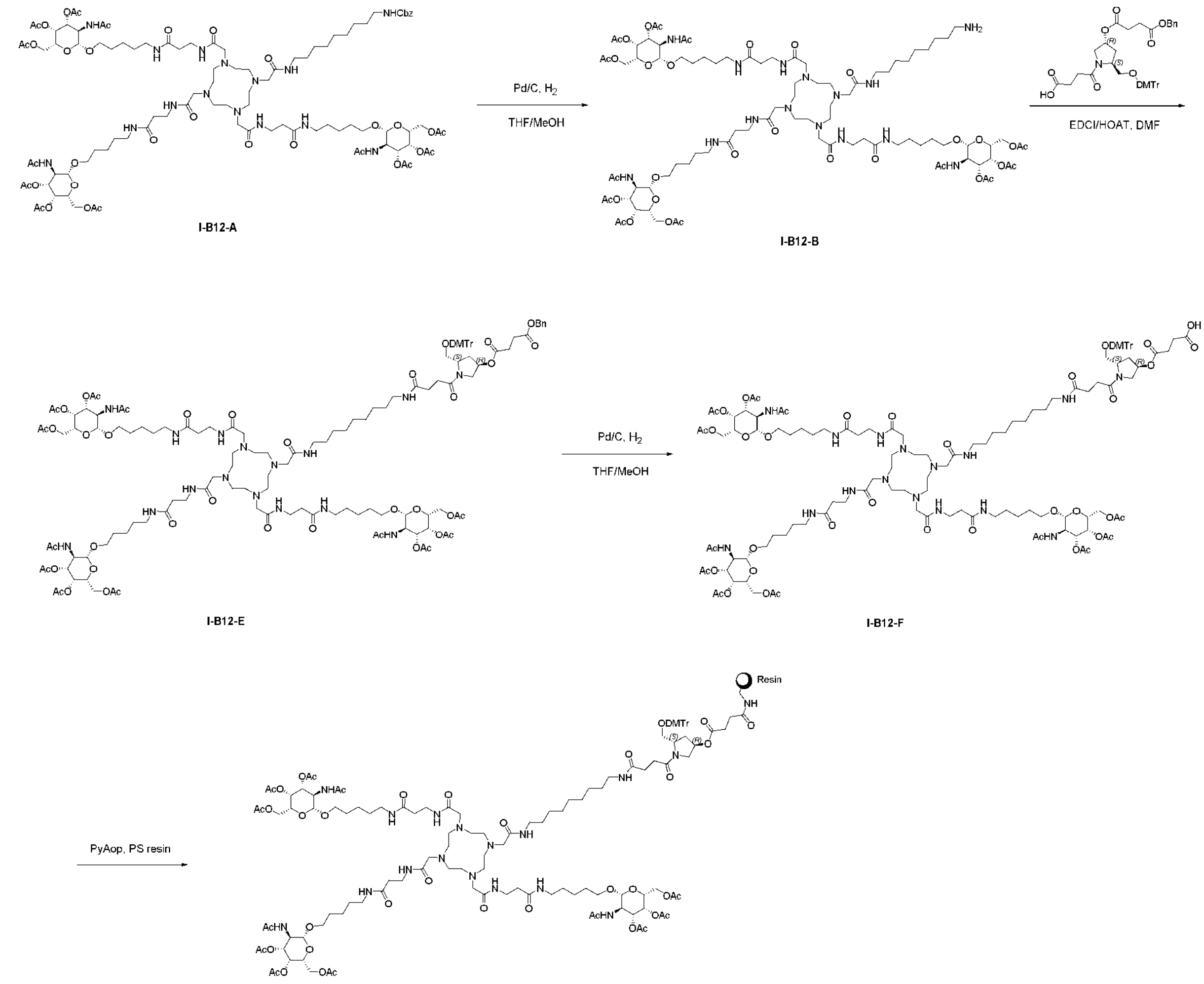


I-B12-A
I-B12-B
I-B12-E
I-B12-F
I-B12-F-PS

Synthesis of intermediate **I-B12-B**

The intermediate **I-B12-A** (400 mg, 0.187 mmol, 1.0 equiv.) was dissolved in THF (10 mL) and MeOH (1 mL). To this solution was added Pd/C (10%, 100 mg), and the reaction mixture was stirred under a hydrogen atmosphere for 16 hours. The reaction mixture was filtered and concentrated to afford **I-B12-B** as a crude product, which was used directly in the next step. LCMS: (ESI) *m/z* = 1001.7 $[M+2H]/2^+$; 668.2 $[M+3H]/3^+$.

Synthesis of intermediate **I-B12-E**

The intermediate **A-13** (153 mg, 0.22 mmol, 1.2 equiv.) was dissolved in DMF (10 mL), followed by sequential addition of **I-B12-B** (crude from the previous step), EDCI (42 mg, 0.22 mmol), HOAt (30 mg, 0.22 mmol), and DIPEA (92 μL, 0.54 mmol). The reaction mixture was stirred at room temperature for 3 hours. Reaction completion was confirmed by LCMS analysis, and the mixture was directly purified by preparative column chromatography to afford **I-B12-E** (350 mg, 72% yield) as a white solid. LCMS: (ESI) *m/z* = 1196.1 $[M+2H]/2^+$.
HPLC preparation conditions: column: Xbridge C18, 19 × 250 mm, 10 μm, 130 Å; mobile phase: A: water

(containing 10 mmol/L $NH_4HCO_3$), B: acetonitrile; gradient program: ramp from 54% B to 64% B over 20 min.

Synthesis of intermediate **I-B12-F**

**I-B12-E** (200 mg, 0.074 mmol, 1.0 equiv.) was dissolved in THF (6 mL) and MeOH (2.0 mL), followed by addition of Pd/C (10%, 40 mg). The reaction mixture was stirred under a hydrogen atmosphere for 6 hours. After filtration and concentration, the crude product was purified by preparative HPLC to afford **I-B12-F** (150 mg, 78% yield) as a white solid. LCMS: (ESI) *m/z* = 1321.2 [M+H+K]/2$^+$; 1169.7 [M+2H-DMTr]/2$^+$.

HPLC preparation conditions: column: Xbridge C18, 19 × 250 mm, 10 μm, 130 Å; mobile phase: A: water (containing 10 mmol/L $NH_4HCO_3$), B: acetonitrile; gradient program: ramp from 39% B to 49% B over 20 min.

HPLC purity: >99% % (214 nm), RT = 15.734 min
Analysis conditions were the same as those for **I-A12-H.**

Synthesis of resin **I-B12-F-PS**

**I-B12-F-PS** was synthesized following a similar procedure to resin **I-A12-H-PS.** The loading capacity was determined by the same method and recorded as 118.0 μmol/g.

$$\text{Loading} = \frac{\frac{938.75 \times 2 - 51.991}{3435.6} \times 4}{18.0 \times 10^{-3}}\ \mu mol/g\ =\ 118\ \mu mol/g$$

The residual resin after DMTr removal was washed with acetonitrile (2 mL × 3), treated with concentrated aqueous ammonia (2 mL), and agitated at 60°C for 3 h. The mixture was filtered, and the filtrate was analyzed by HPLC, confirming the resin-bound product as high-purity peptides. LCMS LCMS: (ESI) *m/z* = 930.8 [M+H+K]/2$^+$911.8 [M+2H]/2$^+$; 608.3 [M+3H]/3$^+$.

12.4 Synthesis of resin **I-A10-H-PS**

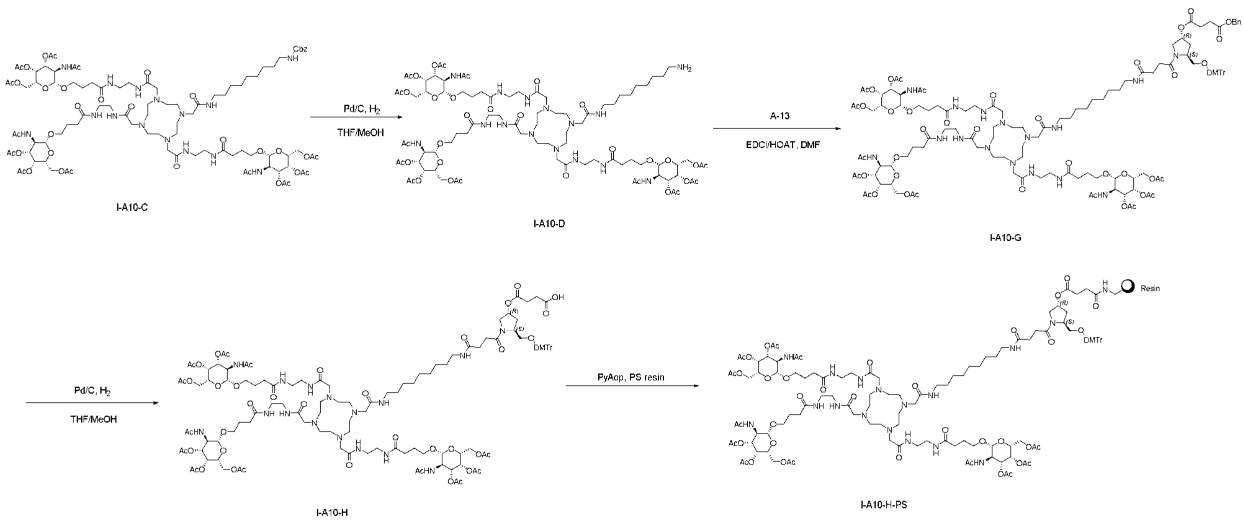


Compound **I-A10-H** was synthesized following the procedure developed for resin **I-A12-H-PS** and was loaded onto the resin using the same method. Starting from 240 mg of compound **I-A10-C,** a four-step reaction sequence afforded 243 mg of resin **I-A12-H-PS** with a loading value of 107 μmol/g.

Analysis data for compound **I-A10-H:** LCMS: (ESI) *m/z* = 1108.4 [M+2H-DMTr]/2$^+$, 1278.5 [M+H+K]/2$^+$.

HPLC: >99% (214 nm), RT = 14.84 min
Mobile phase: A: water (10 mM $NH_4HCO_3$), B: acetonitrile
Gradient: ramping from 20% B to 80% B over 20 min, ramping to 95% B over 2 min, and held at 95% B for 2 min.
Flow rate: 1.0 mL/min

HPLC analysis column: XBridge peptide BEH column C18, 4.6 × 150 mm, 3.5 μm, 300 Å
Column temperature: 45°

12.5 Synthesis of resin **I-A11-H-PS**

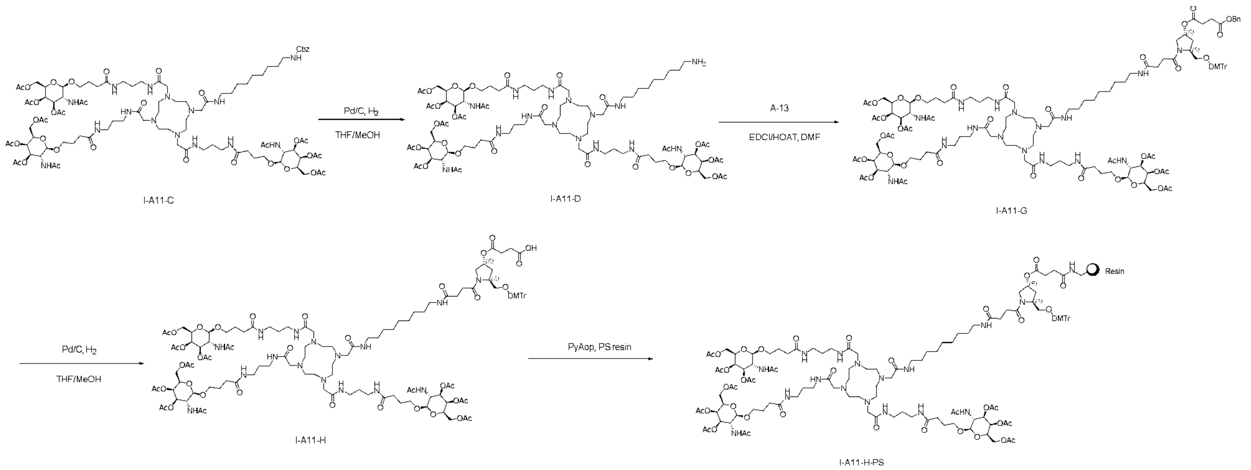


Compound **I-A11-H** was synthesized following the procedure developed for resin **I-A12-H-PS** and was loaded onto the resin using the same method. Starting from 240 mg of compound **I-A11-C,** a four-step reaction sequence afforded 174 mg of resin **I-A12-H-PS** with a loading value of 92.5 μmol/g.
Analysis data for compound **I-A11-H:** LCMS: (ESI) *m*/*z* = 1148.7 [M+H+K-DMTr]/2$^+$; 1299.8 [M+H+K]/2$^+$.
HPLC: >99% (214 nm), RT = 14.642 min. Analysis conditions were the same as those for **I-A12-H.**

12.6 Synthesis of resin **I-A12-H2-PS**

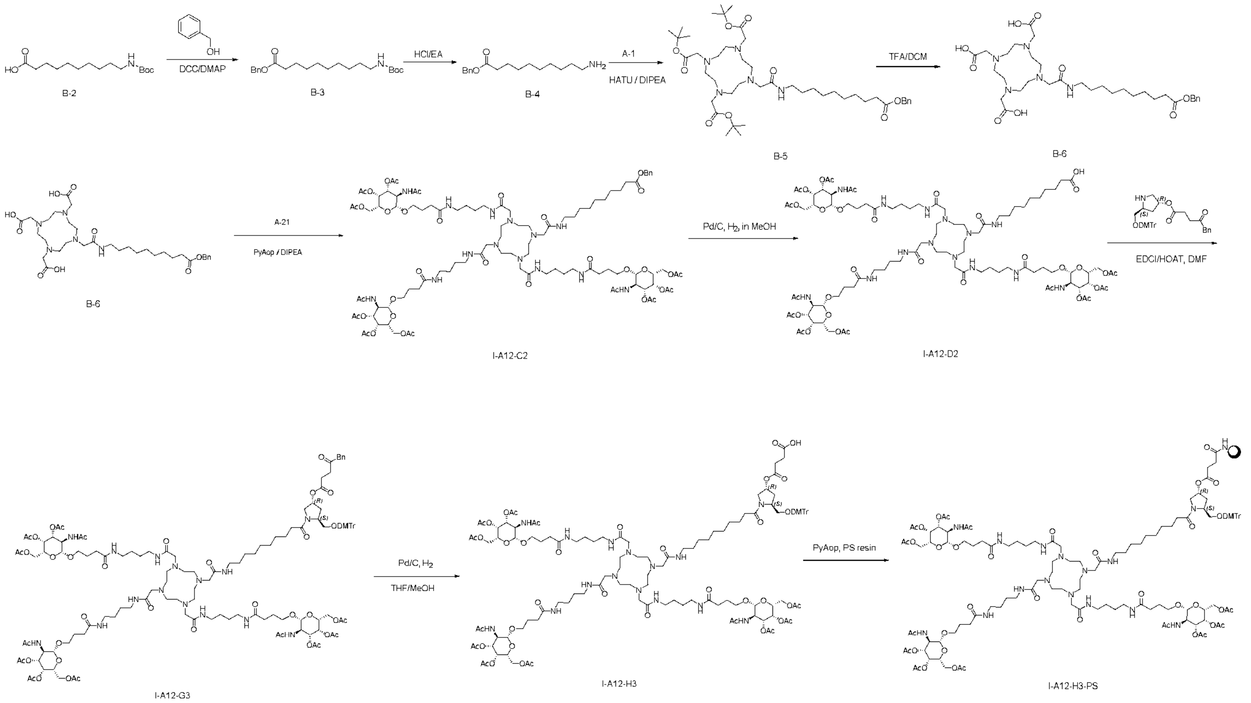

Synthesis of intermediate **B-4**

Starting material **B-2** (2.5 g, 8.7 mmol, 1.0 equiv.) was dissolved in anhydrous dichloromethane (80 mL), followed by sequential addition of benzyl alcohol (1.88 g, 17.4 mmol, 2.0 equiv.), DCC (3.59 g, 17.4 mmol, 2.0 equiv.), and DMAP (0.53 g, 4.35 mmol, 0.5 equiv.). The reaction mixture was stirred overnight. After LCMS confirmed complete conversion, the precipitate was filtered off. The filtrate was concentrated, and the resulting concentrate was dissolved in dichloromethane (30 mL) and re-concentrated (repeated twice) to afford crude **B-3** as a pale-yellow oil, which was used directly in the next step. LCMS: 400.4 $[M+Na]^+$, 278.3 $[M+H-Boc]^+$. The crude product **B-3** was dissolved in EtOAc (30 mL), treated with HCl(g)-saturated EtOAc solution (30 mL), and stirred for 2 hours at room temperature. Upon LCMS confirmation of reaction completion, the mixture was concentrated. Purification of the crude product by reversed-phase flash column chromatography afforded **B-4** (1.0 g) as a white solid. LCMS: 278.3 $[M+H]^+$.

Reversed-phase purification conditions: column: 120 g C18 column; Mobile phase: A: 0.05%TFA/$H_2O$; B: ACN; Gradient: ramped from 5% B to 65% B over 20 min.

Synthesis of intermediate **B-6**

**B-4** (600 mg, 2.16 mmol, 1.0 equiv.), **A-1** (1.36 g, 2.37 mmol, 1.1 equiv.), and HATU (0.9 g, 2.37 mmol, 1.1 equiv.) were sequentially dissolved in DMF (30 mL), followed by addition of DIEA (1.2 mL, 6.48 mmol, 3.0 equiv.). The reaction mixture was allowed to react at room temperature for 2 hours. After LCMS confirmation of complete conversion, the mixture was added with water (20 mL) and then extracted with EtOAc (2 × 50 mL). The combined organic layers were washed with saturated brine (twice), dried over anhydrous $Na_2SO_4$, filtered, and concentrated to afford crude B-5 for direct use in the next step. LCMS: 854.6 $[M+Na]^+$, 832.7 $[M+H]^+$, 416.9 $[M+2H]/2^+$. The intermediate **B-5** was dissolved in DCM (20 mL), and after complete dissolution, TFA (20 mL) was added. The resulting solution was stirred overnight at room temperature. Upon LCMS confirmation of reaction completion, the mixture was concentrated and directly purified by reversed-phase flash column chromatography to afford **B-6** (1.2 g) as a white solid. LCMS: RT: 664.5 $[M+H]^+$. NMR: $^1H$ NMR (400 MHz, DMSO) δ 8.39 (s, 1H), 7.46 - 7.27 (m, 5H), 5.08 (s, 2H), 3.85 (d, *J* = 49.5 Hz, 4H), 3.58 (s, 4H), 3.23 (s, 8H), 3.08 (dd, *J* = 17.4, 11.3 Hz, 11H), 2.34 (t, *J* = 7.4 Hz, 2H), 1.58 - 1.47 (m, 2H), 1.41 (s, 2H), 1.24 (s, 10H).

Reversed-phase purification conditions: column: 120 g C18 column; Mobile phase: A: 0.05%TFA/$H_2O$; B: ACN; Gradient: ramped from 5% B to 65% B over 20 min.

Synthesis of intermediate **I-A12-C2**

The intermediate **B-6** (250 mg, 0.37 mmol, 1.0 equiv.) was dissolved in DMF (10 mL), followed by sequential addition of **A-21** (570 mg, 1.14 mmol, 3.1 equiv.), PyAOP (772 mg, 1.48 mmol, 4.0 equiv.), and DIEA (0.8 mL, 4.44 mmol, 12.0 equiv.). The reaction mixture was stirred at room temperature for 2 hours. After LCMS confirmed reaction completion, the mixture was directly purified by preparative HPLC to afford **I-A12-C2** (370 mg) as a white solid. LCMS: 1060.3 $[M+2H]/2^+$; 707.6 $[M+3H]/3^+$.

Preparative conditions: column: Xbridge C18, 19 × 250 mm, 10 μm, 130 Å; Mobile phase: A: 10M $NH_4HCO_3/H_2O$; B: ACN; Gradient: ramped from 33% B to 43% B over 10 min.

Synthesis of intermediate **I-A12-G2**

**I-A12-C2** (370 mg, 0.17 mmol, 1.0 equiv.) was dissolved in MeOH (10 mL), followed by addition of Pd/C (10%, 40 mg) and DIEA (0.15 mL, 5.0 equiv.). The mixture was purged with $H_2$ twice and stirred under a hydrogen atmosphere for 3 hours. After LCMS confirmed complete conversion, the reaction mixture was filtered and concentrated to afford crude **I-A12-D2** (340 mg) for direct use in the next step. LCMS: 1016.0 $[M+2H]/2^+$, 851.1 $[M-GalNAc+2H]/2^+$, 677.7 $[M+3H]/3^+$. **I-A12-D2** (340 mg, 0.167 mmol, 1.0 equiv.) was dissolved in DMF (10 mL), followed by sequential addition of **A-12** (123 mg, 0.2 mmol, 1.2 equiv.), EDCI (40 mg, 0.2 mmol, 1.2 equiv.), HOAt (28 mg, 0.2 mmol, 1.2 equiv.), and DIEA (90 μL, 0.5 mmol, 3.0 equiv.). The resulting mixture was stirred overnight at room temperature. Upon LCMS confirmation of reaction completion, direct purification by preparative HPLC afforded **I-A12-G2** (220 mg) as a white solid. LCMS: 1161.1 $[M-DMTr+2H]/2^+$, 774.3 $[M-DMTr+3H]/3^+$, 303.3 $[DMTr+H]^+$.

Preparative conditions: column: Xbridge C18, 19 × 250 mm, 10 μm, 130 Å; Mobile phase: A: 10M $NH_4HCO_3/H_2O$; B: ACN; Gradient: ramped from 20% B to 80% B over 20 min.

Synthesis of compound **I-A12-H2**

**I-A12-G2** (220 mg, 0.097 mmol, 1.0 equiv.) was dissolved in MeOH (10 mL), followed by addition of Pd/C (10%, 30 mg) and DIEA (0.08 mL, 5.0 equiv.). The mixture was purged with $H_2$ (twice) and stirred under a hydrogen atmosphere

for 3 hours. After LCMS confirmed complete conversion, the reaction mixture was filtered and concentrated. Purification of the crude product by preparative HPLC afforded **I-A12-H2** (100 mg) as a white solid.

Preparative conditions: column: Xbridge C18, 19 × 250 mm, 10 μm, 130 Å; Mobile phase: A: 10M $NH_4HCO_3/H_2O$; B: ACN; Gradient: ramped from 36% B to 46% B over 20 min.

LCMS: RT: 7.788 min (the product after DMTr removal under acidic conditions), 744.2 $[M\text{-}DMTr+3H]/3^+$; RT: 8.350 min (the product after DMTr removal under acidic conditions), 756.8 $[M\text{-}DMTr+2H+K]/3^+$; RT: 15.133 min, 303.3 $[DMTr+H]^+$.

HPLC: 92.77% (214 nm), RT = 6.85 min
Mobile phase: A: water (0.05%TFA/$H_2O$), B: acetonitrile
Gradient: ramping from 20% B to 80% B over 20 min, ramping to 95% B over 2 min, and held at 95% B for 2 min.
Flow rate: 1.0 mL/min
HPLC Analysis column: XBridge peptide BEH column C18, 4.6 × 150 mm, 3.5 μm
Column temperature: 60°C
NMR: $^1H$ NMR (400 MHz, DMSO) δ 7.96 (s, 3H), 7.84 (d, *J* = 9.1 Hz, 3H), 7.77 (t, *J* = 5.2 Hz, 3H), 7.36 - 7.26 (m, 4H), 7.25 - 7.16 (m, 5H), 6.91 - 6.82 (m, 4H), 5.36 (s, 1H), 5.21 (d, *J* = 3.3 Hz, 3H), 4.96 (dd, *J* = 11.2, 3.4 Hz, 3H), 4.48 (d, *J* = 8.5 Hz, 3H), 4.19 (s, 1H), 4.02 (s, 9H), 3.87 (dd, *J* = 19.9, 8.9 Hz, 3H), 3.78 - 3.71 (m, 7H), 3.71 - 3.61 (m, 4H), 3.43 (dt, *J* = 9.8, 6.5 Hz, 4H), 3.13 - 2.89 (m, 24H), 2.62 (s, 13H), 2.47 (d, *J* = 3.0 Hz, 5H), 2.29 - 2.15 (m, 5H), 2.12 - 2.02 (m, 16H), 1.99 (s, 9H), 1.89 (s, 9H), 1.78 (s, 9H), 1.69 (dd, *J* = 14.0, 7.0 Hz, 6H), 1.51 - 1.29 (m, 17H), 1.22 (s, 11H).

Synthesis of resin **I-A12-H2-PS**

Following the procedure developed for resin **I-A12-H-PS, I-A12-H2** was loaded onto the resin. Starting from 70 mg of compound **I-A12-H2,** 203.2 mg of resin **I-A12-H-PS** was afforded with a loading value of 107 μmol/g.

12.7 Synthesis of resin **I-B12-F2-PS**

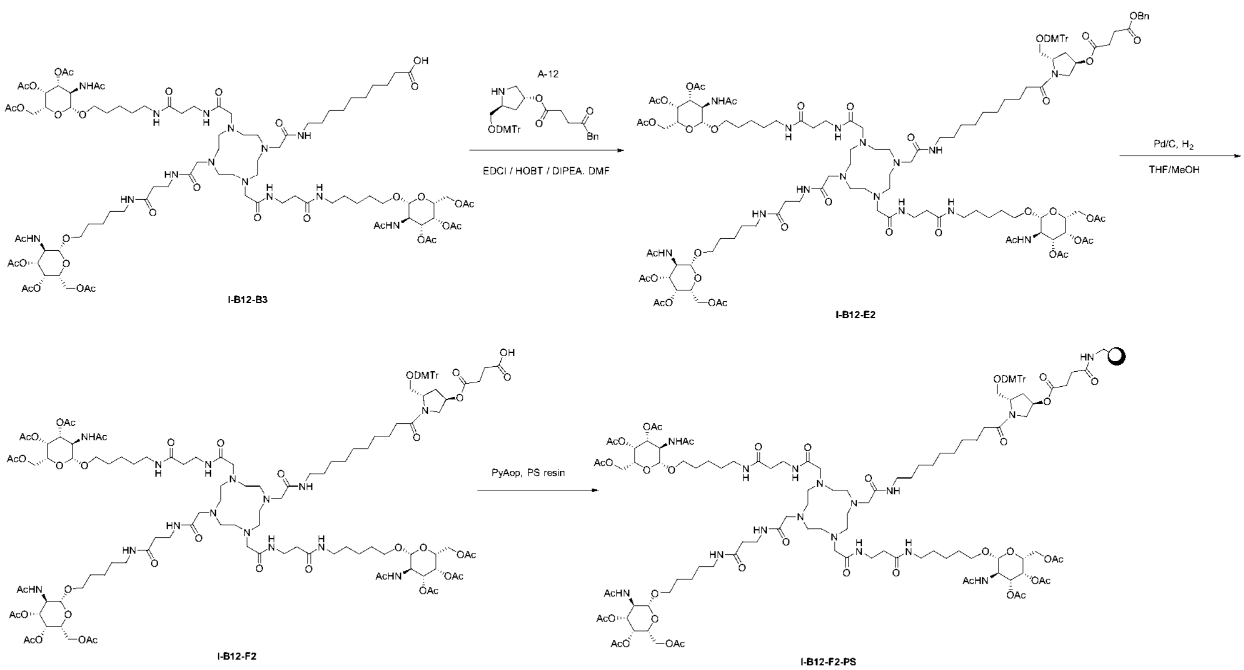

I-B12-B3
I-B12-E2
I-B12-F2
I-B12-F2-PS

Synthesis of intermediate **I-B12-E2**

**I-B12-B3** (3 g, 1.5 mmol) was dissolved in DMF (10 mL), followed by sequential addition of **A-12** (1 g, 1.7 mmol), EDCI (370 mg, 1.9 mmol), HOAt (266 mg, 1.9 mmol), and DIPEA (1.06 g, 8.3 mmol). The reaction mixture was stirred at room temperature for 3 hours. After LCMS confirmed reaction completion, direct purification by preparative HPLC afforded I-B12-E2 (2 g, 50% yield) as a white solid. LCMS: (ESI) *m/z* = 1161.2 $[M\text{-}DMTr+2H]/2^+$.

Preparation conditions: column: Xbridge C18, 19 × 250 mm, 10 μm, 130 Å; mobile phase: A: water (containing 10 mmol/L $NH_4HCO_3$), B: acetonitrile; gradient program: ramp from 10% B to 90% B over 10 min.

Synthesis of intermediate **I-B12-F2**

**I-B12-E2** (2 g, 0.76 mmol) was dissolved in MeOH (40 mL), followed by addition of 10% Pd/C (400 mg). The reaction system was purged with $H_2$ (twice) and stirred under a hydrogen atmosphere at room temperature for 3 hours. After LCMS confirmed reaction completion, the mixture was filtered and concentrated. Direct purification of the crude product by preparative HPLC afforded **I-B12-F2** (730 mg, 39% yield) as a white solid. LCMS: (ESI) *m*/*z* = 1115.7 [M-DMTr+2H]/$2^+$;1285.7 [M+K+H]/$2^+$.

$^1H$ NMR (400 MHz, DMSO) δ 8.26-8.05 (m, 11H), 7.31-7.17 (m, 9H), 6.89-6.85 (m, 4H), 5.36-5.34 (m, 1H), 5.21-5.20 (m, 3H), 4.98-4.95 (m, 3H), 4.49-4.47 (m, 3H), 4.23-4.19 (m, 1H), 4.02 (s, 9H), 3.90-3.83 (m, 3H), 3.73-3.65 (m, 10H), 3.42-3.39 (m, 4H), 3.27-3.26 (m, 7H), 3.01-2.95 (m, 16H), 2.68-2.53 (m, 12H), 2.47-2.42 (m, 3H), 2.24-2.22 (m, 11H), 2.09 (s, 9H), 1.99 (s, 9H), 1.89 (m, 9H), 1.88-1.85 (m, 1H), 1.78 (s, 9H), 1.45-1.36 (m, 17H), 1.25-1.22 (m, 17H).

Preparation conditions: column: Xbridge C18, 19 × 250 mm, 10 μm, 130 Å; mobile phase: A: water (containing 10 mmol/L $NH_4HCO_3$), B: acetonitrile; gradient program: ramp from 36% B to 46% B over 20 min.

Synthesis of resin **I-B12-F2-PS**

Following the procedure developed for resin **I-B12-F-PS, I-B12-F2** was loaded onto the resin. Starting from 700 mg of compound **I-B12-F2,** 2 g of resin **I-B12-F2-PS** was afforded with a loading value of 136.5 μmol/g.

Synthesis of resin **I-B12-F3-PS**

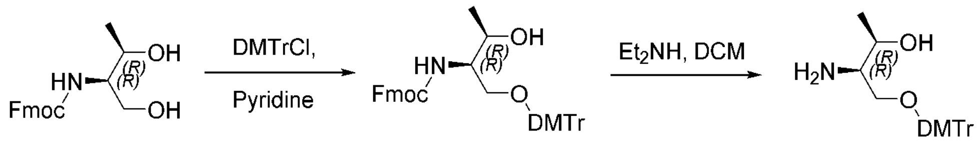


B-7

B-8

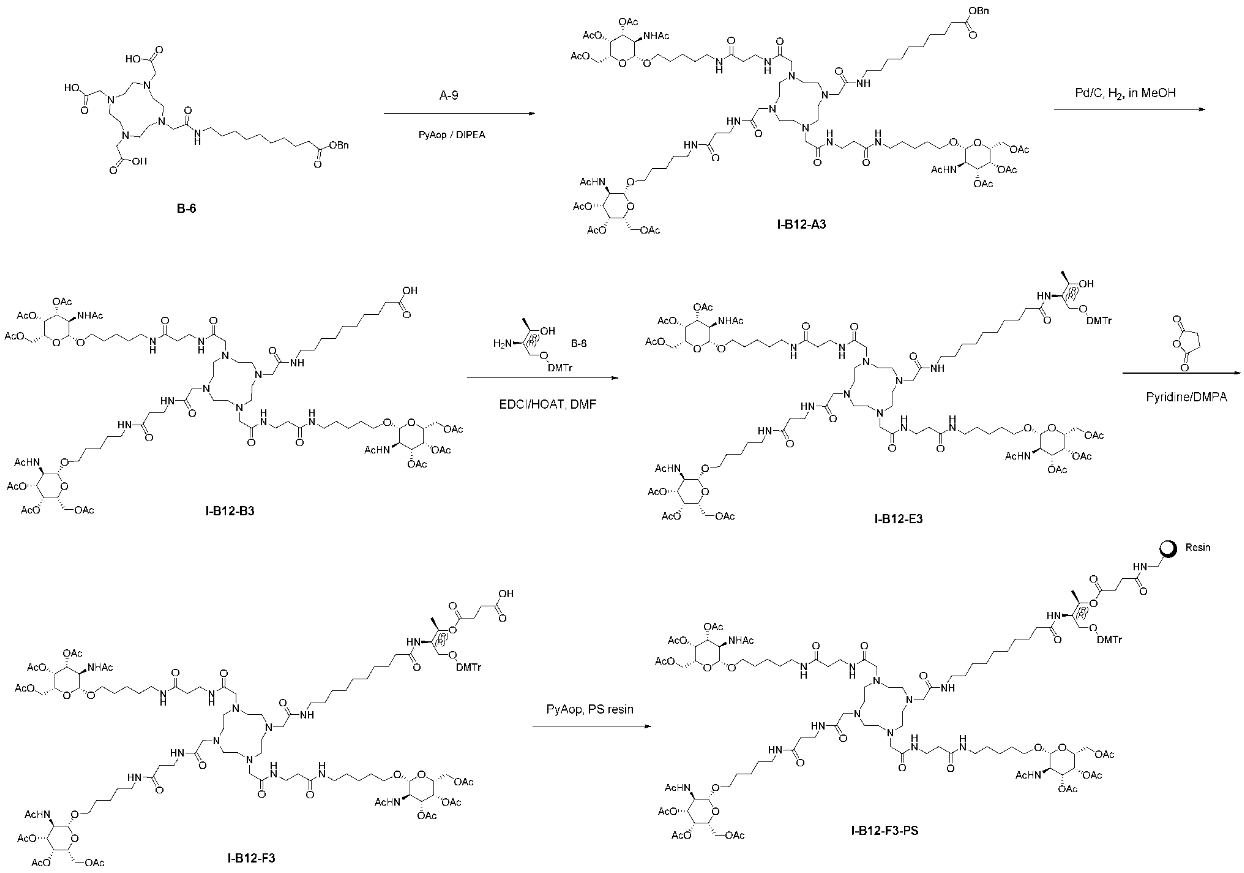


Synthesis of intermediate **B-8**

Fmoc-L-threoninol (1.6 g, 4.89 mmol, 1.0 equiv.) was dissolved in pyridine (20 mL), followed by addition of DMTrCl (2.48 g, 7.34 mmol, 1.5 equiv.). The solution was stirred overnight at room temperature. After LCMS confirmed complete conversion, the mixture was diluted with EtOAc (20 mL) and treated with aqueous $CuSO_4$ solution (5.0 g in 100 mL $H_2O$). The aqueous layer was extracted with EtOAc (3 × 20 mL). The combined organic layers were washed with saturated brine (2 × 50 mL), dried over $Na_2SO_4$, filtered, and concentrated. Purification of the crude product by flash chromatography afforded intermediate **B-7** (1.45 g). LCMS: 633.3 $[M+Na]^+$. The intermediate **B-7** (1.45 g, 2.3 mmol, 1.0 equiv.) was dissolved in DCM (30 mL), treated with diethylamine (30 mL), and stirred at room temperature for 3 hours. After LCMS confirmed reaction completion, the mixture was concentrated to afford a crude product. Purification of the crude product by flash column chromatography afforded **B-8** (600 mg) as a pale-yellow oil.

NMR: $^1H$ NMR (400 MHz, DMSO) δ 7.40 (d, *J* = 7.4 Hz, 2H), 7.35 - 7.17 (m, 7H), 6.88 (d, *J* = 8.9 Hz, 4H), 4.10 (s, 3H), 3.73 (s, 6H), 3.70 - 3.58 (m, 1H), 3.01 (dd, *J* = 8.8, 5.3 Hz, 1H), 2.83 (dd, *J* = 8.9, 6.2 Hz, 1H), 2.56 (dt, *J* = 14.6, 7.2 Hz, 1H), 0.96 (t, *J* = 9.7 Hz, 3H).

Synthesis of intermediate **I-B12-A3**

Compound **B-6** (500 mg, 0.75 mmol, 1.0 equiv.) was dissolved in DMF (20 mL), followed by sequential addition of **A-9** (1.18 g, 2.34 mmol, 3.1 equiv.), PyAOP (1.57 g, 3.0 mmol, 4.0 equiv.), and DIEA (1.6 mL, 9.0 mmol, 12.0 equiv.). The reaction mixture was stirred at room temperature for 2 hours. After LCMS confirmed reaction completion, direct purification by preparative HPLC afforded **I-B12-A3** (860 mg) as a white solid. LCMS: 1061.2 $[M+2H]/2^+$, 707.7 $[M+3H]/3^+$.

Preparation conditions: column: Xbridge C18, 19 × 250 mm, 10 μm, 130 Å; mobile phase: A: 10M $NH_4HCO_3/H_2O$, B: acetonitrile; gradient program: ramp from 20% B to 62% B over 14 min.

Synthesis of intermediate **I-B12-E3**

**I-B12-A3** (860 mg, 0.40 mmol, 1.0 equiv.) was dissolved in MeOH (20 mL), followed by addition of 10% Pd/C (100 mg) and DIEA (0.34 mL, 5.0 equiv.). The mixture was purged with $H_2$ (twice) and stirred under a hydrogen atmosphere for 3 hours. After LCMS confirmed reaction completion, the mixture was filtered and concentrated to afford crude **I-B12-B3** (750

mg) for direct use in the next step. LCMS: 1015.8 $[M+2H]/2^+$, 677.8 **$[M+3H]/3^+$.I-B12-B3** (750 mg, 0.37 mmol, 1.0 equiv.) was dissolved in DMF (15 mL), followed by sequential addition **of B-8** (180 mg, 0.45 mmol, 1.2 equiv.), EDCI (87 mg, 0.45 mmol, 1.2 equiv.), HOAt (62 mg, 0.45 mmol, 1.2 equiv.), and DIEA (200 µL, 1.11 mmol, 3.0 equiv.). The resulting mixture was stirred overnight at room temperature. Upon LCMS confirmation of reaction completion, direct purification by preparative HPLC afforded **I-B12-E3** (350 mg) as a white solid. LCMS: 1059.4 $[M\text{-}DMTr +2H]/2^+$, 707.0 $[M\text{-}DMTr +3H]/3^+$, 303.3 $[DMTr+H]^+$.

Preparation conditions: column: Xbridge C18, 19 × 250 mm, 10 µm, 130 Å; mobile phase: A: 10M $NH_4HCO_3/H_2O$, B: acetonitrile; gradient program: ramp from 41% B to 51% B over 20 min.

Synthesis of compound **I-B12-F3**

**I-B12-E3** (350 mg, 0.144 mmol, 1.0 equiv.) was dissolved directly in pyridine (5 mL), followed by sequential addition of succinic anhydride (280 mg, 2.88 mmol, 20.0 equiv.) and DMAP (176 mg, 1.44 mmol, 10.0 equiv.). The reaction mixture was stirred at room temperature for 3 days. After LCMS confirmed complete conversion, direct purification by preparative HPLC afforded **I-B12-F3** (100 mg) as a white solid.

Preparation conditions: column: Xbridge C18, 19 × 250 mm, 10 µm, 130 Å; mobile phase: A: 10M $NH_4HCO_3/H_2O$, B: acetonitrile; gradient program: ramp from 38% B to 48% B over 20 min.

LCMS: RT: 14.095 min(the product after DMTr removal under acidic conditions), 1109.6 $[M\text{-}DMTr+2H]/2^+$, 740.3 $[M\text{-}DMTr+3H]/3^+$; RT: 14.519 min(the product after DMTr removal under acidic conditions), 1128.4 $[M\text{-}DMTr+H+K]/2^+$, 752.2 $[M\text{-}DMTr+2H+K]/3^+$; RT: 16.645 min, 303.3 $[DMTr+H]^+$.

HPLC: >95% (214 nm), RT = 11.24 min (M+H) + 11.63 min (M+K)
Mobile phase: A: water (0.05%TFA/$H_2O$), B: acetonitrile
Gradient: ramping from 20% B to 80% B over 20 min, ramping to 95% B over 2 min, and held at 95% B for 2 min.
Flow rate: 1.0 mL/min
HPLC Analysis column: XBridge peptide BEH column C18, 4.6 × 150 mm, 3.5 µm
Column temperature: 20°C
NMR: $^1H$ NMR (400 MHz, DMSO) δ 7.86 (s, 7H), 7.38 - 7.26 (m, 4H), 7.21 (d, *J* = 8.8 Hz, 5H), 6.90 - 6.84 (m, 4H), 5.21 (d, *J* = 3.1 Hz, 3H), 4.97 (d, *J* = 11.0 Hz, 3H), 4.48 (d, *J* = 8.4 Hz, 3H), 4.02 (s, 9H), 3.92 - 3.81 (m, 3H), 3.70 (d, *J* = 22.2 Hz, 9H), 3.38 (d, *J* = 6.5 Hz, 5H), 3.27 (s, 7H), 3.01 (s, 17H), 2.60 (s, 10H), 2.24 (s, 8H), 2.10 (s, 11H), 1.99 (s, 9H), 1.89 (s, 9H), 1.77 (s, 9H), 1.45 (s, 9H), 1.36 (s, 9H), 1.20 (s, 18H), 1.02 (d, *J* = 6.2 Hz, 3H).

Synthesis of resin **I-B12-F3-PS**

Following the procedure developed for resin **I-A12-H-PS, I-B12-F3** was loaded onto the resin. Starting from 70 mg of compound **I-B12-F3,** 206.4 mg of resin **I-B12-F3-PS** was afforded with a loading value of 105 µmol/g.

**13. Oligonucleotide Conjugates Synthesis using GalNAc Ligand-Loaded Resins**

13.1 Solid-phase synthesis of GalNAc-siRNA-SS-3'using a DNA/RNA synthesizer according to the following protocol

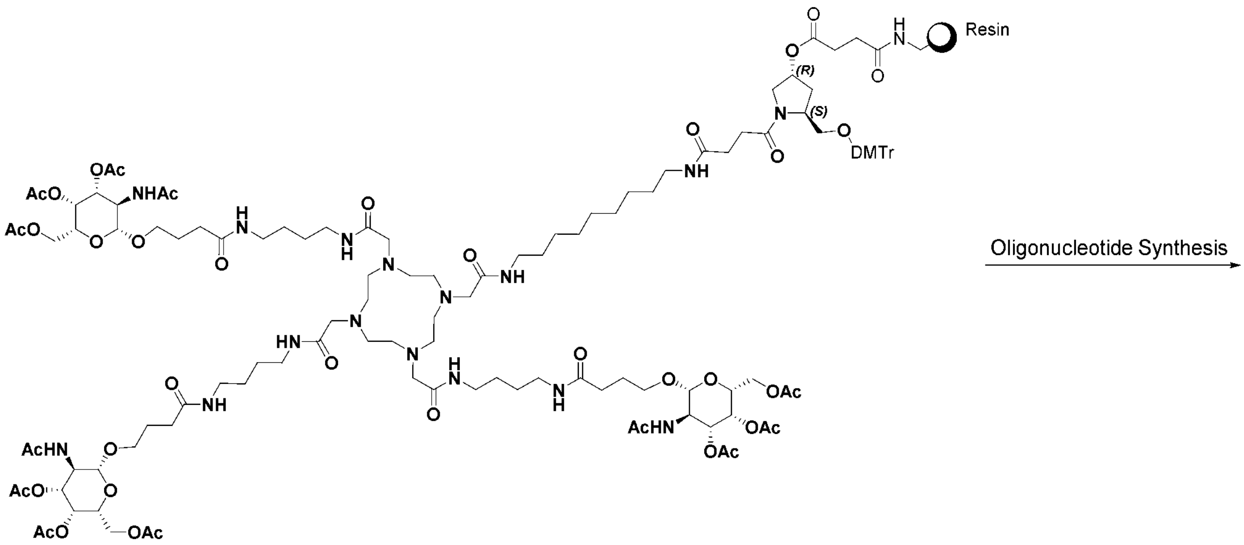


**I-A12-H-PS**

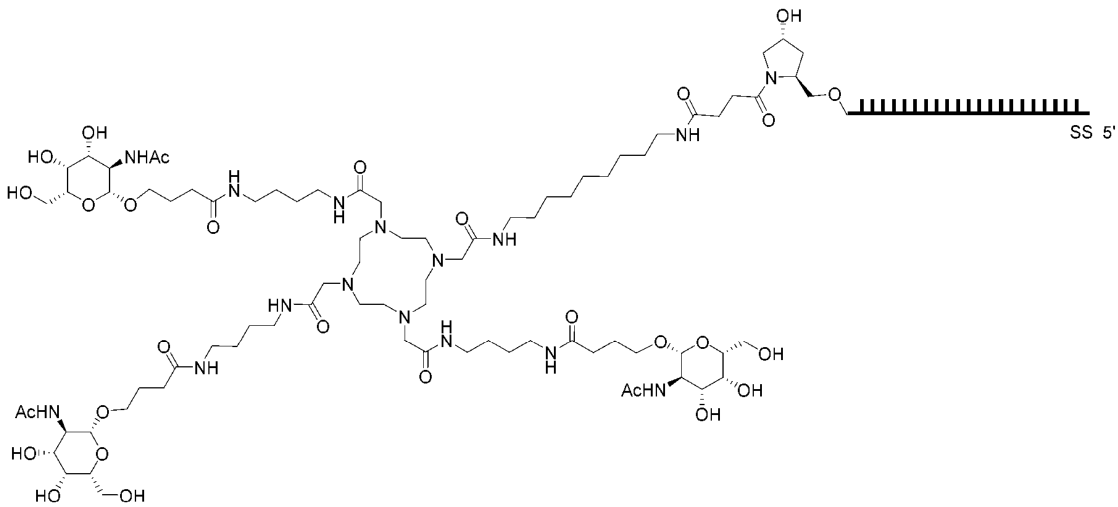

**I-A12-I-siRNA-SS'**

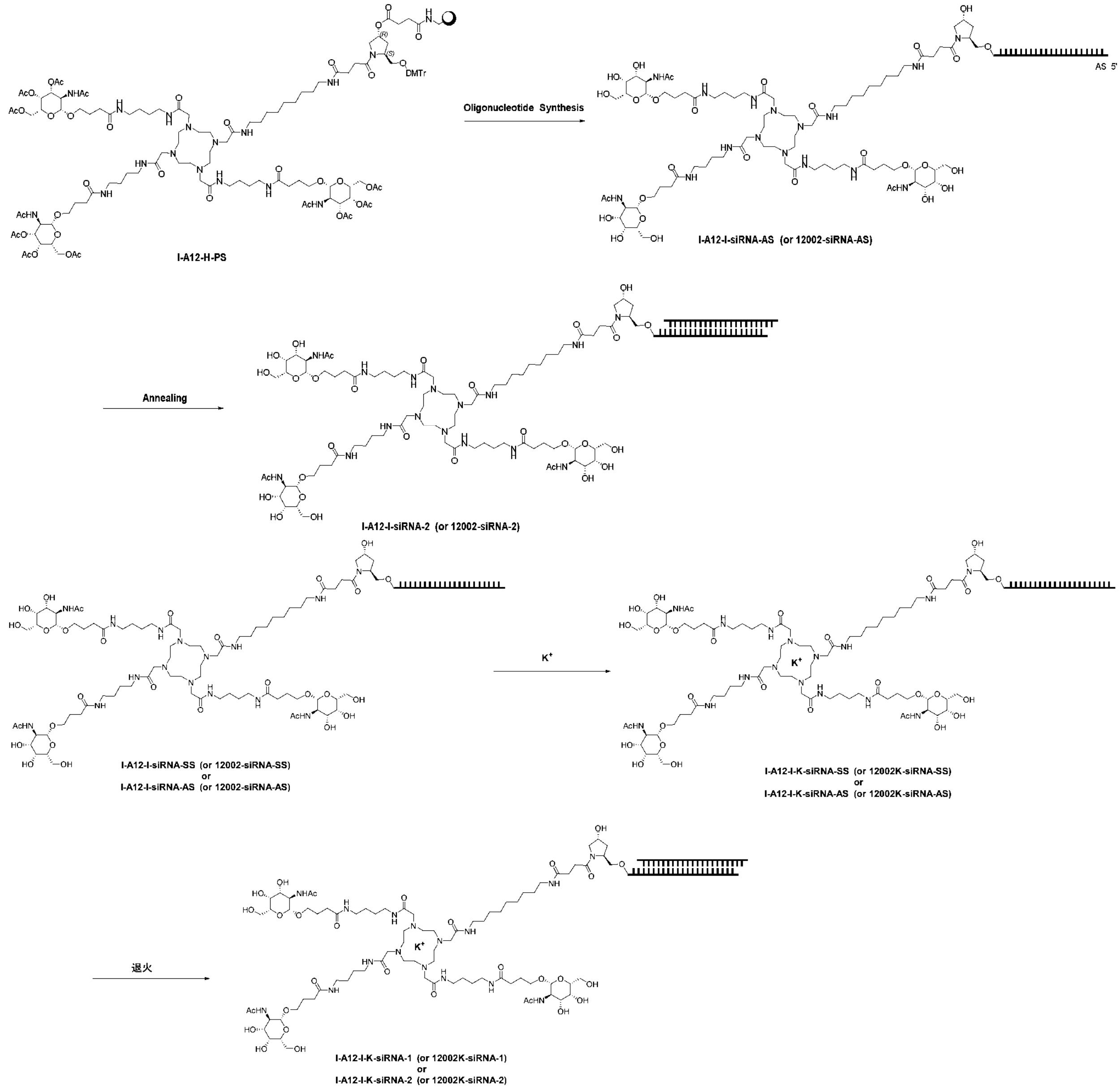


I-A12-H-PS

I-A12-I-siRNA-AS (or 12002-siRNA-AS)

I-A12-I-siRNA-2 (or 12002-siRNA-2)

I-A12-I-siRNA-SS (or 12002-siRNA-SS)
or
I-A12-I-siRNA-AS (or 12002-siRNA-AS)

I-A12-I-K-siRNA-SS (or 12002K-siRNA-SS)
or
I-A12-I-K-siRNA-AS (or 12002K-siRNA-AS)

I-A12-I-K-siRNA-1 (or 12002K-siRNA-1)
or
I-A12-I-K-siRNA-2 (or 12002K-siRNA-2)

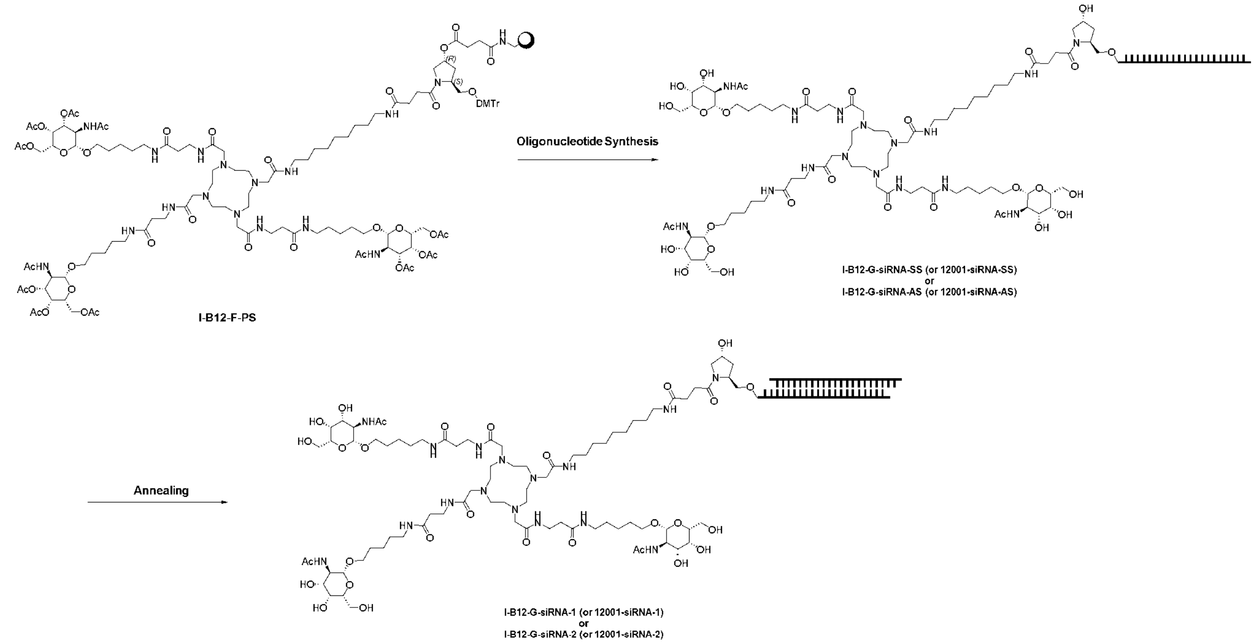


I-B12-F-PS

I-B12-G-siRNA-SS (or 12001-siRNA-SS)
or
I-B12-G-siRNA-AS (or 12001-siRNA-AS)

I-B12-G-siRNA-1 (or 12001-siRNA-1)
or
I-B12-G-siRNA-2 (or 12001-siRNA-2)

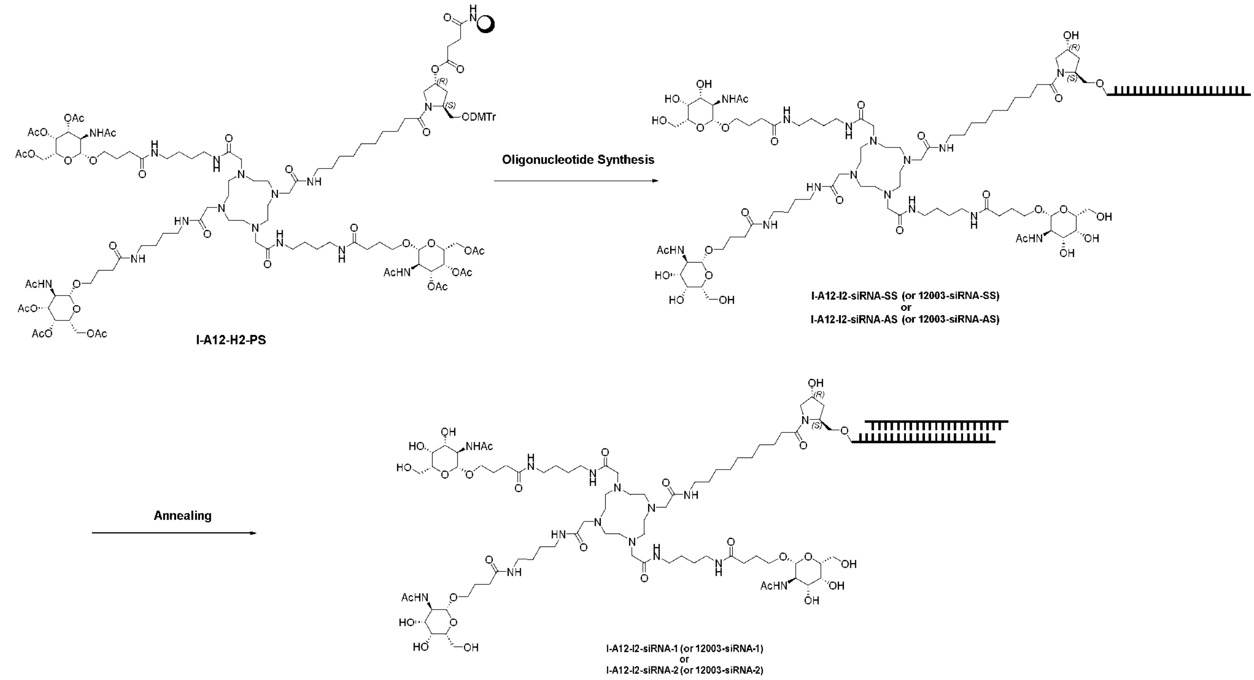


I-A12-H2-PS

I-A12-I2-siRNA-SS (or 12003-siRNA-SS)
or
I-A12-I2-siRNA-AS (or 12003-siRNA-AS)

I-A12-I2-siRNA-1 (or 12003-siRNA-1)
or
I-A12-I2-siRNA-2 (or 12003-siRNA-2)

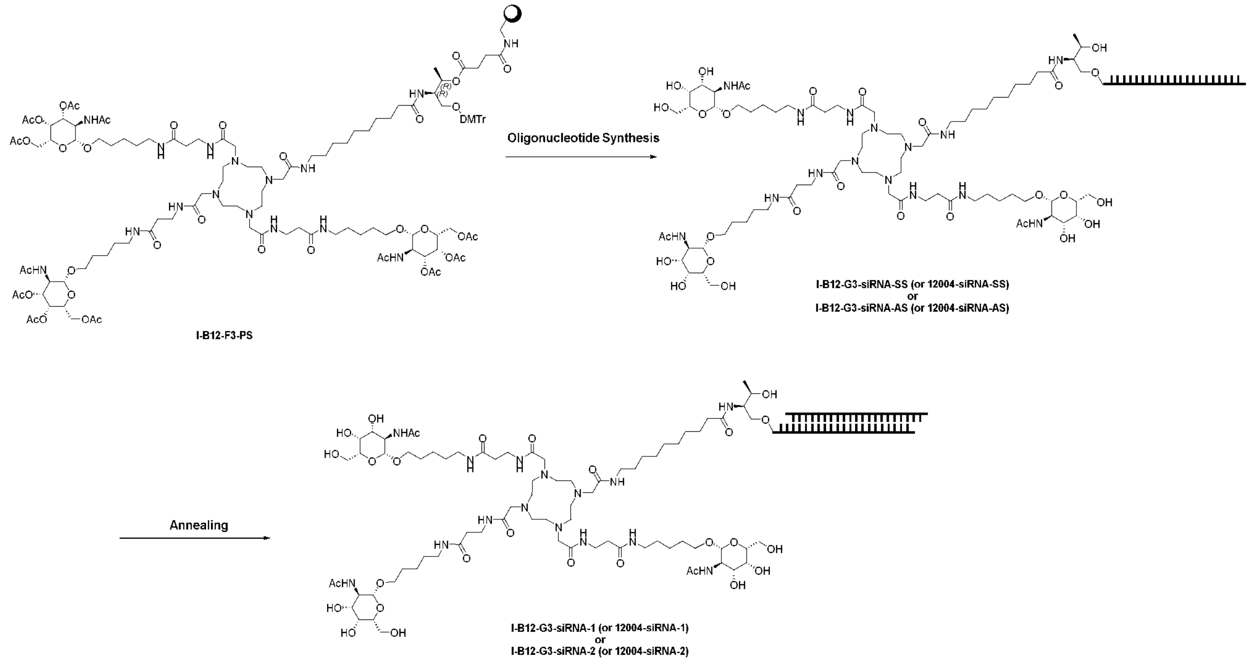


I-B12-F3-PS

I-B12-G3-siRNA-SS (or 12004-siRNA-SS)
or
I-B12-G3-siRNA-AS (or 12004-siRNA-AS)

I-B12-G3-siRNA-1 (or 12004-siRNA-1)
or
I-B12-G3-siRNA-2 (or 12004-siRNA-2)

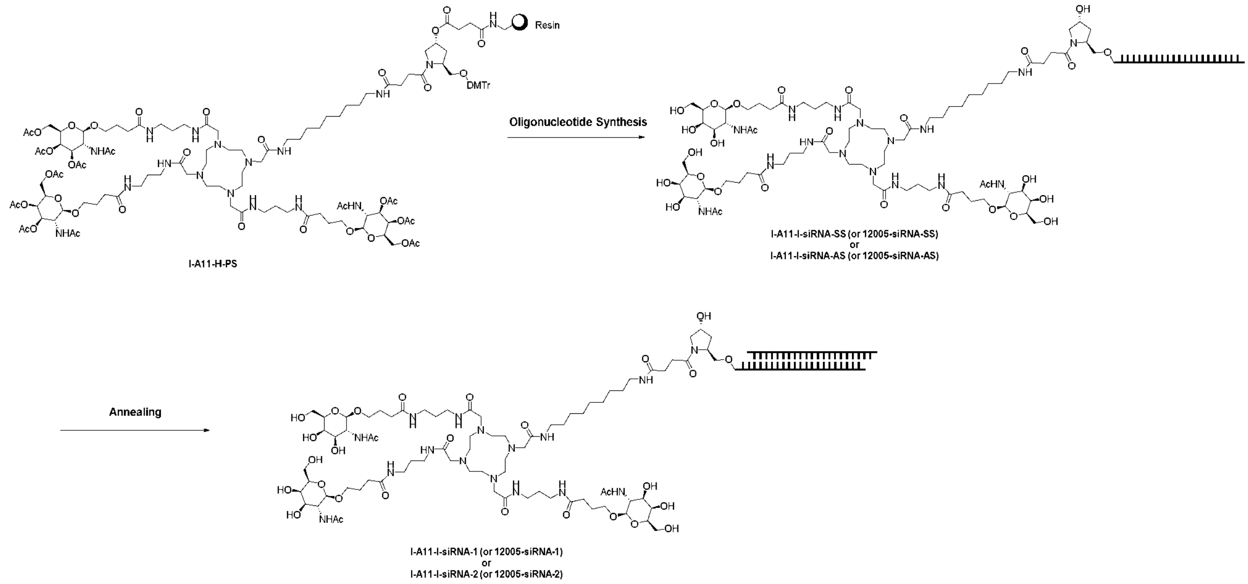


I-A11-H-PS

I-A11-I-siRNA-SS (or 12005-siRNA-SS)
or
I-A11-I-siRNA-AS (or 12005-siRNA-AS)

I-A11-I-siRNA-1 (or 12005-siRNA-1)
or
I-A11-I-siRNA-2 (or 12005-siRNA-2)

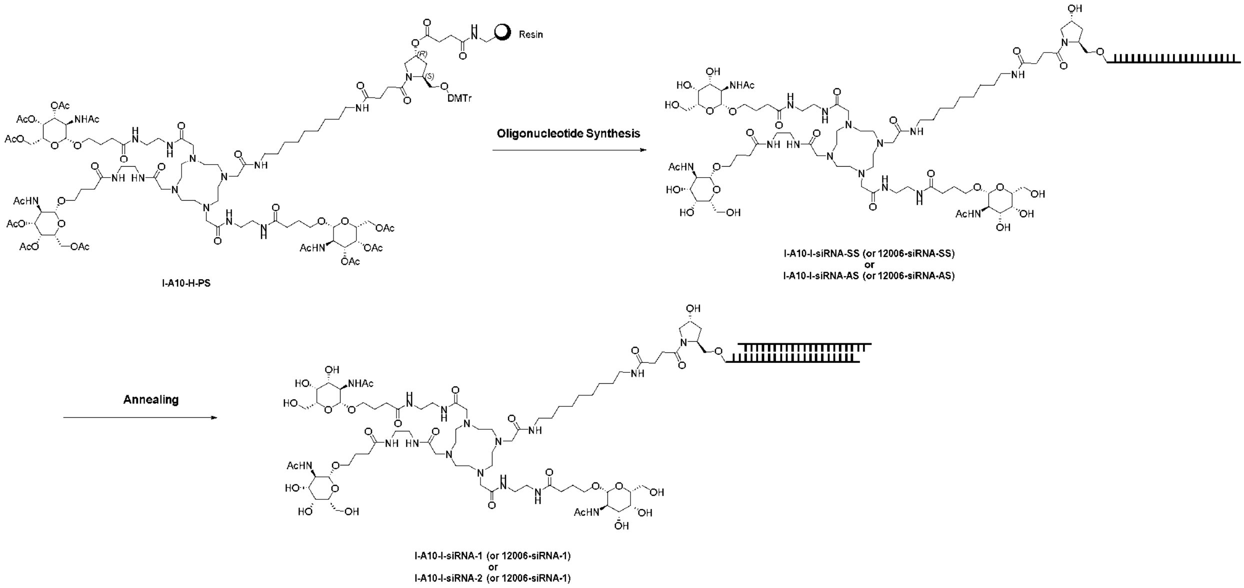


I-A10-H-PS

I-A10-I-siRNA-SS (or 12006-siRNA-SS) or I-A10-I-siRNA-AS (or 12006-siRNA-AS)

I-A10-I-siRNA-1 (or 12006-siRNA-1) or I-A10-I-siRNA-2 (or 12006-siRNA-1)

The synthesis for siRNA was identical to standard phosphoramidite solid-phase synthesis (all synthesized by Suzhou Biosyntech Co., Ltd.), comprising four iterative steps: deprotection, coupling, capping, and oxidation/sulfurization. For the sense strand incorporating the GalNAc delivery ligand, prefunctionalized amino resin supports **(I-A12-H-PS, I-B12-F-PS, I-A12-H2-PS, I-B12-F3-PS, I-A11-H-PS, I-A10-H-PS** resins) were employed to replace the usual Universal-CPG carrier. Synthesis initiated from the solid support, with nucleotides sequentially added in the 3'→5' direction using commercially available phosphoramidite monomers (2'-F RNA, 2'-OMe RNA; Shanghai Hongene Biotechnology Co., Ltd.). Upon completion of solid-phase synthesis, the solid support was transferred to a centrifuge tube and incubated with 28% $NH_4OH$ in ethanol/$H_2O$ (3:1 v/v) at 50°C for 16 hours to cleave the oligonucleotide from the solid support into solution. The supernatant was centrifuged and transferred to a new tube, concentrated to dryness, and redissolved in deionized water. Purification was performed using a C18 reversed-phase column with a mobile phase of 0.1 M TEAA and acetonitrile. The target oligonucleotide fraction was collected, lyophilized, identified as the desired product by LC-MS, and quantified by UV spectroscopy (260 nm). The obtained sense and antisense strands were annealed at an equimolar ratio to form the complementary double-stranded siRNA (dsRNA), which was subsequently adjusted to the desired concentration.

## Huh7 Transfection

On day 1, a certain amount of the diluted compound was added to an equal volume of the RNAiMAX-Opti-MEM mixture, mixed well, and incubated at room temperature for 15 minutes. Huh7 cells were washed with DPBS, digested with trypsin, and adjusted to a density of $2\times10^5$ cells/mL. During cell seeding, 10 μL of the mixture of Opti-MEM RNAiMAX and the compound was added to the cell culture plate. The cells were then seeded into a 96-well plate at a density of 20,000 cells per well (90 μL/well), with a final culture volume of 100 μL per well. The cells were cultured in a 5% $CO_2$ incubator at 37 °C for 24 hours.

## HepG2 Transfection

On day 1, a certain amount of the diluted compound was added to an equal volume of the RNAiMAX-Opti-MEM mixture, mixed well, and incubated at room temperature for 15 minutes. HepG2 cells were washed with DPBS, digested with trypsin, and adjusted to a density of $2.2\times10^5$ cells/mL. During cell seeding, 10 μL of the mixture of Opti-MEM RNAiMax and the compound was added to the cell culture plate. The cells were then seeded into a 96-well plate at a density of 20,000 cells per well (90 μL/well), with a final culture volume of 100 μL per well. The cells were cultured in a 5% $CO_2$ incubator at 37 °C for 24 hours.

## RNA Extraction and Reverse Transcription

RNA was extracted following the instructions of the RNA extraction kit (Qiagen, 74182). Subsequently, following the instructions of the FastKing cDNA First Strand Synthesis Kit (TianGen, KR116-02), the extracted RNA was reverse transcribed into cDNA.

**QPCR Detection and Data Analysis**

qPCR was used to detect the target gene cDNA. The qPCR reaction system preparation is shown in Table 8. GAPDH was used as the internal reference gene. qPCR was performed in 384-well plates. The qPCR reaction program was: 50 °C for 2 minutes, 95 °C for 2 minutes; then 95 °C for 5 seconds, 60 °C for 30 seconds, for 40 cycles; 95 °C for 15 seconds, 60 °C for 1 minute, 95 °C for 15 seconds.

**Table 8. RT-PCR Reaction System**

| Component | Concentration | Volume ( μ L)/well |
|---|---|---|
| FastStart Universal Probe Mast | 2× | 5 |
| KHK/GAPDH forward primer | 10 μM | 0.4 |
| KHK /GAPDH reverse primer | 10 μM | 0.4 |
| RNase-free Water | / | 2.2 |
| Total | / | 8 |

Data Analysis: ΔΔCT method (Comparative CT method). After qRT-PCR, the CT value of the internal reference is also recorded, referred to as Ct (GAPDH), and the CT value of the sample is referred to as Ct (sample).

$$\Delta Ct\ (sample) = Ct\ (sample) - Ct\ (GAPDH)$$

$$\Delta Ct\ (control) = Ct\ (control) - Ct\ (GAPDH)$$

$$\Delta\Delta Ct = \Delta Ct\ (sample) - \Delta Ct\ (control)$$

$$Relative\ gene\ expression = 2\text{^}{-}\Delta\Delta Ct$$

Inhibition % = (1 - Relative expression of sample / Average relative expression of control group) * 100

**Example 1. Study on Hepatic Cellular Uptake Activity of Poly-GalNAc Ligands with Macrocyclic Scaffold Structure**

Flow cytometry was applied to evaluate the uptake efficiency of FAM-labeled test articles in HepG2 cells.

The human hepatocellular carcinoma cell line (HepG2) was purchased from ATCC (USA). Cells were cultured in DMEM medium (Gibco, ThermoFisher Scientific, USA) containing 10% fetal bovine serum (ExCell Bio, South America), 100 units/mL penicillin, and 100 units/mL streptomycin, placed in a 37 °C, 5% $CO_2$ incubator.

On day 0, the HepG2 cell suspension was adjusted to an appropriate density (1.5E+05/wells) and cells were seeded into a 48-well plate. On day 1, the diluted fluorescently labeled test articles were added. The test articles were diluted to different concentrations: 1.6, 8, 40, 200, and 1000 nM. The GalNAc ligand used for Givosiran was the positive control, and 5FAM was the negative control. After incubating the test articles with cells for 4 hours or 24 hours, all liquid was removed, cells were washed twice with PBS, and then cells were collected. After digesting and fixing cells, the corresponding fluorescence in HepG2 cells was detected by flow cytometry (BD FACSCanto™ II, Becton Dickinson, America). The mean fluorescence intensity (MFI) of the test compounds' FAM was analyzed relative to the MFI of the positive compound. Tables 9 and 11 show data at the 4-hour time point, and Tables 10 and 12 show data at the 24-hour time point.

Table 9. Ratio of MFI for Hepatic Cellular Uptake of GalNAc Ligands to MFI of Positive Compound (L96-5FAM) (FAM Fluorescence Channel, 4-hour time point)

| | 1000 nM | 200 nM | 40 nM | 8 nM |
|---|---|---|---|---|
| ID | MFI Ratio | | | |
| I-A10-5FAM | 2.16 | 2.86 | 2.86 | 3.28 |

(continued)

| | 1000 nM | 200 nM | 40 nM | 8 nM |
|---|---|---|---|---|
| ID | MFI Ratio | | | |
| I-A11-5FAM | 1.69 | 2.68 | 2.78 | 2.19 |
| I-A12-5FAM | 2.00 | 3.22 | 3.25 | 3.09 |
| I-A13-5FAM | 0.667 | 0.552 | 0.525 | 0.438 |
| I-B11-5FAM | 1.84 | 2.62 | 3.14 | 3.41 |
| I-B12-5FAM | 1.97 | 2.98 | 3.68 | 3.53 |
| I-C10-5FAM | 0.603477 | 0.507752 | 0.496403 | - |

Table 10. Ratio of MFI for Hepatic Cellular Uptake of GalNAc Ligands to MFI of Positive Compound (FAM Fluorescence Channel, 24-hour time point)

| | 1000 nM | 200 nM | 40 nM | 8 nM |
|---|---|---|---|---|
| ID | MFI Ratio | | | |
| I-A10-5FAM | 2.24 | 3.65 | 2.92 | 2.01 |
| I-A11-5FAM | 1.72 | 5.08 | 3.45 | 3.14 |
| I-A12-5FAM | 3.65 | 6.44 | 4.24 | 4.18 |
| I-A13-5FAM | 1.41 | 1.76 | 0.834 | 0.810 |
| I-B11-5FAM | 2.46 | 4.20 | 3.77 | 3.44 |
| I-B12-5FAM | 2.86 | 6.49 | 4.85 | 4.88 |
| I-C10-5FAM | 1.16 | 0.770 | 0.645 | 0.416 |
| Negative control | 0.0036 | 0.0053 | - | - |

Table 11. Ratio of MFI for Hepatic Cellular Uptake of GalNAc Ligands of the Present Invention to MFI of Positive Compound (L96-5FAM) (FAM Fluorescence Channel, 24-hour time point)

| | 1000 nM | 200 nM | 40 nM |
|---|---|---|---|
| ID | MFI Ratio | | |
| I-A12c-5FAM | 4.12 | 3.88 | 4.68 |
| I-A12d-5FAM | 5.40 | 5.14 | 6.34 |
| I-B12b-5FAM | 4.39 | 4.85 | 5.61 |
| IIB-B12b-5FAM | 1.88 | 1.26 | 1.20 |
| IIB-A12-5FAM | 1.82 | 1.36 | 1.60 |
| IIB-B12-5FAM | 1.80 | 1.56 | 1.76 |
| I-D11-5FAM | 2.60 | 1.95 | 2.57 |
| I-A12-5FAM | 2.93 | 3.32 | 4.25 |
| I-B12-5FAM | 3.23 | 4.59 | 5.61 |

Table 12. Ratio of MFI for Hepatic Cellular Uptake of GalNAc Ligands of the Present Invention to MFI of Positive Compound (FAM Fluorescence Channel, 24-hour time point)

| | 1000 nM | 200 nM | 40 nM | 8 nM |
|---|---|---|---|---|
| ID | MFI Ratio | | | |
| I-B12c-5FAM | 2.42 | 3.48 | 2.97 | 3.11 |
| I-C12-5FAM | 1.78 | 1.56 | 1.31 | 1.18 |

(continued)

| | 1000 nM | 200 nM | 40 nM | 8 nM |
|---|---|---|---|---|
| ID | MFI Ratio | | | |
| I-S12-5FAM | 2.54 | 2.93 | 2.99 | 2.82 |
| IIB-B12c-5FAM | 1.01 | 0.782 | 0.634 | 0.691 |
| IIB-C12-5FAM | 0.959 | 0.684 | 0.563 | 0.545 |
| IIIA-B12-5FAM | 1.82 | 1.90 | 1.97 | 1.78 |
| IIIB-B12-SFAM | 1.06 | 1.09 | 1.18 | 1.09 |
| IV-B12-5FAM | 2.35 | 1.94 | 2.17 | 2.75 |
| VA-A12-5FAM | 1.84 | 1.76 | 1.55 | 1.33 |
| VB-A12-5FAM | 1.41 | 1.60 | 1.47 | 1.25 |
| I-B12-5FAM | 3.13 | 4.02 | - | - |

**Example 2. Screening of Unmodified siRNA Sequences**

To evaluate the *in vitro* inhibition efficacy of modified siRNAs against KHK mRNA in the Huh7 cell line, the designed siRNAs were functionally screened and evaluated *in vitro*. Results are shown in Table 13.

**Table 13. *In Vitro* Inhibition Efficacy of Unmodified siRNAs against KHK mRNA in Huh7 Cell Line**

| Compound ID | Average Inhibition % | | SD | |
|---|---|---|---|---|
| | 1 nM | 0.1 nM | 1 nM | 0.1 nM |
| 4089 | 67.22 | 54.32 | 5.15 | 6.69 |
| 4098 | 75.20 | 57.42 | 5.31 | 7.30 |
| 4101 | 73.16 | 62.95 | 11.45 | 0.37 |
| 4102 | 68.38 | 58.40 | 11.54 | 2.78 |
| 4103 | 60.20 | 47.04 | 20.46 | 10.51 |
| 4118 | 59.88 | 51.87 | 0.94 | 4.10 |
| 4119 | 55.14 | 51.32 | 3.08 | 1.34 |
| 4121 | 60.62 | 60.36 | 6.33 | 6.24 |
| 4122 | 66.36 | 71.06 | 0.80 | 2.91 |
| 4123 | 62.17 | 60.58 | 4.47 | 0.83 |
| 4124 | 64.60 | 63.82 | 2.10 | 2.05 |
| 4125 | 66.45 | 69.01 | 4.02 | 0.59 |
| 4126 | 65.11 | 67.56 | 5.96 | 2.24 |
| 4127 | 52.22 | 58.46 | 1.58 | 2.72 |
| 4128 | 62.16 | 43.68 | 1.09 | 3.33 |
| 4129 | 80.31 | 69.21 | 2.70 | 1.46 |
| 4130 | 83.09 | 75.12 | 1.52 | 0.55 |
| 4132 | 69.61 | 59.70 | 0.44 | 0.85 |
| 4135 | 71.62 | 63.31 | 3.85 | 3.04 |
| 4138 | 60.41 | 44.05 | 1.38 | 0.67 |
| 4141 | 69.57 | 25.89 | 2.75 | 7.46 |
| 4142 | 71.37 | 46.36 | 3.45 | 6.29 |

(continued)

| Compound ID | Average Inhibition % | | SD | |
|---|---|---|---|---|
| | 1 nM | 0.1 nM | 1 nM | 0.1 nM |
| 4145 | 77.79 | 62.74 | 0.78 | 4.66 |
| 4147 | 72.31 | 54.58 | 5.86 | 0.33 |
| 4148 | 73.12 | 51.65 | 5.56 | 0.29 |
| 1nM | 0.1 nM | 1 nM | 0.1 nM | |
| 4149 | 75.32 | 51.48 | 3.74 | 8.85 |
| 4151 | 72.77 | 65.14 | 3.98 | 10.54 |
| 4159 | 60.01 | 40.20 | 1.80 | 4.57 |
| 4161 | 70.19 | 54.31 | 1.50 | 5.38 |
| 4162 | 72.82 | 53.33 | 4.48 | 6.06 |
| 4163 | 63.25 | 50.48 | 1.35 | 8.63 |

To evaluate the *in vitro* inhibition efficacy of unmodified siRNAs against KHK mRNA in the HepG2 cell line, the designed siRNAs were functionally screened and evaluated in vitro. Results are shown in Table 14.

**Table 14. *In Vitro* Inhibition Efficacy of Unmodified siRNAs against KHK mRNA in HepG2 Cell Line**

| Compound ID | Inhibition % (Mean) | | | SD | | |
|---|---|---|---|---|---|---|
| | 10 nM | 0.1 nM | 0.1 nM | 10 nM | 0.1 nM | 0.1 nM |
| 4023 | 73.07 | 68.37 | 55.36 | 1.22 | 1.44 | 1.54 |
| 4089 | 74.63 | 73.94 | 63.52 | 1.85 | 1.28 | 0.84 |
| 4098 | 75.20 | 73.70 | 66.19 | 2.00 | 2.54 | 1.42 |
| 4101 | 73.45 | 75.79 | 69.29 | 2.53 | 0.00 | 0.99 |
| 4102 | 67.27 | 66.66 | 62.29 | 3.95 | 0.01 | 2.97 |
| 4103 | 74.18 | 66.80 | 57.42 | 0.08 | 11.40 | 2.19 |
| 4118 | 75.13 | 74.01 | 56.50 | 2.81 | 0.78 | 3.63 |
| 4119 | 78.62 | 74.52 | 63.80 | 1.84 | 0.26 | 2.30 |
| 4122 | 81.91 | 78.87 | 70.27 | 0.77 | 0.95 | 0.87 |
| 4123 | 79.27 | 75.25 | 65.40 | 4.19 | 1.19 | 1.88 |
| 4125 | 81.05 | 79.11 | 72.79 | 0.66 | 1.21 | 0.25 |
| 4126 | 81.92 | 81.10 | 74.04 | 0.77 | 0.58 | 1.40 |
| 4132 | 76.22 | 68.51 | 55.82 | 1.62 | 0.88 | 1.69 |
| 4135 | 71.69 | 69.76 | 62.63 | 0.32 | 1.70 | 0.68 |
| 4138 | 68.09 | 65.80 | 46.50 | 1.99 | 0.75 | 2.50 |
| 4141 | 67.12 | 67.74 | 49.51 | 0.98 | 4.12 | 9.13 |
| 4142 | 77.45 | 73.84 | 63.78 | 1.27 | 0.65 | 2.89 |
| 4145 | 77.30 | 75.66 | 70.51 | 1.99 | 2.13 | 1.72 |
| 4147 | 74.38 | 71.23 | 59.50 | 1.64 | 1.95 | 1.39 |
| 4148 | 74.43 | 72.51 | 69.13 | 0.69 | 0.69 | 3.97 |
| 4149 | 79.50 | 75.59 | 66.99 | 1.42 | 0.36 | 0.41 |
| 4151 | 81.22 | 80.30 | 75.33 | 0.26 | 0.74 | 1.91 |

(continued)

| Compound ID | Inhibition % (Mean) | | | SD | | |
|---|---|---|---|---|---|---|
| | 10 nM | 0.1 nM | 0.1 nM | 10 nM | 0.1 nM | 0.1 nM |
| 4161 | 71.25 | 68.02 | 53.01 | 3.28 | 1.36 | 0.87 |
| 4162 | 73.24 | 68.60 | 62.99 | 0.60 | 2.61 | 4.79 |
| 4163 | 74.17 | 64.81 | 50.16 | 0.13 | 1.92 | 1.60 |

**Example 3. *In vitro* Screening of Modified GalNAc-siRNA Compounds**

To evaluate the *in vitro* inhibition efficacy of modified GalNAc-siRNA compounds against KHK mRNA in the HepG2 cell line, the designed GalNAc-siRNAs were functionally screened and evaluated *in vitro.* Results are shown in Table 15. AD-1613400 served as the positive control (WO2022182574A1).

Sense strand:
mG*mC*mAmGmGmAmAmGfCfAfCmUmGmAmGmAmUmUmCmGmU-L96 (SEQ ID NO. 33);
Antisense strand:

mA*dC*mGmAdAmUdCmUmCmAmGdTmGfCmUmUmCmCmUmGmC*mA*mC (SEQ ID NO. 66).

**Table 15. *In Vitro* Inhibition Efficacy of Modified GalNAc-siRNA Compounds against KHK mRNA in HepG2 Cell Line**

| Compound | Average Inhibition % | | SD | |
|---|---|---|---|---|
| | 1nM | 10nM | 1nM | 10nM |
| ALN212-60-L96 | 67.12 | 83.28 | 5.81 | 5.77 |
| AD-1613400 | 81.00 | 87.21 | 2.34 | 2.03 |
| AD-63821 | 22.00 | 49.88 | 12.64 | 6.75 |
| 4122-60-L96 | 40.65 | 71.51 | 10.64 | 7.65 |
| 4122-60-001 | 41.61 | 74.92 | 11.20 | 11.67 |
| 4122-50-001 | 54.00 | 78.93 | 7.68 | 9.52 |
| 4122-4-001 | 33.56 | 67.86 | 11.57 | 18.07 |
| 4122-64-001 | 40.92 | 75.92 | 11.01 | 4.96 |
| 4122-67-001 | 40.45 | 71.60 | 18.54 | 10.74 |
| 4122-10-001 | 73.37 | 85.34 | 7.62 | 3.17 |
| 4122-11-001 | 79.69 | 85.46 | 12.28 | 5.33 |
| 4122-70-001 | 80.31 | 82.18 | 3.88 | 9.31 |
| 4122.1-60-001 | 48.48 | 67.55 | 13.04 | 9.70 |
| 4122-60-002 | 48.77 | 71.63 | 9.19 | 10.23 |
| 4122-10-002 | 66.51 | 80.47 | 12.43 | 8.38 |
| 4122-11-002 | 66.10 | 82.64 | 15.56 | 14.66 |
| 4122-60-004 | 41.84 | 71.62 | 15.68 | 22.16 |
| 4122-10-004 | 58.45 | 72.65 | 19.65 | 16.02 |
| 4122-11-004 | 57.58 | 74.45 | 18.93 | 16.62 |
| 4023-65-001 | 54.27 | 61.44 | 11.84 | 19.85 |

(continued)

| Compound | Average Inhibition % | | SD | |
|---|---|---|---|---|
| | 1nM | 10nM | 1nM | 10nM |
| 4023-44-001 | 46.48 | 61.02 | 15.84 | 19.00 |
| 4122-44-001 | 80.74 | 84.66 | 4.46 | 5.06 |
| 4122-18-001 | 80.26 | 88.06 | 3.62 | 3.00 |
| 4122-19-001 | 74.79 | 88.47 | 8.26 | 4.72 |
| 4122-74-001 | 79.02 | 90.23 | 3.79 | 3.37 |
| 4122-75-001 | 68.72 | 88.45 | 5.63 | 4.48 |
| 4122-101-001 | 70.05 | 85.45 | 7.93 | 4.90 |
| 4122-34-001 | 61.63 | 86.16 | 7.76 | 4.83 |
| 4122-35-001 | 66.15 | 80.07 | 7.21 | 6.35 |
| 4122-98-001 | 34.44 | 77.24 | 19.90 | 12.10 |
| 4122-26-001 | 55.63 | 83.81 | 17.22 | 5.18 |
| 4122-27-001 | 57.36 | 87.18 | 8.55 | 5.24 |
| 4122-97-001 | 28.56 | 66.38 | 18.29 | 8.82 |
| 4122-85-001 | 42.89 | 79.27 | 11.00 | 1.48 |
| 4122-86-001 | 71.02 | 79.54 | 33.46 | 3.68 |
| 4122-99-001 | 28.82 | 59.09 | 11.56 | 6.48 |
| 4122-80-001 | 78.99 | 89.62 | 1.39 | 1.88 |
| 4122-81-001 | 73.17 | 90.22 | 3.40 | 1.37 |
| 4122-100-001 | 58.99 | 82.81 | 2.32 | 1.91 |
| 4122.3-60-001 | 42.35 | 78.94 | 13.32 | 1.66 |
| 4132-60-L96 | 47.99 | 74.52 | 8.10 | 11.30 |
| 4132-60-001 | 39.67 | 82.77 | 3.79 | 5.39 |
| 4132-10-001 | 56.92 | 90.55 | 7.99 | 2.80 |
| 4132-11-001 | 66.69 | 85.41 | 3.82 | 5.44 |
| 4132-44-001 | 49.85 | 82.94 | 14.81 | 5.30 |
| 4132-26-001 | 29.43 | 75.80 | 17.91 | 5.57 |
| 4132-27-001 | 54.55 | 82.69 | 6.65 | 6.03 |
| 4132-34-001 | 38.14 | 78.13 | 8.03 | 6.05 |
| 4132-35-001 | 43.39 | 82.63 | 24.13 | 5.09 |
| 4132-60-004 | 39.31 | 78.17 | 14.23 | 11.25 |
| 4132-10-004 | 60.33 | 81.59 | 1.89 | 6.81 |
| 4132-11-004 | 59.69 | 82.04 | 2.25 | 7.92 |
| 4089-60-001 | 79.44 | 87.89 | 1.44 | 7.21 |
| 4098-60-001 | 80.84 | 89.39 | 6.66 | 8.09 |
| 4118-60-001 | 46.15 | 78.06 | 8.14 | 5.08 |
| 4123-60-001 | 75.75 | 79.38 | 7.02 | 10.83 |
| 4129-60-001 | 72.00 | 86.57 | 6.55 | 6.17 |
| 4135-60-001 | 53.94 | 74.86 | 6.84 | 9.10 |

(continued)

| Compound | Average Inhibition % | | SD | |
|---|---|---|---|---|
| | 1nM | 10nM | 1nM | 10nM |
| 4145-60-001 | 75.54 | 86.34 | 2.93 | 1.23 |

**Example 4. Multi-concentration Point Inhibition Efficacy Screening of Modified GalNAc-siRNA Compounds in HepG2 Cell Line**

The *in vitro* inhibition efficacy of modified GalNAc-siRNAs against KHK mRNA in the HepG2 cell line was screened at final compound concentrations of 10, 2, 0.4, 0.08, and 0.016 nM. $IC_{50}$ values for inhibition rates were calculated as follows.

**Table 16. KHK $IC_{50}$ Values of GalNAc-siRNAs in HepG2 Cell Line**

| Compound | Average Inhibition % | | | | | $IC_{50}$(nM) |
|---|---|---|---|---|---|---|
| | 10 nM | 2 nM | 0.4 nM | 0.08 nM | 0.016 nM | |
| AD1613400 | 92.73 | 87.46 | 71.22 | 17.53 | 16.74 | 0.284 |
| AD63821 | 68.94 | 40.37 | 38.27 | 31.03 | -3.88 | 1.061 |
| 4122-10-001 | 93.53 | 83.69 | 79.72 | 29.09 | -26.86 | 0.083 |
| 4122-11-001 | 93.07 | 85.85 | 67.44 | -6.60 | -12.65 | 0.238 |
| 4122-74-001 | 92.76 | 77.64 | 80.37 | 28.75 | 24.62 | 0.198 |
| 4122-35-001 | 86.33 | 68.02 | 61.67 | -12.15 | -12.13 | 0.268 |
| 4122-27-001 | 83.17 | 83.89 | 60.06 | 25.26 | -3.18 | 0.171 |
| 4122-80-001 | 88.80 | 79.19 | 63.47 | -0.79 | -35.91 | 0.23 |
| 4122-81-001 | 90.35 | 74.24 | 64.41 | 2.33 | -3.97 | 0.226 |
| 4132-10-001 | 84.37 | 72.36 | 52.82 | 24.85 | -2.26 | 0.232 |
| 4132-11-001 | 69.77 | 75.13 | 48.29 | 30.27 | -17.44 | 0.167 |
| 4089-60-001 | 84.88 | 79.15 | 49.02 | 53.33 | -21.93 | 0.071 |
| 4098-60-001 | 90.69 | 87.05 | 52.03 | 55.11 | -3.59 | 0.131 |
| 4123-60-001 | 84.13 | 77.64 | 56.73 | 49.37 | 8.54 | 0.08 |
| 4129-60-001 | 83.44 | 77.38 | 62.53 | 46.97 | -7.07 | 0.051 |
| 4145-60-001 | 76.13 | 65.49 | 67.55 | 47.43 | -16.68 | 0.073 |

**Example 5. *In vivo* Screening of Modified GalNAc-siRNA Compounds in HFD + Fructose Water-induced Mouse Model**

Male C57BL/6 mice were fed a HFD (Research diets, D12492) and provided with 30% (wt/v) fructose drinking water for 6 weeks to induce the model (30% fructose water was changed twice weekly). Control mice were fed regular chow and normal drinking water. Feeding conditions from the modeling period were maintained during the efficacy period.

Dosing: Day 0 was defined as the day of first dosing. Dosing was administered on Day 0 and Day 14. Route: s.c. Volume: 10 μL/g. Dose: 20 mg/kg, with 5 mice per group. On Day 28 post-dosing, the target gene KHK in the liver was measured.

G1: HFD + fructose, PBS
G2: HFD + fructose, 4023-60-L96, 20 mpk;
G3: HFD + fructose, 4122-60-L96, 20 mpk;
G4: HFD + fructose, 4132-60-L96, 20 mpk;
G5: HFD + fructose, 4149-60-L96, 20 mpk;

To evaluate the inhibitory efficiency of KHK siRNAs on the target gene, KHK mRNA in mouse liver tissue was measured at the experimental endpoint. Data shows that 4023-60-L96, 4122-60-L96, 4132-60-L96, and 4149-60-L96 can all significantly inhibit mouse liver KHK mRNA expression (Fig. 1).

### Example 6. *In vivo* Screening of Modified GalNAc-siRNA Compounds in HDI Mouse Model

A mouse model expressing human KHK (hKHK) was constructed by hydrodynamically injecting a stable plasmid carrying the human KHK and encoding and expressing the SEAP protein into Balb/c mice via tail vein. Specifically, approximately 6-week-old male Balb/c mice were injected hydrodynamically via the tail vein with 25 μg of plasmid carrying the human KHK cDNA sequence and the SEAP protein, along with 50 μg of helper plasmid. Two weeks later, the target knockdown ability of GalNAc-siRNAs was tested. All mice began subcutaneous administration on Day 0 with a single dose at 20 mg/kg, and an administration volume of 10 μL/g per mouse. Two days prior to administration, SEAP protein expression in mouse serum was detected using the Phospha-Light SEAP Reporter Gene Assay Kit. Mice were randomly grouped based on the average SEAP protein expression levels, with 5 mice per group. On Day 7 and Day 14 post-administration, blood was collected from the submandibular vein of all mice to obtain plasma for measuring SEAP protein expression in serum. The *in vivo* screening results in the HDI model are shown in Table 17. AD-1613400 served as the positive control (WO2022182574A1).

**Table 17. Normalized Serum SEAP Protein Relative to Pre-dose and Saline Group in KHK-SEAP Mouse Experiment**

| Group | Treatment | Dose | Average SEAP | | SD | |
|---|---|---|---|---|---|---|
| | | | Day7 | Day14 | Day7 | Day14 |
| G1 | Saline | NA | 1.00 | 1.00 | 0.076 | 0.123 |
| G2 | 4089-60-L96 | 20mpk | 0.496 | 0.287 | 0.317 | 0.404 |
| G3 | 4098-60-L96 | 20mpk | 0.619 | 0.479 | 0.114 | 0.276 |
| G4 | 4118-60-L96 | 20mpk | 0.630 | 0.514 | 0.116 | 0.171 |
| G5 | 4123-60-L96 | 20mpk | 0.490 | 0.411 | 0.111 | 0.175 |
| G6 | 4125-60-L96 | 20mpk | 0.528 | 0.491 | 0.169 | 0.321 |
| G7 | 4129-60-L96 | 20mpk | 0.396 | 0.373 | 0.184 | 0.329 |
| G8 | 4135-60-L96 | 20mpk | 0.745 | 0.600 | 0.102 | 0.246 |
| G9 | 4145-60-L96 | 20mpk | 0.846 | 0.516 | 0.163 | 0.086 |
| G10 | 4147-60-L96 | 20mpk | 0.667 | 0.615 | 0.215 | 0.412 |
| G11 | 4151-60-L96 | 20mpk | 0.699 | 0.726 | 0.318 | 0.544 |
| G12 | AD-1613400 | 20mpk | 0.543 | 0.635 | 0.160 | 0.191 |
| G13 | 4122-60-L96 | 20mpk | 0.609 | 0.383 | 0.187 | 0.289 |

## Claims

1. An RNAi agent for inhibiting expression of a ketohexokinase (KHK) gene in a cell, comprising a sense strand and an antisense strand that form a duplex region, wherein the antisense strand comprises at least 15 contiguous nucleotides of a nucleotide sequence selected from SEQ ID NOs: 34 to 65 and nucleotide sequences having 1 to 3 nucleotide differences therefrom.

2. The RNAi agent according to claim 1, wherein the duplex region is 17 to 23 base pairs in length, preferably 18 to 21 base pairs, more preferably 19 base pairs.

3. The RNAi agent according to claim 1 or 2, wherein each of the sense strand and the antisense strand is 17 to 23 nucleotides in length, preferably 19 to 21 nucleotides.

4. The RNAi agent according to any one of claims 1 to 3, wherein the RNAi agent comprises one or two blunt ends,

preferably one blunt end.

5. The RNAi agent according to any one of claims 1 to 4, wherein the RNAi agent comprises one or two overhangs, preferably one overhang, each overhang comprising 1 to 4 unpaired nucleotides, preferably 2 unpaired nucleotides.

6. The RNAi agent according to claim 5, wherein the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

7. The RNAi agent according to any one of claims 1 to 6, wherein the sense strand comprises at least 15 contiguous nucleotides of any nucleotide sequence selected from SEQ ID NOs: 1 to 32 and nucleotide sequences having 1 to 3 nucleotide differences therefrom.

8. The RNAi agent according to any one of claims 1 to 7, wherein the antisense strand has no more than 23 nucleotides and comprises a nucleotide sequence selected from SEQ ID NOs: 34 to 65; and the sense strand has no more than 21 nucleotides and comprises a nucleotide sequence from SEQ ID NOs: 1 to 32.

9. The RNAi agent according to claim 8, wherein:

(a1) the sense strand comprises or consists of a sequence set forth in SEQ ID NO:1 or a nucleotide sequence having 1, 2, or 3 nucleotide differences therefrom, and the antisense strand comprises or consists of a sequence set forth in SEQ ID NO:34 or a nucleotide sequence having 1, 2, or 3 nucleotide differences therefrom;
(a2) the sense strand comprises or consists of a sequence set forth in SEQ ID NO:2 or a nucleotide sequence having 1, 2, or 3 nucleotide differences therefrom, and the antisense strand comprises or consists of a sequence set forth in SEQ ID NO:35 or a nucleotide sequence having 1, 2, or 3 nucleotide differences therefrom;
(a3) the sense strand comprises or consists of a sequence set forth in SEQ ID NO:3 or a nucleotide sequence having 1, 2, or 3 nucleotide differences therefrom, and the antisense strand comprises or consists of a sequence set forth in SEQ ID NO:36 or a nucleotide sequence having 1, 2, or 3 nucleotide differences therefrom;
(a4) the sense strand comprises or consists of a sequence set forth in SEQ ID NO:4 or a nucleotide sequence having 1, 2, or 3 nucleotide differences therefrom, and the antisense strand comprises or consists of a sequence set forth in SEQ ID NO:37 or a nucleotide sequence having 1, 2, or 3 nucleotide differences therefrom;
(a5) the sense strand comprises or consists of a sequence set forth in SEQ ID NO:5 or a nucleotide sequence having 1, 2, or 3 nucleotide differences therefrom, and the antisense strand comprises or consists of a sequence set forth in SEQ ID NO:38 or a nucleotide sequence having 1, 2, or 3 nucleotide differences therefrom;
(a6) the sense strand comprises or consists of a sequence set forth in SEQ ID NO:6 or a nucleotide sequence having 1, 2, or 3 nucleotide differences therefrom, and the antisense strand comprises or consists of a sequence set forth in SEQ ID NO:39 or a nucleotide sequence having 1, 2, or 3 nucleotide differences therefrom;
(a7) the sense strand comprises or consists of a sequence set forth in SEQ ID NO:7 or a nucleotide sequence having 1, 2, or 3 nucleotide differences therefrom, and the antisense strand comprises or consists of a sequence set forth in SEQ ID NO:40 or a nucleotide sequence having 1, 2, or 3 nucleotide differences therefrom;
(a8) the sense strand comprises or consists of a sequence set forth in SEQ ID NO:8 or a nucleotide sequence having 1, 2, or 3 nucleotide differences therefrom, and the antisense strand comprises or consists of a sequence set forth in SEQ ID NO:41 or a nucleotide sequence having 1, 2, or 3 nucleotide differences therefrom;
(a9) the sense strand comprises or consists of a sequence set forth in SEQ ID NO:9 or a nucleotide sequence having 1, 2, or 3 nucleotide differences therefrom, and the antisense strand comprises or consists of a sequence set forth in SEQ ID NO:42 or a nucleotide sequence having 1, 2, or 3 nucleotide differences therefrom;
(a10) the sense strand comprises or consists of a sequence set forth in SEQ ID NO:10 or a nucleotide sequence having 1, 2, or 3 nucleotide differences therefrom, and the antisense strand comprises or consists of a sequence set forth in SEQ ID NO:43 or a nucleotide sequence having 1, 2, or 3 nucleotide differences therefrom;
(a11) the sense strand comprises or consists of a sequence set forth in SEQ ID NO:11 or a nucleotide sequence having 1, 2, or 3 nucleotide differences therefrom, and the antisense strand comprises or consists of a sequence set forth in SEQ ID NO:44 or a nucleotide sequence having 1, 2, or 3 nucleotide differences therefrom;
(a12) the sense strand comprises or consists of a sequence set forth in SEQ ID NO:12 or a nucleotide sequence having 1, 2, or 3 nucleotide differences therefrom, and the antisense strand comprises or consists of a sequence set forth in SEQ ID NO:45 or a nucleotide sequence having 1, 2, or 3 nucleotide differences therefrom;
(a13) the sense strand comprises or consists of a sequence set forth in SEQ ID NO:13 or a nucleotide sequence having 1, 2, or 3 nucleotide differences therefrom, and the antisense strand comprises or consists of a sequence set forth in SEQ ID NO:46 or a nucleotide sequence having 1, 2, or 3 nucleotide differences therefrom;

(a14) the sense strand comprises or consists of a sequence set forth in SEQ ID NO:14 or a nucleotide sequence having 1, 2, or 3 nucleotide differences therefrom, and the antisense strand comprises or consists of a sequence set forth in SEQ ID NO:47 or a nucleotide sequence having 1, 2, or 3 nucleotide differences therefrom;
(a15) the sense strand comprises or consists of a sequence set forth in SEQ ID NO:15 or a nucleotide sequence having 1, 2, or 3 nucleotide differences therefrom, and the antisense strand comprises or consists of a sequence set forth in SEQ ID NO:48 or a nucleotide sequence having 1, 2, or 3 nucleotide differences therefrom;
(a16) the sense strand comprises or consists of a sequence set forth in SEQ ID NO:16 or a nucleotide sequence having 1, 2, or 3 nucleotide differences therefrom, and the antisense strand comprises or consists of a sequence set forth in SEQ ID NO:49 or a nucleotide sequence having 1, 2, or 3 nucleotide differences therefrom;
(a17) the sense strand comprises or consists of a sequence set forth in SEQ ID NO:17 or a nucleotide sequence having 1, 2, or 3 nucleotide differences therefrom, and the antisense strand comprises or consists of a sequence set forth in SEQ ID NO:50 or a nucleotide sequence having 1, 2, or 3 nucleotide differences therefrom;
(a18) the sense strand comprises or consists of a sequence set forth in SEQ ID NO:18 or a nucleotide sequence having 1, 2, or 3 nucleotide differences therefrom, and the antisense strand comprises or consists of a sequence set forth in SEQ ID NO:51 or a nucleotide sequence having 1, 2, or 3 nucleotide differences therefrom;
(a19) the sense strand comprises or consists of a sequence set forth in SEQ ID NO:19 or a nucleotide sequence having 1, 2, or 3 nucleotide differences therefrom, and the antisense strand comprises or consists of a sequence set forth in SEQ ID NO:52 or a nucleotide sequence having 1, 2, or 3 nucleotide differences therefrom;
(a20) the sense strand comprises or consists of a sequence set forth in SEQ ID NO:20 or a nucleotide sequence having 1, 2, or 3 nucleotide differences therefrom, and the antisense strand comprises or consists of a sequence set forth in SEQ ID NO:53 or a nucleotide sequence having 1, 2, or 3 nucleotide differences therefrom;
(a21) the sense strand comprises or consists of a sequence set forth in SEQ ID NO:21 or a nucleotide sequence having 1, 2, or 3 nucleotide differences therefrom, and the antisense strand comprises or consists of a sequence set forth in SEQ ID NO:54 or a nucleotide sequence having 1, 2, or 3 nucleotide differences therefrom;
(a22) the sense strand comprises or consists of a sequence set forth in SEQ ID NO:22 or a nucleotide sequence having 1, 2, or 3 nucleotide differences therefrom, and the antisense strand comprises or consists of a sequence set forth in SEQ ID NO:55 or a nucleotide sequence having 1, 2, or 3 nucleotide differences therefrom;
(a23) the sense strand comprises or consists of a sequence set forth in SEQ ID NO:23 or a nucleotide sequence having 1, 2, or 3 nucleotide differences therefrom, and the antisense strand comprises or consists of a sequence set forth in SEQ ID NO:56 or a nucleotide sequence having 1, 2, or 3 nucleotide differences therefrom;
(a24) the sense strand comprises or consists of a sequence set forth in SEQ ID NO:24 or a nucleotide sequence having 1, 2, or 3 nucleotide differences therefrom, and the antisense strand comprises or consists of a sequence set forth in SEQ ID NO:57 or a nucleotide sequence having 1, 2, or 3 nucleotide differences therefrom;
(a25) the sense strand comprises or consists of a sequence set forth in SEQ ID NO:25 or a nucleotide sequence having 1, 2, or 3 nucleotide differences therefrom, and the antisense strand comprises or consists of a sequence set forth in SEQ ID NO:58 or a nucleotide sequence having 1, 2, or 3 nucleotide differences therefrom;
(a26) the sense strand comprises or consists of a sequence set forth in SEQ ID NO:26 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
(a27) the sense strand comprises or consists of a sequence set forth in SEQ ID NO:27 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
(a28) the sense strand comprises or consists of a sequence set forth in SEQ ID NO:28 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
(a29) the sense strand comprises or consists of a sequence set forth in SEQ ID NO:29 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
(a30) the sense strand comprises or consists of a sequence set forth in SEQ ID NO:30 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
(a31) the sense strand comprises or consists of a sequence set forth in SEQ ID NO:31 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom; or
(a32) the sense strand comprises or consists of a sequence set forth in SEQ ID NO:32 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom.

**10.** The RNAi agent according to any one of claims 1 to 9, wherein the RNAi agent comprises duplex 4023, 4089, 4098, 4101, 4102, 4103, 4118, 4119, 4121, 4122, 4123, 4124, 4125, 4126, 4127, 4128, 4129, 4130, 4132, 4135, 4138, 4141, 4142, 4145, 4147, 4148, 4149, 4151, 4159, 4161, 4162 or 4163.

**11.** The RNAi agent according to any one of claims 1 to 10, wherein the sense strand and/or the antisense strand of the RNAi agent comprises at least one modified nucleotide, the modified nucleotide independently selected from 2'-deoxythymidine (dT) nucleotide, 2'-O-methyl-modified nucleotide, 2'-fluoro-modified nucleotide, 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide, 2'-amino-modified nucleotide, 2'-O-allyl-modified nucleotide, 2'-C-alkyl-modified nucleotide, 2'-methoxyethyl-modified nucleotide, abasic nucleotide, inverted abasic nucleotide, inverted nucleotide, morpholino nucleotide, phosphoramidate, tetrahydropyran-modified nucleotide, 1,5-anhydrohexitol-modified nucleotide, cyclohexenyl-modified nucleotide, nucleotide comprising a phosphorothioate group, nucleotide comprising a methylphosphonate group, nucleotide comprising a 5'-phosphate, nucleotide comprising a 5'-phosphate mimic, and combinations thereof; preferably selected from 2'-O-methyl-modified nucleotide, 2'-fluoro-modified nucleotide, 2'-deoxy-modified nucleotide, nucleotide comprising a phosphorothioate internucleotide linkage, and combinations thereof; and/or preferably, each nucleotide of the sense strand and/or the antisense strand of the RNAi agent is modified.

**12.** The RNAi agent according to claim 11, wherein, in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

**13.** The RNAi agent according to claim 11 or 12, wherein the antisense strand comprises at least one phosphorothioate internucleotide linkage; preferably, the phosphorothioate internucleotide linkage is present at one or more of the following locations:

(i) between nucleotide positions 1 and 2 at 5'-end of the antisense strand;
(ii) between nucleotide positions 2 and 3 at 5'-end of the antisense strand;
(iii) between nucleotide positions 1 and 2 at 3'-end of the antisense strand; and
(iv) between nucleotide positions 2 and 3 at 3'-end of the antisense strand.

**14.** The RNAi agent according to any one of claims 11 to 13, wherein, in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

**15.** The RNAi agent according to any one of claims 11 to 14, wherein the sense strand comprises at least one phosphorothioate internucleotide linkage; preferably, the phosphorothioate internucleotide linkage is present (i) between nucleotide positions 1 and 2 at 5'-end of the sense strand; and/or (ii) between nucleotide positions 2 and 3 at 5'-end of the sense strand.

**16.** The RNAi agent according to claim 11, wherein the modification is selected from one of STC, ESC, Advanced ESC, ESC+, AD1-3, AD5 and GalXC.

**17.** The RNAi agent according to any one of claims 11 to 16, wherein the antisense strand of the RNAi agent comprises or consists of a sequence set forth in any one of SEQ ID NOs: 104 to 130 or a nucleotide sequence having 1, 2 or 3 nucleotide differences from each thereof.

**18.** The RNAi agent according to any one of claims 11 to 17, wherein the sense strand of the RNAi agent comprises or consists of a sequence set forth in any one of SEQ ID NOs: 132 to 153 or a nucleotide sequence having 1, 2 or 3 nucleotide differences from each thereof.

**19.** The RNAi agent according to any one of claims 11 to 18, wherein in the RNAi agent:

(b1) the sense strand comprises or consists of a nucleotide sequence set forth in SEQ ID NO: 132 or a nucleotide sequence having 1, 2 or 3 nucleotide differences therefrom, and the antisense strand comprises or consists of a nucleotide sequence set forth in SEQ ID NO: 104 or a nucleotide sequence having 1, 2 or 3 nucleotide differences

therefrom;

(b2) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;

(b3) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;

(b4) the sense strand comprises or consists of a nucleotide sequence set forth in SEQ ID NO: 135 or a nucleotide sequence having 1, 2 or 3 nucleotide differences therefrom, and the antisense strand comprises or consists of a nucleotide sequence set forth in SEQ ID NO: 107 or a nucleotide sequence having 1, 2 or 3 nucleotide differences therefrom;

(b5) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 108 or a nucleotide sequence having 1, 2 or 3 nucleotide differences therefrom;

(b6) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 109 or a nucleotide sequence having 1, 2 or 3 nucleotide differences therefrom;

(b7) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 110 or a nucleotide sequence having 1, 2 or 3 nucleotide differences therefrom;

(b8) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 111 or a nucleotide sequence having 1, 2 or 3 nucleotide differences therefrom;

(b9) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b10) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b11) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 114 or a nucleotide sequence having 1, 2 or 3 nucleotide differences therefrom;

(b12) the sense strand comprises or consists of a nucleotide sequence set forth in SEQ ID NO: 133 or a nucleotide sequence having 1, 2 or 3 nucleotide differences therefrom, and the antisense strand comprises or consists of a nucleotide sequence set forth in SEQ ID NO: 115 or a nucleotide sequence having 1, 2 or 3 nucleotide differences therefrom;

(b13) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 116 or a nucleotide sequence having 1, 2 or 3 nucleotide differences therefrom;

(b14) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b15) the sense strand comprises or consists of a nucleotide sequence set forth in SEQ ID NO: 136 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b16) the sense strand comprises or consists of a nucleotide sequence set forth in SEQ ID NO: 137 or a nucleotide

sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b17) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b18) the sense strand comprises or consists of a nucleotide sequence set forth in SEQ ID NO: 137 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b19) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b20) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b21) the sense strand comprises or consists of a nucleotide sequence set forth in SEQ ID NO: 138 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b22) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b23) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b24) the sense strand comprises or consists of a nucleotide sequence set forth in SEQ ID NO: 139 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b25) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b26) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b27) the sense strand comprises or consists of a nucleotide sequence set forth in SEQ ID NO: 140 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(b28) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(b29) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(b30) the sense strand comprises or consists of a nucleotide sequence set forth in SEQ ID NO: 141 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences

therefrom;

(b31) the sense strand comprises or consists of a nucleotide sequence set forth in SEQ ID NO: 142 or a nucleotide sequence having 1, 2 or 3 nucleotide differences therefrom, and the antisense strand comprises or consists of a nucleotide sequence set forth in SEQ ID NO: 118 or a nucleotide sequence having 1, 2 or 3 nucleotide differences therefrom;

(b32) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b33) the sense strand comprises or consists of a nucleotide sequence set forth in SEQ ID NO: 134 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b34) the sense strand comprises or consists of a nucleotide sequence set forth in SEQ ID NO: 143 or a nucleotide sequence having 1, 2 or 3 nucleotide differences therefrom, and the antisense strand comprises or consists of a nucleotide sequence set forth in SEQ ID NO: 121 or a nucleotide sequence having 1, 2 or 3 nucleotide differences therefrom;

(b35) the sense strand comprises or consists of a nucleotide sequence set forth in SEQ ID NO: 144 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b36) the sense strand comprises or consists of a nucleotide sequence set forth in SEQ ID NO: 144 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b37) the sense strand comprises or consists of a nucleotide sequence set forth in SEQ ID NO: 145 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;

(b38) the sense strand comprises or consists of a nucleotide sequence set forth in SEQ ID NO: 145 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;

(b39) the sense strand comprises or consists of a nucleotide sequence set forth in SEQ ID NO: 132 or a nucleotide sequence having 1, 2 or 3 nucleotide differences therefrom, and the antisense strand comprises or consists of a nucleotide sequence set forth in SEQ ID NO: 122 or a nucleotide sequence having 1, 2 or 3 nucleotide differences therefrom;

(b40) the sense strand comprises or consists of a nucleotide sequence set forth in SEQ ID NO: 146 or a nucleotide sequence having 1, 2 or 3 nucleotide differences therefrom, and the antisense strand comprises or consists of a nucleotide sequence set forth in SEQ ID NO: 123 or a nucleotide sequence having 1, 2 or 3 nucleotide differences therefrom;

(b41) the sense strand comprises or consists of a nucleotide sequence set forth in SEQ ID NO: 147 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;

(b42) the sense strand comprises or consists of a nucleotide sequence set forth in SEQ ID NO: 148 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;

(b43) the sense strand comprises or consists of a nucleotide sequence set forth in SEQ ID NO: 149 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;

(b44) the sense strand comprises or consists of a nucleotide sequence set forth in SEQ ID NO: 150 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;

(b45) the sense strand comprises or consists of a nucleotide sequence set forth in SEQ ID NO: 151 or a nucleotide

sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;

(b46) the sense strand comprises or consists of a nucleotide sequence set forth in SEQ ID NO: 152 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom; or

(b47) the sense strand comprises or consists of a nucleotide sequence set forth in SEQ ID NO: 153 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom.

**20.** The RNAi agent according to claim 19, wherein the RNAi agent comprises duplex 4023-60, 4122-60, 4132-60, 4149-60, 4122-50, 4122-4, 4122-64, 4122-67, 4122-10, 4122-11, 4122-70, 4122.1-60, 4122-44, 4122-18, 4122-19, 4122-74, 4122-75, 4122-101, 4122-34, 4122-35, 4122-98, 4122-26, 4122-27, 4122-97, 4122-85, 4122-86, 4122-99, 4122-80, 4122-81, 4122-100, 4122.3-60, 4132-10, 4132-11, 4132-44, 4132-26, 4132-27, 4132-34, 4132-35, 4023-65, 4023-44, 4089-60, 4098-60, 4118-60, 4123-60, 4129-60, 4135-60 or 4145-60.

**21.** The RNAi agent according to any one of claims 1 to 20, wherein the RNAi agent further comprises a ligand that targets a hepatocyte; preferably, the ligand comprises a galactose moiety, a galactosamine moiety, or an N-acetylgalactosamine moiety; more preferably, the ligand is a trivalent or tetravalent N-acetylgalactosamine moiety, still more preferably, the ligand that targets a hepatocyte is L96, NAG25 or NAG37; or wherein the RNAi agent further comprises a ligand that targets a non-hepatocyte, preferably the ligand comprises a lipophilic monomer, the lipophilic monomer preferably being Y132 to Y135, Y158, Y165 to Y168, L10, L57, L321, L322, Q361 to Q367, Q361s to Q367s, Q370, Q377 to Q379, or Q383.

**22.** The RNAi agent according to claim 21, wherein the ligand comprises a structure of formula (I):

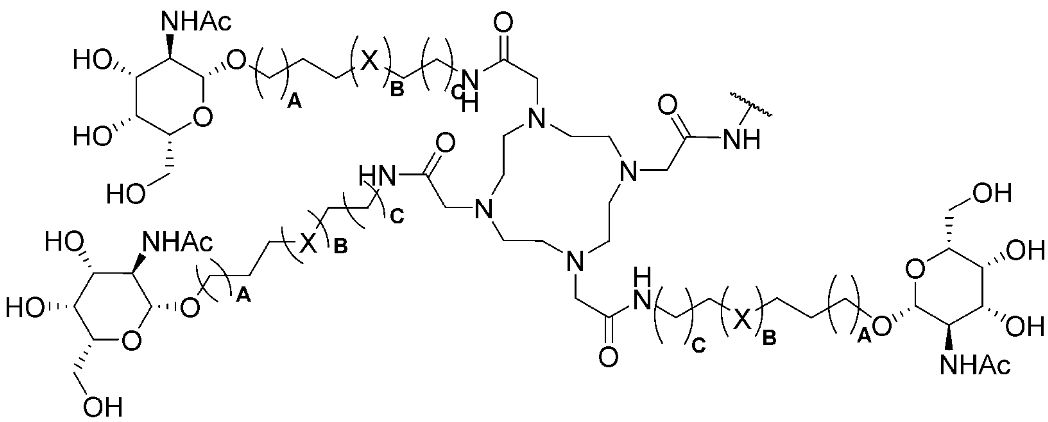

(I)

wherein,

X is -C(O)-NH-, -NH-C(O)-, $-OCH_2-CH_2O-$, -S-,

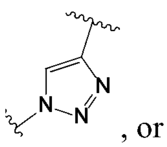

, or

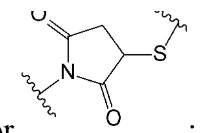

;

A and C are independently 0 or an integer between 1 and 14;
B is 0 or an integer between 1 and 12;
the wave line represents a site to which other part of the RNAi agent is attached.

**23.** The RNAi agent according to claim 22, wherein the ligand has a structure of formula (II) or formula (III):

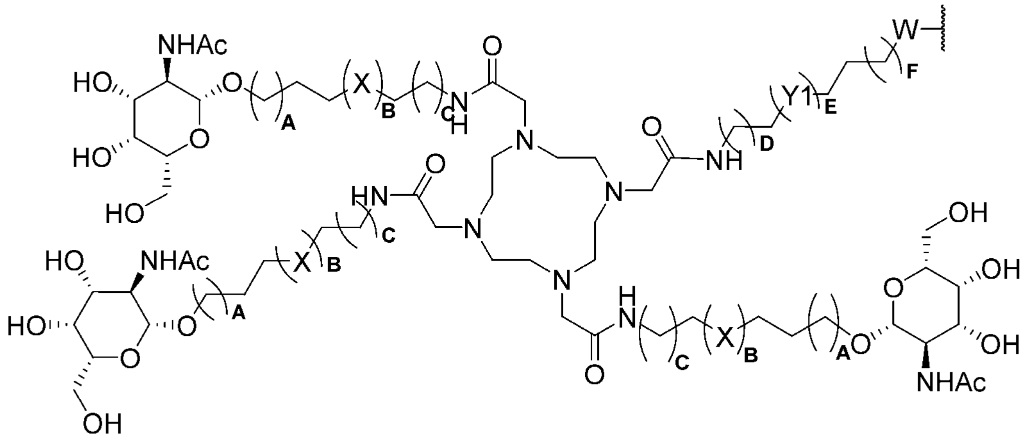

(II)

wherein,

X is -C(O)-NH-, -NH-C(O)-, $-OCH_2-CH_2O-$, -S-,

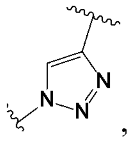, or 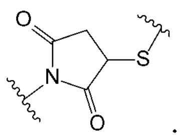;

Y1 is $-C(O)-NR_1-$, -NH-C(O)-, $-OCH_2-CH_2O-$, -S-, -S-S-,

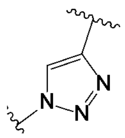, or 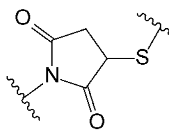,

wherein $R_1$ is an alkyl group having 1-6 carbons or hydrogen;
W is -NH-, -O- or

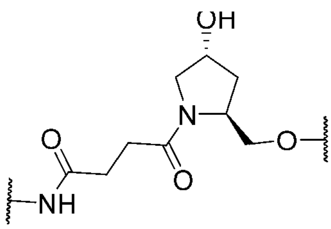,

wherein the wave line on the right represents a site to which the sense strand or antisense strand is attached; the wave line connected to W in formula (II) represents a site to which the sense strand or antisense strand is attached, wherein the ligand is connected to 5'- and/or 3'-end of the sense strand and/or antisense strand;
A, C and F are independently 0 or an integer between 1 and 14;
B and E are independently 0 or an integer between 1 and 12;
D is an integer between 1 and 20;

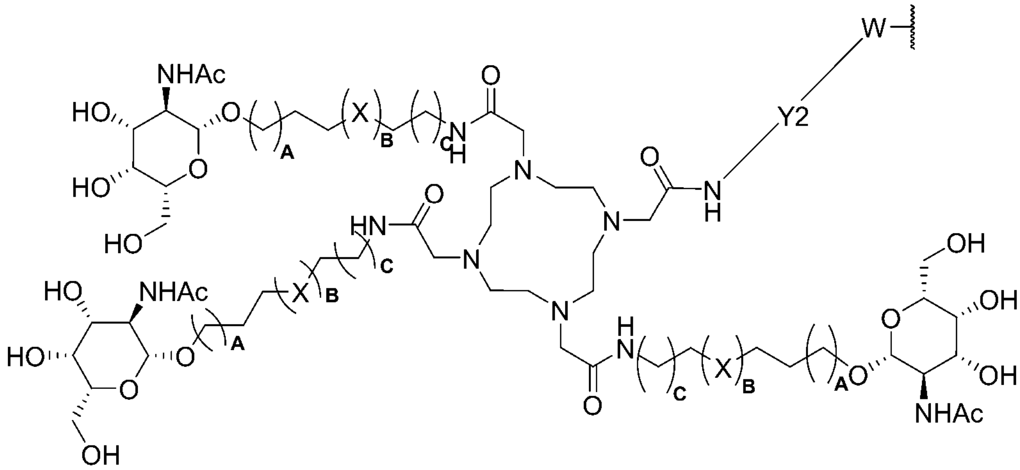

(III)

wherein,

X is -C(O)-NH-, -NH-C(O)-, $-OCH_2-CH_2O-$, -S-,

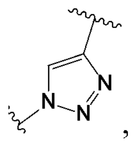
, or
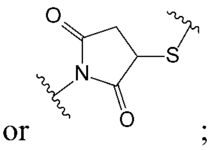
;

Y2 is a linear short chain or polypeptide structure, selected from the group consisting of:

(1) $-(CH_2)p-(O-CH_2-CH_2)q-(CH_2)j-Z_3-$, wherein $Z_3$ is O, NH or C(O), p is an integer from 1 to 3, q is an integer from 3 to 10; j is 0 or 1;
(2) $-CH_2-C(O)-(Sar)n_1-NH-(CH_2)r-NH-$, wherein $n_1$ is an integer between 2 and 8; r is an integer from 2 to 10;
(3) $-CH_2-C(O)-(Gly)n_2-(Z_1)m_1-(Gly)o_1-NH-(CH_2)r-NH-$, wherein $Z_1$ is Ser, Thr, His, Arg, Arg(Me), Trp, Lys, Lys(Me), $Lys(Me)_2$, Orn, Orn(Me), $Orn(Me)_2$, Dap, Dap(Me), $Dap(Me)_2$, Dab, Dab(Me), $Dab(Me)_2$ or Val-Cit, $n_2$ is an integer between 1 and 5; $m_1$ is an integer between 1 and 6; $o_2$ is an integer between 1 and 5; r is an integer from 2 to 10;
(4) $-CH_2-C(O)-(Sar)n_3-NH-(CH_2)r-O-$, wherein $n_3$ is an integer between 1 and 5; r is an integer from 2 to 10;
(5) $-CH_2-CO-(Gly)n_4-(Z_2)m_2-(Gly)o_2-NH-(CH_2)r-O-$, wherein $Z_2$ is Ser, Thr, His, Arg, Arg(Me), Trp, Lys, Lys(Me), $Lys(Me)_2$, Orn, Orn(Me), $Orn(Me)_2$, Dap, Dap(Me), $Dap(Me)_2$, Dab, Dab(Me), $Dab(Me)_2$ or Val-Cit, $n_4$ is an integer between 1 and 5; $m_2$ is an integer between 1 and 6; $o_2$ is an integer between 1 and 5; r is an integer from 2 to 10;
(6)

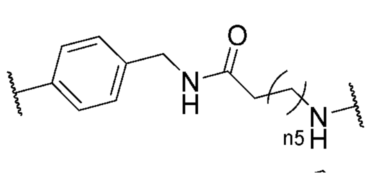
,
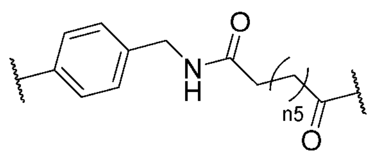
,
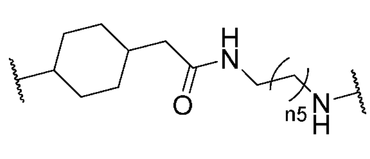
, or
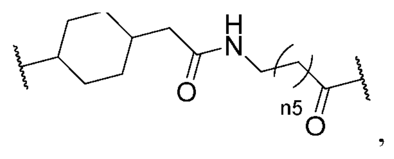
,

wherein $n_5$ is an integer between 1 and 19;

W is absent, or is

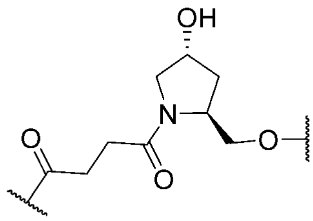

or

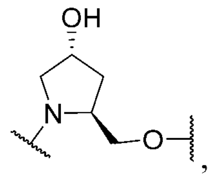

,

wherein the wave line on the right represents a site to which the sense strand or antisense strand is attached; the wave line connected to W in formula (III) represents a site to which the sense strand or antisense strand is attached, wherein the ligand is connected to 5'- and/or 3'-end of the sense strand and/or antisense strand;
A and C are independently 0 or an integer between 1 and 14;
B is 0 or an integer between 1 and 12.

**24.** The RNAi agent according to claim 22, wherein the ligand has a structure of formula (II') or formula (III'):

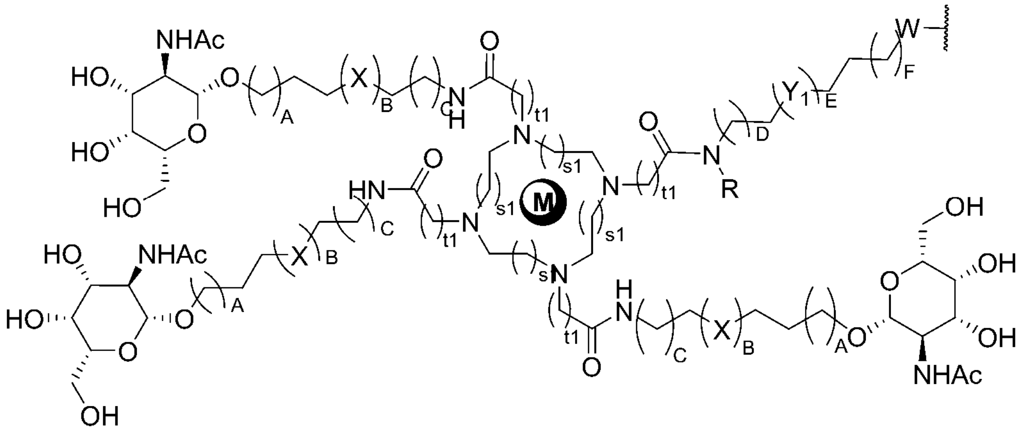

(II')

wherein,

X is -C(O)-NH-, -NH-C(O)-, $-OCH_2-CH_2O-$, -S-,

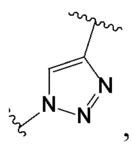

, or

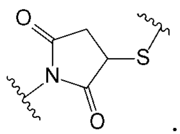

;

each $s_1$ and each $t_1$ are independently an integer between 1 and 5, preferably independently 1 or 2;
$Y_1$ is $-C(O)-NR_1-$, $-NR_1-C(O)-$, $-OCH_2-CH_2O-$, -S-, -S-S-,

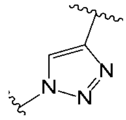

,

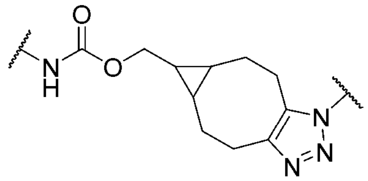, 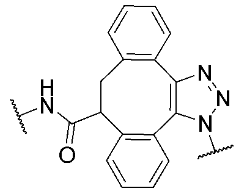, 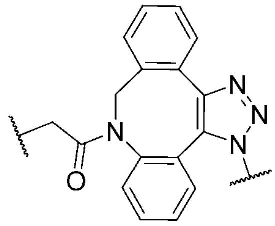,

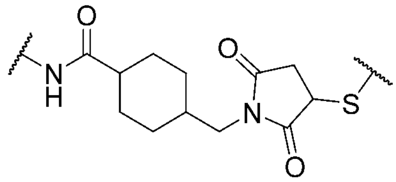, or 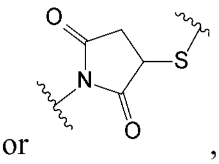,

$R_1$ is an alkyl group having 1-6 carbons or hydrogen;
R is an alkyl group having 1-16 carbons or hydrogen;
W is -NH-, -O-,

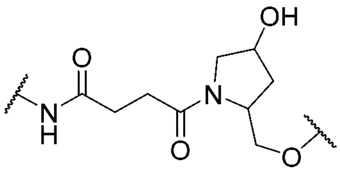, 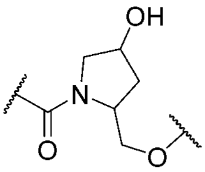, 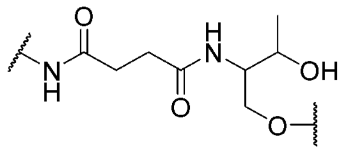,

or

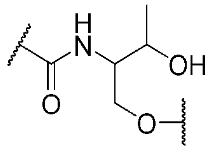,

or a tautomer, a mesomer, a racemate, an enantiomer, or a diastereoisomer thereof, wherein the wave line on the right represents a site to which other part of the conjugate is attached;
A and C are independently 0 or an integer between 1 and 14;
B is 0 or an integer between 1 and 12;
D is an integer between 1 and 20;
E is 0 or an integer between 1 and 12;
F is 0 or an integer between 1 and 14; and
M is present or absent, and when present M is a cation, preferably $H^+$ or a metal cation, wherein the metal cation is preferably a monovalent metal cation, preferably $Na^+$, $K^+$, or $Ag^+$; a divalent metal cation, preferably $Ca^{2+}$, $Mg^{2+}$, $Cu^{2+}$, $Fe^{2+}$, $Zn^{2+}$, or $Mn^{2+}$; or a trivalent metal cation, preferably $Fe^{3+}$, $Al^{3+}$ or a lanthanide metal ion; alternatively, when present, M is a radionuclide, preferably Lu-177, Zr-89, Sc-44, Cu-64, Ga-67, Ga-68, Tc-99m, In-111, Sc-47, Y-90, Y-86, Sm-153, Ho-166, Re-188, Pb-212, Bi-213 or Th-232;

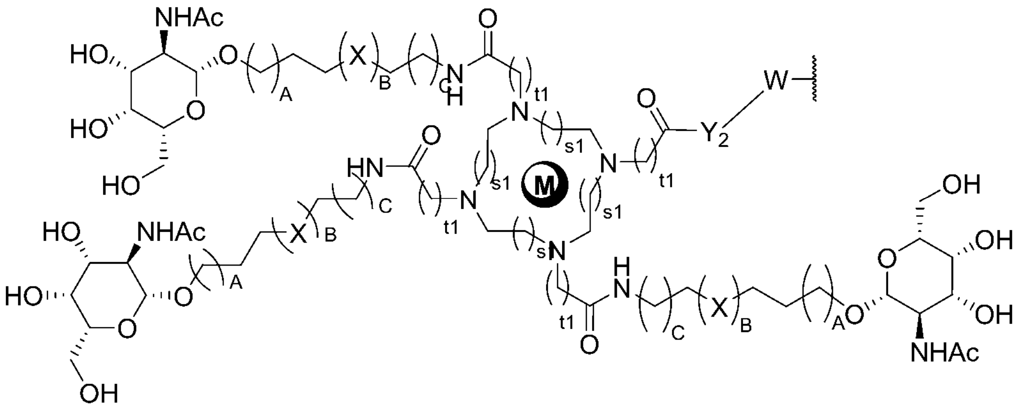

(III')

wherein,

X is -C(O)-NH-, -NH-C(O)-, $-OCH_2-CH_2O-$, -S-,

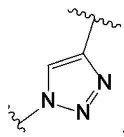, or 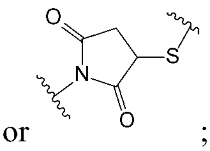 ;

each $s_1$ and each $t_1$ are independently an integer between 1 and 5, preferably independently 1 or 2;
$Y_2$ is selected from the group consisting of:

(1) $NH-(CH_2)p-(O-CH_2-CH_2)q-(CH_2)j-Z_1-$, wherein $Z_1$ is O, NH, C(O) or absent, p is an integer from 1 to 3, q is an integer from 3 to 10, j is 0 or 1;
(2) $(Sar)n_1-NH-(CH_2)r-Z_2-$, wherein $n_1$ is an integer between 2 and 8, r is an integer from 2 to 10, $Z_2$ is O, NH, C(O) or absent;
(3) $(Gly)n_2-(Z_3)m_1-(Gly)o_1-NH-(CH_2)r-Z_4-$, wherein $Z_3$ is Ser, HomoSer, (NMe)Ser, Thr, (NMe)Thr, His, (NMe)His, Arg, (NMe)Arg, Arg(Me), Trp, (NMe)Trp, Trp(Me), Lys, (NMe)Lys, Lys(Me), $Lys(Me)_2$, Orn, (NMe)Orn, Orn(Me), $Orn(Me)_2$, Dap, (NMe)Dap, Dap(Me), $Dap(Me)_2$, Dab, (NMe)Dab, Dab(Me), $Dab(Me)_2$, Cit or Val-Cit, $n_2$ is an integer between 0 and 5, $m_1$ is an integer between 1 and 10, $o_1$ is an integer between 0 and 5, r is an integer from 2 to 16, $Z_4$ is O, NH, C(O) or absent;
(4)

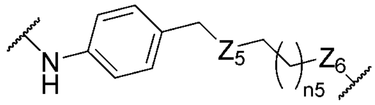 , 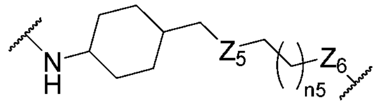 ,

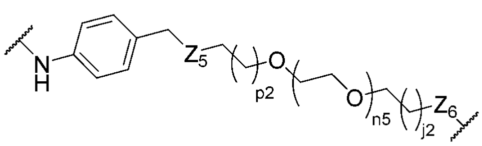 , 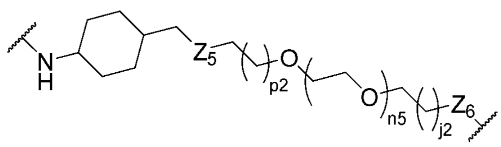 ,

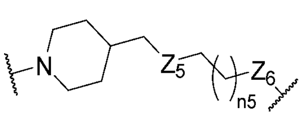 , 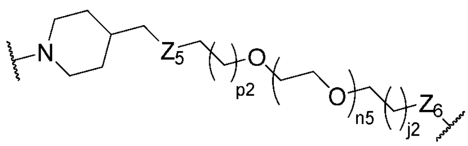 ,

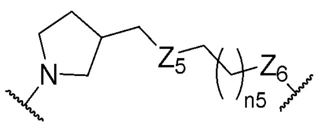 , 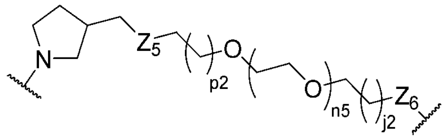 ,

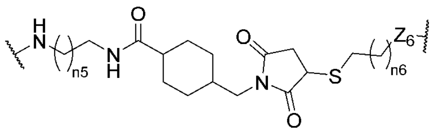 , 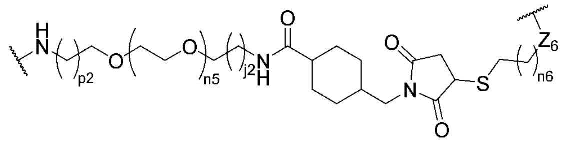 ,

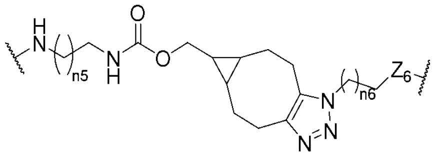 , 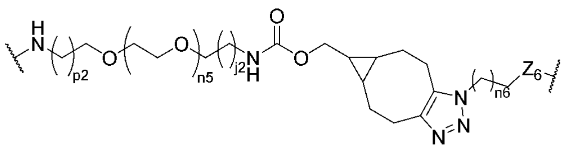 ,

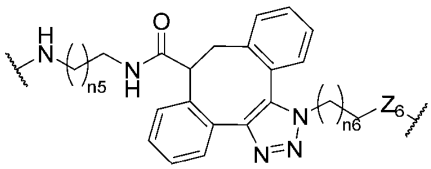 , 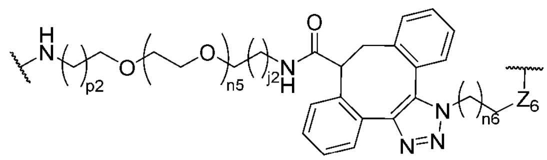 ,

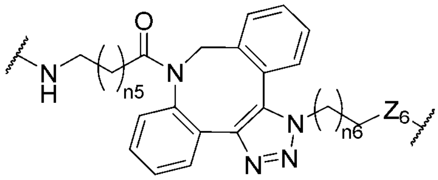 , 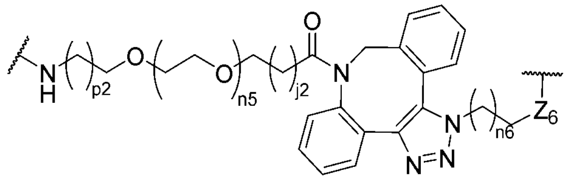 ,

or a tautomer, a mesomer, a racemate, an enantiomer or a diastereoisomer thereof, wherein $Z_5$ is -C(O)-NH-, -NH-C(O)- or absent, $Z_6$ is O, NH, C(O) or absent, $p_2$ is an integer from 1 to 2, $j_2$ is an integer of 1 or 2, $n_5$ is an integer between 1 and 19, $n_6$ is an integer between 1 and 19;

W is absent or is

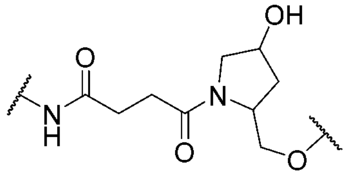 , 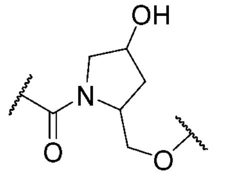 ,

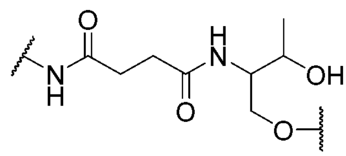 , or 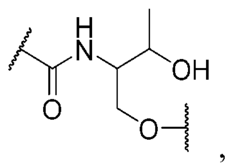 ,

or a tautomer, a mesomer, a racemate, an enantiomer or a diastereoisomer thereof, wherein the wave line on the right represents a site to which other part of the conjugate is attached;
A and C are independently 0 or an integer between 1 and 14;
B is 0 or an integer between 1 and 12; and
M is present or absent, and when present M is a cation, preferably $H^+$ or a metal cation, wherein the metal

cation is preferably a monovalent metal cation, preferably $Na^+$, $K^+$, or $Ag^+$; a divalent metal cation, preferably $Ca^{2+}$, $Mg^{2+}$, $Cu^{2+}$, $Fe^{2+}$, $Zn^{2+}$, or $Mn^{2+}$; or a trivalent metal cation, preferably $Fe^{3+}$, $Al^{3+}$ or a lanthanide metal ion; alternatively, when present, M is a radionuclide, preferably Lu-177, Zr-89, Sc-44, Cu-64, Ga-67, Ga-68, Tc-99m, In-111, Sc-47, Y-90, Y-86, Sm-153, Ho-166, Re-188, Pb-212, Bi-213 or Th-232.

**25.** The RNAi agent according to claim 23 or 24, wherein the ligand has a structure of YHZY12001 represented by formula (IV-1), YHZY12002 represented by formula (IV-2), YHZY12003 represented by formula (IV-3), YHZY12004 represented by formula (IV-4), YHZY12005 represented by formula (IV-5), YHZY12006 represented by formula (IV-6), or YHZY12007 represented by formula (IV-7):

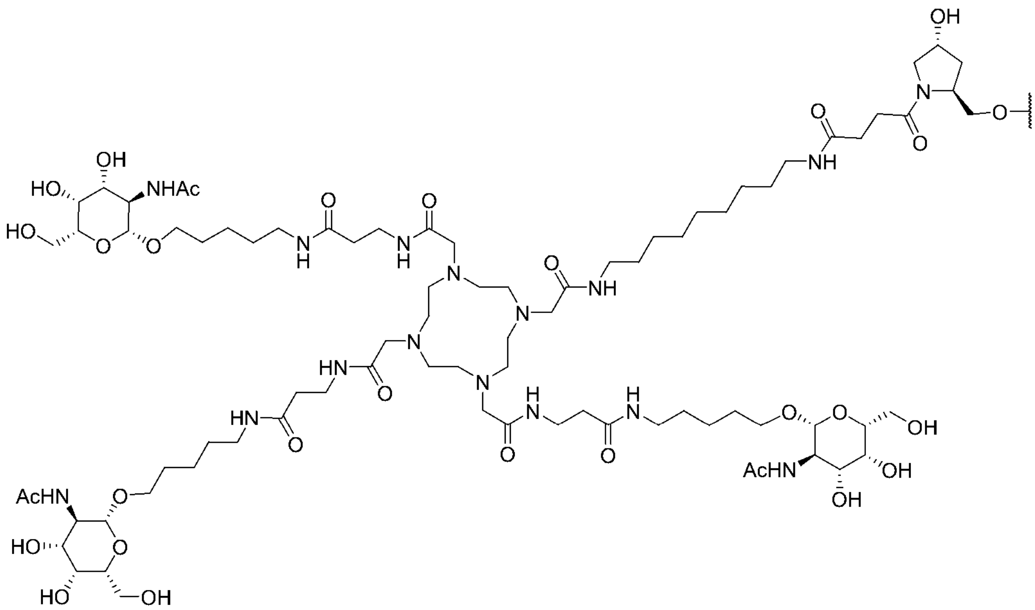

(IV-1) YHZY12001

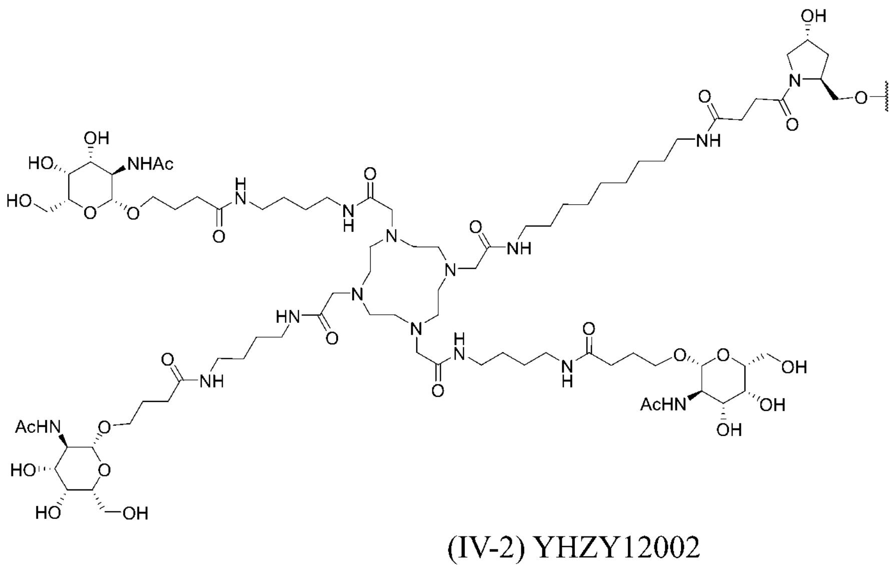

(IV-2) YHZY12002

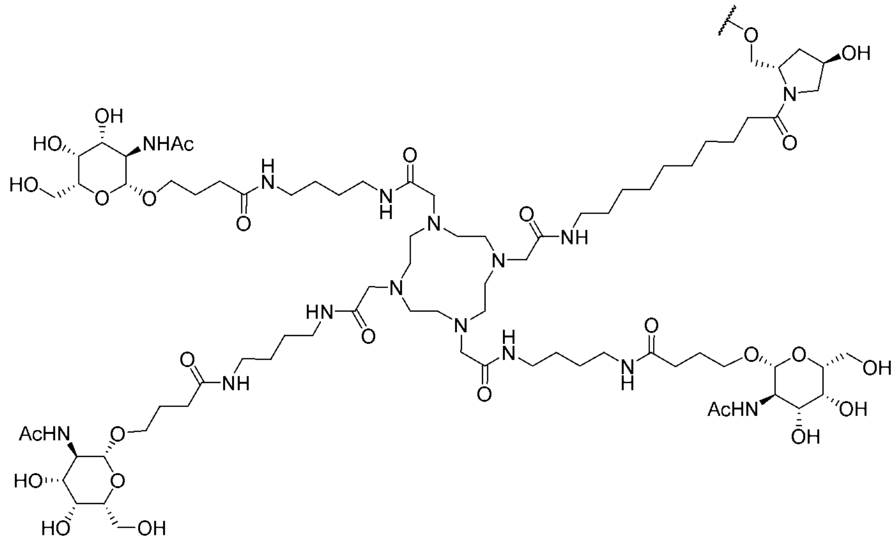

(IV-3) YHZY12003

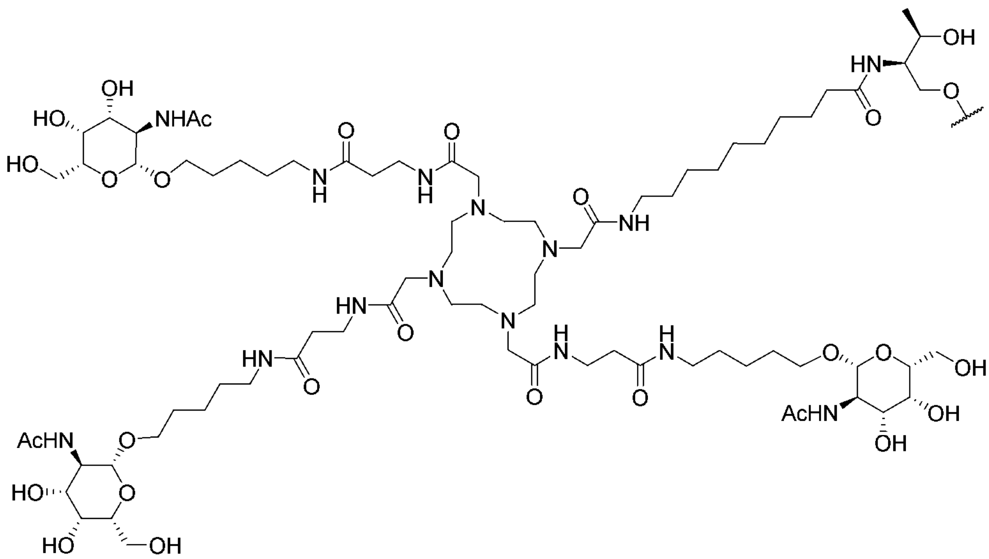

(IV-4) YHZY12004

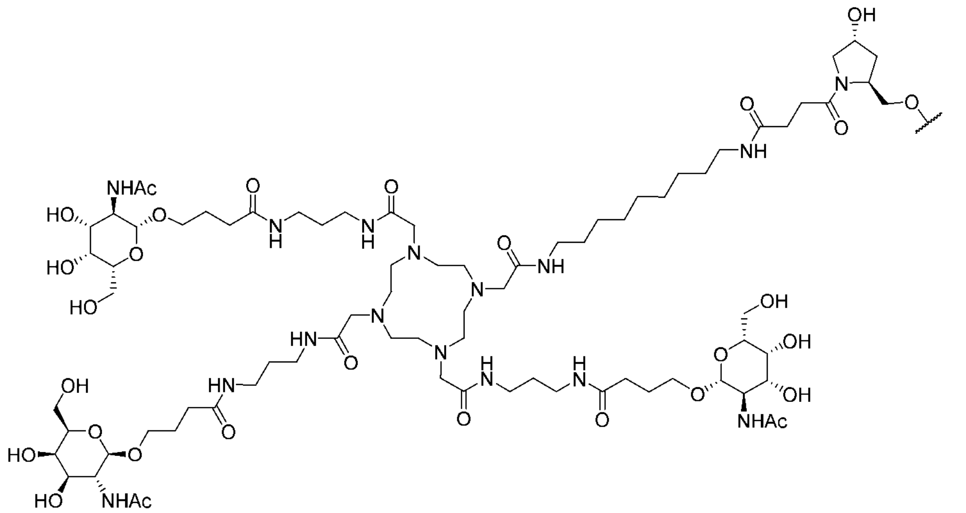

(IV-5) YHZY12005

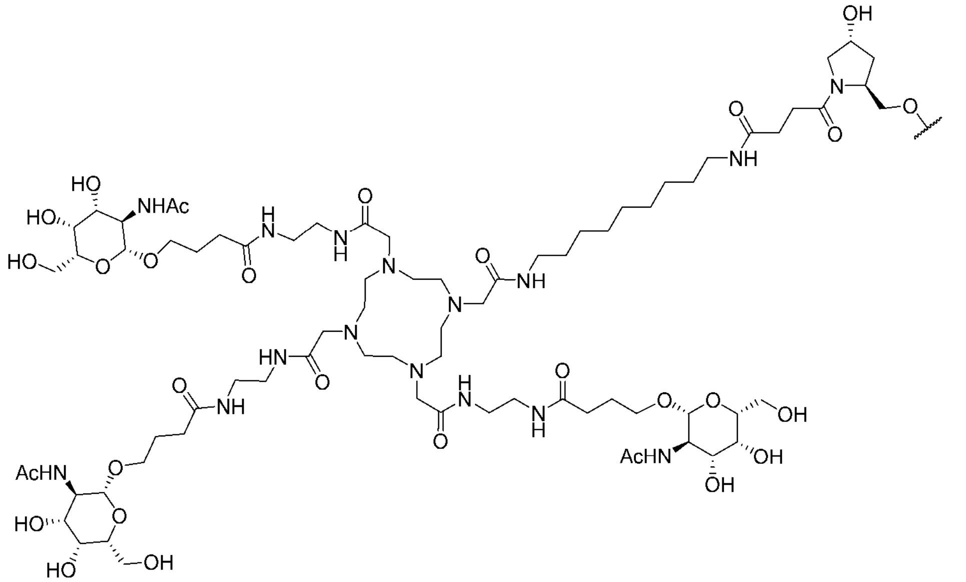

(IV-6) YHZY12006

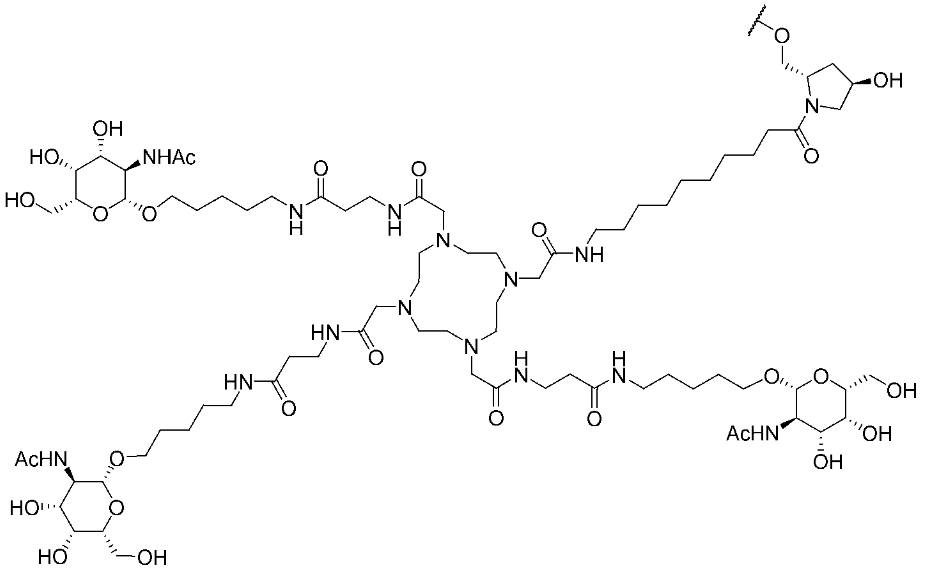

(IV-7) YHZY12007

wherein the wave line represents a site to which the sense strand or antisense strand is attached, and preferably the ligand is connected to 5'- and/or 3'-end of the sense strand; preferably, the ligand is connected to 3'-end of the sense strand; preferably, wherein the ligand is connected to the sense strand or antisense strand via a phosphate ester group, a thiophosphate ester group or a phosphonate ester group.

**26.** The RNAi agent according to any one of claims 1 to 25, wherein the RNAi agent has a structure of any one of the following formulas VII-1 to VII-8:

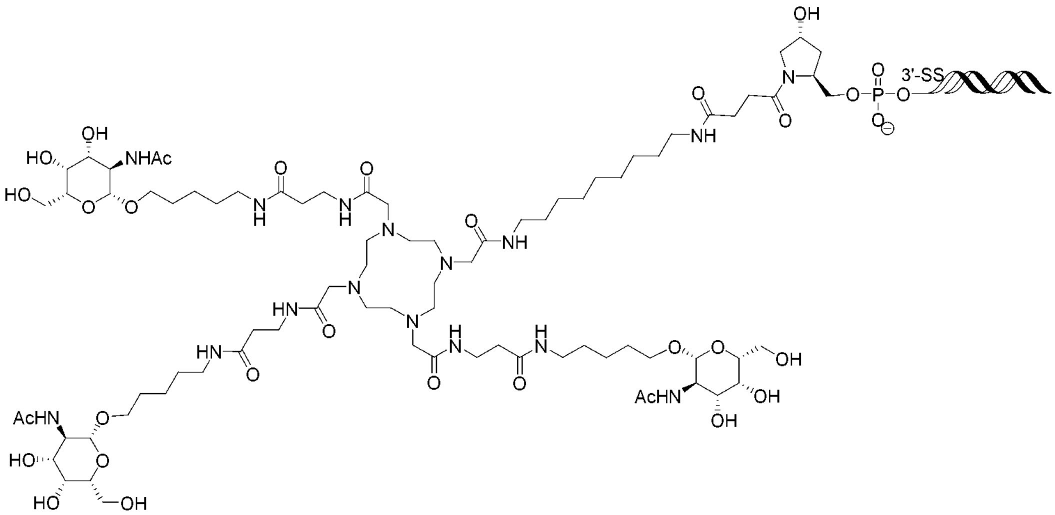

(VII-1)

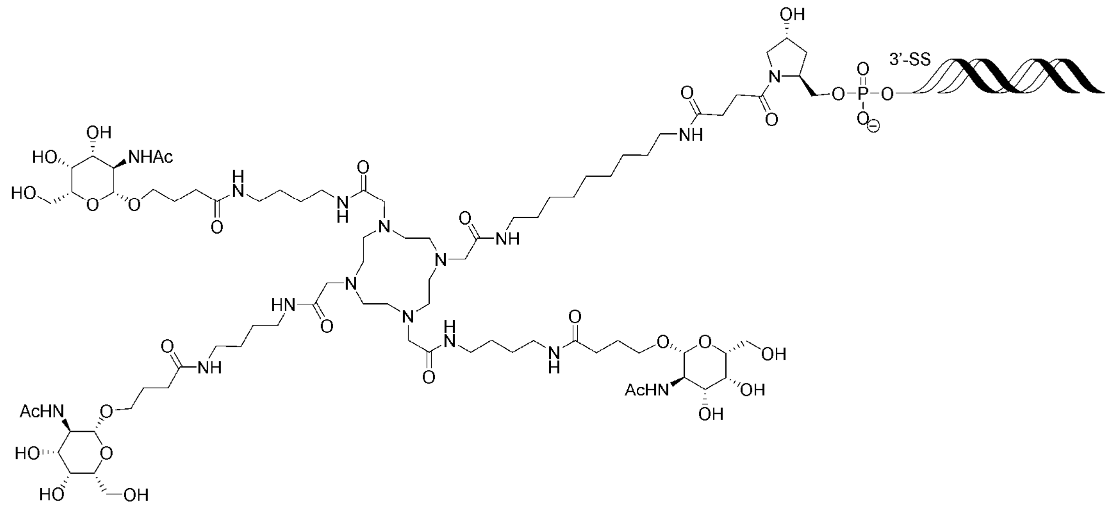

(VII-2)

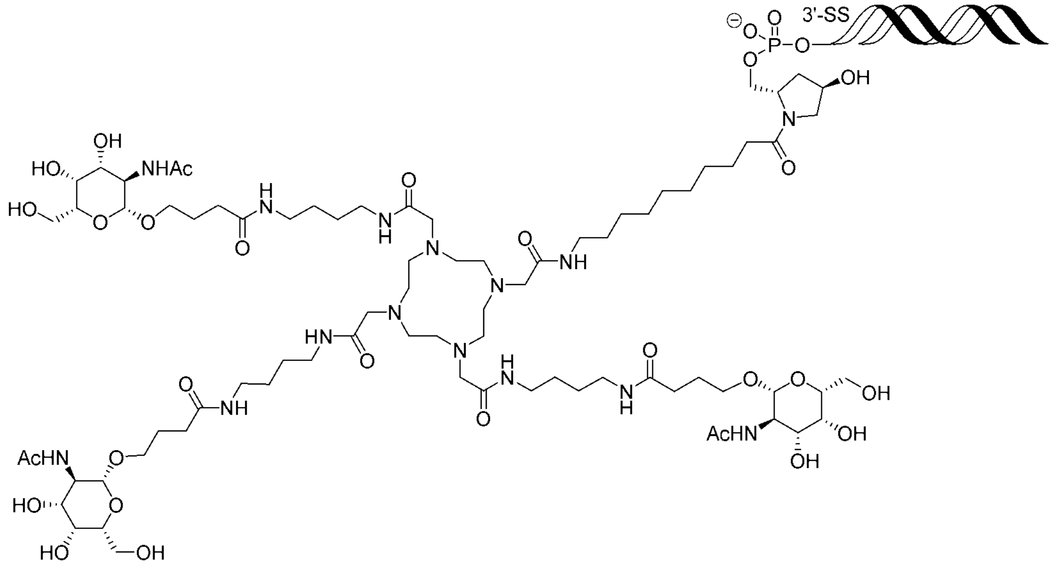

(VII-3)

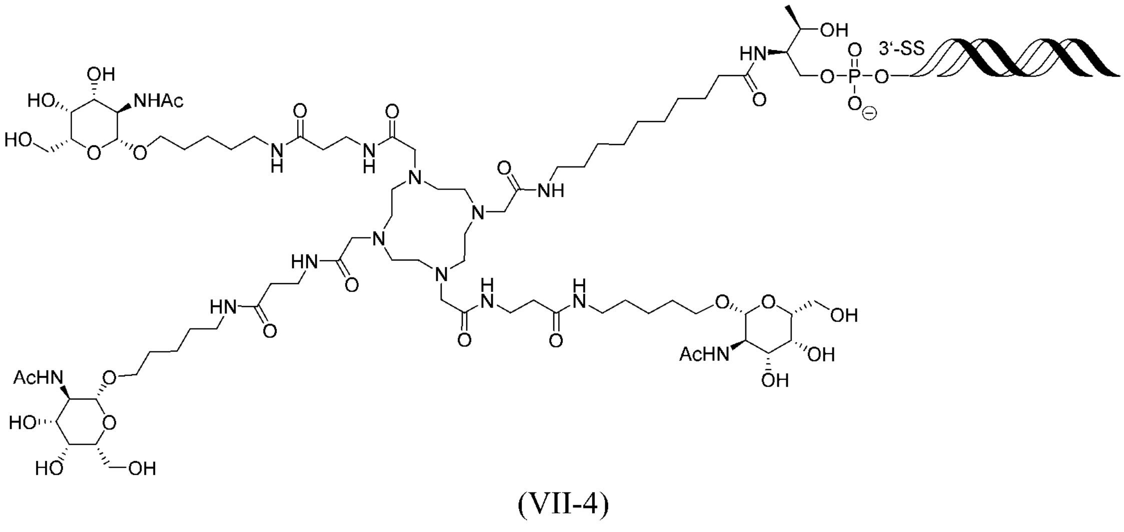

(VII-4)

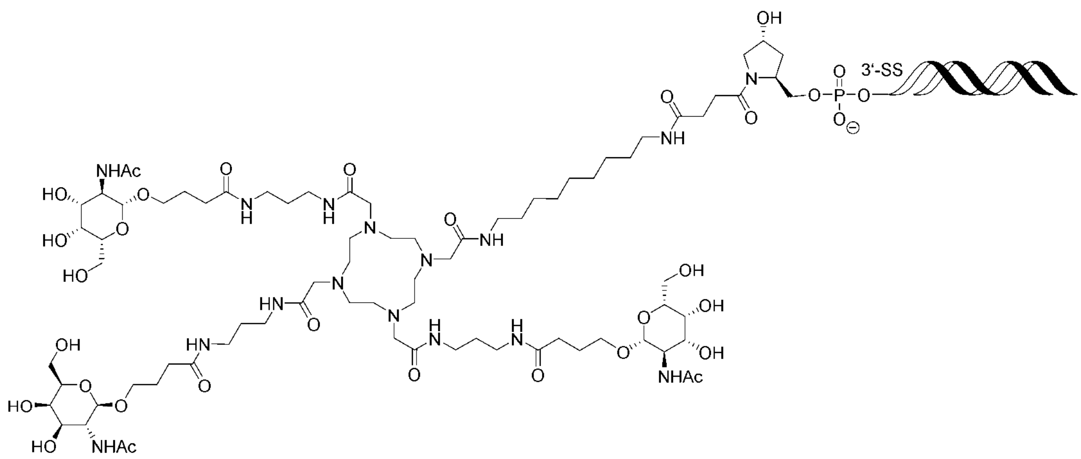

(VII-5)

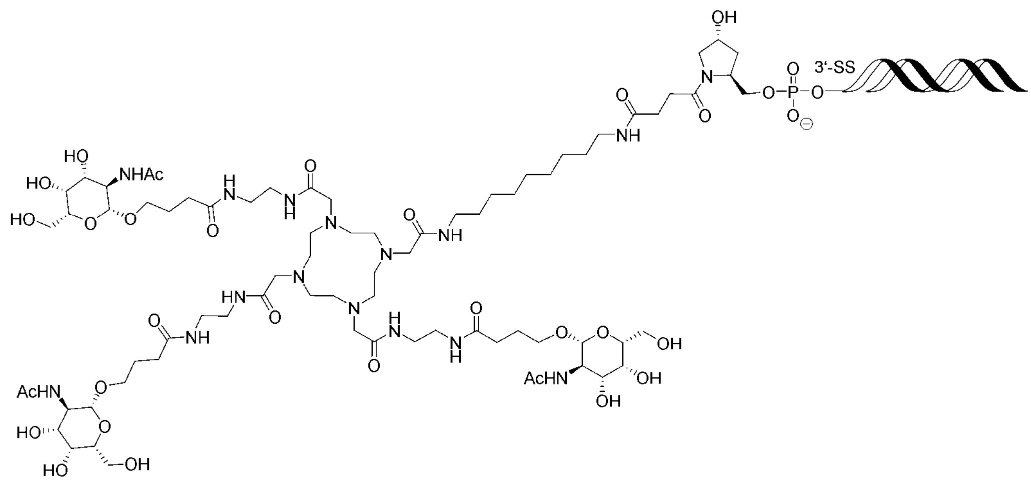

(VII-6)

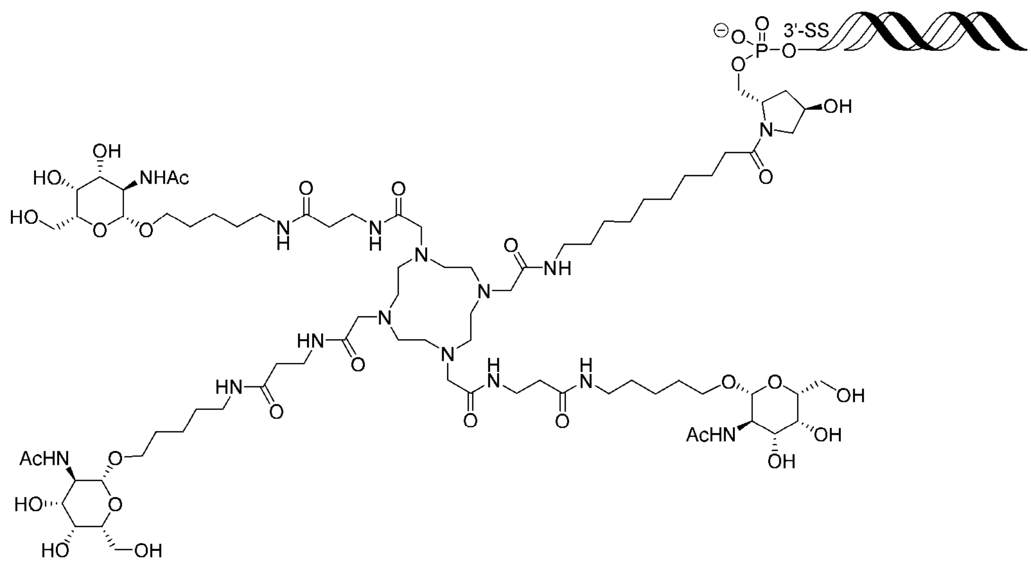

(VII-7)

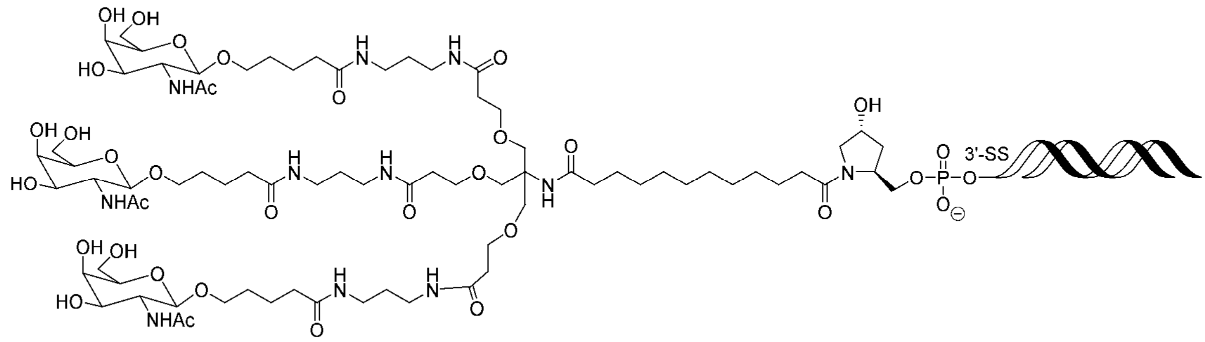

(VII-8).

**27.** The RNAi agent according to any one of claims 21 to 26, wherein the sense strand comprises or consists of a nucleotide sequence set forth in any one of SEQ ID NOs: 68 to 102 or a nucleotide sequence having 1, 2 or 3 nucleotide differences from each thereof.

**28.** The RNAi agent according to claim 27, wherein:

(c1) the sense strand comprises or consists of a nucleotide sequence set forth in SEQ ID NO: 68 or a nucleotide sequence having 1, 2 or 3 nucleotide differences therefrom, and the antisense strand comprises or consists of a nucleotide sequence set forth in SEQ ID NO: 104 or a nucleotide sequence having 1, 2 or 3 nucleotide differences therefrom;
(c2) the sense strand comprises or consists of a nucleotide sequence set forth in SEQ ID NO: 69 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;
(c3) the sense strand comprises or consists of a nucleotide sequence set forth in SEQ ID NO: 70 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;
(c4) the sense strand comprises or consists of a nucleotide sequence set forth in SEQ ID NO: 71 or a nucleotide sequence having 1, 2 or 3 nucleotide differences therefrom, and the antisense strand comprises or consists of a nucleotide sequence set forth in SEQ ID NO: 107 or a nucleotide sequence having 1, 2 or 3 nucleotide differences therefrom;
(c5) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;
(c6) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 108 or a nucleotide sequence having 1, 2 or 3 nucleotide differences therefrom;
(c7) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 109 or a nucleotide sequence having 1, 2 or 3 nucleotide differences therefrom;
(c8) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 110 or a nucleotide sequence having 1, 2 or 3 nucleotide differences therefrom;
(c9) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 111 or a nucleotide sequence having 1, 2 or 3 nucleotide differences therefrom;
(c10) the sense strand comprises or consists of a nucleotide sequence set forth in SEQ ID NO: 72 or a nucleotide sequence having 1, 2 or 3 nucleotide differences therefrom, and the antisense strand comprises or consists of a

nucleotide sequence set forth in SEQ ID NO: 112 or a nucleotide sequence having 1, 2 or 3 nucleotide differences therefrom;

(c11) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c12) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 114 or a nucleotide sequence having 1, 2 or 3 nucleotide differences therefrom;

(c13) the sense strand comprises or consists of a nucleotide sequence set forth in SEQ ID NO: 72 or a nucleotide sequence having 1, 2 or 3 nucleotide differences therefrom, and the antisense strand comprises or consists of a nucleotide sequence set forth in SEQ ID NO: 115 or a nucleotide sequence having 1, 2 or 3 nucleotide differences therefrom;

(c14) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 116 or a nucleotide sequence having 1, 2 or 3 nucleotide differences therefrom;

(c15) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c16) the sense strand comprises or consists of a nucleotide sequence set forth in SEQ ID NO: 73 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c17) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c18) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c19) the sense strand comprises or consists of a nucleotide sequence set forth in SEQ ID NO: 74 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c20) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c21) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c22) the sense strand comprises or consists of a nucleotide sequence set forth in SEQ ID NO: 75 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;

(c23) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;

(c24) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;

(c25) the sense strand comprises or consists of a nucleotide sequence set forth in SEQ ID NO: 76 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;
(c26) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;
(c27) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;
(c28) the sense strand comprises or consists of a nucleotide sequence set forth in SEQ ID NO: 77 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;
(c29) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;
(c30) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;
(c31) the sense strand comprises or consists of a nucleotide sequence set forth in SEQ ID NO: 78 or a nucleotide sequence having 1, 2 or 3 nucleotide differences therefrom, and the antisense strand comprises or consists of a nucleotide sequence set forth in SEQ ID NO: 117 or a nucleotide sequence having 1, 2 or 3 nucleotide differences therefrom;
(c32) the sense strand comprises or consists of a nucleotide sequence set forth in SEQ ID NO: 79 or a nucleotide sequence having 1, 2 or 3 nucleotide differences therefrom, and the antisense strand comprises or consists of a nucleotide sequence set forth in SEQ ID NO: 118 or a nucleotide sequence having 1, 2 or 3 nucleotide differences therefrom;
(c33) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;
(c34) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;
(c35) the sense strand comprises or consists of a nucleotide sequence set forth in SEQ ID NO: 80 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;
(c36) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 105 or a nucleotide sequence having 1, 2 or 3 nucleotide differences therefrom;
(c37) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 112 or a nucleotide sequence having 1, 2 or 3 nucleotide differences therefrom;
(c38) the sense strand comprises or consists of a nucleotide sequence set forth in SEQ ID NO: 81 or a nucleotide sequence having 1, 2 or 3 nucleotide differences therefrom, and the antisense strand comprises or consists of a nucleotide sequence set forth in SEQ ID NO: 113 or a nucleotide sequence having 1, 2 or 3 nucleotide differences therefrom;
(c39) the sense strand comprises or consists of a nucleotide sequence set forth in SEQ ID NO: 82 or a nucleotide sequence having 1, 2 or 3 nucleotide differences therefrom, and the antisense strand comprises or consists of a

nucleotide sequence set forth in SEQ ID NO: 106 or a nucleotide sequence having 1, 2 or 3 nucleotide differences therefrom;
(c40) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c41) the sense strand comprises or consists of a nucleotide sequence set forth in SEQ ID NO: 82 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c42) the sense strand comprises or consists of a nucleotide sequence set forth in SEQ ID NO: 83 or a nucleotide sequence having 1, 2 or 3 nucleotide differences therefrom, and the antisense strand comprises or consists of a nucleotide sequence set forth in SEQ ID NO: 121 or a nucleotide sequence having 1, 2 or 3 nucleotide differences therefrom;
(c43) the sense strand comprises or consists of a nucleotide sequence set forth in SEQ ID NO: 84 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c44) the sense strand comprises or consists of a nucleotide sequence set forth in SEQ ID NO: 84 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c45) the sense strand comprises or consists of a nucleotide sequence set forth in SEQ ID NO: 85 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c46) the sense strand comprises or consists of a nucleotide sequence set forth in SEQ ID NO: 85 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c47) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 106 or a nucleotide sequence having 1, 2 or 3 nucleotide differences therefrom;
(c48) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 119 or a nucleotide sequence having 1, 2 or 3 nucleotide differences therefrom;
(c49) the sense strand comprises or consists of a nucleotide sequence set forth in SEQ ID NO: 86 or a nucleotide sequence having 1, 2 or 3 nucleotide differences therefrom, and the antisense strand comprises or consists of a nucleotide sequence set forth in SEQ ID NO: 120 or a nucleotide sequence having 1, 2 or 3 nucleotide differences therefrom;
(c50) the sense strand comprises or consists of a nucleotide sequence set forth in SEQ ID NO: 87 or a nucleotide sequence having 1, 2 or 3 nucleotide differences therefrom, and the antisense strand comprises or consists of a nucleotide sequence set forth in SEQ ID NO: 122 or a nucleotide sequence having 1, 2 or 3 nucleotide differences therefrom;
(c51) the sense strand comprises or consists of a nucleotide sequence set forth in SEQ ID NO: 88 or a nucleotide sequence having 1, 2 or 3 nucleotide differences therefrom, and the antisense strand comprises or consists of a nucleotide sequence set forth in SEQ ID NO: 123 or a nucleotide sequence having 1, 2 or 3 nucleotide differences therefrom;
(c52) the sense strand comprises or consists of a nucleotide sequence set forth in SEQ ID NO: 89 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;
(c53) the sense strand comprises or consists of a nucleotide sequence set forth in SEQ ID NO: 90 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;

(c54) the sense strand comprises or consists of a nucleotide sequence set forth in SEQ ID NO: 91 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;
(c55) the sense strand comprises or consists of a nucleotide sequence set forth in SEQ ID NO: 92 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;
(c56) the sense strand comprises or consists of a nucleotide sequence set forth in SEQ ID NO: 93 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;
(c57) the sense strand comprises or consists of a nucleotide sequence set forth in SEQ ID NO: 94 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom;
(c58) the sense strand comprises or consists of a nucleotide sequence set forth in SEQ ID NO: 95 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom;
(c59) the sense strand comprises or consists of a nucleotide sequence set forth in SEQ ID NO: 96 or a nucleotide sequence having 1, 2 or 3 nucleotide differences therefrom, and the antisense strand comprises or consists of a nucleotide sequence set forth in SEQ ID NO: 124 or a nucleotide sequence having 1, 2 or 3 nucleotide differences therefrom;
(c60) the sense strand comprises or consists of a nucleotide sequence set forth in SEQ ID NO: 97 or a nucleotide sequence having 1, 2 or 3 nucleotide differences therefrom, and the antisense strand comprises or consists of a nucleotide sequence set forth in SEQ ID NO: 125 or a nucleotide sequence having 1, 2 or 3 nucleotide differences therefrom;
(c61) the sense strand comprises or consists of a nucleotide sequence set forth in SEQ ID NO: 98 or a nucleotide sequence having 1, 2 or 3 nucleotide differences therefrom, and the antisense strand comprises or consists of a nucleotide sequence set forth in SEQ ID NO: 126 or a nucleotide sequence having 1, 2 or 3 nucleotide differences therefrom;
(c62) the sense strand comprises or consists of a nucleotide sequence set forth in SEQ ID NO: 99 or a nucleotide sequence having 1, 2 or 3 nucleotide differences therefrom, and the antisense strand comprises or consists of a nucleotide sequence set forth in SEQ ID NO: 127 or a nucleotide sequence having 1, 2 or 3 nucleotide differences therefrom;
(c63) the sense strand comprises or consists of a nucleotide sequence set forth in SEQ ID NO: 100 or a nucleotide sequence having 1, 2 or 3 nucleotide differences therefrom, and the antisense strand comprises or consists of a nucleotide sequence set forth in SEQ ID NO: 128 or a nucleotide sequence having 1, 2 or 3 nucleotide differences therefrom;
(c64) the sense strand comprises or consists of a nucleotide sequence set forth in SEQ ID NO: 101 or a nucleotide sequence having 1, 2 or 3 nucleotide differences therefrom, and the antisense strand comprises or consists of a nucleotide sequence set forth in SEQ ID NO: 129 or a nucleotide sequence having 1, 2 or 3 nucleotide differences therefrom; or
(c65) the sense strand comprises or consists of a nucleotide sequence set forth in SEQ ID NO: 102 or a nucleotide sequence having 1, 2 or 3 nucleotide differences therefrom, and the antisense strand comprises or consists of a nucleotide sequence set forth in SEQ ID NO: 130 or a nucleotide sequence having 1, 2 or 3 nucleotide differences therefrom.

**29.** The RNAi agent according to claim 28, wherein the RNAi agent comprises or consists of duplex 4023-60-L96, 4122-60-L96, 4132-60-L96, 4149-60-L96, 4122-60-001, 4122-50-001, 4122-4-001, 4122-64-001, 4122-67-001, 4122-10-001, 4122-11-001, 4122-70-001, 4122.1-60-001, 4122-44-001, 4122-18-001, 4122-19-001, 4122-74-001, 4122-75-001, 4122-101-001, 4122-34-001, 4122-35-001, 4122-98-001, 4122-26-001, 4122-27-001, 4122-97-001, 4122-85-001, 4122-86-001, 4122-99-001, 4122-80-001, 4122-81-001, 4122-100-001, 4122.3-60-001, 4122-60-002, 4122-10-002, 4122-11-002, 4122-60-004, 4122-10-004, 4122-11-004, 4132-60-001, 4132-10-001, 4132-11-001, 4132-44-001, 4132-26-001, 4132-27-001, 4132-34-001, 4132-35-001, 4132-60-004, 4132-10-004, 4132-11-004, 4023-65-001, 4023-44-001, 4089-60-L96, 4098-60-L96, 4118-60-L96, 4123-60-L96, 4129-60-L96, 4135-60-L96, 4145-60-L96, 4089-60-001, 4098-60-001, 4118-60-001,

4123-60-001, 4129-60-001, 4135-60-001 or 4145-60-001.

**30.** A pharmaceutical composition comprising the RNAi agent according to any one of claims 1 to 29, and a pharmaceutically acceptable carrier; preferably, the pharmaceutical composition is formulated for intravenous or subcutaneous injection.

**31.** Use of the RNAi agent according to any one of claims 1 to 29 or the pharmaceutical composition according to claim 30, in the manufacture of a medicament for:

(i) reducing expression level of KHK in a cell;
(ii) preventing or treating a disease caused by an elevated KHK expression level; or
(iii) preventing or treating a metabolic disease, wherein the metabolic disease is, for example, hypertriglyceridemia, obesity, hyperlipidemia, abnormal lipid and/or cholesterol metabolism, atherosclerosis, Type 2 diabetes mellitus, cardiovascular disease, coronary artery disease, non-alcoholic steatohepatitis, non-alcoholic fatty liver disease, homozygous or heterozygous familial hypercholesterolemia, hyperuricemia or gout.

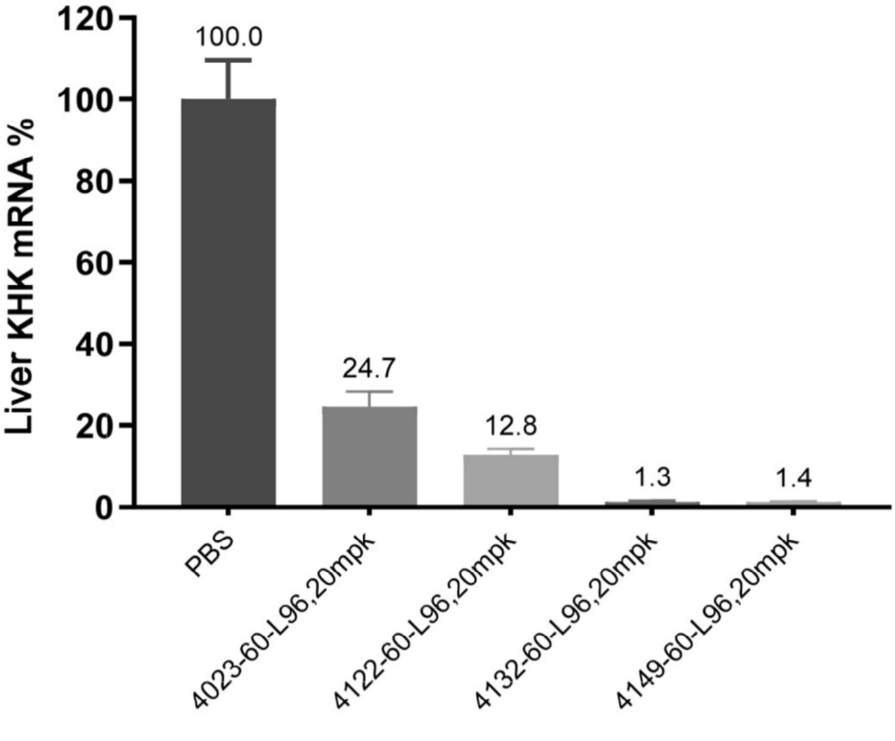
Liver KHK mRNA %
120
100
80
60
40
20
0
100.0
24.7
12.8
1.3
1.4
PBS
4023-60-L96,20mpk
4122-60-L96,20mpk
4132-60-L96,20mpk
4149-60-L96,20mpk

FIG. 1

## INTERNATIONAL SEARCH REPORT

International application No.
**PCT/CN2024/105105**

**A. CLASSIFICATION OF SUBJECT MATTER**

C12N 15/113(2010.01)i; A61K 48/00(2006.01)i; A61K 31/713(2006.01)i; A61P 1/16(2006.01)i; A61P 3/10(2006.01)i

According to International Patent Classification (IPC) or to both national classification and IPC

**B. FIELDS SEARCHED**

Minimum documentation searched (classification system followed by classification symbols)

IPC:C12N, A61K, A61P

Documentation searched other than minimum documentation to the extent that such documents are included in the fields searched

Electronic data base consulted during the international search (name of data base and, where practicable, search terms used)

CJFD, CNTXT, ENTXTC, VEN, WPABS, CNKI, ISI WEB OF KNOWLEDGE, 万方, WANFANG, PUBMED, STN: 申请人, applicant, 发明人, inventor, 靶向, 半乳糖胺, 己酮糖激酶, 修饰, RNA干扰, 抑制表达, 肝, 代谢, target+, liver, AD5, dsRNA, ESC, GalXC, ketohexokinase, KHK, RNAi, STC, AD1-3, SEQ ID NOs: 1, 34, 132, 68 and 104, 式I-VII化合物, compounds of formulas I-VII

**C. DOCUMENTS CONSIDERED TO BE RELEVANT**

| Category* | Citation of document, with indication, where appropriate, of the relevant passages | Relevant to claim No. |
|---|---|---|
| X | CN 106103718 A (ALNYLAM PHARMACEUTICALS, INC.) 09 November 2016 (2016-11-09)<br>claims 1-108 | 1-21, 27-31 |
| X | CN 112424355 A (ALNYLAM PHARMACEUTICALS, INC.) 26 February 2021 (2021-02-26)<br>claims 1-111, and tables 2-3 | 1-21, 27-31 |
| X | WO 2022182574 A1 (ALNYLAM PHARMACEUTICALS, INC.) 01 September 2022 (2022-09-01)<br>claims 1-108, and tables 2-3 | 1-21, 27-31 |
| X | WO 2021178736 A1 (ALNYLAM PHARMACEUTICALS, INC.) 10 September 2021 (2021-09-10)<br>claims 1-79, and tables 2-3 | 1-21, 27-31 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 October 2024** | **25 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/105105**

**C. DOCUMENTS CONSIDERED TO BE RELEVANT**

| Category* | Citation of document, with indication, where appropriate, of the relevant passages | Relevant to claim No. |
|---|---|---|
| X | US 2021332367 A1 (ALNYLAM PHARMACEUTICALS, INC.) 28 October 2021 (2021-10-28)<br>claims 1-111, and tables 2-3 | 1-21, 27-31 |
| X | US 2016340679 A1 (ALNYLAM PHARMACEUTICALS, INC.) 24 November 2016 (2016-11-24)<br>claims 1-30, and tables 2-5 | 1-21, 27-31 |
| A | WO 2022222986 A1 (MICROBIO (SHANGHAI) CO., LTD.) 27 October 2022 (2022-10-27)<br>claims 1-26 | 1-31 |
| A | 孙晶等 (SUN Jing et al.). "siRNA缀合物的研究进展 (Recent Progress on siRNA Conjugates)"<br>*中国科学: 化学 (Scientia Sinica (Chimica)),*<br>Vol. 46, No. (07), 20 July 2016 (2016-07-20), 633-642<br>pages 633-642 | 1-31 |
| A | WANG, G.Y. et al. "Emerging Novel Targets for Nonalcoholic Fatty Liver Disease Treatment: Evidence from Recent Basic Studies"<br>*World Journal of Gastroenterology,* Vol. 29, No. (1), 07 January 2023 (2023-01-07), 75-95<br>pages 75-95 | 1-31 |

Form PCT/ISA/210 (second sheet) (July 2022)

**INTERNATIONAL SEARCH REPORT**

International application No.
**PCT/CN2024/105105**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/105105**

**Box No. III Observations where unity of invention is lacking (Continuation of item 3 of first sheet)**

This International Searching Authority found multiple inventions in this international application, as follows:

Invention 1: claims 1-31 (in part) relate to an RNAi agent with a sense strand containing or being the sequence as shown in SEQ ID NO: 1 and an antisense strand containing or being the sequence as shown in SEQ ID NO: 34, and a derivative thereof, a corresponding pharmaceutical composition, and a pharmaceutical use;

invention 2: claims 1-31 (in part) relate to an RNAi agent with a sense strand containing or being the sequence as shown in SEQ ID NO: 2 and an antisense strand containing or being the sequence as shown in SEQ ID NO: 35, and a derivative thereof, a corresponding pharmaceutical composition, and a pharmaceutical use;

invention 3: claims 1-31 (in part) relate to an RNAi agent with a sense strand containing or being the sequence as shown in SEQ ID NO: 3 and an antisense strand containing or being the sequence as shown in SEQ ID NO: 36, and a derivative thereof, a corresponding pharmaceutical composition, and a pharmaceutical use;

invention 4: claims 1-31 (in part) relate to an RNAi agent with a sense strand containing or being the sequence as shown in SEQ ID NO: 4 and an antisense strand containing or being the sequence as shown in SEQ ID NO: 37, and a derivative thereof, a corresponding pharmaceutical composition, and a pharmaceutical use;

invention 5: claims 1-31 (in part) relate to an RNAi agent with a sense strand containing or being the sequence as shown in SEQ ID NO: 5 and an antisense strand containing or being the sequence as shown in SEQ ID NO: 38, and a derivative thereof, a corresponding pharmaceutical composition, and a pharmaceutical use;

invention 6: claims 1-31 (in part) relate to an RNAi agent with a sense strand containing or being the sequence as shown in SEQ ID NO: 6 and an antisense strand containing or being the sequence as shown in SEQ ID NO: 39, and a derivative thereof, a corresponding pharmaceutical composition, and a pharmaceutical use;

...

invention 32: claims 1-31 (in part) relate to an RNAi agent with a sense strand containing or being the sequence as shown in SEQ ID NO: 32 and an antisense strand containing or being the sequence as shown in SEQ ID NO: 65, and a derivative thereof, a corresponding pharmaceutical composition, and a pharmaceutical use.

The common technical feature included in inventions 1-32 is an RNAi agent used for inhibiting the expression of ketohexokinase (KHK) genes in cells. However, CN 112424355 A (publication date: 26 February 2021) discloses an RNAi reagent targeting ketohexokinase (KHK) genes, a pharmaceutical composition, and a use (see claims 1-111, and table 3). However, RNAi agents involved in inventions 1-32 are different from each other in terms of sequence, do not share a common structural feature related to the functions thereof, and therefore the inventions do not share a same or corresponding special technical feature, and do not comply with PCT Article 13.2.

Form PCT/ISA/210 (continuation of first sheet) (July 2022)

**INTERNATIONAL SEARCH REPORT**

International application No.
**PCT/CN2024/105105**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-31 (in part) relate to an RNAi agent with a sense strand containing or being the sequence as shown in SEQ ID NO: 1 and an antisense strand containing or being the sequence as shown in SEQ ID NO: 34, and a derivative thereof, a corresponding pharmaceutical composition, and a pharmaceutical use.**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/105105**

| Patent document cited in search report | Publication date (day/month/year) | Patent family member(s) | Publication date (day/month/year) |
|---|---|---|---|
| CN 106103718 A | 09 November 2016 | KR 20160118346 A | 11 October 2016 |
| | | KR 102389968 B1 | 25 April 2022 |
| | | CA 2938857 A1 | 20 August 2015 |
| | | EP 3960860 A2 | 02 March 2022 |
| | | EP 3960860 A3 | 08 June 2022 |
| | | JP 2017509354 A | 06 April 2017 |
| | | JP 6594902 B2 | 23 October 2019 |
| | | AU 2023266339 A1 | 01 February 2024 |
| | | WO 2015123264 A1 | 20 August 2015 |
| | | US 2020172909 A1 | 04 June 2020 |
| | | US 11136582 B2 | 05 October 2021 |
| | | KR 20230152154 A | 02 November 2023 |
| | | EP 3105331 A1 | 21 December 2016 |
| | | EP 3105331 B1 | 23 June 2021 |
| | | JP 2020010712 A | 23 January 2020 |
| | | JP 7062623 B2 | 06 May 2022 |
| | | EA 201691587 A1 | 30 January 2017 |
| | | KR 20220054702 A | 03 May 2022 |
| | | KR 102592370 B1 | 25 October 2023 |
| | | AU 2021204336 A1 | 22 July 2021 |
| | | JP 2022109966 A | 28 July 2022 |
| | | AU 2015217301 A1 | 25 August 2016 |
| | | US 2022127618 A1 | 28 April 2022 |
| | | US 2016340679 A1 | 24 November 2016 |
| | | US 10370666 B2 | 06 August 2019 |
| | | US 2024182902 A1 | 06 June 2024 |
| CN 112424355 A | 26 February 2021 | JP 2022500003 A | 04 January 2022 |
| | | JP 2024056730 A | 23 April 2024 |
| | | WO 2020060986 A1 | 26 March 2020 |
| | | EP 3853364 A1 | 28 July 2021 |
| | | AU 2019344776 A1 | 21 January 2021 |
| | | US 2021332367 A1 | 28 October 2021 |
| | | US 2024158796 A1 | 16 May 2024 |
| | | CA 3105385 A1 | 26 March 2020 |
| WO 2022182574 A1 | 01 September 2022 | EP 4298218 A1 | 03 January 2024 |
| | | US 2023323363 A1 | 12 October 2023 |
| | | US 11926832 B2 | 12 March 2024 |
| | | BR 112023016645 A2 | 14 November 2023 |
| | | CO 2023012562 A2 | 20 November 2023 |
| | | MX 2023009704 A | 23 October 2023 |
| | | JP 2024515423 A | 10 April 2024 |
| | | AU 2022226098 A1 | 24 August 2023 |
| | | IL 305153 A | 01 October 2023 |
| | | TW 202302847 A | 16 January 2023 |
| | | KR 20230150844 A | 31 October 2023 |
| | | CL 2023002479 A1 | 05 April 2024 |
| | | CA 3211059 A1 | 01 September 2022 |
| | | US 2024150769 A1 | 09 May 2024 |
| WO 2021178736 A1 | 10 September 2021 | JP 2023516095 A | 17 April 2023 |
| | | EP 4114948 A1 | 11 January 2023 |

Form PCT/ISA/210 (patent family annex) (July 2022)

**INTERNATIONAL SEARCH REPORT**
**Information on patent family members**

International application No.
**PCT/CN2024/105105**

| Patent document cited in search report | Publication date (day/month/year) | Patent family member(s) | Publication date (day/month/year) |
|---|---|---|---|
| | | US 2023416748 A1 | 28 December 2023 |
| | | MX 2022011009 A | 07 October 2022 |
| | | KR 20220150380 A | 10 November 2022 |
| | | BR 112022017822 A2 | 08 November 2022 |
| | | IL 296109 A | 01 November 2022 |
| | | AU 2021232014 A1 | 06 October 2022 |
| | | CA 3174725 A1 | 10 September 2021 |
| US 2021332367 A1 | 28 October 2021 | JP 2022500003 A | 04 January 2022 |
| | | JP 2024056730 A | 23 April 2024 |
| | | WO 2020060986 A1 | 26 March 2020 |
| | | EP 3853364 A1 | 28 July 2021 |
| | | AU 2019344776 A1 | 21 January 2021 |
| | | US 2024158796 A1 | 16 May 2024 |
| | | CA 3105385 A1 | 26 March 2020 |
| US 2016340679 A1 | 24 November 2016 | KR 20160118346 A | 11 October 2016 |
| | | KR 102389968 B1 | 25 April 2022 |
| | | CA 2938857 A1 | 20 August 2015 |
| | | EP 3960860 A2 | 02 March 2022 |
| | | EP 3960860 A3 | 08 June 2022 |
| | | JP 2017509354 A | 06 April 2017 |
| | | JP 6594902 B2 | 23 October 2019 |
| | | AU 2023266339 A1 | 01 February 2024 |
| | | WO 2015123264 A1 | 20 August 2015 |
| | | US 2020172909 A1 | 04 June 2020 |
| | | US 11136582 B2 | 05 October 2021 |
| | | KR 20230152154 A | 02 November 2023 |
| | | EP 3105331 A1 | 21 December 2016 |
| | | EP 3105331 B1 | 23 June 2021 |
| | | JP 2020010712 A | 23 January 2020 |
| | | JP 7062623 B2 | 06 May 2022 |
| | | EA 201691587 A1 | 30 January 2017 |
| | | KR 20220054702 A | 03 May 2022 |
| | | KR 102592370 B1 | 25 October 2023 |
| | | AU 2021204336 A1 | 22 July 2021 |
| | | JP 2022109966 A | 28 July 2022 |
| | | AU 2015217301 A1 | 25 August 2016 |
| | | US 2022127618 A1 | 28 April 2022 |
| | | US 10370666 B2 | 06 August 2019 |
| | | US 2024182902 A1 | 06 June 2024 |
| WO 2022222986 A1 | 27 October 2022 | IL 307882 A | 01 December 2023 |
| | | EP 4326744 A1 | 28 February 2024 |
| | | US 2024218023 A1 | 04 July 2024 |
| | | AU 2022261265 A1 | 09 November 2023 |
| | | AU 2022261265 A9 | 16 November 2023 |
| | | KR 20240044385 A | 04 April 2024 |
| | | JP 2024516613 A | 16 April 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017058944 A
- WO 2021092371 A
- US 7491805 B
- US 8106022 B
- US 8877917 B
- US 20030130186 A
- WO 2013166155 A
- US 7723509 B
- US 8017762 B
- US 8828956 B
- US 9181551 B
- WO 0003683 A
- US 5976567 A
- US 5981501 A
- US 6534484 A
- US 6586410 A
- US 6815432 A
- US 20100324120 A
- WO 9640964 A
- US 056230
- WO 2009127060 A
- US 2010022614 W
- US 0963933 W
- WO 2010129709 A
- WO 2021037205 A
- WO 2015042447 A1
- US 20120035115 A
- WO 2021049504 A1
- WO 2020093053 A
- WO 2022182574 A1

### Non-patent literature cited in the description

- *J. Org. Chem.*, 2020, vol. 85 (10), 6593-6604
- *J. Med. Chem.*, 2016, vol. 59 (6), 2718-2733